(19) Europäisches Patentamt
European Patent Office
Office européen des brevets

(11) EP 3 049 535 B1

# (12) EUROPEAN PATENT SPECIFICATION

(45) Date of publication and mention of the grant of the patent:
**01.12.2021 Bulletin 2021/48**

(21) Application number: **14789914.0**

(22) Date of filing: **29.09.2014**

(51) Int Cl.:
***C12Q 1/6876*** *(2018.01)*

(86) International application number:
**PCT/US2014/058089**

(87) International publication number:
**WO 2015/048665 (02.04.2015 Gazette 2015/13)**

(54) **METHOD TO ESTIMATE THE AGE OF TISSUES AND CELL TYPES BASED ON EPIGENETIC MARKERS**

VERFAHREN ZUR SCHÄTZUNG DES ALTERS VON GEWEBEN UND ZELLTYPEN AUF BASIS EPIGENETISCHER MARKER

PROCÉDÉ PERMETTANT D'ESTIMER L'ÂGE DES TISSUS ET DE TYPES CELLULAIRES SUR LA BASE DES MARQUEURS ÉPIGÉNÉTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2013 US 201361883875 P**

(43) Date of publication of application:
**03.08.2016 Bulletin 2016/31**

(73) Proprietor: **The Regents of the University of California**
**Oakland, CA 94607 (US)**

(72) Inventor: **HORVATH, Stefan**
**Los Angeles, CA 90049 (US)**

(74) Representative: **J A Kemp LLP**
**80 Turnmill Street**
**London EC1M 5QU (GB)**

(56) References cited:
- **D. G. HERNANDEZ ET AL: "Distinct DNA methylation changes highly correlated with chronological age in the human brain", HUMAN MOLECULAR GENETICS, vol. 20, no. 6, 7 January 2011 (2011-01-07), pages 1164-1172, XP055069768, ISSN: 0964-6906, DOI: 10.1093/hmg/ddq561**
- **JORDANA T. BELL ET AL: "Epigenome-Wide Scans Identify Differentially Methylated Regions for Age and Age-Related Phenotypes in a Healthy Ageing Population", PLOS GENETICS, vol. 8, no. 4, 19 April 2012 (2012-04-19), page e1002629, XP055151172, DOI: 10.1371/journal.pgen.1002629**
- **GREGORY HANNUM ET AL: "Genome-wide Methylation Profiles Reveal Quantitative Views of Human Aging Rates", MOLECULAR CELL, vol. 49, no. 2, 1 January 2013 (2013-01-01), pages 359-367, XP055160619, ISSN: 1097-2765, DOI: 10.1016/j.molcel.2012.10.016**
- **DANNY BEN-AVRAHAM ET AL: "Epigenetic genome-wide association methylation in aging and longevity", EPIGENOMICS, vol. 4, no. 5, 1 October 2012 (2012-10-01) , pages 503-509, XP055160612, ISSN: 1750-1911, DOI: 10.2217/epi.12.41**
- **Carmen M Koch ET AL: "Epigenetic-aging-signature to determine age in different tissues", Aging, 1 October 2011 (2011-10-01), pages 1018-1027, XP055160615, United States Retrieved from the Internet: URL:http://www.ncbi.nlm.nih.gov/pubmed/22067257 [retrieved on 2015-01-08]**

EP 3 049 535 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KRISTOPHERL NAZOR ET AL: "Recurrent Variations in DNA Methylation in Human Pluripotent Stem Cells and Their Differentiated Derivatives", CELL STEM CELL, ELSEVIER, CELL PRESS, AMSTERDAM, NL, vol. 10, no. 5, 6 February 2012 (2012-02-06), pages 620-634, XP028481935, ISSN: 1934-5909, DOI: 10.1016/J.STEM.2012.02.013 [retrieved on 2012-02-21]

- None

SEQUENCE LISTING

<110> HORVATH, STEFAN

<120> METHOD TO ESTIMATE THE AGE OF TISSUES AND CELL TYPES BASED ON EPIGENETIC MARKERS

<130> G&C 30435.276-WO-U1

<140>
<141>

<150> 61/883,875
<151> 2013-09-27

<160> 358

<170> PatentIn version 3.5

<210> 1
<211> 122
<212> DNA
<213> Homo sapiens

<400> 1

**ggtgtggcca ggagccaccc ccaccccgc acctgacttc acacacatac ctgccttcag 60**

**cgcctgcccc agagctccca agcccctgcc cgccacatct gcagtgccgc acacagacag 120**

**ga 122**

<210> 2
<211> 122
<212> DNA
<213> Homo sapiens

<400> 2

aaaccttaca gaaacatgaa gccctcaacc atctgctact cagttattcg gggctgacgg 60

cggcttctag aacatccagg tgttctgcag atgcgagaac tcatcctgta gtcaccagat 120

gg 122

<210> 3
<211> 122
<212> DNA
<213> Homo sapiens

<400> 3

agtacaagac cgtattattt gagagaaagt ctcgaacgct gctggctaag ggaaaagtg 60

cgataacttg tgatgattca gggaatgact agacaggatg ggaaaatacc cacgtgtctc 120

tt 122

<210> 4
<211> 122
<212> DNA
<213> Homo sapiens

<400> 4

tgggattaca gacgtgagcc accgcgcccg gccatgtttc cttttagcaa tggagcataa 60

cgggatctga ggaacaatat aactcaggaa gagctgatgg aacattaaga cgtgttacaa 120

ct 122

<210> 5
<211> 122
<212> DNA
<213> Homo sapiens

<400> 5

ccttaactgt agctaagctt ccactcttaa gtatcaatta agcttctctg ttcagtccag 60

cgtttagggc gcctactgcg cgccccgccc cacacacttt tgacaaaaag gtcgcctgct 120

ct 122

<210> 6
<211> 122
<212> DNA
<213> Homo sapiens

<400> 6

gcccagcctc ggtgagcaca cacgccctcc ctgtctctcg ccttcgcttc cctgcatctg 60

cgctgattgg taagtgcttc agatttttac tccaagaact tttgtggtga gaaaagcaag 120

tt 122

<210> 7
<211> 122
<212> DNA
<213> Homo sapiens

<400> 7

cagggaccaa aggtctctgg cacccattta tttatcagtt tccttctctg aggctcattt 60

cgccagctcc tctgggggtg acaggcaagt gagacgtgct cagagctccg atgccaaggc 120

ca 122

<210> 8
<211> 122
<212> DNA
<213> Homo sapiens

<400> 8

acagcacctc agaatacaag ttcgcagagg tcaaagcagt ggacacactc cgaagagctc 60

cgtggagttt tggaaactac attatccaga gtgcagagcg caaaacggcg gcggagttga 120

gc 122

<210> 9
<211> 122
<212> DNA
<213> Homo sapiens

<400> 9

catgtgcata atactgtgga aattagtaaa cagtcacaaa caagtgattc atattcaggg 60

cgcagccttt ttgacaggaa aacagtaatc aagagtttgg gatttgaaga tttttaaaag 120

ga 122

<210> 10
<211> 122
<212> DNA
<213> Homo sapiens

<400> 10

ttggttttct ttcccctcat ccttttgcct gctcccggcg aggggtggct ttgatttcgg 60

cgatgagctc ccagaaaggc aacgtggctc gttccagacc tcagaagcac cagaatacgt 120

tt 122

<210> 11
<211> 122
<212> DNA
<213> Homo sapiens

<400> 11

ctgcagcggc cccgtttgca gggcagggac ccgggtgctg ccccaccctc agcgttccag 60

cggagaaact gaagtccgaa cctgaacctc gggaatctgt ctgcacctgt ctaggtggga 120

tg 122

<210> 12
<211> 122
<212> DNA
<213> Homo sapiens

<400> 12

ctgggggagg gaaggcagga tgcggtgcgg gagttaatgg acctggcctt ggcgaaggcg 60

cgtcctgggt tggatcgaaa ccctctcatc cgccctgtgg ccggagggac cagaccatta 120

gt 122

<210> 13
<211> 122
<212> DNA
<213> Homo sapiens

<400> 13

tggggaacgc gagtggggac agggggcct tcagctgggc cccagggaac cgccccgtgg 60

cgctctcggc ctcgctctca ctcacggtgc tacaggtggt aagcaaattg actatgttgt 120

gg 122

<210> 14
<211> 122
<212> DNA
<213> Homo sapiens

<400> 14

tatttccgat gacctacatc tcagggacgc agtaggatgt tcattgataa acaaataaag 60

cggctcgaag aaatattgtg cagagacatg attgaggtgt acaatcatta ggatattgaa 120

tt 122

<210> 15
<211> 122
<212> DNA
<213> Homo sapiens

<400> 15

cagcggcggt agccgagcga gggcgcggtg gcctctgaca ggaatgactc tgcgcacgtg 60

cgtttcgcag cagtggaagt cttcacaccc ggaaactcga ctttggccgt ttctccattt 120

ct 122

<210> 16
<211> 122
<212> DNA
<213> Homo sapiens

<400> 16

cggggcagct gtcagtgaag ctctacggta tgtgggggcc agcctctgtg accaggcagg 60

cgctcaagct ctgcacactc actgggccac cccgaggggc tgggtgagcc catggggaca 120

ca 122

<210> 17
<211> 122
<212> DNA
<213> Homo sapiens

<400> 17

gggtcgctgt gcctgtcccc gtgtgatccg aaaagtgctg gcaaaatgcg gctgctgctt 60

cgcccggggg ggacgtggtg agtgccaggt cgagagggtc cagtgttgag tgggggggcgg 120

gc 122

<210> 18
<211> 122
<212> DNA
<213> Homo sapiens

<400> 18

aacctatgaa aataaacaaa agctgctcca agcattctct cggcctttct gaactttcta 60

cgctttgggt ttttgttttt tcctcccgtc tcagaggtta aaaacttcga tagggactcg 120

ga 122

<210> 19
<211> 122
<212> DNA
<213> Homo sapiens

<400> 19

gagggacagc tctccaccga ccgaaggagg agaatgctat ttatttcagc accaaatatc 60

cggacagcgc ctctcgggag gtccgagaag agaaccgcga tctgtttcag caccggggct 120

ca 122

<210> 20
<211> 122
<212> DNA
<213> Homo sapiens

<400> 20

ctcctccccc cacctctgga attccacctc ccttgttgcg cccatcgcta tggtgacggg 60

cgctctcagt acactgtctc tacaggccag gaaagagttg tgtgtctttg gggtcccttc 120

cg 122

<210> 21
<211> 122
<212> DNA
<213> Homo sapiens

<400> 21

aacctaaatt ttgggagcac ctactctgca tgaagcactg tgctccatgc ctgtgcacag 60

cgtgactctg tcattggtga tgggtcctgc ttgctgagcc tccactgtgc accaggcaca 120

gt 122

<210> 22
<211> 122
<212> DNA
<213> Homo sapiens

<400> 22

gcctcgaaga gcattatggc cgtagatctg ggtgctgagg actgagccac ccccagactg 60

cgacatgggc ggcggtgcct ccttccccaa gccccaggga gtgttttttt gtttgttttg 120

tt 122

<210> 23
<211> 122
<212> DNA
<213> Homo sapiens

<400> 23

aattgttgcg gcctaacaat gaagcgcagc cataacagtc ctgagccact ggcatgtttg 60

cgggcccttt attgccttgg gaataaactg ctgtggcatt gtatcgtata ttgttttcat 120

gg 122

<210> 24
<211> 122
<212> DNA
<213> Homo sapiens

<400> 24

acccttteet gtgagattct tccgccaagt ggaaggctca tcttcggtcg acagcctacg 60

cggttgaaga acaatccagt aggcacttat agctcagggt ctcgccattc agtcttatct 120

at 122

<210> 25
<211> 122
<212> DNA
<213> Homo sapiens

<400> 25

aagctagaag taagaagtac tgaaatttta gttacaagtt tcatacaggt aaacccaagg 60

cgctacaaat gaagaattaa aggaatgaaa ggcgaaagaa taaaggggcc aaagaggtga 120

tc 122

<210> 26
<211> 122
<212> DNA
<213> Homo sapiens

<400> 26

gcctggacgg tgttagtctc ctggaagcag ctcgcccagg caggagctgc taaccagacg 60

cgcattgtga aggagaccgt ggaaaatcaa aagtgggttc ctgcaaaaat gtagcattgg 120

tt 122

<210> 27
<211> 122
<212> DNA
<213> Homo sapiens

<400> 27

aagagagtgg gcccgccttc agggtctggg gccttccagg ttgggtcgta ggggcgggag 60

cgcacaggct gcgagagagg agcaaaggtt ggtggaggga gaagagcagt ctggggcctg 120

gc 122

<210> 28
<211> 122
<212> DNA
<213> Homo sapiens

<400> 28

tttcctagag gaagaatggg cagggaagat gtgggtctaa aggcagaaag acttaatgtg 60

cggtttcggg ctttactgtg catacatact aactgtgaaa ggttttcact tcctcctcag 120

ga 122

<210> 29
<211> 122
<212> DNA
<213> Homo sapiens

<400> 29

gccagcgcgc acgcagatgg cggggtggcc tggggaggtc ttcgggtccc ttcctgggaa 60

cgcagggcca agttgtgctc cgattccacg cccccccac ccacgtcggg cacacgcagc 120

cc 122

<210> 30
<211> 122
<212> DNA
<213> Homo sapiens

<400> 30

acagccggct ctaccgctct gctcgcaggt ttgggctagt ctggggcggg gacttgggag 60

cgcctaaaac ttgcgaggag ggcggggccg cagaccggtc ctttaaaggt tggaagtggc 120

cc 122

<210> 31
<211> 122
<212> DNA
<213> Homo sapiens

<400> 31

agggtgcctg cctctcccgg cctgcgcctg cgcgctgggg ccttcggctg aaggggtgtg 60

cgctagcgga gctccgggaa atgaatgaat gaatgaatga atgaaatgct gaagcgggca 120

gg 122

<210> 32
<211> 122
<212> DNA
<213> Homo sapiens

<400> 32

ctccaccaac aggagctcct tgaggcgagg cacagtgtct tctgtgtccc tggagccaag 60

cgcatggctc agcccaggtc acgtgtccag tgaatgggtg gcatctgagc ctcctgcacc 120

tg 122

<210> 33

<211> 122
<212> DNA
<213> Homo sapiens

<400> 33

gcagcccggg aaggggcatt ggtggcgctt ggcagcaggt gtgacagacc tcctccgggg 60

cgcctgatcc gcggcggggg cggggcctgc ccctagggcc cctccagaga acccaccaga 120

gg 122

<210> 34
<211> 122
<212> DNA
<213> Homo sapiens

<400> 34

ctctgcgggg acagaggtct caggaaagta gcctttattt atgtggcacc gatcggaacc 60

cgcggccggc caggcggacc tggacggagc gtccctgctc ggaacctggc gcggggcgcc 120

gc 122

<210> 35
<211> 122
<212> DNA
<213> Homo sapiens

<400> 35

ttaattggct tgtgcctctt attttactct aatgcaatga ataaagacag tcccagcctt 60

cgccctaagg gagcaggagc acctgcgatg ccccgttccc aagtcctcag ggcgaatccg 120

cc 122

<210> 36
<211> 122
<212> DNA
<213> Homo sapiens

<400> 36

gatgtctcca ggcacccccg acctgggctt ggccctctgc ttggggcgga gcttccagga 60

cgtgctggga cctaggtctg accccgccca aggcagagtt gaacccactg tgaactttca 120

gg 122

<210> 37
<211> 122
<212> DNA
<213> Homo sapiens

<400> 37

acataataca cgctcaatta aagctgccga atgaaagtgt tcagaaactt gcacccatct 60

cgcctgggtt tcacctccct tttcctgtag gggaaaacc gatcctgaac cagtaaataa 120

ac 122

<210> 38
<211> 122
<212> DNA
<213> Homo sapiens

<400> 38

aaggaggaga tggccaaggg cgaggcgtcg gagaagatca tcatcaacgt gggcggcacg 60

cgacatgaga cctaccgcag caccctgcgc accctaccgg gaacccgcct cgcctggctg 120

gc 122

<210> 39
<211> 122
<212> DNA
<213> Homo sapiens

<400> 39

cctggtacta tttcttttgc aaattcagag tctgggtctg gatattgata gccgtcctac 60

cgctgaagtc tgtgccacac acacaatttc accaggaccc aaaggtgagg aaagaaaacc 120

ac 122

<210> 40
<211> 122
<212> DNA
<213> Homo sapiens

<400> 40

aagaattcca gtaaagagct gatcatggtt ctcactcctt gaataccagg aacaccatct 60

cgtatcacat aatgagacag ggagacattc tggtcctcat ctcacagatg aaaaatgtca 120

ag 122

<210> 41
<211> 122
<212> DNA
<213> Homo sapiens

<400> 41

caaggaaagt agcagatcat tacccaagta tttttataat tccttgtcct atgcttccac 60

cggtacactg caaattccac ccaaccatga ttaagggaaa agaaacaaag atagcatacc 120

tt 122

<210> 42
<211> 122
<212> DNA
<213> Homo sapiens

<400> 42

ccagtcccac tctgcttaac tgctctggca tgcttgaagg cctagcttag cgtagcaggc 60

cgttgcagcc gttctcgctc tgtggcattg ctctttgcct tcttggtcca gctgcctcca 120

gc 122

<210> 43
<211> 122
<212> DNA
<213> Homo sapiens

<400> 43

ctgacctcac cacccaccag ggaggtgggt cttattctgg gcatcgtgcc aagttcttag 60

cggggccctc tagaatctct aaagcaaatc aggctgaaga ggggaaaacc agcaggggga 120

gg 122

<210> 44
<211> 122
<212> DNA
<213> Homo sapiens

<400> 44

ggtcagcgtt ccgcggggga gacttcccag cgtcagctcc gacctcctct ttctctacca 60

cgatcccggc cagcatcccc gcccagcagc ggctcagcca caaacccaag ggtctccacc 120

tg 122

<210> 45
<211> 122
<212> DNA
<213> Homo sapiens

<400> 45

tctctccgca ttaatggcct ctggcagtct aattaatggc agtctggacc tcccctggat 60

cgtggggccc ctctgagacg tccccgatcc ccagcttaaa tttatccagg aggacctgtg 120

ag 122

<210> 46
<211> 122
<212> DNA
<213> Homo sapiens

<400> 46

ggagagcaag tcaagaaata cggtgaagga gtccttccca aagttgtcta ggtccttccg 60

cgccggtgcc tggtcttcgt cgtcaacacc atggacagct cccgggaacc gactctgggg 120

cg 122

<210> 47
<211> 122
<212> DNA
<213> Homo sapiens

<400> 47

agcatcgaga cagcgggcga acgggcgtcc ggggacaggg tgggggcggc ggggaggagg 60

cgtcggagac tctgaacccc agaaaagttc aaggtttgtg caggttcccc cagggaaggc 120

ga 122

<210> 48
<211> 122
<212> DNA
<213> Homo sapiens

<400> 48

acttcattgt ttggtgagtt gctttgcttt gctcgttgcc ccgatcttct gtgtattctg 60

cgcagacccc gcaagtgctc ctgcactccc tcccagccct ctgctggggc ttaacgcttc 120

cc 122

<210> 49
<211> 122
<212> DNA
<213> Homo sapiens

<400> 49

tgccgcgggg gagaggaacc cctcgcccca gccgggctcc accctagctc acccatcccg 60

cggcctacac tgaggctctc aatttgggtg gcacttatgg ggcatgtgtc ccctctctcc 120

tt 122

<210> 50
<211> 122
<212> DNA
<213> Homo sapiens

<400> 50

tggcatgggc tagagaataa aatgagaata gattttaaaa ggtctttgaa cagtcaaaag 60

cgaacaggat acctaagagg ttatttttag tcattgtcag cagaagctgg agattcccgc 120

ct 122

<210> 51
<211> 122
<212> DNA
<213> Homo sapiens

<400> 51

gaggcgcggg gtggagactg ggccgagcag gggatagaga tgaactccag aaaggaacag 60

cgacttgctg aaagtcacag ggcaaaatgt ggcgcgtctg tagtcaataa ataatatata 120

tt 122

<210> 52
<211> 122
<212> DNA
<213> Homo sapiens

<400> 52

ggcctcaggt ctttctccca aatagcagag aactcaaatg aagagtcatt tcattcccag 60

cggtttgggc agctcatggg atgacaggca actttttcct ttttttaaaa aaagaggccc 120

ag 122

<210> 53
<211> 122
<212> DNA
<213> Homo sapiens

<400> 53

cgctacgcga aggggaggag ctggtcatgg acgaggaggc ctatgtgctc taccaccgag 60

cgcagactgg tagggctgag tccggactcc agggtcctga ggtggctgat cccgagcctt 120

ta 122

<210> 54
<211> 122
<212> DNA
<213> Homo sapiens

<400> 54

ggctgtgttt agacctgagg gagccagctg tgaggctgga gcagttgctg catggcgggg 60

cgggggctcc acagggctgt tcacctgctg ctctgtgcag agacagcctc aagtccagct 120

gc 122

<210> 55
<211> 122
<212> DNA
<213> Homo sapiens

<400> 55

ggtaacagag cactgtgaga gcccgcagaa agctcctaac ccatctggga tgagacctag 60

cgcttccagg acgagccgat gttgagctga gacctcgaag gacaggttag tcattcacct 120

tc 122

<210> 56
<211> 122
<212> DNA
<213> Homo sapiens

<400> 56

cttcggcttc tcagggcgct gacgacgacg gcagtcgtag gaagccccgc ctggctgcat 60

cgttgcagat cagcccccag ccccgcccct ggcgaccgct acccgcccag gcccaaagtg 120

cc 122

<210> 57
<211> 122
<212> DNA
<213> Homo sapiens

<400> 57

ggcgagggtg aagttacctg cgtgcgtgct ggggctggca tctgcctggt tcgcatttgg 60

cggtaaatat caccgtctgc acacggggag gcctccgatt tccccattgt ttggaaactg 120

tg 122

<210> 58
<211> 122
<212> DNA
<213> Homo sapiens

<400> 58

tcttactccg tgggaaaatg gccctgagcc cgactggctt gaggcttaga caggtgaccc 60

cgcgaagcgg gtgggcaggc gcggccgagg ggcgggaggc gggcagcctc cgtgattggc 120

cg 122

<210> 59
<211> 122
<212> DNA
<213> Homo sapiens

<400> 59

cttccagcag aatttgggat cagggtgatc aaagacagga ggcttctggg gatgggtgtg 60

cgggctgttt ccagataccg ggagacccag aatctggtct gtggaagccc agcttccaga 120

aa 122

<210> 60
<211> 122
<212> DNA
<213> Homo sapiens

<400> 60

atcttgttca ctgttcagtc accagggcct gatggccgct catgctcaat atagacttgg 60

cgcggagcgg agtggaggaa ggaaagaggg caggtgctag ttggctggcc tgcagttaga 120

ag 122

<210> 61
<211> 122
<212> DNA
<213> Homo sapiens

<400> 61

ccctcccgcg cccccctttt tagcatattt gatcactttg attctctgtt cttttctctc 60

cgcggtgtgt gtgtgcgtgc gcgcgtgtgt gttttcttct tctcctcctc ctctccccga 120

gt 122

<210> 62
<211> 122
<212> DNA
<213> Homo sapiens

<400> 62

gctgcgtcct ggggctccag tagctggcgc gggctggggt gggctgggct ggcctgggac 60

cgcctcgatg ggacaggctc gggtttccct ggcgctgttt ctccctcctg cggtctacgg 120

cg 122

<210> 63
<211> 122
<212> DNA
<213> Homo sapiens

<400> 63

aggtgcccaa ctccgcggaa gcgccccttg ctgggtagaa gagtgggtct cccgccgcgg 60

cgcacctgtc tcggctgccg gctccccgca cctacctgta cgagacctgc ttccggaaag 120

tt 122

<210> 64
<211> 122
<212> DNA
<213> Homo sapiens

<400> 64

tgaaagcgat ccaaacacag ccagagggcg ccaaaatgcc gcaaataaaa gttccaaagg 60

cgtcaactgg cttttgcggg aaggtaaaat tggcttttgt gtaatcaaag agctaccgtt 120

gt 122

<210> 65
<211> 122
<212> DNA
<213> Homo sapiens

<400> 65

acccacgcgg aagccggagc ccgtgagcgt gtctgtgctg tggccgttct ctccgatgag 60

cgtcatgttg gagccctgct gacaactgtc ccgacactgg cccttgagac aggtccgctt 120

gc 122

<210> 66
<211> 122
<212> DNA
<213> Homo sapiens

<400> 66

ctggagttgg atcagaagga cgaactgatc cagaagctgc agaacgagct ggacaagtac 60

cgctcggtga tccgaccagc cacccagcag gcgcagaagc agagcgcgag caccttgcag 120

gg 122

<210> 67
<211> 122
<212> DNA
<213> Homo sapiens

<400> 67

gcagggcggg cagaagccgc aaccgcttca gcagcttctg ttccttggag ccaaagctgg 60

cgttacccat cgttgggatt cggaggggag atacgtgcac aagttctccc acacttagct 120

gg 122

<210> 68
<211> 122
<212> DNA
<213> Homo sapiens

<400> 68

gctccgtgct cccggctgag gccctggtgc tcaagaccgg gctgaaggcg ccgggactgg 60

cgctggccga ggttatcacc tccgacatcc tgcacagctt cctgtacggc cgctggcgca 120

ac 122

<210> 69
<211> 122
<212> DNA
<213> Homo sapiens

<400> 69

ggagcttgta ggggacgagg cgtagggctg ggatccggct cccaggtgtg ccgaagctgg 60

cgcgcgctct tccgccgcgc ggaaagtgcc gcggcaaact cgcggtgcgg agctccaggc 120

aa 122

<210> 70
<211> 122
<212> DNA
<213> Homo sapiens

<400> 70

ccacaacccc agcctcacac caccagccca tttatctgga ggacccctag tctgagacag 60

cgccaagaat cctgaataag ccataggatg gcagaggccc attgccaggt ggggaatccc 120

at 122

<210> 71
<211> 122
<212> DNA
<213> Homo sapiens

<400> 71

ggctcttcag cagcgagtgc agattgctcc cccgcggccg cagatctccc gtttgcgccg 60

cgttcagctg ctcccgaaca acttttctgc cggcccagag gccccagggc gtcgcagcgc 120

cg 122

<210> 72
<211> 122
<212> DNA
<213> Homo sapiens

<400> 72

gttggatctg acaatccctt ccaggttctc agactttaat ctcgagtttt cctgcccatg 60

cgccaggttg aacagttgct ggtgggttaa agagaatccc ccagcctgtt gctgtgtaga 120

ga 122

<210> 73
<211> 122
<212> DNA
<213> Homo sapiens

<400> 73

gtagagggct tgtttttaaa atccatccga aagggccaat cagacgcggc agtctgagtg 60

cgcaggcgcg gattggtccg cagctactta gagtgaccaa taggcgtgga ggtaagtttg 120

gt 122

<210> 74
<211> 122
<212> DNA
<213> Homo sapiens

<400> 74

ttgggatgcg ataactcagt gccctcttgc agacttgcat agaaataatt actgggttgt 60

cgtggagggg acacgagaca gagggagttc tccgtaatgt gccttgcgga gagaaaggtc 120

ca 122

<210> 75
<211> 122
<212> DNA
<213> Homo sapiens

<400> 75

tcaggtctcc ttggcagttc cccttctgct gttcttgttg ctgcttggtg ctgtgtgaag 60

cgcaccaggg cagagcccgc tgggggctca caagtgggag cggtaattgc gattggctgt 120

gg 122

<210> 76
<211> 122
<212> DNA
<213> Homo sapiens

<400> 76

aaaaggaaaa ggaggaagtg gaatgctggc ttttcaggtg tcgcttggcc aaatctaaag 60

cgtggcaact tcaggaattt caggttgtcc ccattgtcag attccaggca cccacaggta 120

ag 122

<210> 77
<211> 122
<212> DNA
<213> Homo sapiens

<400> 77

cgacccatcc cgctagaatc cgtccagtct ctgctcgcgc accgtgactt ctaaggggcg 60

cggatttcag ccgagctgtt ttcgcctctc agttgcagca gagaagcccc tggcacccga 120

ct 122

<210> 78
<211> 122
<212> DNA
<213> Homo sapiens

<400> 78

ctcgctgctt ctcccctagt cttcgggtcc cttgaacgca ggtcgcttgt ttgccttacg 60

cgtagtcagc ggccagtggc tatttatggc agtaaggaat attatccaca tttcacatgg 120

ag 122

<210> 79
<211> 122
<212> DNA
<213> Homo sapiens

<400> 79

tacctgttgg ccagggcgca gggcgcacgg aattcgggtg actttgctcc aagatacacg 60

cgtgtgtccc gactctcact caatttatag gggagaggga ctcgccaaat ccctgttttc 120

tg 122

<210> 80
<211> 122
<212> DNA
<213> Homo sapiens

<400> 80

ccctacacac ggaactcacc gtccttgtct ccgtcggggg cctctgcgga ggacgcgccg 60

cgaagccgcc gctgtcgccg cctccagctc accagaccca ccaggaccag cgccaggacc 120

ag 122

<210> 81
<211> 122
<212> DNA
<213> Homo sapiens

<400> 81

cccttccaca cacccttccc tgccggcccg cccctgccct ccccctctta ccgcgcaccc 60

cgctgagtct gctctgcctt gacctgcgac agtgcccagt gacccaataa cctccttcct 120

gc 122

<210> 82
<211> 122
<212> DNA
<213> Homo sapiens

<400> 82

tggcgatcca ggagcaccag tacaggtcgg tgacggcgat gaggtacagg tccagcaggc 60

cgccctgcgc cagcagcagc accacggaca gcgcctggta gccccagcgg cacctgggac 120

tg 122

<210> 83
<211> 122
<212> DNA
<213> Homo sapiens

<400> 83

tttgggacgg cgcgtcccaa gggtttctgg aagttgtaac ctgtgctccg agtgcgtagg 60

cgcaggaacc cttcggggga atccctttag cagggagcgt atattgaaga gtgcgtgcgg 120

ag 122

<210> 84
<211> 122
<212> DNA
<213> Homo sapiens

<400> 84

ccactggccc ggttcaacga atatctatta agtatccact ctataccaga cactgcttta 60

cgctccaggg atagagcagg gaacaaaaca gacaaaacca gtcccacgca gttgacagtt 120

gt 122

<210> 85
<211> 122
<212> DNA
<213> Homo sapiens

<400> 85

ccggcgggcg gcaaggctcc gggccagcat gggggcttcg tggtgactgt caagcaagag 60

cgcggcgagg gtccacgcgc gggcgagaag gggtcccacg aggaggaggt gagagtccct 120

gc 122

<210> 86
<211> 122
<212> DNA
<213> Homo sapiens

<400> 86

gacctcaagt gatccaccga cctgggcctc ccaaaatgtt aggattactg gcatgaacca 60

cggcgcccag cccatccgac ttttgtaaca ctcagaattg tagttttgtt tgtttgtttg 120

ag 122

<210> 87
<211> 122
<212> DNA
<213> Homo sapiens

<400> 87

tacctggggt ggaccaagca caggtcagcc ccctcccctt ggcgtcgggt cctactcgag 60

cgccccgccc cacatccacc aagagaggct gagctcagca gagtcgtccc ctcccccgcc 120

gc 122

<210> 88
<211> 122
<212> DNA
<213> Homo sapiens

<400> 88

agaaagctcc ctcaccggct cccctgctcc tgctcaacag gccctggtgg ctgcagatgt 60

cgtgcccccc agttggttcc atggtgaaca cactccagta gcggattact tttgcccttt 120

gt 122

<210> 89
<211> 122
<212> DNA
<213> Homo sapiens

<400> 89

atctctcacc ttgctacttt ctcggtagcc gtttctgttg tccctggatt gggggctcgg 60

cgttcgctgt ccctgggcac caacccttt aaagacagta acgttgtagg aaatcaaatt 120

ag 122

<210> 90
<211> 122
<212> DNA
<213> Homo sapiens

<400> 90

agtggttggg accctgtgag aaccggaact gcgaaaaccg gagaagggaa ttgttgaccg 60

cgaaagggac taaggaaatt gggattccag ttcgacccct aaattcacac catccttgct 120

aa 122

<210> 91
<211> 122
<212> DNA
<213> Homo sapiens

<400> 91

cctcacaggc tgagtggagt gttttgcagt ctcaaagcct tatcgctggc gtgcgcatac 60

cgcagggagt gacatcagat cgaaactaca gggtttcgcc ggggaccaac cactcctcca 120

aa 122

<210> 92
<211> 122
<212> DNA
<213> Homo sapiens

<400> 92

aataataaat aataatgaat ccattcttcc ttcggtcgtg ggtctggcag gcataaattc 60

cggccgggat tccgacccca gggccagagc aggactcgcc ttggcgtcta tgagtgggcg 120

gg 122

<210> 93
<211> 122
<212> DNA
<213> Homo sapiens

<400> 93

gggctgaaga gacccccccc caacacacca gccccgaaaa ccgtctgccg tcccctatag 60

cgctgcatgg aaaagaacca agacaaggac ttggagtgga gaagacagaa attgtccact 120

ga 122

<210> 94
<211> 122
<212> DNA
<213> Homo sapiens

<400> 94

ccatttgagg gcaagggctg tgtctttggg tacttcgctc ctcgcagtca caagtactgg 60

cgtgcgtacg cggggagaga tcgctcctca aaacggggtc ctgaacgctg ccccgcggcc 120

cc 122

<210> 95
<211> 122
<212> DNA
<213> Homo sapiens

<400> 95

gtcttccctc tgaggactgg atcctcaaga tggtggagat tatgcaaatg taggaaagta 60

cgatacaaag gaaaggagtc caaccaatga agaccccagt ggatagcagt gccaactcat 120

tg 122

<210> 96
<211> 122
<212> DNA
<213> Homo sapiens

<400> 96

ctgggggcct gtttgggaga tgccacaaga accttgccat tgggggcccc ctttgggga 60

cgacatagat attgctttgg ggccctggct gggtgatgga tgacacagag cttgtctttg 120

gg 122

<210> 97
<211> 122
<212> DNA
<213> Homo sapiens

<400> 97

ttccttttgg gaaacgcagt gtgctaaaaa agtgcatgca gcccaggctg tggcctaggc 60

cgtcggttcc cggccatgcc tagctcctct gaggtcgccc ttagtgagga cacgaggtgc 120

cc 122

<210> 98
<211> 122
<212> DNA
<213> Homo sapiens

<400> 98

taagcgataa ggagtttcac acgatgtctt tttatttcgc agttgagtcc cagtttctgc 60

cgctttatct ttcccgcctc ccggcaggca ggccgttaac cgtcttccgg aagacgctgc 120

ta 122

<210> 99
<211> 122
<212> DNA
<213> Homo sapiens

<400> 99

gaagggccac gccgagagag gcaggcaaca agggcacggc tggaggccgg aaggtcaccc 60

cgtccccggc ggggcgggcg cggcccagcc tcacttcccg ggcacgttcg ggcggggcga 120

tt 122

<210> 100
<211> 122
<212> DNA
<213> Homo sapiens

<400> 100

gaagggtggg cttagggcca ggggtgcaaa tccctcggta aaagccggca aactaaaagt 60

cgcacacatc ccaggtcccg gtccaggccc cggcggggca gggtccccga agtcccgggg 120

cg 122

<210> 101
<211> 122
<212> DNA
<213> Homo sapiens

<400> 101

ctggggttct aggctggagc aggctttgtg gaccccagcg gcctggtggt gagcagtacc 60

cgccttccac ttcctaaatc gggatgcaga gattctagtg gacaggcctt gtggtccggg 120

ga 122

<210> 102
<211> 122
<212> DNA
<213> Homo sapiens

<400> 102

gcggacagag atagaaaggc tctcagagat ccgagcctca ccgcgaacac ccggggcaaa 60

cgacattgcg gtgcatgtta agcagcatct tgcagtgcct ggcccttact cacaggtctc 120

ag 122

<210> 103
<211> 122
<212> DNA
<213> Homo sapiens

<400> 103

ctgctgggcc caggtcggct catgaacccg ctgcaggccg gcggaggccc gcttcagcag 60

cggctgcgtg ccaccccaca gagcggccac cagcaccaga gccaacacct gccctgaatg 120

ca 122

<210> 104
<211> 122
<212> DNA
<213> Homo sapiens

<400> 104

gcagcgggat catagctgct atggggctga gatccaggaa tctgtgtcgg gactgcgggg 60

cgctgggtta catcagaggc caggactggc acctggcgcc tttcacttcc ctaaacttgc 120

ct 122

<210> 105
<211> 122
<212> DNA
<213> Homo sapiens

<400> 105

gcagcctggg ccccgccgcc agccgctgct cggagggagc gagcgagaaa ggggagccgg 60

cgcagctcgc tgccctgttc cagaactcag aatttgagag gcgagagttc ggtaagccgt 120

gc 122

<210> 106
<211> 122
<212> DNA
<213> Homo sapiens

<400> 106

ctcctcctct tgaaaactct gctatggctg agttacccag aggaatctta gtcctgctag 60

cgctgcgatg cccattgccc agtgtgtcag tcctcattct ggggcgccaa atggggcagc 120

at 122

<210> 107
<211> 122
<212> DNA
<213> Homo sapiens

<400> 107

ttgttaatct ttaatttaat taaagaattt atcccccaaa taggaaagaa agcagcggag 60

cggctaaagc gtcatttgat ttttctgtcg atgacttgag ttgcctttga agggggtgaa 120

ta 122

<210> 108
<211> 122
<212> DNA
<213> Homo sapiens

<400> 108

tgaggccgtc gcatcaaatc ctcaatagag gctggatcct ggaagtccgg cctcgggggg 60

cgttgccagg aaggctagag acctggaagt ttgtccccag cccctcctcc ctcagacact 120

cc 122

<210> 109
<211> 122
<212> DNA
<213> Homo sapiens

<400> 109

ccttctagtc tccgggcagc ctggggagcg gcctttaatc ctggtccctt ctccgggata 60

cgtcgtcccc caggtgtctc agaccaccaa aactcaggtt cctgggtaga ccaggggggt 120

ct 122

<210> 110
<211> 122
<212> DNA
<213> Homo sapiens

<400> 110

tgtggtctgt ggcaacaggt gtcacttgaa tgaatgtccc agaggaagct gggtgtctcc 60

cgccctggct cctttccttg acctccctgc cccttcttgg cccaggtgtc ctggctcaca 120

gc 122

<210> 111
<211> 122
<212> DNA
<213> Homo sapiens

<400> 111

ggcacagctc cagggtgggc acggcggcca tggagtcgat gtaggcgctg aagtcgatgg 60

cgctctcgtc gtcgtacatg gcgggggcgg cggcgcctgg ctcgcctagg gcccctggct 120

cg 122

<210> 112
<211> 122
<212> DNA
<213> Homo sapiens

<400> 112

ctcccgccca gcgatgtatt cagcgccctc cgcctgcact tgcctgtaag cgcccgcgcg 60

cggggctgcc caccttgcct ggctgtctgt ccgtatgcct gtgccctgta cctctgtctg 120

cc 122

<210> 113
<211> 122
<212> DNA
<213> Homo sapiens

<400> 113

cactggcttg ttaactcttc aagggcagaa ttatgggcac cgagcctcta aaatgttgaa 60

cgaatgactg aatatcatca agaggcagta ctaaaagatg atgaaagaat gaatgagcgg 120

tg 122

<210> 114
<211> 122
<212> DNA
<213> Homo sapiens

<400> 114

gcagaaatgg gagaaggtgg cgtcgcgcgt gtcggaggga acggcagaac gcacgcttgg 60

cgtattatag tgggaaaggg cacagcctca actcagcacc cgcaactcac tcagcactcc 120

cg 122

<210> 115
<211> 122
<212> DNA
<213> Homo sapiens

<400> 115

gcctgttgtt gtggctgctg ctgttcagga tgtcccgggt gggaacttgg aggcgtcccc 60

cgcagcctct acccaggcct gccaggctcc aaaatactgg caaacatgtg aacaatgcta 120

ct 122

<210> 116
<211> 122
<212> DNA
<213> Homo sapiens

<400> 116

tttaactcag agttcttaac cttttctgcg ccgtgggccc cttggcaagc aagtgaagtt 60

cgtggactcc tacaataatg ctataaatgc atagaagaaa agacacagga ctgtgaaaga 120

aa 122

<210> 117
<211> 122
<212> DNA
<213> Homo sapiens

<400> 117

ccgtgtctgc ctcccgcttc cccgcctcgc gacttgagcc ccgcccgtac ctgcttaggg 60

cgctgccctc gcccgcttgc tccggatccc agcccaggta cccggcctcg cccgcgggtc 120

gg 122

<210> 118
<211> 122
<212> DNA
<213> Homo sapiens

<400> 118

gggggaaga cggagactct tataccgcgg gagactaacc tgtgagcaac agaagcacca 60

cgctacaaag agcatgacga gttcttccag gcttgggaaa gcacgggtaa atgcccgcgg 120

tc 122

<210> 119
<211> 122
<212> DNA
<213> Homo sapiens

<400> 119

aaacaaaaga actcagccaa gtgtaaaagc cctttctgat cccaggtctt agtgagccac 60

cggcggggct gggattcgaa cccagtggaa tcagaaccgt gcaggtccca taacccacct 120

ag 122

<210> 120
<211> 122
<212> DNA
<213> Homo sapiens

<400> 120

cgcaaatctc agggcggctc tggccagttt ggagcctggg gtgacccttg gagctgacct 60

cgctggtccc tgtcggagcc ctgcgcgctg cggagcttgg cggttcgcag ctctcggggt 120

ag 122

<210> 121
<211> 122
<212> DNA
<213> Homo sapiens

<400> 121

agttgctggc cttccacttg tcttcaggag ctgaaacaca tggcatttga aaaaaactgg 60

cgaacagagg aaactcttgc agcctcgcag ccgccctggt ccagtgccaa cggcaggagc 120

ac 122

<210> 122
<211> 122
<212> DNA
<213> Homo sapiens

<400> 122

gaaggagccc cgcccgcgcc ggccctggag tcgccggtgt cgccgccctg cccgcgggcc 60

cgccctcctg gcccagccca gggccctgcg agctattttg aaagtgaccc tgggctgggg 120

cg 122

<210> 123
<211> 122
<212> DNA
<213> Homo sapiens

<400> 123

tctggccggc cctggcgacg gggctgcaaa cgcttcgtag acctcagaac agcgcaacgg 60

cggaccggcg gaccggcacg aaacatagca gccccaccac aaacatttcc cttcttaatt 120

cc 122

<210> 124
<211> 122
<212> DNA
<213> Homo sapiens

<400> 124

agccaggatc tgccttttaa cctccatttg ctgttgagat gctcagttca acctgctgtg 60

cgggatagac atcgatgtct ccctgagaag cacatatagg ctctctgagg tttcttttct 120

tc 122

<210> 125
<211> 122
<212> DNA
<213> Homo sapiens

<400> 125

gtagccctgt tcctgtctgc cctccccgcc cccacagaaa tagagatgag aaggggcagg 60

cgaagaacta ggagtgtctg cgagaccatc ccaggaccct gagcccccca actctctgca 120

tc 122

<210> 126
<211> 122
<212> DNA
<213> Homo sapiens

<400> 126

gccgtgaatg gagtggagac tggccgcagg tcaggagagc tcaccacttg aaggtgaagt 60

cgccctgctc ggattccatc tgcagatttt gtttctcccc caaatcagcc actgctggag 120

ct 122

<210> 127
<211> 122
<212> DNA
<213> Homo sapiens

<400> 127

ggcagccaga aaggcagctc caagttgtgg atttcctggg ggctcttcat ttaaagcggc 60

cgcaccactt tccacaattc tgttttttca gagaatgctc tcaaggcctg gagggagggc 120

at 122

<210> 128
<211> 122
<212> DNA
<213> Homo sapiens

<400> 128

tcccttggcc tcgctctctg cccagccccg ggctcctttt ctccacacgt ggctgtcaag 60

cgccttctgt atgccccaca ctcctgggag cttgggctac atcgatgaac aaaaacaaag 120

ga 122

<210> 129
<211> 122
<212> DNA
<213> Homo sapiens

<400> 129

gcgcgcgtgc cgccgccgcg ggcactgcgc ccgtttgcct gcccctcgtc ggggatcggg 60

cgctccctct gagacctgaa agggcaccca agtgcccccct gtctgcgaag tccggcgcgg 120

gc 122

<210> 130
<211> 122

<212> DNA
<213> Homo sapiens

<400> 130

ttttctcttt gcagcgaggc tggagggtgg gctttttttt tttttttcc tttttgcgcg 60

cgtatgtatg tgtgtgcgcg caaagtatct ctatctaggg aatgaaaaat gggcgctggc 120

gg 122

<210> 131
<211> 122
<212> DNA
<213> Homo sapiens

<400> 131

gggcggggct gagacctgcg agaggcaggc tgggaagcgg cgccatattg gcgtcggccg 60

cgctgtattg tcataaatag agccggtttt gtggtgtttt cactactcgg ttggatgcct 120

ca 122

<210> 132
<211> 122
<212> DNA
<213> Homo sapiens

<400> 132

aaaacatata atatttaact tgagaggtgc agtcctcctc tacattgagg gcaggctcag 60

cgaaggaggg cccaagacat aaaactaacc aatggcagga aagcccccat gccccaccca 120

ag 122

<210> 133
<211> 122
<212> DNA
<213> Homo sapiens

<400> 133

atccagccca tcagtaaatc ctgttatcca gacatttctc agcactaatt ctgagaccat 60

cgtagtccac acctctatca tctcttgcct ggactactat ttaatgtaac agcttttaac 120

cg 122

<210> 134
<211> 122
<212> DNA
<213> Homo sapiens

<400> 134

aagcaggagc aggagcacgc gggacccggg ccgcaagtcc cgtcccatct cggggctccg 60

cggactctgc ggggatggag ccacctcgct ctgactccca gacatgctcc ggcgcgtgac 120

gt 122

<210> 135
<211> 122
<212> DNA
<213> Homo sapiens

<400> 135

gggtgcaaac ctttgggcat ccagggagag ctttcttgtt agagcccaca cacaatcggg 60

cgcatcaagt gggtaagtcc ccctcccccg ccgccacctt ctgaaacaag tagctcttat 120

tt 122

<210> 136
<211> 122
<212> DNA
<213> Homo sapiens

<400> 136

caaaataaaa cagagccctg tgagtcttca atttccgagt tgagtgacct ttcacagggt 60

cgcagaatca gccccagctc tcccccagtc ctttcactga ctcctctctg tggcagagct 120

ga 122

<210> 137
<211> 122
<212> DNA
<213> Homo sapiens

<400> 137

gcgcgccctg gagcgggagc aggcgcggca cggggacctg ctgctgctgc ccgcgctgcg 60

cgacgcctac gaaaacctca cggccaaggt gctggccatg ctggcctggc tggacgagca 120

cg 122

<210> 138
<211> 122
<212> DNA
<213> Homo sapiens

<400> 138

tcggacgcag gctggctggg cagggacact cggccggcgg ggctggcggt ggtggtcact 60

cgttcctccg gctcgcgggg atggccgag ggcgtgcagg gcccgcagct ccagaggctg 120

ag 122

<210> 139
<211> 122
<212> DNA
<213> Homo sapiens

<400> 139

ggttggggac gaggaggggg cgctcctcgg gcagggatgg ctcctcaggt gctttctggg 60

cgcggagcgg cggaggtggg agagcagctt gggaaaagga gcgcccggaa aagggcagcg 120

ct 122

<210> 140
<211> 122
<212> DNA
<213> Homo sapiens

<400> 140

tcgggggtgg tgttaagcag gttattaagt tccacgaaca ttccgagctc ctgggactag 60

cgctctggag gagaacccgg agtgctgcag agacgacgga ggctggagag caaaacacac 120

cc 122

<210> 141
<211> 122
<212> DNA
<213> Homo sapiens

<400> 141

cgacccggag cgcgggcgcg gggctgcgcc gtgccaggcg gtggagatcc ccatgtgccg 60

cggcatcggc tacaacctga cccgcatgcc caacctgctg ggccacacgt cgcagggcga 120

gg 122

<210> 142
<211> 122
<212> DNA
<213> Homo sapiens

<400> 142

cacctggtag ttgtctagct gctcttcggt gaagatggtc tgcttgttcc ccatggtggc 60

cgccgcgccg ccgctcgccc gcccgggctc cgactcccat cagcggccgc cagacccgga 120

gc 122

<210> 143
<211> 122
<212> DNA
<213> Homo sapiens

<400> 143

ttttccttgt gcagcttttg cccttctcag ttttattttc tcacatcgtc ctaatattaa 60

cgttcactgt ggttgaatga aagactgata gattacattt atttctcaaa gaagctaagt 120

tt 122

<210> 144
<211> 122
<212> DNA
<213> Homo sapiens

<400> 144

gactccatat gccctaggga tgtgttgtga tgaacttttc ctactggtac tgtttcctcc 60

cgcgagggaa tgtctagacc agccgcacct tcttgctttg acccttcaga actttggcct 120

gt 122

<210> 145
<211> 122
<212> DNA
<213> Homo sapiens

<400> 145

agagcaccag agagagaggg agagagagag agagcgctag agagagggag cgagcatgtg 60

cgatgagcaa tagctgtgga ccttacagtt gctgctaact gccctggtgt gtgtgaggga 120

ga 122

<210> 146
<211> 122
<212> DNA
<213> Homo sapiens

<400> 146

ccgcccgggg gcgggtggaa ggtggctccc ggggcaggga gcctgcaggg cggctcacag 60

cgcttctgct cttgtgtgtg tgtgaccccc aaaatgcctt ttatggtatt tttccagtcc 120

cc 122

<210> 147
<211> 122
<212> DNA
<213> Homo sapiens

<400> 147

gggccccgct tggggagggc gtggagggcg ccgaaggggt taacctccct ggggctggac 60

cgcggggcga gcccggggtg tggagtgggg ccctccccgc cgcgccggcc gggggaggcg 120

gc 122

<210> 148
<211> 122
<212> DNA
<213> Homo sapiens

<400> 148

ctgactggcc gaggtggcag cgaggagaag ctgtcccgga tgcccggagt cgccccgggt 60

cgaagccagc caggctcacc gctgctcagc ccctgccagc caatgtagcc cctaggggac 120

ct 122

<210> 149
<211> 122
<212> DNA
<213> Homo sapiens

<400> 149

aaataggggga gtctacaccc tgtggagctc aagatggtcc tgagtggggc gctgtgcttc 60

cggtgagtgt atgaggccct ggtttggtgg tgtcctccgg aggaagtgag ttctggatag 120

ac 122

<210> 150
<211> 122
<212> DNA
<213> Homo sapiens

<400> 150

cggggcgacc ccctccttgc ctcgctctct ccgggatcag agagagagcg agagagagag 60

cgcgcgcagg ttgcgactgg agggcctgtt ggggcgctag gcagagcgca aaccctagat 120

cc 122

<210> 151
<211> 122
<212> DNA
<213> Homo sapiens

<400> 151

ccacgaagag cttgatggcg tcgtggtcct tcatgggtac ggcgggaccg gggtttagcc 60

cgctcatgcc gacgccgctg tccgcggtgc tgaaacccag gcgcgggccg gggccagcgg 120

gc 122

<210> 152
<211> 122
<212> DNA
<213> Homo sapiens

<400> 152

```
gggcccgcgg cggctggtgg ataccttcgt gctgcacctg gcggcagctg acctgggctt        60
cgtgctcacg ctgccgctgt gggccgcggc ggcggcgcta ggcggccgct ggccgttcgg       120
cg                                                                      122
```

<210> 153
<211> 122
<212> DNA
<213> Homo sapiens

<400> 153

```
acacgggtgc gatcgcaggc agaagcagta cgggggaact taagaggggg actgtcaaag        60
cgagaaatag aaaccaagac caggtgaaga gcaagagtgg aatacaggga gggggcggaa       120
ta                                                                      122
```

<210> 154
<211> 122
<212> DNA
<213> Homo sapiens

<400> 154

```
ttttcatgaa cagaggtaca gctcagggag tgtggctaaa tcagtcccag tctccagctc        60
cgcgtgaacc tgggatccag acatctcctg gatatctggc gctctctgag atccagccct       120
cg                                                                      122
```

<210> 155
<211> 122
<212> DNA
<213> Homo sapiens

<400> 155

```
aggccgagcc gggagagccc ccgccccggg aggaagggga ggaggccgag tgtttcctgg        60
cgcattcccg gccagcccga gtgactcact cggccaagga aactcccagg gcccgcccag       120
ga                                                                      122
```

<210> 156
<211> 122
<212> DNA
<213> Homo sapiens

<400> 156

gggcctgggc attaagtcag tggttctggg cttggggtgc cgcacccagc acgaattcca 60

cgtcgcttcc ccctggcctc gttggggacc cctgcacctc tccggttccc gcagaggcgc 120

tg 122

<210> 157
<211> 122
<212> DNA
<213> Homo sapiens

<400> 157

aaaaaaatta ccgggcgtaa ctgcacgcgc ccgtagtccc agcactttgg gaggctaagg 60

cggaggatca cttgaaagag agagaaaagc agctacacat ctatagattc ggttcacaga 120

tg 122

<210> 158
<211> 122
<212> DNA
<213> Homo sapiens

<400> 158

tgcgccaggg cggccacgca ggccaggcag accacgtggc cgcaggacag gttgcgcggg 60

cgccgctgct gccggtggcc aaacttctca aagcacacct tgcactcgag caggctgatc 120

tc 122

<210> 159
<211> 122
<212> DNA
<213> Homo sapiens

<400> 159

tcacatctgt catctctcag gtcatatcca acacactggg ccacccacgc acagggacga 60

cgcgacagcc ctgtggctcc accgcacagg acagccacga ctggcaatcc tgtgccggcc 120

ct 122

<210> 160
<211> 122
<212> DNA
<213> Homo sapiens

<400> 160

gtgcagggaa agcacaccgt ggctgcagcc cagcaactgg cagtaggtat tttcaatggt 60

cggcaggtac tcatgacgga agttgccgct cgcccacttg tgcagcagcg tacttttccc 120

ca 122

<210> 161
<211> 122
<212> DNA
<213> Homo sapiens

<400> 161

```
cgaagatccg gccaatttgc ccagcgcgct gtgctccgcg acggcgcatg cccgcttttg        60
cgcaggcgcg gggactacgg cgcaggcgcg gagactattg cgcaggcaag cgcgtacgca       120
ga                                                                      122
```

<210> 162
<211> 122
<212> DNA
<213> Homo sapiens

<400> 162

```
ctccagtgcc ggcaggtggg agggctgagg tggcacaggc tgctccgcca cctcggactg        60
cggctcctac tcggccactg gccagagtcc ctccagccaa ctgcccctgg tgagaccacc       120
gt                                                                      122
```

<210> 163
<211> 122
<212> DNA
<213> Homo sapiens

<400> 163

```
tggctgcccc ggcaaatcgg agtgtaaagc cgccccggat tggctgaaac acttcctgag        60
cgattatctt tgtgaggctc gggtgagcaa gagccatcct gtgcatagaa aaagacaggc       120
ta                                                                      122
```

<210> 164
<211> 122
<212> DNA
<213> Homo sapiens

<400> 164

```
ctgagatctc gctggctctt ctcctctcgg attttcgggg tgctccctta gggaatcttt        60
cggtcccatc tcagagaccc cagaagggaa gtgtattagt gcgttttcac gctgctgata       120
aa                                                                      122
```

<210> 165
<211> 122
<212> DNA
<213> Homo sapiens

<400> 165

ctggtttata ctgccacatt cattcttgga ggtgagtaca tttcgatctt ggtccggctg 60

cgcagagagt caaagcagga aaatcacaga ttcttcccag cagtctacag cctacacagc 120

gg 122

<210> 166
<211> 122
<212> DNA
<213> Homo sapiens

<400> 166

gcaagcaatc ttaaaggaac tgggaagagt tctgactcct gtccttcttc cttaggactg 60

cgagtagact gtgagaaaaa caggttttct ggacttgaga tgtgtacaaa tggcacaaag 120

aa 122

<210> 167
<211> 122
<212> DNA
<213> Homo sapiens

<400> 167

gttggagtgc agacccagtc agtctcagaa taagacgaga agccgttgga gcattttgag 60

cggagatgac accatgtgat ttactttcta gctggcttaa gatttctcga tgtcattgtc 120

at 122

<210> 168
<211> 122
<212> DNA
<213> Homo sapiens

<400> 168

ctctgcaagc tccatgagga caggcgtgaa gttcaggcta catgcctggt acgtaataga 60

cgctctgaca gacatttgct gaatgaataa gttagtcact acggcgtttg tgggctttaa 120

aa 122

<210> 169
<211> 122
<212> DNA
<213> Homo sapiens

<400> 169

ggggcgcgcg aggggcgcag cgcccggagg gctgcccggg ggaacctgga gcccccgccc 60

cgggcctccc gacccgctcg cccgctccgg cctggtctgc agcagagact gcggcggcgg 120

cc 122

<210> 170
<211> 122
<212> DNA
<213> Homo sapiens

<400> 170

tcttctgaag gatttgatgc tggtgctttt caggtgtggg tcctgacagt gatgttggga 60

cggcagctag ccagacagca actgtaccat gtaaactcac ttcagaggtg tagaatgggg 120

gc 122

<210> 171
<211> 122
<212> DNA
<213> Homo sapiens

<400> 171

gggatgagga tggggcgggg aggtggtccc agcctgctat cacctagctg gggcgcgggg 60

cgctttggcc aagggacgat agcttgagat aaatgggagt gtggggactc tggaaagacg 120

gg 122

<210> 172
<211> 122
<212> DNA
<213> Homo sapiens

<400> 172

tgccaatcgg cgtgtaatcc tgtaggaatt tctcccgggt ttatctggga gtcacactgc 60

cgcctcctct ccccagtcgc ccaggggagc ccggagaagc aggctcagga gggagggagc 120

ca 122

<210> 173
<211> 122
<212> DNA
<213> Homo sapiens

<400> 173

gggtgagtgt gtgtgagtgc atgggagggt gctgaatatt ccgagacact gggaccacag 60

cggcagctcc gctgaaaact gcattcagcc agtcctccgg acttctggag cggggacagg 120

gc 122

<210> 174
<211> 122
<212> DNA
<213> Homo sapiens

<400> 174

gaggcgccag cgggaggcaa catcaatgca gttagctaca cgggcctgaa aactggaggc 60

cgcgacaagc gtcgctgagt ggaggcccag taagtcccac ccactaggcc agcccgagcg 120

cg 122

<210> 175
<211> 122
<212> DNA
<213> Homo sapiens

<400> 175

gcaggggggc gtcttggggg gcctcttagc gctgacttgc agcatgaggc agaagccgag 60

cgcggagagc gccagcagcc ccggccccgg gcccccctg gcccgcagcc ccgccatgct 120

gc 122

<210> 176
<211> 122
<212> DNA
<213> Homo sapiens

<400> 176

atcctcccaa actgtgagct gggaactagc aagaatcaaa aagccagtgt atgcttcctg 60

cgaaccacac agcctgaact gctgtagggt gatgtccctg tgtgacagac tggggtgggg 120

ag 122

<210> 177
<211> 122
<212> DNA
<213> Homo sapiens

<400> 177

gataagcgcc taatatacat ccctgcctgt cattattcac attgtggcat gcagtcaaag 60

cgacactctg aggaaaatgt atcgccttaa atacattgat tagaaaataa gaaagcccga 120

ac 122

<210> 178
<211> 122
<212> DNA
<213> Homo sapiens

<400> 178

ggggaagcac tctctaaacg ttagcaaata ccatggtagg acacaaggcc cctgactctc 60

cgctttcagc ttactgaaga tcctcaaaac caacagcaca cagcttccag cgcatgctcc 120

tt 122

<210> 179
<211> 122
<212> DNA
<213> Homo sapiens

<400> 179

ttgttgagag gcggacactg actcgggagg tctggggtag ggcctgaacg tttgcctttg 60

cggttctaac aagctctcag gtgatggcga tgctactgtt ccctggcccc gaggtagagg 120

aa 122

<210> 180
<211> 122
<212> DNA
<213> Homo sapiens

<400> 180

agctctccac cgaccgaagg aggagaatgc tatttatttc agcaccaaat atccggacag 60

cgcctctcgg gaggtccgag aagagaaccg cgatctgttt cagcaccggg gctcaggaca 120

gt 122

<210> 181
<211> 122
<212> DNA
<213> Homo sapiens

<400> 181

gggcttccta actttcaggt gtcagaatgt gtggcccagc ccacaggggc acggggaaca 60

cgctccgtac gggcaccgca ggctcggctc agaaatcccc cgccacgagt gtccccagac 120

gg 122

<210> 182
<211> 122
<212> DNA
<213> Homo sapiens

<400> 182

ataagccacg tctctcctca cccctagcac ttaatcacaa aggcctgtag agagtcccga 60

cgagaacttc tgagcaggcc ccgctgtcag tccctgagga cagcatgcaa gggaggttga 120

cg 122

<210> 183
<211> 122
<212> DNA
<213> Homo sapiens

<400> 183

ccggcgcctc tgcccgcagc gctcgccgtc gggctagggc tccgccgccg ccacgcctcg 60

cgcccggcac tcaccgcccc atgctggtgc acacctactc cgccatggtg agtagtctcg 120

gg 122

<210> 184
<211> 122
<212> DNA
<213> Homo sapiens

<400> 184

ggctgcccac ccgcccaccc cgcctggaag ctttctgatt tctctgttcg ccccgccagg 60

cgctgtgggg tccgtctcac caggtctgca cgtgagcccc ctgcccccaa tccctcccag 120

tc 122

<210> 185
<211> 122
<212> DNA
<213> Homo sapiens

<400> 185

ggtgggaagg aaatgtccct gagagccggg acgcgctgcc tccgctgcct ggaggagctg 60

cgctgtcctg ccagctaact tttgcccacg gtttccactg cccgggtgac ctttctgagc 120

gg 122

<210> 186
<211> 122
<212> DNA
<213> Homo sapiens

<400> 186

cgacgacgac ctcaacagcg tgctggactt catcctgtcc atggggctgg atggcctggg 60

cgccgaggcc gccccggagc cgccgccgcc gcccccgccg cctgcgttct attaccccga 120

ac 122

<210> 187
<211> 122
<212> DNA
<213> Homo sapiens

<400> 187

cgcaacaccc caggcgtggg gcaaagacag cggggttgcg gggctcctgt ctgcccgggg 60

cgtcgagagt tcctgccgcc ccctcccgcc tcatgcacgg aaagcgccga gccacggcgt 120

gc 122

<210> 188
<211> 122
<212> DNA
<213> Homo sapiens

<400> 188

gtgtgaccac ggaacggccc tgctggtgcc gggagcttgg ggggtcgagg gcttggcagc 60

cgcagcgcac aggccccgcg cgggtgggcg gtcagagccc gggaaccgag gaacgggtgg 120

gt 122

<210> 189
<211> 122
<212> DNA
<213> Homo sapiens

<400> 189

gacggaatga aatgaagtgc cctggagaag ccaactggag gtggtggccc cgagagtaga 60

cgcggagggg ctgaggccgc aggatcctgg agcccaggag ctgacggaga tcgcccacag 120

ct 122

<210> 190
<211> 122
<212> DNA
<213> Homo sapiens

<400> 190

ggaattcctg attccctggt ggaccctgga agttgtcctt aaataaatat atcgctggcc 60

cgcggttgag cagccacctc gtcagagcag catgtggact ggctcgccgg gtcccctccg 120

tg 122

<210> 191
<211> 122
<212> DNA
<213> Homo sapiens

<400> 191

ctacacaaag gcgctcacac tttatccgaa acagcagtgg ggcttgggtg cggtggctca 60

cgcctataat cccagcactt tgggaggccg aggagggtgg atcatctgag gtcaggagtt 120

ca 122

<210> 192
<211> 122
<212> DNA
<213> Homo sapiens

<400> 192

gaaaccaaga ctaggggcgc gccgtcacca gagaccgggc ctcaggctgg tgcggggcag 60

cggagaccca ggctgcggtc ccagttttgg cctgggctct acctcaaagc ttaaggaccg 120

gc 122

<210> 193
<211> 122
<212> DNA
<213> Homo sapiens

<400> 193

caagcctagg aaagtgcctc aggctggacg gtcccctgac cgccagatag cacttacccg 60

cggctccgaa ccacaccagc agctgtcccc agcagcccat ccctgttggg tccacccggc 120

aa 122

<210> 194
<211> 122
<212> DNA
<213> Homo sapiens

<400> 194

ctcctcctct gctgacatgt cactaggatt ggcaccacag tccaccttgc cttacttcca 60

cgccccccgc tttgtatagc aatatgttaa tatgcttaat tcaattccag aaaataccac 120

ta 122

<210> 195
<211> 122
<212> DNA
<213> Homo sapiens

<400> 195

ctttgctttc ttatctccag ctcacacctt taagtcttat gtagttaaag gacatttatc 60

cgcctccttg gagaacacag ccctccagtg tctcctgcag cctggagcct gggacattct 120

gg 122

<210> 196
<211> 122
<212> DNA
<213> Homo sapiens

<400> 196

taactgctgg acctgactgt gttacacagg atgctgctct ggtgcagaag ttttggccat 60

cgtatgcttg gggacagacc tgggcaaaag cccacagagg aagttgccac aaacacatga 120

tc 122

<210> 197
<211> 122
<212> DNA
<213> Homo sapiens

<400> 197

ctcaccaggt cactggctgg aacccctggg ggccaccatt gcgggaatca gcctttgaaa 60

cgatggccaa cagcagctaa taataaacca gtaatttggg atagacgagt agcaagaggg 120

ca 122

<210> 198
<211> 122
<212> DNA
<213> Homo sapiens

<400> 198

cggtttggag acggggggcg ctgtcggagg gagggaggaa gggagggagc gggggtgggg 60

cgcacagagg attccaacag gagactggaa gagattttga aaggtcatct cgtccttccc 120

cc 122

<210> 199
<211> 122
<212> DNA
<213> Homo sapiens

<400> 199

aagccggatc ctctccgttc ccttggagtg agcaagcggg acagttctgc ggaaagtttc 60

cgcccccaat cccccagccc tgcgcccgga ctgaagcggc ggcccccacc tccagcatcc 120

tc 122

<210> 200
<211> 122
<212> DNA
<213> Homo sapiens

<400> 200

gttccaagaa atctgccacc agctccaagc ctcatgtcct gaagtgccac ctcattcccg 60

cggggtgagc cagcagcctc tgaaaagagg aagccattga acagatcaca ctgtgcctcc 120

cg 122

<210> 201
<211> 122
<212> DNA
<213> Homo sapiens

<400> 201

tgattatatg tactattatt atctcatttt actactgtgg aaactgagat acgaaacttg 60

cggagtgagg atttgaacct aggtcatact cttggccagc cagagacacc ctaagcccca 120

gc 122

<210> 202
<211> 122
<212> DNA
<213> Homo sapiens

<400> 202

gcaagtttaa aagtactcac aaaatctaat aggcaattca acataaaact ccatggctat 60

cgctgttcct cactttctga acctttacct gcctgacttt actccatacc actccaactc 120

ac 122

<210> 203
<211> 122
<212> DNA
<213> Homo sapiens

<400> 203

cccccgcccg gtcctggaag accgggtcag gcattgtttt cttgcctatt gttccagttc 60

cgcgcccccc accctaagtt gagggagttt ggggagagtc tagggagcaa tgagtgaact 120

cc 122

<210> 204
<211> 122
<212> DNA
<213> Homo sapiens

<400> 204

gtagttttat tgtatcagac ttagtacagg ggtggggtgg gggtgtgtat tggaatgatg 60

cgtgcccgtt tctctgcaaa atagtttcta tgtcatggaa aggagtcgat gggacaagaa 120

ga 122

<210> 205
<211> 122
<212> DNA
<213> Homo sapiens

<400> 205

ggttttagcc agagagaagc ggatggaggc ggaacgctgg cagaggacgt tggtgggctg 60

cgtcccagct tcgtcagccc cacctggcct gaccccacca cacaggggtc ggcttccatg 120

ca 122

<210> 206
<211> 122
<212> DNA
<213> Homo sapiens

<400> 206

ggaggagggt tggagagcag ggccgtgttg caaggctctc tgggtggcca cagcagcttg 60

cgctgcgccc acattgcttc tgcgtgttta cagttgggca cgagaaggct cagcacgcac 120

gc 122

<210> 207
<211> 122
<212> DNA
<213> Homo sapiens

<400> 207

gggaggctca gttcctgggc ttgctgtttc tgcagccgct ttgggtggct ccaggtaaaa 60

cggggatggc gggagggttg acctccagcc ccacaggagg ggaccagcag ggatctctgt 120

gg 122

<210> 208
<211> 122
<212> DNA
<213> Homo sapiens

<400> 208

agcctgccgg cctggtgtgt ctcgggccgt aggtggcgac gtgggcgaag gatcagcgtc 60

cgcgcgggcc gggggcgcag ccatggcgct cggaggcctc tttgcgggcc tggccgggcg 120

gc 122

<210> 209
<211> 122
<212> DNA
<213> Homo sapiens

<400> 209

tgcctgatgg ataatccatc acttgctttt ctagtatgaa tggtctattt acgggtccag 60

cgcccctgct ggcttacgac cttttccagg gcggggaggg gctgtcctca tctctgtgac 120

cc 122

<210> 210
<211> 122
<212> DNA
<213> Homo sapiens

<400> 210

gtttgaatgt tgctgaagga cgctggtttt caaacggtaa ggaatctcct gataaaggca 60

cgaatcttgg tgtgcagata agccagcgat tcttgcttct ggctagttct acgttgttcc 120

tg 122

<210> 211
<211> 122
<212> DNA
<213> Homo sapiens

<400> 211

ccctgcgagg gggaaggtaa tggtttcaag ctgcccgggc tgggttccga atctctagga 60

cgccatggct gcgatctcct cgctttcctg gacatcttac ctccggatgt actccagtct 120

ca 122

<210> 212
<211> 122
<212> DNA
<213> Homo sapiens

<400> 212

tgagcatagt tgtcaccttc cccacctccc accaaaagtc cgggattttc acgaggggag 60

cgttttatct ttgggcccct agaagagtgc tttgtagttt gtaggtcctc agaaatttga 120

gg 122

<210> 213
<211> 122
<212> DNA
<213> Homo sapiens

<400> 213

tttccccgcc tcccaaccgt gaggtgttgg gtttggggga cgctggcagc tgggttctcc 60

cggttccctt gggcaggtgc agggtcgggt tcaaagcctc cggaacgcgt tttggcctga 120

tt 122

<210> 214
<211> 122
<212> DNA
<213> Homo sapiens

<400> 214

ataatcggcc tccggtccct gaggattcgg aaactcctga cgcagctaaa gtgaatctgg 60

cgctgagatg ccccctccat gggccggacg cggagggaag gggtgcccag ttgggttctg 120

gg 122

<210> 215
<211> 122
<212> DNA
<213> Homo sapiens

<400> 215

tgaatgaata aagggagcta ttgaaatgtc aggatgttct aaaacactgc caccttttca 60

cgtgtaactt caaattgagt tccatctcac ctctccaaat gtgacccaga aactagggac 120

ag 122

<210> 216
<211> 122
<212> DNA
<213> Homo sapiens

<400> 216

tggcagagca ggctgcctgc ctacttgtgc ttgattgaag tggcggtgta gttgtggtgg 60

cgcgaatcag cgtccagcaa cagtttgtgg aaactgtggg tttgctgagt atggcggggg 120

aa 122

<210> 217
<211> 122
<212> DNA
<213> Homo sapiens

<400> 217

accatctcac actgtcacat acacaatcat atccactgat agactgcaca cgcagtggca 60

cgcttaaacc gtcacacgtg ctcttgtcca tgcattcatt cccattctag gcactgtccg 120

gg 122

<210> 218
<211> 122
<212> DNA
<213> Homo sapiens

<400> 218

ctgccccgcg cgagggcctc acctgtgggt agaggtgctg catgaactgc tcccgagaaa 60

cgccctccag ccggggtacc gggaggtgct gcccggccat ggttgctcac gcctgccctc 120

tt 122

<210> 219
<211> 122
<212> DNA
<213> Homo sapiens

<400> 219

ggtggcggcc ccggcacggc ggctgctgct gctgctacag ctccggacgc ccgggccgcg 60

cgtgcctgct ccaaatcccc gggaaatgcc tgactcatac aggaggaaga ggaggaggag 120

gc 122

<210> 220
<211> 122
<212> DNA
<213> Homo sapiens

<400> 220

ctctgaccaa tcaccctttg ccttacaaca tgtaaaacgg ttatcaaatg ccttttaggg 60

cgggatttat cactaaactg ctccaggttt ggactataga aatgcggctg ttcgctgcaa 120

cc 122

<210> 221
<211> 122
<212> DNA
<213> Homo sapiens

<400> 221

agcttacgtc agtttctcgg tggcagcgaa tttactgcca gagtcttgtg gcatgagatc 60

cgcgcaggcc tggggccctg gccgggaacc cctcactccc caaacgtccc aagcccaacc 120

ca 122

<210> 222

<211> 122
<212> DNA
<213> Homo sapiens

<400> 222

tgacgttacg tactggaagt cccaggagga atgcccagca agtggaatcc aagacgttct 60

cgccttctcg gggacagggc catcaccagg attcggaaag gaacagggag gttcggtttg 120

tg 122

<210> 223
<211> 122
<212> DNA
<213> Homo sapiens

<400> 223

gatgaccttg gctaactgat cttatccctt gggccgctgt ggcacaggat gagtgagcta 60

cgcctggtaa caagagtgcc actctcgtgt aagggggctg cgaagtagaa aggaggccag 120

cc 122

<210> 224
<211> 122
<212> DNA
<213> Homo sapiens

<400> 224

cctctctacc gctcatctaa gggcgtctcc ggactgtcgc ccaccccacc atcctccctg 60

cgctgggggt actaaatccc gtgcaaaaag acctggtcca ttcccaagac tggtccagac 120

ac 122

<210> 225
<211> 122
<212> DNA
<213> Homo sapiens

<400> 225

ccagccaagt ggccttgatc gttttcccaa tgcccccgag cctgtttcct gccagtagag 60

cgggtcagat gttgccaacc tctgcagagt agcaataagc agtaaacgcc acgctctgca 120

ca 122

<210> 226
<211> 122
<212> DNA
<213> Homo sapiens

<400> 226

gagggagccg cggaggactg gcagctgcag atgctggagc aggccagcct gtggctgggc 60

cgtagcttcc tgctggcagg cttcctggta tcgagcagct gccccagcct ggagcaggcg 120

gc 122

<210> 227
<211> 122
<212> DNA
<213> Homo sapiens

<400> 227

gccaggtcac cctctcactc tgtgcctctt agttatcttg catgctctgg tctttgcata 60

cgctgctccc tgcaccagga acctccatcc ccatctttgt ctgcttgtcg aacttcagaa 120

at 122

<210> 228
<211> 122
<212> DNA
<213> Homo sapiens

<400> 228

gcagccagcg cagcacccaa ggcagcgcct ccagagtcag agccaggccc acagccgccg 60

cggccgccac ctgccaactc aaccgtccca tgccgccgct aatccgggac ccacagccac 120

gc 122

<210> 229
<211> 122
<212> DNA
<213> Homo sapiens

<400> 229

tcgacctgtc cgcgcagtga gtttccaaga ttcccgaggg atcttcaacc ctgtagaggg 60

cgccgccgtg cgcgttaggg acccgcgggc ggagactgca cctccgcagc tcgcggccct 120

gg 122

<210> 230
<211> 122
<212> DNA
<213> Homo sapiens

<400> 230

gggttacccg gccctcgata aggaaacact ccggccatat ccggagaatc tggggagcgg 60

cgggatagaa aaattcacta accacaggcc cgggcccaca agaagcgcag cagaaaggcg 120

tc 122

<210> 231
<211> 122
<212> DNA
<213> Homo sapiens

<400> 231

atatcgggtt tgtcagacat ggttgcggag gaaaagcgga gcgaggcgcg cgagtacgag 60

cgaagtctgg tctgcgcagt ggccaccacc gagttgtcgc cataatattt ttaataatgt 120

tt 122

<210> 232
<211> 122
<212> DNA
<213> Homo sapiens

<400> 232

tggggagggt ttcctggaca gaggtccttt ggctgctgcc ttaagacgtg cagcctgggc 60

cgtggctgtc actgcgttcg gacccagacc cgctgcaggc agcagcagcc cccgcccgcg 120

ca 122

<210> 233
<211> 122
<212> DNA
<213> Homo sapiens

<400> 233

agggggagta atttcatttg acgaccatat acaggcctaa tgggagcctg caaagtacag 60

cggccgcagt catgggtaga ttacaggatt cccatctgta agatcagtac tgtgggggtg 120

ga 122

<210> 234
<211> 122
<212> DNA
<213> Homo sapiens

<400> 234

aacgagccgg agagacttga ttgggccatt cacgcctcag gatgaggact ggccagtctg 60

cgcctggagg gcgggccggt cccgctgatc acgtgacacg atttttgaaa ggtgattggc 120

tg 122

<210> 235
<211> 122
<212> DNA
<213> Homo sapiens

<400> 235

cagaataagt agaggaggac aattcaagag agcacagagc tgcgtgcatt ctccctgtgc 60

cgcgacctgt atccaaaagc ctcagacgag acttgaggag cttcctagag gctctcctgc 120

ca 122

<210> 236
<211> 122
<212> DNA
<213> Homo sapiens

<400> 236

cgtcacagcc ggtccccaga gcaggattcc ttccggcgcc tgcgcctgat caccgctctg 60

cgcttgagct gataaactca gctgatggga taagagtctt gttttatcgg attttgggga 120

ag 122

<210> 237
<211> 122
<212> DNA
<213> Homo sapiens

<400> 237

tacagggctt aactcatttt atccttacca caatcctatg aagtaggaac ttttataaaa 60

cgcattttat aaacaaggca cagagaggtt aattaacttg ccctctggtc acacagctag 120

ga 122

<210> 238
<211> 122
<212> DNA
<213> Homo sapiens

<400> 238

gggagccagt gttctttctc tcctgtgact ttggtgaagt ctctcaccac tcagtgttgt 60

cgtgagcatg ctaggcagag tgcaagaaag gagcaagaac tcactaatgg ctaggccttc 120

cc 122

<210> 239
<211> 122
<212> DNA
<213> Homo sapiens

<400> 239

ggcctggaga ccaggtggtt cagactccat aaactctgcc cattctccag tgaggtggac 60

cgaggcaacc cctcaagtcc tgtccctccc catagtgacg gctctgtagc cgctgctggc 120

ca 122

<210> 240
<211> 122
<212> DNA
<213> Homo sapiens

<400> 240

gatggtgctt atggggcagg ttccctaaca gtcaggattc cggttgcagt ttttctcccc 60

cgccccaaag atacgtggtt gcagacgtaa gtaacaggaa tccatctttc tttgaaagtc 120

ct 122

<210> 241
<211> 122
<212> DNA
<213> Homo sapiens

<400> 241

tggtaacacg ctcagccgct gccacgctat ttaaacgcgg gctatggatc caggaaccgg 60

cgcgaatcaa tgagatcaaa tgcgagggag atgcaccgtc aattacaaac acttggacaa 120

gt 122

<210> 242
<211> 122
<212> DNA
<213> Homo sapiens

<400> 242

agtgggccag cagtcgggcc agagtccagc tcagcaactc cgggttacag gcagcccagg 60

cgggcctagc caccggcagc tgcactcaga ggccactgtg tcctggctga gctcatctgc 120

ct 122

<210> 243
<211> 122
<212> DNA
<213> Homo sapiens

<400> 243

ctctcttcct attttgtgat taggatgctc catcagtttc tgccaccagc ttgctggaga 60

cgctgcgtgt ccctgactcc tctcaaaggg tgaaaagctc agtcgcaccc gagacctgct 120

cc 122

<210> 244
<211> 122
<212> DNA
<213> Homo sapiens

<400> 244

agcagcaaca agttttgcat ttcagcaatc aatttcagcc attacatttg caccaatcag 60

cgccgcccaa gttccgggct cggggcgggg ctcgctctta aggtggtccg gggtcctggc 120

tg 122

<210> 245
<211> 122
<212> DNA
<213> Homo sapiens

<400> 245

gctaacggaa accgaggcac gtggactgca attatgcatt ttcattggtc ctcaggatca 60

cgcgacagga agtattgcgt aaccggttga ctgccacatg cgcattggct tccagggccg 120

ga 122

<210> 246
<211> 122
<212> DNA
<213> Homo sapiens

<400> 246

tcggggtccc ttggcctgga gaccctttgt ccaacccgtc gcccacctca agacctgcct 60

cgatgctgcg catacagtag gtatccaata aatgttcctg ggatagaagg caaaggcgct 120

gg 122

<210> 247
<211> 122
<212> DNA
<213> Homo sapiens

<400> 247

tcactaacat cgcgctccag ggccagccgg atctgcgtgg ccgcatccac cagatagtca 60

cgttttgtca tgtcaggcac tcccagagcc accctgttgc gaatctgctc caggtacacg 120

tg 122

<210> 248
<211> 122
<212> DNA
<213> Homo sapiens

<400> 248

gcagaaacgc ggggcggcct ctccccatcc ccgtgtagtt ctccgggctg aaccgttggg 60

cgcctatttg cagaaaaggc agctcctgag cctcaagaca gactcggggg ccaggcgtgc 120

gt 122

<210> 249
<211> 122
<212> DNA
<213> Homo sapiens

<400> 249

tcactattct tagtccacag gggagtagtg actacccagg gcttggtaag tgctcagtaa 60

cgtttgttga aagatgaatc aatatttcaa tgctggggca aagcagtgaa aaactgggga 120

at 122

<210> 250
<211> 122
<212> DNA
<213> Homo sapiens

<400> 250

tcggggtatt tttaggccgg cgataaataa ttcataggga acgtggcatc aggctccccc 60

cgcgggagga gggggcgcga gcagcgagag ccaccgtcac ccgcggctca aggacactcg 120

cg 122

<210> 251
<211> 122
<212> DNA
<213> Homo sapiens

<400> 251

atcagcatta ggggttggga ctgaggtcag agtcaggggt atcaggggtg ggagctcaca 60

cgaaagcctg gaggtgacag tccccgtcag cctcctgcag ttccacctgg atgaccttcc 120

tc 122

<210> 252
<211> 122
<212> DNA
<213> Homo sapiens

<400> 252

ccccagagag ctttcatcta gaaggtttga ctctggccag acaaccagcg agcatcttct 60

cgcaatctgt tgcttcttcc atggcaaact ccagagaatt aagaagccaa actcaacatc 120

gc 122

<210> 253
<211> 122
<212> DNA
<213> Homo sapiens

<400> 253

tcacaagtct gccaggggaa gtccctggac ttcttgcttc tttcgtgtag gacaggctgt 60

cgaaacctca gtggataaaa gacctagaga atgtgtatcc cagaagaagc tggccaagga 120

ta 122

<210> 254
<211> 122
<212> DNA
<213> Homo sapiens

<400> 254

tgggggtgcc tggagtttgg ctggggctgg gtgcccagtg ggcgggcaca ggccccttga 60

cgtggctgtg gcctagctgg cagcctcgtc cttcctctcc gctaggcggg cactggagct 120

tt 122

<210> 255
<211> 122
<212> DNA
<213> Homo sapiens

<400> 255

aacggggaag aggctgagat tgtatgactc ccagccacag tttgctgggc aagatactgg 60

cgccaggagg tggtgagatt tgtctaaggt cacacatgaa atccaggata gaactctgca 120

gc 122

<210> 256
<211> 122
<212> DNA
<213> Homo sapiens

<400> 256

actctggggc tcgagcttag gataacttca ggttcagctg aggcctctga actgtgactc 60

cgccccgtgg ccgcatgcgt cggaactcct acctgccctt tgcccttctc gaggccggtg 120

ct 122

<210> 257
<211> 122
<212> DNA
<213> Homo sapiens

<400> 257

tcttgccctc agattaccag acacgacgca gctggacttg tctcatgcct gcgataggga 60

cggcccccac cctgacttgc atggaacagt cgacataatg tggcctactg cttccacctg 120

ag 122

<210> 258
<211> 122
<212> DNA
<213> Homo sapiens

<400> 258

tggtctcccc tggagggtgg gcgggttatc tgagggagtc ctcggagggt cgcccccttg 60

cgcgtcagag ttgctgcgtg gggtctcaga gatagcgcct gggctgggga aatcattgtg 120

gg 122

<210> 259
<211> 122
<212> DNA
<213> Homo sapiens

<400> 259

tgttaggctt ctccatcgaa tcttctttct ccccatttcc acggagaaaa gcccttagtt 60

cgtccagaaa tgagtgatga ggcagctcag cctctctgag aaagacctgg gttcaaatgc 120

ca 122

<210> 260
<211> 122
<212> DNA
<213> Homo sapiens

<400> 260

aaatgctcaa aatcaagaat tacaaaaaaa tcccttaata acaagcaaat tcctaacaca 60

cgttaaatat atcatttctc tcttactaga catagcatga cacagtttaa cagtatcaga 120

aa 122

<210> 261
<211> 122
<212> DNA
<213> Homo sapiens

<400> 261

ggtcttgtgt gttcagaggc tggttttaca ggtgaagaga agaaacagcc gcagaagttg 60

cgattgtcca aggtcactta ataagtggca agaattagga tgttaagtgt tctcaccccc 120

ag 122

<210> 262
<211> 122
<212> DNA
<213> Homo sapiens

<400> 262

**acccctggac gctgcgtcct gatttcccca gggacgcagg cctggttggg agaaggggtg 60**

**cgagctccga ttccggactc tgcttgggtt taaaacccag attgagggct gggcgcggtg 120**

**gc 122**

<210> 263
<211> 122
<212> DNA
<213> Homo sapiens

<400> 263

**accaggggt gatgccagac attgctcact ttttccatgt agtcaatgtc agtcctgcag 60**

**cgtcagctgg gatgggggta aggacatctg ggaacccct cttcctggtc tccctccctc 120**

**tt 122**

<210> 264
<211> 122
<212> DNA
<213> Homo sapiens

<400> 264

**gggccccgag ctgcgcctgt ccagccagct gctgcccgag ctctgtacct tcgtggtgcg 60**

**cgtgctgttc tacctggggc ctgtctacct agctggctac ctggggctca gcataacctg 120**

**gt 122**

<210> 265
<211> 122
<212> DNA
<213> Homo sapiens

<400> 265

**tcacatgttt cgtttctagt cctgaaacat ggttaagtgc ttgcctccta gggcctctgc 60**

**cgcaggcttt tggtttggag gctctccttt gccactccac ccctctccac tcttctcctc 120**

**tt 122**

<210> 266
<211> 122
<212> DNA
<213> Homo sapiens

<400> 266

attcacattt agttcgccta ggaaaactag cagttagtga aaaactggcc acatcacagc 60

cgcacagctc cagcagcccg ggtagcttcc ccaccctcac tttctccagc cccgcctcca 120

gg 122

<210> 267
<211> 122
<212> DNA
<213> Homo sapiens

<400> 267

ctactcaagg ggcatccacg gagctgggtc agcaaacata acactggtca tctgagcctg 60

cgcccgccct tcctcccagg ccagggcgcc cccaccccct gggtttttcc tccgtggacg 120

cc 122

<210> 268
<211> 122
<212> DNA
<213> Homo sapiens

<400> 268

cagacaccga gccgcggcca cagggccagc cgcacagtcg gaggaagggc cggagcgagg 60

cggggcccgg ggctgtcaag gagaaaaaca tcccaaggcc tgcaaattgc tgctctcagc 120

tt 122

<210> 269
<211> 122
<212> DNA
<213> Homo sapiens

<400> 269

aagggttcat caggatggag atatccggtg caccatgagt tctgtttcct taatcaacac 60

cgttgtaact tgcccatcca gttttgtgac attaattcaa acctgtgccc tagtcctctt 120

tt 122

<210> 270
<211> 122
<212> DNA
<213> Homo sapiens

<400> 270

gcagtgcatc gagctggagc agcagtttga cttcttgaag gacctggtgg catctgttcc 60

cgacatgcag ggggacgggg aagacaacca catggatggg gacaagggcg cccgcaggtg 120

gg 122

<210> 271
<211> 122
<212> DNA
<213> Homo sapiens

<400> 271

tcaacatact acatgatttg cttacaatac ttgtctgtct tgccttcacc agaatgtaag 60

cgctctacaa aggcagaggg aaggctatct tgctctctga tgtatcctcc agcccttaga 120

ac 122

<210> 272
<211> 122
<212> DNA
<213> Homo sapiens

<400> 272

ggtgtgaatc acactgcccg gtcgggcctt tgggaaaaaa ttaatgaagg acacagtcag 60

cgccgtagaa cctgccaaat acacatcaga tccagtggag tctgtgaagg gggaggggga 120

ga 122

<210> 273
<211> 122
<212> DNA
<213> Homo sapiens

<400> 273

gcctttctcg ggatctatct ttctgtgtct ctttcccttg ctgattttct gtccatttcc 60

cgcaccacca ctaccaccaa accctcctcc cgccttcccc cacccctagt ctctgtcttc 120

tc 122

<210> 274
<211> 122
<212> DNA
<213> Homo sapiens

<400> 274

actttgctcc tggtggtttt cactgttctg ccatggtggg gttctgaaga ccaggctcat 60

cgtactcacc ttgcaacacc tgcccctcta atccacactt tttctagaag cactttaaga 120

ta 122

<210> 275
<211> 122
<212> DNA
<213> Homo sapiens

<400> 275

cgcacaaaat cccagcctca agggcagaac attttaaatg acccacccat cctagagatg 60

cgccagttag gtcatcttat atatcttgag atagctgaga tggtcagatc aaccaaggac 120

ct 122

<210> 276
<211> 122
<212> DNA
<213> Homo sapiens

<400> 276

actgacaatg ctatagcatc ctggccatat ccagttttga aaacactacg gtgtcagcca 60

cgcaccattt aggacgggga gaatggaaag ccagtttgga gaacagacgc tttcttaaga 120

gt 122

<210> 277
<211> 122
<212> DNA
<213> Homo sapiens

<400> 277

tccctagtat cacattctca gctacttctg cctccttgaa agtttctcat gatgaaattt 60

cgcaaaattg taactaacat aaaagataac attattttcc ccatgctgtg gttcaagttt 120

ag 122

<210> 278
<211> 122
<212> DNA
<213> Homo sapiens

<400> 278

gtcagtgttc ttttagtttg cttaaactgt gtgggtactt gagtcctttt aaacgattaa 60

cgctgggaag aggcaccatt taattaatta atttgttctg gaagggatca gtgtacaatt 120

tt 122

<210> 279
<211> 122
<212> DNA
<213> Homo sapiens

<400> 279

ggagacagaa ctttcccctt ttttcccatc ccttcttctt gctcagagag gcaagcaagg 60

cgcggagctt tagaaagttc ttaagtggtc aggaaggtag gtgcttccct ttttctcctc 120

ac 122

<210> 280
<211> 122
<212> DNA
<213> Homo sapiens

<400> 280

```
tgtcctttgt gtcttgagcg gatggtgggg ccgtggaaca tgaaggagta tctttgtgta        60
cgttcacaac gttcacatcg gtgtaggcca ggttgctgga ctctgactca aagtgttata       120
ga                                                                      122
```

<210> 281
<211> 122
<212> DNA
<213> Homo sapiens

<400> 281

```
ccaacttcga gacttgcagt caaagcgatt tttaaaatga cttgttttca agcctctggc        60
cgccgcccac tcttctggcc cttggacttt gaccaagatg ttttctcgca gtttttgcaa       120
gg                                                                      122
```

<210> 282
<211> 122
<212> DNA
<213> Homo sapiens

<400> 282

```
ccccctcgcc cggcccggcg cccactagcc acagggcccg cttccccctg gagatcagcg        60
cgcacttccc gagccctcgt agcactcaga ggtcgcatcc acacctggga tgcctagggg       120
gc                                                                      122
```

<210> 283
<211> 122
<212> DNA
<213> Homo sapiens

<400> 283

```
ggagtcctgg ctcccattgg ctgcagcggg aaatggtgaa ccaatgctca tagaccttaa        60
cgccctcctc tcgggatcac ttccgcctct ggggtcaggc tccgcccagc ttgcccggca       120
tc                                                                      122
```

<210> 284
<211> 122
<212> DNA
<213> Homo sapiens

<400> 284

ccagaaattg ggcggcagtg aggtcgccgc aaggcttccc gtggaccctg caaaacgtgg 60

cgtgggcatt gcacaccatt gtactgtatg gaaacttctg cagaggttag caccgtgcct 120

ga 122

<210> 285
<211> 122
<212> DNA
<213> Homo sapiens

<400> 285

ggctaaattg atcaggttct cccatgtact tttcctttta aaatttccag tggctcattc 60

cgttatcagt aatgagtaat tgattagtgc caactgccga aggacttagt attctcattt 120

ag 122

<210> 286
<211> 122
<212> DNA
<213> Homo sapiens

<400> 286

gttgaaaaag ctaagtaatt ctgtaaaaat gtctactttc tcattacagt aagatgtttt 60

cgcagagtta acagtgctct ggtgtagata accaagactg cttctgtaaa ttaggcctac 120

tc 122

<210> 287
<211> 122
<212> DNA
<213> Homo sapiens

<400> 287

cctcagccag gaggaggccc aggccgtgga ccaggagcta tttaacgaat accagttcag 60

cgtggaccaa cttatggaac tggccgggct gagctgtgct acagccatcg ccaaggtcag 120

tg 122

<210> 288
<211> 122
<212> DNA
<213> Homo sapiens

<400> 288

ccgcactcta gtcccagtat ttgctaagct attgctttaa agacacccca tttctttacc 60

cgcctccacc agacacgcgc acaccctccg ctttgctgct ccatcctttt ctggagagga 120

gg 122

<210> 289
<211> 122
<212> DNA
<213> Homo sapiens

<400> 289

ttatccccaa agcagcccac gcccgggtgg gcagggtccc ccggggctgt atgaacagaa 60

cgtcagacct gggaaggccc cattccagaa atggggcccc tcactctggc acccccgggt 120

gt 122

<210> 290
<211> 122
<212> DNA
<213> Homo sapiens

<400> 290

cccgcaacct ggcagttact agaggtcttg gaatccagac ttctttgctt tcgccatcac 60

cgtcatcaaa gtgggaaatg cacacttact gttaaaacct agtgtagggc cgggcgcggt 120

gg 122

<210> 291
<211> 122
<212> DNA
<213> Homo sapiens

<400> 291

cagctggatg cacttgttct ggagctcctc tgtgagttca gcaatggcca cagtctgctt 60

cgacagctgc tcccgcagct ccttcaaatg gtactcccgc tcctggatct cagcatcctt 120

cc 122

<210> 292
<211> 122
<212> DNA
<213> Homo sapiens

<400> 292

cggtgctgcc tccacgcccg gcttccccat ggctgctgct gccactggca ctgctaagtg 60

cgttgccaag gcctctgttg gtcccaggtg actcccaggg caccgcccac aggggccggc 120

ca 122

<210> 293
<211> 122
<212> DNA
<213> Homo sapiens

<400> 293

tttcttcaaa ttaaattgct acagcaggaa attactgaac tgtggctctt ctcctacgtc 60

cgccttccct atgtcaattc ccatttccct tgctttctcc aatagttagg actgtaaatt 120

ct 122

<210> 294
<211> 122
<212> DNA
<213> Homo sapiens

<400> 294

aaaataataa ttaaaactcc ctcaactttt aaggccgagc aacataatct attaattggt 60

cgctattaac atgcagtttt attgaccata gcacacagaa gtctgattgt gagggaggag 120

tg 122

<210> 295
<211> 122
<212> DNA
<213> Homo sapiens

<400> 295

ccctcccccg ccagcctggc gcattgcggg cctcgggctc attgctgaga gggggcactg 60

cgcctggcac ctctgttaag caatttaggg gctacaacct gagcaagaca gatgagcccg 120

gc 122

<210> 296
<211> 122
<212> DNA
<213> Homo sapiens

<400> 296

tggaaggtgc tgtttcctgg tacctgtcca gccctctgag cttttctctc agcttccaaa 60

cgctgcagtt gagaactagc agatcctatt ggtagtgccc tgtggcccac actccttggt 120

aa 122

<210> 297
<211> 122
<212> DNA
<213> Homo sapiens

<400> 297

ccaggggacc agttccttgg tgttgctttg gcattgatgc ctgaagtggg aggagaaagc 60

cgagcccaca aacacacaga gcagagtggg gctctgagta tataactgtt aggtgcctcc 120

ct 122

<210> 298
<211> 122
<212> DNA
<213> Homo sapiens

<400> 298

tctgaggttt gtgttattaa ccccctatta tctttggtct acccagggca gccaaagagg 60

cgcagagaag aatgacaagg tgcccagcaa gcggcaggat caaagcctgg gtctctaatt 120

cc 122

<210> 299
<211> 122
<212> DNA
<213> Homo sapiens

<400> 299

ggcgattccg taatttccgc ttccggtagt gagaaccctt ccggtgggct aggtactgag 60

cgcgcgaggt gaggagttgt gcagggtttg gggaaaggaa ggctggcttg gcgagagggc 120

ag 122

<210> 300
<211> 122
<212> DNA
<213> Homo sapiens

<400> 300

gcagagcagg ctgcctgcct acttgtgctt gattgaagtg gcggtgtagt tgtggtggcg 60

cgaatcagcg tccagcaaca gtttgtggaa actgtgggtt tgctgagtat ggcgggggaa 120

tt 122

<210> 301
<211> 122
<212> DNA
<213> Homo sapiens

<400> 301

ccagtagagc gggtcagatg ttgccaacct ctgcagagta gcaataagca gtaaacgcca 60

cgctctgcac agcctcccag tgctgggcct ggtcgccacg cggagccttg ggctgggaca 120

gg 122

<210> 302
<211> 122
<212> DNA
<213> Homo sapiens

<400> 302

actgctggat cgtgagaggt aagcatgctg gcttctactg aaacgcccct tgtcatcaca 60

cgcccatccc ctggggcgac acgacccagg ccccgcccct cgggggctg ctgcgagtcc 120

gg 122

<210> 303
<211> 122
<212> DNA
<213> Homo sapiens

<400> 303

ctcgacctcg gcttgggagg cagcggccac gacagccagc agtgtggtca gcagcttcaa 60

cgcgcgtacc gccatcgctc cctcagacct aacggaaccg ccagccaccc gccaccaagg 120

cc 122

<210> 304
<211> 122
<212> DNA
<213> Homo sapiens

<400> 304

cagtagcagc agcagcagcg aagacagggg tgtcagagtc cccagcatgg cgtccgtgga 60

cgtgctgcaa agaagaacag agaaagtcat caagccagcc ctgggtggtt tggcactagg 120

cc 122

<210> 305
<211> 122
<212> DNA
<213> Homo sapiens

<400> 305

cgattatctg tacccaaaac agtatgagtg ggtcctcatc gcagcctatg tggctgtgtt 60

cgtcgtggcc ctggtgggca acacgctggg taggtccagg gcttgcccgg cagtgctgcc 120

gg 122

<210> 306
<211> 122
<212> DNA
<213> Homo sapiens

<400> 306

gggccctcca tgccatcgga gctggcatct ccagctagaa aatggccagt tgttctgatt 60

cgtagctctc ctagtcagct tccagtccag ggcagagggc agggactgct agggacctgg 120

gc 122

<210> 307
<211> 122
<212> DNA
<213> Homo sapiens

<400> 307

ataacaataa taataatggt agcaagcaac gctctgcagt aggggcttct ctcgccattt 60

cgtactgagg aggaaacata cttaagaggt tacaaaactt gcaccaaaca gataaccctc 120

gg 122

<210> 308
<211> 122
<212> DNA
<213> Homo sapiens

<400> 308

tctgcttaca gctgcttcca aattaagcat atctggatgg tgtgacactt tttgttagtc 60

cgagaactgt atgggcatcg caactgggcc tgttccaaga tagacttgtt gggaccttca 120

aa 122

<210> 309
<211> 122
<212> DNA
<213> Homo sapiens

<400> 309

cattcttatg cgactgtgtg ttcagaatat agctctgatg ctaggctgga ggtctggaca 60

cgggtccaag tccaccgcca gctgcttgct agtaacatga cttgtgtaag ttatcccagc 120

tg 122

<210> 310
<211> 122
<212> DNA
<213> Homo sapiens

<400> 310

ggacaaagcc accacctttc acaaaatgag gccagaccac ctgcctccct ccagtccctg 60

cggcctggag acggagtcaa cattcttatc tgtgttggat ctgaatgttc ctccttgcaa 120

ag 122

<210> 311
<211> 122
<212> DNA
<213> Homo sapiens

<400> 311

aaaagggtgg gagcgtccgg gggcccatct ctctcgggtg gagtcttctg acagctggtg 60

cgcctgcccg ggaacatcct cctggactca atcatggctt gtgtgagtgt ggggaccccc 120

cc 122

<210> 312
<211> 122
<212> DNA
<213> Homo sapiens

<400> 312

gactagcatt ttatttccat tggacagcgc tggctgagaa caaaacctaa ccctctgtgc 60

cgccctcgcg gccgggatgc ggtgcgcccc gggcctcccc attcggaaaa cgaggagcct 120

gg 122

<210> 313
<211> 122
<212> DNA
<213> Homo sapiens

<400> 313

actgcgatga aaggccataa ggatgctcac acccgaatct aaaaagccct ttgtgtgggc 60

cgcagccaag catactttgg caagaaattt ctgtggctct aacctccttt gaaaactgga 120

ga 122

<210> 314
<211> 122
<212> DNA
<213> Homo sapiens

<400> 314

gacggagaca gagggtggtt ccgggattca cagtgcagag gcggccagag cagtgcacag 60

cgccccgaga aatgggcccg gattccctgg gattgaaggg aaacattttg gcgcggggtc 120

cc 122

<210> 315
<211> 122
<212> DNA
<213> Homo sapiens

<400> 315

gtagtccccg aggtcacaag gcagtggcag gtgtctgtag tcctcgggtt gactgcagct 60

cgcggtggtc cctctccgag cccaggaagc cactccagtg ccgagggaga ggcctgggag 120

cg 122

<210> 316
<211> 122
<212> DNA
<213> Homo sapiens

<400> 316

agacccaacc ccagtcctaa agctacctgg cttcttcccc ggctcaggca tcctgagaga 60

cgtcacacca ggcacgaagc aggcacaggt cacccaaaga gggactgagt ggggtcctgt 120

cc 122

<210> 317
<211> 122
<212> DNA
<213> Homo sapiens

<400> 317

ggcctgcgca acaccccaga ggcaaggtga acgcgagggc ctataatgca agaaccaagg 60

cgagtcacgc cctgtctggg caaaagagga gtaaagaccc ctcagctgca gcccggcagc 120

gc 122

<210> 318
<211> 122
<212> DNA
<213> Homo sapiens

<400> 318

gtcggcctgg caggcgcggc ccccggttca gctgcgccgg ggcggcccag cgcgactccg 60

cgggcctttt ggctgctcgc cccggctccg gaacactgtc agatccttct ccgcagaggt 120

ag 122

<210> 319
<211> 122
<212> DNA
<213> Homo sapiens

<400> 319

ctgtgtcccc tctcaccaaa gtccagtagc tgcttcatgg acagcgggga cgggctgtag 60

cgcgagaaat gctccacctc tcggggcacc aggccggcgc cgttgagcga gccagcgctg 120

cg 122

<210> 320
<211> 122
<212> DNA
<213> Homo sapiens

<400> 320

ttttattgtt ttatgtctct gcaggtctcg tgtttctctc ttccaatcgg ttgtctttat 60

cgtggacact gaggtgttct ctgccttgac taaagatgag tgacgtgaat ccaccctctg 120

ac 122

<210> 321
<211> 122
<212> DNA
<213> Homo sapiens

<400> 321

gaaggctcct gggcctttct ggctctggga atgaagcgtg gaaaaccctc cttaggcggg 60

cgcagtgctt caagtagcca agctctgact tccgagggaa gaaaggaggc catgggcctc 120

tg 122

<210> 322
<211> 122
<212> DNA
<213> Homo sapiens

<400> 322

gaccacgagc atggacatga tggtcgcgct cactccggtg cagtgagtgt ctggggtgag 60

cgtctgcagc aatgaggccc caagggaggg cggtggggtg gctcgggcac tgacctcttc 120

cc 122

<210> 323
<211> 122
<212> DNA
<213> Homo sapiens

<400> 323

attagggtag gcccctggtc ctcgcgcttc ccagggtaac ctggagcagg ggtcccggag 60

cgcactcctg ggctcagct cagcttcact taccagggtc tgctcgtact gcagcgcccg 120

tg 122

<210> 324
<211> 122
<212> DNA
<213> Homo sapiens

<400> 324

gcctgtgatt gggagttgct ggagtcggtg cttcactctt aaggttccga tcacagactg 60

cggagtgggt caggggctgc gagggctgcc ccaagtccta ccgggtttgc acgggcgcgc 120

cc 122

<210> 325
<211> 122
<212> DNA
<213> Homo sapiens

<400> 325

tagctatgac acatggcttg gaaattaacc tttaaccaaa catcttataa gtaacgccag 60

cgcagcttcc cttgtgaatg taaagagatc cagggctctt ggagagggac aagtgagagc 120

ca 122

<210> 326
<211> 122
<212> DNA
<213> Homo sapiens

<400> 326

caaaaaaggc gggctgtttt gtaaatattt gtctctatgt aaggaaatca aaactgaaag 60

cggagtaaca ccaagtatgc ccgtttcttg agctcaagca ctggaaggat caaaagtagc 120

ga 122

<210> 327
<211> 122
<212> DNA
<213> Homo sapiens

<400> 327

tcagtctccc catatttaca ataaaagggg agcgaggtgg gatggcgctg aggatcccta 60

cgtccgatcc taatctccag ctcaggcagg ctcggccgcc actagcatcc tggagcgaca 120

ac 122

<210> 328
<211> 122
<212> DNA
<213> Homo sapiens

<400> 328

aaccccggca tgaccaccag cctcccggct ctgcagtcgg cgcccaggcc ggccgcttcg 60

cgtcacttga ctaaggaccc acggcctggc accgcccctc gtcggcccag cagccagccc 120

tc 122

<210> 329
<211> 122
<212> DNA
<213> Homo sapiens

<400> 329

agagactccc agctctgaca ccaattagct gtgtgatctt gggcaagtga cctagcctcg 60

cggagcctgg ctacatcatc tgaagagctg ggacagtact agtgcccacc tcacagggct 120

gt 122

<210> 330
<211> 122
<212> DNA
<213> Homo sapiens

<400> 330

gggccatgag tggccctacc atggctcttc cccagcatct cagggagtat ctacctcgtg 60

cgaggaccag gcttggacac caggtcccga ttccattgtc atcttggtgg aatcactttg 120

ct 122

<210> 331
<211> 122
<212> DNA
<213> Homo sapiens

<400> 331

cgcgctgggc ttgcagccca gctttcagat tgctcctgtg ccggagccct gcgaatcatg 60

cgaatcatga aactgaagac ctggccctga agtcccagtg catatgagga gatccgttgt 120

ct 122

<210> 332
<211> 122
<212> DNA
<213> Homo sapiens

<400> 332

tttttcttgt gctgtctttg tactctttcc tgtgaattgc tttttccctt taacttccat 60

cgtagcaact ctggaaaacc aaaaccaaaa ccaaaaacaa tcactgcagt tctcttcatc 120

aa 122

<210> 333
<211> 122
<212> DNA
<213> Homo sapiens

<400> 333

agcattgctg gttctattta atggacatga gataatgtta gaggttttaa agtgattaaa 60

cgtgcagact atgcaaacca ggcccagtct ccagtgtggt accgttgctc ctgcatcgca 120

gc 122

<210> 334
<211> 122
<212> DNA
<213> Homo sapiens

<400> 334

ggaggaactg gctatcctaa aggtgatttt aaaccggggt agctagagcc caaagaaggg 60

cgaaaccagg actaactgcc ccatagcatg aggggcagcg cctgtaaaat tacataggat 120

tt 122

<210> 335
<211> 122
<212> DNA
<213> Homo sapiens

<400> 335

ctggcccacc cgtgagtcac ggacagaaca tgcagactca ggccttggtg acataagctc 60

cgcattgcta aaaccgcgtg acctcgaggg ctgactggcc tgagaaccct ggatggcgct 120

ct 122

<210> 336
<211> 122
<212> DNA
<213> Homo sapiens

<400> 336

gccatcttgt ggaatgttcc ggaatgccgt taggtgtcga agtgggcagc ggttgacaac 60

cgtgggcctt tgacagttac tagtactaaa catcgatgcc gattgtgagt ttccaatcag 120

ag 122

<210> 337
<211> 122
<212> DNA
<213> Homo sapiens

<400> 337

cgtggtccct gcagggtgtg tgggctgctc ggccttggcc agcatcaggg acagctctgg 60

cgcccggtca ctctgccccc tacccgcggc ctgctgcggg ccagcagggt gacagctaat 120

gt 122

<210> 338
<211> 122
<212> DNA
<213> Homo sapiens

<400> 338

tctacctgtc tcatttgagt tgagtgtgaa ttgtttagga tattgcaatt agaggtggtg 60

cgggctggct ggttgctata agccatctta acatttggct aagctcactc ctgtgtgctg 120

gg 122

<210> 339
<211> 122
<212> DNA
<213> Homo sapiens

<400> 339

gatggaatga atgatggaat gattgaaggc tgagggagta ttacaaaatt agtaggtcag 60

cgcctcgtgt ctaaagggct cacatgcagc atgaatgcag gaagcttctg gacattcctt 120

tt 122

<210> 340
<211> 122
<212> DNA
<213> Homo sapiens

<400> 340

cgagctgcct ggttagtgag cacctcctct tctctgggaa cctctagaac tgggaggaca 60

cgccccccgaa agggtgtccc tgagccaacg tgggaccgcg agtgccagcc cgttagcgtc 120

gg 122

<210> 341
<211> 122
<212> DNA
<213> Homo sapiens

<400> 341

acttgattct ggttgggggc tttgcctagg ggagccttcc ctgactcctc aggctggccg 60

cgtgggctaa cacacgtagg cacagcattg agcacactgt ttactcttgg tccgttcaca 120

gg 122

<210> 342
<211> 122
<212> DNA
<213> Homo sapiens

<400> 342

aatgagttgt ttcatatttt gcactgtctt ttcatgatca tttgcatcca ttagagaccc 60

cgcatcctat tggcttcttc gtactcctcc cggacagaac gcagagcgag ggtgagagcg 120

ag 122

<210> 343
<211> 122
<212> DNA
<213> Homo sapiens

<400> 343

aactcctgcc tccctctccc cccggccgag gtctgggaga tgagaaggga gcgcgttccc 60

cgggaaggga gcccccccgcg agccccagcc ggctacagat ctgggaggga gccgctcccg 120

tc 122

<210> 344
<211> 122
<212> DNA
<213> Homo sapiens

<400> 344

aagcgcccac atgcgcccgt ctccaccaaa actgagaaag ccgccggtca cctacgcccg 60

cgtttcccgt gcaccaccta gccgctccgc atggcggatc cagccaatca gcgcgccgtg 120

ca 122

<210> 345
<211> 122
<212> DNA
<213> Homo sapiens

<400> 345

taaataaata aggcttttg tttgtttgcc ggctcctgca catggctgct gggactcaag 60

cgctcgtgtt gtctgcgcct ctgtgggact ctggggacgg gaggcagggg aggcccccgc 120

ag 122

<210> 346
<211> 122
<212> DNA
<213> Homo sapiens

<400> 346

ccgggtaaag gggatgaata gcagactgcc ccggggcagt taggaattcg actggacagc 60

cgcgtgggag ggagtgcggg gagaggcaga gttgttttgt tattgttgtt ttattttgtt 120

tt 122

<210> 347
<211> 122
<212> DNA
<213> Homo sapiens

<400> 347

cttgggcaac gtaggagacc tccgtctcca caagtaaaat taattagccg gctgtggtgg 60

cgcgcacctg tggtcccagc tactcaggag gctgaggtag gaggatcacc tgagcccggg 120

ag 122

<210> 348
<211> 122
<212> DNA
<213> Homo sapiens

<400> 348

agcctgcagg tgggtttgtt agggggagac cgctctgcca atactggctt tcccatcgcc 60

cggccatctg caactgccag acgcaaagtg aggctcgtcc accgagcccc acttcccaga 120

gc 122

<210> 349
<211> 122
<212> DNA
<213> Homo sapiens

<400> 349

ggactggtac aggacaggca tctttgaacc tatttctggg agttctgaaa ctactgttct 60

cgtgggcctt ggcgactgat ttgggaaagc tgaccctggg ttggcctggc ttccagccac 120

cg 122

<210> 350
<211> 122
<212> DNA
<213> Homo sapiens

<400> 350

tgtttttgtg ggaggccttc tgcatggtcc cgggaggtca ggcagcccgg gagggcctcc 60

cggagcagag gctggagtca gtcccaatgc caacagtttc gaaccttgcc cgcgggcact 120

gc 122

<210> 351
<211> 122
<212> DNA
<213> Homo sapiens

<400> 351

tctctccctg gccaggagac ggtggccaag ggacttgact ttgaactacc aacaagctca 60

cgtttggcag ctgcaaagac aaaggctaga cttttagcag gtttttgggg gagcctgggg 120

ca 122

<210> 352
<211> 122
<212> DNA
<213> Homo sapiens

<400> 352

cgggcaaggt ctgaagactg cgaggaccca gctgccaggc gcattgtgaa gtggcccgag 60

cgtcacaggc gacccggacc tcgggaccgg ggggcagggc gggtgtctgc agcgtcctcg 120

gg 122

<210> 353
<211> 122
<212> DNA
<213> Homo sapiens

<400> 353

gaaccctcga ctgggggcag ccgcaccagt ggacacggcg gggtaggatt aaagttgagg 60

cgtgctcaca gacacttgtc tggtgtgagc ccttggcata tagatggctg cgagtgaagt 120

gg 122

<210> 354
<211> 122
<212> DNA
<213> Homo sapiens

<400> 354

ggtgcgttgt tcgcgggggt gaattgtgaa gaaccatcgc ggggtccttc ctgctgaggc 60

cgcggacacc gtgacctcgc tgctctgggt ctgcagggaa acgtaggaaa aaaagttgtc 120

ag 122

<210> 355
<211> 1201
<212> DNA
<213> Homo sapiens

<400> 355

```
ttgtatggga actctggtga atgcgaatca tttttaaatt actttttttg taaagtgcaa      60
aacaacaata gcacccattt gcgtcatact ttatagttcg caaagcacat gggaaaaata     120
aaggtaatga tggggatcgt tgcaattcat aggaaaggag gcacgaggaa atgaaaatga     180
aagggagtaa taactacgta actagtcaat cttccttaaa aaaaaaaacc cttaaaatat     240
accaccatct tctatttgat ataatgcaga atgggaatga taaaaacatg aattacattt     300
cagagtttca aaaagcaaac cagctttata gcaatgcttg aggttgggct gctaacaagc     360
tcactcaact agtgtttcct gacggccaac gtcagaataa ttccatctcc atgagaagta     420
cagaaagaac cacaaaccaa acctccaaat tgattctaag ataaaatacc cttaaaaaaa     480
atttcccttc ctatccgggc ggcagaccaa gaggaagttt atcctcccac ctacaaattc     540
cccagagagc tttcatctag aaggtttgac tctggccaga caaccagcga gcatcttctc     600
gcaatctgtt gcttcttcca tggcaaactc cagagaatta agaagccaaa ctcaacatcg     660
ccatgggcct caggacgact aaacagatgg ggagaggcac tggcagacca agaggaagtt     720
tatcctccca cctacaaatt ccccagagag ctttcatcta gaaggtttga ctctggccag     780
acaaccagcg agcatcttct cgcaatctgt tgcttcttcc atggcaaact ccagagaatt     840
aagaagccaa actcaacatc gccatgggcc tcaggacgac taaacagatg gggagaggca     900
ctaaagctcc tggtcaccaa gagggtatgt aggcatttgc tgtcttcctg gatttctcag     960
agctgagttt ttagccagag gttgcttatt tacgataatt cttggatata ttatacacta    1020
aatactatta ttatcttttt cgacccgact tttatctttc tgttcttatg tgtgaaggca    1080
gagaaagatt atttagagct cttcaaagat tcctatttaa tttaaaatgc ctgtcgcctt    1140
cctataatag gcttatgatg gatgatagct ttagttaaaa tgtagcaatc ttaaatatat    1200
t                                                                    1201
```

<210> 356
<211> 4164
<212> DNA
<213> Homo sapiens

<400> 356

```
atttaaacgg gagacggcgc gatgcctggc actcggtgcg ccttccgcgg accgggcgac      60
ccagtgcacg gccgccgcgt cactctcggt cccgctgacc ccgcgccgag ccccggcggc     120
tctggccgcg gccgcactca gcgccacgcg tcgaaagcgc aggccccgag gacccgccgc     180
actgacagta tgagccgcac agcctacacg gtgggagccc tgcttctcct cttggggacc     240
ctgctgccgg ctgctgaagg gaaaaagaaa gggtcccaag gtgccatccc cccgccagac     300
aaggcccagc acaatgactc agagcagact cagtcgcccc agcagcctgg ctccaggaac     360
cgggggcggg gccaagggcg gggcactgcc atgcccgggg aggaggtgct ggagtccagc     420
caagaggccc tgcatgtgac ggagcgcaaa tacctgaagc gagactggtg caaaacccag     480
ccgcttaagc agaccatcca cgaggaaggc tgcaacagtc gcaccatcat caaccgcttc     540
tgttacggcc agtgcaactc tttctacatc cccaggcaca tccggaagga ggaaggttcc     600
tttcagtcct gctccttctg caagcccaag aaattcacta ccatgatggt cacactcaac     660
tgccctgaac tacagccacc taccaagaag aagagagtca cacgtgtgaa gcagtgtcgt     720
tgcatatcca tcgatttgga ttaagccaaa tccaggtgca cccagcatgt cctaggaatg     780
cagccccagg aagtcccaga cctaaaacaa ccagattctt acttggctta aacctagagg     840
ccagaagaac ccccagctgc ctcctggcag gagcctgctt gtgcgtagtt cgtgtgcatg     900
agtgtggatg ggtgcctgtg ggtgttttta gacaccagag aaaacacagt ctctgctaga     960
gagcactccc tattttgtaa acatatctgc tttaatgggg atgtaccaga aacccacctc    1020
accccggctc acatctaaag ggcggggccc gtggtctggt tctgactttg tgtttttgtg    1080
ccctcctggg gaccagaatc tcctttcgga atgaatgttc atggaagagg ctcctctgag    1140
ggcaagagac ctgttttagt gctgcattcg acatggaaaa gtccttttaa cctgtgcttg    1200
catcctcctt tcctcctcct cctcacaatc catctcttct taagttgata gtgactatgt    1260
cagtctaatc tcttgtttgc caaggttcct aaattaattc acttaaccat gatgcaaatg    1320
tttttcattt tgtgaagacc ctccagactc tgggagaggc tggtgtgggc aaggacaagc    1380
aggatagtgg agtgagaaag ggagggtgga gggtgaggcc aaatcaggtc cagcaaaagt    1440
cagtagggac attgcagaag cttgaaaggc caataccaga acacaggctg atgcttctga    1500
```

gaaagtcttt tcctagtatt taacagaacc caagtgaaca gaggagaaat gagattgcca 1560
gaaagtgatt aactttggcc gttgcaatct gctcaaacct aacaccaaac tgaaaacata 1620
aatactgacc actcctatgt tcggacccaa gcaagttagc taaaccaaac caactcctct 1680
gctttgtccc tcaggtggaa aagagaggta gtttagaact ctctgcatag gggtgggaat 1740
taatcaaaaa ccgcagaggc tgaaattcct aatacctttc ctttatcgtg gttatagtca 1800
gctcatttcc attccactat ttcccataat gcttctgaga gccactaact tgattgataa 1860
agatcctgcc tctgctgagt gtacctgaca gtagtctaag atgagagagt ttagggacta 1920
ctctgtttta gcaagagata ttttggggt ctttttgttt taactattgt caggagattg 1980
ggctaaagag aagacgacga gagtaaggaa ataaagggaa ttgcctctgg ctagagagta 2040
gttaggtgtt aatacctggt agagatgtaa gggatatgac ctccctttct ttatgtgctc 2100
actgaggatc tgaggggacc ctgttaggag agcatagcat catgatgtat tagctgttca 2160
tctgctactg gttggatgga cataactatt gtaactattc agtatttact ggtaggcact 2220
gtcctctgat taaacttggc ctactggcaa tggctactta ggattgatct aagggccaaa 2280
gtgcagggtg ggtgaacttt attgtacttt ggatttggtt aacctgtttt cttcaagcct 2340
gaggttttat atacaaactc cctgaatact ctttttgcct tgtatcttct cagcctccta 2400
gccaagtcct atgtaatatg gaaaacaaac actgcagact tgagattcag ttgccgatca 2460
aggctctggc attcagagaa cccttgcaac tcgagaagct gtttttattt cgtttttgtt 2520
ttgatccagt gctctcccat ctaacaacta aacaggagcc atttcaaggc gggagatatt 2580
ttaaacaccc aaaatgttgg gtctgatttt caaactttta aactcactac tgatgattct 2640
cacgctaggc gaatttgtcc aaacacatag tgtgtgtgtt ttgtatacac tgtatgaccc 2700
caccccaaat ctttgtattg tccacattct ccaacaataa agcacagagt ggatttaatt 2760
aagcacacaa atgctaaggc agaattttga gggtgggaga gaagaaaagg gaaagaagct 2820
gaaaatgtaa aaccacacca gggaggaaaa atgacattca gaaccagcaa acactgaatt 2880
tctcttgttg ttttaactct gccacaagaa tgcaatttcg ttaacggaga tgacttaagt 2940
tggcagcagt aatcttcttt taggagcttg taccacagtc ttgcacataa gtgcagattt 3000
ggctcaagta aagagaattt cctcaacact aacttcactg ggataatcag cagcgtaact 3060
accctaaaag catatcacta gccaaagagg gaaatatctg ttcttcttac tgtgcctata 3120
ttaagactag tacaaatgtg gtgtgtcttc caactttcat tgaaaatgcc atatctatac 3180
catattttat tcgagtcact gatgatgtaa tgatatattt tttcattatt atagtagaat 3240
atttttatgg caagatattt gtggtcttga tcatacctat taaaataatg ccaaacacca 3300
aatatgaatt ttatgatgta cactttgtgc ttggcattaa aagaaaaaaa cacacatcct 3360

ggaagtctgt aagttgtttt ttgttactgt aggtcttcaa agttaagagt gtaagtgaaa 3420
aatctggagg agaggataat ttccactgtg tggaatgtga atagttaaat gaaaagttat 3480
ggttatttaa tgtaattatt acttcaaatc ctttggtcac tgtgatttca agcatgtttt 3540
ctttttctcc tttatatgac tttctctgag ttgggcaaag aagaagctga cacaccgtat 3600
gttgttagag tcttttatct ggtcagggga aacaaaatct tgacccagct gaacatgtct 3660
tcctgagtca gtgcctgaat ctttattttt taaattgaat gttccttaaa ggttaacatt 3720
tctaaagcaa tattaagaaa gactttaaat gttattttgg aagacttacg atgcatgtat 3780
acaaacgaat agcagataat gatgactagt tcacacataa agtcctttta aggagaaaat 3840
ctaaaatgaa aagtggataa acagaacatt tataagtgat cagttaatgc ctaagagtga 3900
aagtagttct attgacattc ctcaagatat ttaatatcaa ctgcattatg tattatgtct 3960
gcttaaatca tttaaaaacg gcaaagaatt atatagacta tgaggtacct tgctgtgtag 4020
gaggatgaaa ggggagttga tagtctcata aaactaattt ggcttcaagt ttcatgaatc 4080
tgtaactaga atttaatttt caccccaata atgttctata tagcctttgc taaagagcaa 4140
ctaataaatt aaacctattc tttc 4164

<210> 357
<211> 2134
<212> DNA
<213> Homo sapiens

<400> 357

gcacaggacg cgccatggcg gccgaagcct cggagagcgg gccagcgctg catgagctca 60
tgcgcgaggc ggagatcagc ctgctcgagt gcaaggtgtg ctttgagaag tttggccacc 120
ggcagcagcg gcgcccgcgc aacctgtcct gcggccacgt ggtctgcctg gcctgcgtgg 180
ccgccctggc gcacccgcgc actctggccc tcgagtgccc attctgcagg cgagcttgcc 240
ggggctgcga caccagcgac tgcctgccgg tgctgcacct catagagctc ctgggctcag 300
cgcttcgcca gtccccggcc gcccatcgcg ccgcccccag cgcccccgga gccctcacct 360
gccaccacac cttcggcggc tgggggaccc tggtcaaccc caccggactg gcgctttgtc 420
ccaagacggg gcgtgtcgtg gtggtgcacg acggcaggag gcgtgtcaag atttttgact 480
caggggggagg atgcgcgcat cagtttggag agaaggggga cgctgcccaa gacattaggt 540
accctgtgga tgtcaccatc accaacgact gccatgtggt tgtcactgac gccggcgatc 600
gctccatcaa agtgtttgat ttttttggcc agatcaagct tgtcattgga ggccaattct 660
ccttaccttg ggtgtggag accacccctc agaatgggat tgtggtaact gatgcggagg 720
cagggtccct gcacctcctg gacgtcgact tcgcggaagg ggtccttcgg agaactgaaa 780
ggttgcaagc tcatctgtgc aatccccgag ggtggcagt gtcttggctc accggggcca 840

```
ttgcggtcct ggagcacccc ctggccctgg ggactggggt ttgcagcacc agggtgaaag      900
tgtttagctc aagtatgcag cttgtcggcc aagtggatac ctttgggctg agcctctact      960
ttccctccaa aataactgcc tccgctgtga cctttgatca ccagggaaat gtgattgttg     1020
cagatacatc tggtccagct atcctttgct taggaaaacc tgaggagttt ccagtaccga     1080
agcccatggt cactcatggt ctttcgcatc ctgtggctct taccttcacc aaggagaatt     1140
ctcttcttgt gctggacaca gcatctcatt ctataaaagt ctataaagtt gactgggggt     1200
gatgggctgg ggtgggtccc tggaatcaga agcactagtg ctgccattaa tgaattgttt     1260
aaccctggat aagtcactta aactcatcta tccaggcagg gataattaaa accatctggc     1320
agacttacaa agcttgggac agttattgga gattaatcta ccatttattg aatgcatact     1380
ctgtgcaagg aaatttgcaa atattagctt atttaatctg tactatccag tgaggtaatt     1440
tcttcccccc caagatagag tcaagctctg tcacccaggc tggagtgcag aagcatgatc     1500
acagctcact acagtttcaa cgtcccccgc tcaggtggtc cttccacctc agcctcccaa     1560
gtagctggga ccacaagtgt gcattaccac actcagctaa tttttgtatt ttggcagaga     1620
tggggtttca ccatgttgcc caggctggtc tcaaactcct gagttcaagc aatccacctt     1680
cctcggcctc ccaaagtact aggagtacag gcatagccac ttgctcagcc ataattttta     1740
ttattaatct cattgtacaa gtgagaaaac tgagacccag agagcttaag tgacttcctc     1800
gaggtcatag ttacttactg ccttagtccc aatttgaatt caattctgat tccaaataag     1860
ttgcgcttaa ataagacaac agatgtggga aaaatatgtg aatgtgtagt gttgctatgt     1920
gtactgtctt tacaagtagc taattatttt agcacaaaga tgtgcaaaga aaggagactt     1980
tatggagagt tcaggagaaa aaggattttg tggtggccat cactttcatt caatttgcga     2040
ctgctctgat ggcacattag atgaagttac tgttgatcct gagttacgtg aataagaaaa     2100
acaattgaac tgcttattaa aaaagtaaac atgt                                 2134
```

<210> 358
<211> 1492
<212> DNA
<213> Homo sapiens

<400> 358

```
cagccgctgg ttttgctgag ggctgaggga cggctcagcg acgccacggc cagcagcgct      60
cgcgtcctcc ccagcaacag ttactcaaag ctaatcagat agcgaaagaa gcaggagagc     120
aagtcaagaa atacggtgaa ggagtccttc ccaaagttgt ctaggtcctt ccgcgccggt     180
gcctggtctt cgtcgtcaac accatggaca gctcccggga accgactctg gggcgcttgg     240
acgccgctgg cttctggcag gtctggcagc gctttgatgc ggatgaaaaa ggttacatag     300
aagagaagga actcgatgct ttctttctcc acatgttgat gaaactgggt actgatgaca     360

cggtcatgaa agcaaatttg cacaaggtga aacagcagtt tatgactacc caagatgcct     420
ctaaagatgg tcgcattcgg atgaaagagc ttgctggtat gttcttatct gaggatgaaa     480
actttcttct gctctttcgc cgggaaaacc cactggacag cagcgtggag tttatgcaga     540
tttggcgcaa atatgacgct gacagcagtg gctttatatc agctgctgag ctccgcaact     600
tcctccgaga cctctttctt caccacaaaa aggccatttc tgaggctaaa ctggaagaat     660
acactggcac catgatgaag atttttgaca gaaataaaga tggtcggttg gatctaaatg     720
acttagcaag gattctggct cttcaggaaa acttccttct ccaatttaaa atggatgctt     780
gttctactga agaaaggaaa agggactttg agaaaatctt tgcctactat gatgttagta     840
aaacaggagc cctggaaggc ccagaagtgg atgggtttgt caaagacatg atggagcttg     900
tccagcccag catcagcggg gtggaccttg ataagttccg cgagattctc ctgcgtcact     960
gcgacgtgaa caaggatgga aaaattcaga agtctgagct ggctttgtgt cttgggctga    1020
aaatcaaccc ataatcccag actgctttgc cttttgctct tactatgttt ctgtgatctt    1080
gctggtagaa ttgtatctgt gcattgatgt tgggaacaca gtgggcaaac tcacaaatgg    1140
tgtgctattc ttgggcaaga acagggacgc tagggccttc cttccaccgg cgtgatctat    1200
ccctgtctca ctgaaagccc ctgtgtagtg tctgtgttgt tttcccttga ccctgggctt    1260
tcctatcctc ccaaagactc agctcccctg ttagatggct ctgcctgtcc ttccccagtc    1320
accagggtgg gggggacagg ggcagctgag tgcattcatt ttgtgctttt cttgtgggct    1380
ttctgcttag tctgaaaggt gtgtggcatt catggcaatc ctgtaacttc aacatagatt    1440
tttttgtgtg tgtggaaata aatctgcaat tggaaacaaa aaaaaaaaaa aa             1492
```

## Description

BACKGROUND OF THE INVENTION

(Note: This application references a number of different publications as indicated throughout the specification by reference numbers enclosed in brackets, e.g., [x]. A list of these different publications ordered according to these reference numbers can be found below in the section entitled "REFERENCES".)

From the moment of conception, we begin to age. A decay of cellular structures, gene regulation, and DNA sequence ages cells and organisms. An increasing body of evidence suggests that many manifestations of aging are epigenetic [1, 2]. DNA methylation patterns have been found to change with increasing age and contribute to age-related diseases. Methylation in promoter regions is generally accompanied by gene silencing and loss of methylation or loss of the proteins that bind to certain methylated cytosine DNA nucleotides. This can lead to diseases in humans, for example, Immunodeficiency Craniofacial Syndrome and Rett Syndrome (see, e.g. Bestor (2000) Hum. Mol. Genet. 9:2395-2402). DNA methylation may be gene-specific or occur genome-wide.

One particular type of epigenetic control is the cytosine-5 methylation within Cytosine-phosphate-Guanine (CpG) dinucleotides (also known as DNA methylation or "DNAm"). Age-related DNA hypomethylation has long been observed in a variety of species including salmon [3], rats [4], and mice [5]. More recent studies have shown that many CpGs are subject to age-related hypermethylation or hypomethylation [6-14]. Previous studies have shown that age-related hypermethylation occurs preferentially at CpG islands [8], at bivalent chromatin domain promoters that are associated with key developmental genes [15], and at Polycomb-group protein targets [10]. The epigenomic landscape varies markedly across tissue types [16-18] and many age-related changes depend on tissue type [8, 19]. Some studies have suggested that age-dependent CpG signatures may be defined independently of sex, tissue type, disease state, and array platform [10, 13-15, 20-22].

While there are articles that describe age predictors based on DNA methylation (DNAm) levels in specific tissues (e.g. saliva or blood [23, 24]), it is not yet known whether age can be predicted irrespective of tissue type using a single predictor. Articles that describe age-related changes in various tissues (e.g. blood, saliva, and brain [13, 21, 23, 24, 90, 91]) typically only focus on the biological impact of aging. For example, various DNA CpG methylation markers have been included in a list of aging-related genes by Teschendorff et al. [10], who showed that these markers correlated with age. However, Teschendorff et al. [10] did not investigate brain tissue and saliva and further did not build (multivariate) predictors of age. There have also been publications describing age predictors based on DNA methylation levels (see, e.g. Bockland et al. [23], Koch et al. [21], Hannum et al. [24]). Notably, however, Hannum et al. [24] found that computing a DNA methylation-based age predictor for different tissues gave basically no overlap, e.g. blood-derived predictive CpGs were different from those from other tissues.

Thus, there is a need for an age predictor based on DNA methylation levels that can accurately predict age across a broad spectrum of human tissues/cell types.

SUMMARY OF THE INVENTION

In one aspect of the present invention, a method is provided for estimating the chronological and/or biological age of an individual’s tissue or cell sample by measuring the methylation of specific DNA Cytosine-phosphate-Guanine (CpG) methylation markers attached to the individual’s DNA. Specifically, the present invention provides a method for determining an age of a biological sample, the method comprising: selectively measuring the methylation levels of a set of methylation markers in genomic DNA of the biological sample, said set of methylation markers comprising the 6 CpG methylation markers in Table 7; and comparing the measured methylation marker levels to reference levels for the methylation markers to determine the age of the sample based on said methylation levels. Optionally, the measured methylation levels are transformed. In one or more embodiments, the method comprises forming a linear combination of a predetermined set of CpG methylation markers (or optionally, forming a linear combination of the transformed methylation levels), which is then transformed to an age estimate using a calibration function. The linear combination of the CpGs, referred to as "clock CpGs" (or of the transformed methylation levels), can be interpreted as an epigenetic clock. The resulting predicted age is referred to as the "DNA methylation (DNAm) age". In one embodiment, the age is estimated based on a set of 354 CpG methylation markers (see Table 3 below). In other embodiments, the age is estimated based on a set of 110, 38, 17 or 6 CpG methylation markers (see Tables 4, 5, 6, and 7, respectively). The sets of 110, 38, 17, and 6 CpGs are subsets of methylation markers taken from the set of 354 CpG methylation markers shown in Table 3.

A multi-tissue age predictor is also provided that uses a set of CpG methylation markers for estimating age. An advantage of the multi-tissue age predictor lies in its wide applicability: for most tissues it does not require any adjustments or offsets. The invention allows for the comparison of the ages of different parts of the human body. Furthermore, the multi-tissue age predictor and CpG methylation markers allow for easily accessible tissues (e.g. blood, saliva, buccal cells, epidermis) to be used to measure age in inaccessible tissues (e.g. brain, kidney, liver). For example, the methods disclosed herein can be used to estimate the age of inaccessible human brain tissue by measuring the age of more accessible tissues such as blood, saliva, skin or adipose tissue. In further aspects, the sample comprises tissue culture cells or pluripotent stem cells (e.g. induced pluripotent stem (iPS) cells). Thus, in some aspects, a method of the embodiments can be used to determine the passage number or amount of time in culture for a population of tissue culture cells. In additional aspects, a method of the disclosure can be used to assess the differentiation status (or the pluripotency) of a population of cells comprising pluripotent stem cells (e.g. iPS cells).

In one or more embodiments, a method is provided comprising a first step of extracting genomic DNA from a sample. In a second step, the DNAm levels at multiple loci in the genome are measured. In specific instances, this results in thousands of quantitative measurements per sample. Each measurement measures the extent of methylation at a particular genomic location (CpG). The more CpGs measured allows for normalization of the data, though in certain embodiments, the DNAm levels of only 354, 110, 38, 17 or 6 CpG methylation markers are measured (see, Table 3-7 respectively). A third step comprises calculating the (weighted) average of the (optionally, transformed) DNAm levels across the measured CpGs. In certain instances, the result is a real number that lies between -4 and 4. The DNAm level of each CpG is multiplied by a coefficient value (of a regression model) and the individual products are summed up. In a fourth step, the weighted average is transformed to a new scale, such as a number that measures DNAm age in years. In this instance, age zero corresponds to age at birth and a prenatal sample results in a negative age. A monotonic, non-linear transformation is used.

The method may further comprise an additional step after the second step, wherein the measurements are normalized/transformed such that the two peaks of their frequency distribution are located at the same two locations as that of a gold standard measurement. The result is the same as that of the second step but the values are slightly changed. The peaks of the frequency distribution correspond to values for completely methylated or un-methylated CpGs, respectively. This normalization step is possible because most CpGs are either perfectly methylated or un-methylated. In one exemplary implementation, the gold standard is based on the average DNAm value across 715 blood samples.

The present invention can be used to study the effects of medication, food compounds and/or special diets on the biological age of humans or chimpanzees (which may serve as model organisms since DNAm age is also applicable to chimpanzee tissues). Since DNA methylation patterns change with increasing age and contribute to age-related diseases, the CpGs can be used as biomarkers of chronological age (e.g. for forensic applications). The invention can also be used for determining and/or increasing an individual's likelihood of longevity, in particular, by determining and decreasing an individual's likelihood of developing an age-related disease (e.g. cancer). This is accomplished, for example, by diagnosing and determining the existence or likelihood of disease (e.g. cancer) or providing an assay for identifying a compound which counters the age-related increase or decrease of methylation in the CpG markers disclosed herein.

In a further aspect of the disclosure there is provided a method for determining age of a biological sample comprising selectively measuring the methylation levels of a set of methylation markers in genomic DNA of the biological sample, said set of methylation markers comprising markers in least 6 of the genes listed in Table 3 (SEQ ID NO: 1-354) and determining the age of the sample based on said methylation levels. In some aspects, the set of methylation markers may comprise markers in at least or at most 6, 7, 8, 9, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, or 354 of the genes listed in Table 3. In further aspects, the set of methylation markers may comprise markers in at least or at most 6, 7, 8, 9, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, or 354 of the CpG positions listed in Table 3.

In a further aspect, a method of the disclosure comprises selectively measuring the methylation levels of a set of methylation markers in genomic DNA of the biological sample, said set of methylation markers comprising markers in least 6 of the genes listed in Table 4 and determining the age of the sample based on said methylation levels. In some aspects, the set of methylation markers may comprise markers in at least or at most 6, 7, 8, 9, 10, 15, 20, 25, 30, 35, 40, 45, 50, 55, 60, 65, 70, 75, 80, 85, 90, 95, 100, 105 or 110 of the genes listed in Table 4. In further aspects, the set of methylation markers may comprise markers in at least or at most 6, 7, 8, 9, 10, 15, 20, 25, 30, 35, 40, 45, 50, 55, 60, 65, 70, 75, 80, 85, 90, 95, 100, 105 or 110 of the CpG positions listed in Table 4.

In yet a further aspect, a method of the disclosure comprises selectively measuring the methylation levels of a set of methylation markers in genomic DNA of the biological sample, said set of methylation markers comprising markers in least 3 of the genes listed in Table 5 and determining the age of the sample based on said methylation levels. In some aspects, the set of methylation markers may comprise markers in at least or at most 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37 or 38 of the genes listed in Table 5. In further aspects, the set of methylation markers may comprise markers in at least or at most 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37 or 38 of the CpG positions listed in Table 5.

In yet still a further aspect, a method of the disclosure comprises selectively measuring the methylation levels of a set of methylation markers in genomic DNA of the biological sample, said set of methylation markers comprising markers in least 3 of the genes listed in Table 6 and determining the age of the sample based on said methylation levels. In some aspects, the set of methylation markers may comprise markers in at least or at most 3, 4, 5, 6, 7, 8, 9, 10, 11, 12, 13, 14, 15, 16 or 17 of the genes listed in Table 6. In further aspects, the set of methylation markers may comprise markers in at least or at most 3, 4, 5, 6, 7, 8, 9, 10, 11, 12, 13, 14, 15, 16 or 17 of the CpG positions listed in Table 6.

In still a further aspect, a method of the disclosure comprises selectively measuring the methylation levels of a set of methylation markers in genomic DNA of the biological sample, said set of methylation markers comprising markers in least 2 of the genes listed in Table 7 and determining the age of the sample based on said methylation levels. In some aspects, the set of methylation markers may comprise markers in at least or at most 2, 3, 4, 5 or 6 of the genes listed in Table 7. In further aspects, the set of methylation markers may comprise markers in at least or at most 2, 3, 4, 5 or 6 of the CpG positions listed in Table 7.

In some aspects, the biological sample is a solid tissue, blood, urine, fecal or saliva sample that comprises genomic DNA. In particular aspects, the biological sample is a blood sample.

In further aspects, selectively measuring the methylation levels of a set of methylation markers in genomic DNA, further comprises transforming the measured methylation marker levels. In certain aspects of the invention determining the age of the biological sample comprises applying a statistical prediction algorithm to the measured methylation marker levels (or the transformed methylation marker levels). In certain aspects, applying a statistical prediction algorithm comprises (a) obtaining a linear combination of the methylation marker levels (or the transformed methylation marker levels), and (b) applying a transformation to the linear combination to determine the age of the biological sample. For example, obtaining a linear combination of the methylation marker levels can comprise obtaining weighted average of the methylation marker levels (or a weighted average of the transformed methylation marker levels). In further aspects, applying a transformation to the linear combination comprises applying a logarithmic and/or linear transformation to the linear combination.

In a further aspect determining the age of the biological sample comprises applying a linear regression model to predict sample age based on a weighted average of the methylation marker levels plus an offset.

In still further aspects, the set of methylation markers for use accordingly to the embodiments may comprise methylation markers in all of the gene or at all of the CpG positions of Table 3, Table 4, Table 5, Table 6 or Table 7. In certain aspects, the set of methylation markers may comprise markers in or near the NHLRC1 (SEQ ID NO: 357), GREM1 (SEQ ID NO: 356), SCGN (SEQ ID NO: 358) or EDARADD (SEQ ID NO: 355) genes. In one aspect of the disclosure, probes cg22736354 (SEQ ID NO: 158) near gene NHLRC1, cg21296230 near gene GREM1 (SEQ ID NO: 354), cg06493994 (SEQ ID NO: 46) near gene SCGN, and/or cg09809672 (SEQ ID NO: 252) near gene EDARADD are used.

In some aspects the age of an individual is determined based on the age of the biological sample. For example, the age of individual can be determined by determining the age of biological sample from a peripheral tissue sample (e.g., a blood or saliva sample) from the individual. A method may further comprise, for instance, reporting the age of the sample or of the individual, e.g., by preparing a written, oral or electronic report.

In another aspect of the disclosure there is provided a tangible computer-readable medium comprising computer-readable code that, when executed by a computer, causes the computer to perform operations comprising receiving information corresponding to methylation levels of a set of methylation markers in a biological sample, said markers comprising markers in at least 2 of the genes listed in Table 3, Table 4, Table 5, Table 6 or Table 7 and determining the age of the biological sample by applying a statistical prediction algorithm to the measured methylation marker levels. In some aspects, the set of methylation markers may comprise markers in at least, or at most, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, or 354 of the genes listed in Table 3, Table 4, Table 5, Table 6 or Table 7. In further aspects, the set of methylation markers may comprise markers at least, or at most, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, or 354 of the CpG positions listed in Table 3, Table 4, Table 5, Table 6 or Table 7.

The invention provides a tangible computer-readable medium comprising computer-readable code that, when executed by a computer, causes the computer to perform operations comprising: a) receiving information corresponding to methylation levels of a set of methylation markers in a biological sample, said markers comprising the 6 CpG methylation markers in Table 7; and b) determining the age of the biological sample by comparing the measured methylation marker levels to reference marker levels and by applying a statistical prediction algorithm to the measured methylation marker levels.

In the invention, determining the age of the biological sample further comprises comparing the measured methylation marker levels to reference marker levels. The reference levels may, optionally, be stored in said tangible computer-readable medium. In certain aspects, determining the age of the biological sample may comprise applying a linear regression model to predict sample age based on a weighted average of the methylation marker levels plus an offset.

In some aspects the receiving information may comprise receiving from a tangible data storage device information corresponding to the methylation levels of the set of methylation markers in the biological sample. In other aspects of the disclosure the receiving information may further comprise receiving information corresponding to methylation levels of a set of methylation markers in a biological sample, said markers comprising markers in at least, or at most, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, or 354 of the genes listed in Table 3, Table 4, Table 5, Table 6 or Table 7.

Further aspects of the tangible computer-readable medium may comprise computer-readable code that, when executed by a computer, causes the computer to perform one or more additional operations comprising: sending information corresponding to the methylation levels of the set of methylation markers in the biological sample to a tangible data storage device.

In certain aspects of the embodiments measuring methylation marker comprises, performing methylation specific PCR (MSP), real-time methylation specific PCR, methylation-sensitive single-strand conformation analysis (MS-SSCA), quantitative methylation specific PCR (QMSP), PCR using a methylated DNA-specific binding protein, high resolution melting analysis (HRM), methylation-sensitive single-nucleotide primer extension (MS-SnuPE), base-specific cleavage/MALDI-TOF, PCR, real-time PCR, Combined Bisulfite Restriction Analysis (COBRA), methylated DNA immunoprecipitation (MeDIP), a microarray-based method, pyrosequencing, or bisulfite sequencing. For example, measuring a methylation marker can comprise performing array-based PCR (e.g., digital PCR), targeted multiplex PCR, or direct sequencing without bisulfite treatment (e.g., via a nanopore technology). In some aspects, determining methylation status comprises methylation specific PCR, real-time methylation specific PCR, quantitative methylation specific PCR (QMSP), or bisulfite sequencing. In certain aspects, a method according to the embodiments comprises treating DNA in or from a sample with bisulfite (e.g., sodium bisulfite) to convert unmethylated cytosines of CpG dinucleotides to uracil.

## BRIEF DESCRIPTION OF THE DRAWINGS

Referring now to the drawings in which like reference numbers represent corresponding parts throughout:

**Figure 1:** Univariate predictor of age in blood tissue from multiple independent studies. The predictor of true (chronological) age is highly accurate: Median absolute deviation between predicted and true age is only 7.2 years. Correlation between true and predicted age is 0.76.

**Figure 2:** Univariate linear predictor of age in brain tissues (using samples from temporal cortex, frontal cortex, and PONS). The predictor of true (chronological) age is highly accurate: Median absolute deviation between predicted and true age is only 6.1 years. Correlation between true and predicted age is 0.88.

**Figure 3:** Univariate linear predictor of age by brain region (frontal cortex, temporal cortex, PONS and overall).

**Figure 4:** Multivariate predictor of age in whole blood tissue from multiple independent studies. The multivariate predictor of true (chronological) age is highly accurate: Median absolute deviation between predicted and true age is only 5.4 years. Correlation between true and predicted age is 0.90.

**Figure 5:** Multivariate predictor of age in brain tissues (using samples from temporal cortex, frontal cortex, and PONS). The multivariate predictor of true (chronological) age is highly accurate: Median absolute deviation between predicted and true age is only 5.9 years. Correlation between true and predicted age is 0.89.

**Figure 6:** Multivariate predictor of age by brain region (e.g. frontal cortex, temporal cortex, PONS and overall).

**Figure 7:** Multivariate predictor of age in saliva tissue. The multivariate predictor of true (chronological) age is highly accurate: Median absolute deviation between predicted and true age is only 4.9 years. Correlation between true and predicted age is 0.67.

**Figure 8:** Multivariate predictor of age in whole blood tissue from multiple independent studies. The multivariate predictor of true (chronological) age is highly accurate: Median absolute deviation between predicted and true age is only 5.1 years. Correlation between true and predicted age is 0.91.

**Figure 9:** Multivariate predictor of age in brain tissues (using samples from temporal cortex, frontal cortex, and PONS). The multivariate predictor of true (chronological) age is highly accurate: Median absolute deviation between predicted and true age is only 5.8 years. Correlation between true and predicted age is 0.90.

**Figure 10:** Multivariate predictor of age by brain region (frontal cortex, temporal cortex, PONS and overall).

**Figure 11:** Multivariate predictor of age in saliva tissue. The multivariate predictor of true (chronological) age is highly accurate: Median absolute deviation between predicted and true age is only 4.4 years. Correlation between true and predicted age is 0.71.

**Figure 12:** Multivariate predictor of age in brain tissues (using samples from temporal cortex, frontal cortex, and PONS). The multivariate predictor of true (chronological) age is highly accurate: Median absolute deviation between predicted and true age is only 8.2 years. Correlation between true and predicted age is 0.84.

**Figure 13:** Multivariate predictor of age by brain region (frontal cortex, temporal cortex, PONS and overall).

**Figure 14:** Multivariate predictor of age in saliva tissue. The multivariate predictor of true (chronological) age is highly accurate: Median absolute deviation between predicted and true age is only 4.2 years. Correlation between true and predicted age is 0.72.

**Figure 15:** Although the markers work particularly well in saliva and brain, they also work quite well in blood tissue. The multivariate predictor of true (chronological) age is highly accurate: Median absolute deviation between predicted and true age is only 6.1 years. Correlation between true and predicted age is 0.988.

**Figure 16:** Each column corresponds to different embodiments of the multi-tissue age predictor. The first and second rows show the results in the training data sets and test sets respectively. Each dot corresponds to a human subject and is colored and labeled according to the data set (Table 1 in Horvath 2013). Each panel reports the median error and correlation coefficient between predicted age and chronological age. The first column (panels A, F) shows how one embodiment of the multi-tissue age predictor (based on 354 CpGs, Table 3) performs in the training data (A) and test data (F). The second column (panels B,G) shows the performance of another embodiment of the multi-tissue age predictor based on a "shrunken" subset of 110 CpGs. Similarly, columns three, four, and five report the results of other embodiments of the multi-tissue age predictor based on 38, 17, and 6 CpGs, respectively. Even 6 CpGs (panel J) lead to a very high correlation 0.89 in the test data but the error rate (8.9 years) is substantially higher than that (3.6 years, panel F) observed for the predictor that uses 354 CpGs.

**Figure 17:** Chronological age (y-axis) versus DNAm age (x-axis) in the test data. (A) Across all test data, the age correlation is 0.96 and the error is 3.6 years. Results for (B) CD4 T cells measured at birth (age zero) and at age 1 (cor = 0.78, error = 0.27 years), (C) CD4 T cells and CD14 monocytes (cor = 0.90, error = 3.7), (D) peripheral blood mononuclear cells (cor = 0.96, error = 1.9), (E) whole blood (cor = 0.95, error = 3.7), (F) cerebellar samples (cor = 0.92, error = 5.9), (G) occipital cortex (cor = 0.98, error = 1.5), (H) normal adjacent breast tissue (cor = 0.87, error = 13), (I) buccal epithelium (cor = 0.83, error = 0.37), (J) colon (cor = 0.85, error = 5.6), (K) fat adipose (cor = 0.65, error = 2.7), (L) heart (cor = 0.77, error = 12), (M) kidney (cor = 0.86, error = 4.6), (N) liver (cor = 0.89, error = 6.7), (O) lung (cor = 0.87, error = 5.2), (P) muscle (cor = 0.70, error = 18), (Q) saliva (cor = 0.83, error = 2.7), (R) uterine cervix (cor = 0.75, error = 6.2), (S) uterine endometrium (cor = 0.55, 11), (T) various blood samples composed of 10 Epstein Barr Virus transformed B cell, three naive B cell, and three peripheral blood mononuclear cell samples (cor = 0.46, error = 4.4). Samples are colored by disease status: brown for Werner progeroid syndrome, blue for Hutchinson-Gilford progeria, and turquoise for healthy control subjects.

## DETAILED DESCRIPTION OF THE INVENTION

In the description of embodiments, reference may be made to the accompanying figures which form a part hereof, and in which is shown by way of illustration a specific embodiment in which the invention may be practiced. Publications cited herein are cited for their disclosure prior to the filing date of the present application. Nothing here is to be construed as an admission that the inventors are not entitled to antedate the publications by virtue of an earlier priority date or prior date of invention. Further, the actual publication dates may be different from those shown and require independent verification.

Many of the techniques and procedures described or referenced herein are well understood and commonly employed by those skilled in the art. Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art.

The term "epigenetic" as used herein means relating to, being, or involving a modification in gene expression that is independent of DNA sequence. Epigenetic factors include modifications in gene expression that are controlled by changes in DNA methylation and chromatin structure. For example, methylation patterns are known to correlate with gene expression.

The term "nucleic acids" as used herein may include any polymer or oligomer of pyrimidine and purine bases, preferably cytosine, thymine, and uracil, and adenine and guanine, respectively. The present invention contemplates any deoxyribonucleotide, ribonucleotide or peptide nucleic acid component, and any chemical variants thereof, such as methylated, hydroxymethylated or glucosylated forms of these bases, and the like. The polymers or oligomers may be heterogeneous or homogeneous in composition, and may be isolated from naturally-occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

The terms "oligonucleotide" and "polynucleotide" as used herein refers to a nucleic acid ranging from at least 2, preferable at least 8, and more preferably at least 20 nucleotides in length or a compound that specifically hybridizes to a polynucleotide. Polynucleotides of the present invention include sequences of deoxyribonucleic acid (DNA) or ribonucleic acid (RNA) which may be isolated from natural sources, recombinantly produced or artificially synthesized and mimetics thereof.

The term "methylation marker" as used herein refers to a CpG position that is potentially methylated. Methylation typically occurs in a CpG containing nucleic acid. The CpG containing nucleic acid may be present in, e.g., in a CpG island, a CpG doublet, a promoter, an intron, or an exon of gene. For instance, in the genetic regions provided herein the potential methylation sites encompass the promoter/enhancer regions of the indicated genes. Thus, the regions can begin upstream of a gene promoter and extend downstream into the transcribed region.

The term "genome" or "genomic" as used herein is all the genetic material in the chromosomes of an organism. DNA derived from the genetic material in the chromosomes of a particular organism is genomic DNA.

The term "gene" as used herein refers to a region of genomic DNA associated with a given gene. For example, the region can be defined by a particular gene (such as protein coding sequence exons, intervening introns and associated expression control sequences) and its flanking sequence. It is, however, recognized in the art that methylation in a particular region is generally indicative of the methylation status at proximal genomic sites. Accordingly, determining a methylation status of a gene region can comprise determining a methylation status of a methylation marker within or flanking about 10 bp to 50 bp, about 50 to 100 bp, about 100 bp to 200 bp, about 200 bp to 300 bp, about 300 to 400 bp, about 400 bp to 500 bp, about 500 bp to 600 bp, about 600 to 700 bp, about 700 bp to 800 bp, about 800 to 900 bp, 900 bp to 1kb, about 1 kb to 2 kb, about 2 kb to 5 kb, or more of a named gene, or CpG position.

The phrase "selectively measuring" as used herein refers to methods wherein only a finite number of methylation marker or genes (comprising methylation markers) are measured rather than assaying essentially all potential methylation marker (or genes) in a genome. For example, in some aspects, "selectively measuring" methylation markers or genes comprising such markers can refer to measuring no more than 1,000, 900, 800, 700, 600, 500, 400 or 354 different methylation markers or genes comprising methylation markers.

The term "probes" as used herein are oligonucleotides capable of binding in a base-specific manner to a complementary strand of nucleic acid. The term "probe" as used herein refers to a surface-immobilized molecule that can be recognized by a particular target as well as molecules that are not immobilized and are coupled to a detectable label.

The term "label" as used herein refers, for example, to colorimetric (e.g. luminescent) labels, light scattering labels or radioactive labels. Fluorescent labels include, inter alia, the commercially available fluorescein phosphoramidites such as Fluoreprime™ (Pharmacia™), Fluoredite™ (Millipore™) and FAM™ (ABI™) (see, e.g. U.S. Patent Nos. 6,287,778 and 6,582,908).

The term "primer" as used herein refers to a single-stranded oligonucleotide capable of acting as a point of initiation for template-directed DNA synthesis under suitable conditions for example, buffer and temperature, in the presence of four different nucleoside triphosphates and an agent for polymerization, such as, for example, DNA or RNA polymerase or reverse transcriptase. The length of the primer, in any given case, depends on, for example, the intended use of the primer, and generally ranges from 15 to 30 nucleotides. A primer need not reflect the exact sequence of the template but must be sufficiently complementary to hybridize with such template. The primer site is the area of the template to which a primer hybridizes. The primer pair is a set of primers including a 5' upstream primer that hybridizes with the 5' end of the sequence to be amplified and a 3' downstream primer that hybridizes with the complement of the 3' end of the sequence to be amplified.

The term "complementary" as used herein refers to the hybridization or base pairing between nucleotides or nucleic acids, such as, for instance, between the two strands of a double stranded DNA molecule or between an oligonucleotide primer and a primer binding site on a single stranded nucleic acid to be sequenced or amplified. Complementary nucleotides are, generally, A and T (or A and U), or C and G. Two single stranded RNA or DNA molecules are said to be complementary when the nucleotides of one strand, optimally aligned and compared and with appropriate nucleotide insertions or deletions, pair with at least about 80% of the nucleotides of the other strand, usually at least about 90% to 95%, and more preferably from about 98 to 100%. Alternatively, complementarity exists when an RNA or DNA strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary. See, M. Kanehisa, Nucleic Acids Res. 12:203 (1984).

The term "hybridization" as used herein refers to the process in which two single-stranded polynucleotides bind non-covalently to form a stable double-stranded polynucleotide; triple-stranded hybridization is also theoretically possible. Factors that can affect the stringency of hybridization, including base composition and length of the complementary strands, presence of organic solvents and extent of base mismatching, the combination of parameters is more important than the absolute measure of any one alone. Hybridization conditions suitable for microarrays are described in the Gene Expression Technical Manual, 2004 and the GeneChip Mapping Assay Manual, 2004, available at Affymetrix.com.

The term "array" or "microarray" as used herein refers to an intentionally created collection of molecules which can be prepared either synthetically or biosynthetically (e.g. Illumina™ HumanMethylation27 microarrays). The molecules in the array can be identical or different from each other. The array can assume a variety of formats, for example, libraries of soluble molecules; libraries of compounds tethered to resin beads, silica chips, or other solid supports.

The term "solid support", "support", and "substrate" as used herein are used interchangeably and refer to a material or group of materials having a rigid or semi-rigid surface or surfaces. In many embodiments, at least one surface of the solid support will be substantially flat, although in some embodiments it may be desirable to physically separate synthesis regions for different compounds with, for example, wells, raised regions, pins, etched trenches, or the like. According to other embodiments, the solid support(s) will take the form of beads, resins, gels, microspheres, or other geometric configurations. See U.S. Pat. No. 5,744,305 for exemplary substrates.

In the following description, embodiments utilizing a linear combination are discussed. Those of skill in the art understand that this aspect of the invention is not limited to linear combinations and is merely a typical example. For example, a product or ratio may be used instead. Such a product would be mathematically equivalent to forming a linear combination of log transformed methylation levels.

DESCRIPTION OF ILLUSTRATIVE ASPECTS

As disclosed herein, a number of locations have been identified in the human genome for which the percentage of DNA methylation is linearly correlated with age. By measuring the DNA methylation at just a few of the 3 billion nucleotides in an individual's genome, the present invention allows for accurate estimations of the individual's chronological age. While previous studies have shown that DNA methylation in certain parts of the genome changes with age, the present invention identifies loci where methylation is continuously correlated with age, over a range of at least 5 decades. This allows for a highly accurate prediction of an individual's age. In certain aspects of the disclosure, the link between age and this chemical change in the DNA is so strong that it is possible to estimate the age of an individual by examining, for example, just two spots in the genome of the individual (see Bockland et al., et al. (2011) PLoS ONE 6(6): e14821. doi:10.1371/journal.pone.0014821). In addition, certain aspects of this invention have been confirmed by other studies (see, e.g. Koch et al., (2011) AGING, Vol. 3, No 10, pp 1,018-1,027). A related publication is United States Application Publication No. 2014/0228231, filed by Eric Vilain et al. on August 14, 2014 and titled "Method to Estimate Age of Individual Based On Epigenetic Markers in Biological Sample,". A further publication is "DNA methylation age of human tissues and cell types" by Steve Horvath (Horvath (2013) Genome Biology 14:R115).

The present invention relates to methods for estimating the chronological and/or biological age of an individual human tissue or cell type sample based on measuring DNA Cytosine-phosphate-Guanine (CpG) methylation markers that are attached to our DNA. In a general embodiment of the invention, a method is disclosed comprising a first step of choosing a biological cell or tissue sample (e.g. whole blood, individual blood cells, saliva, brain). In a second step, genomic DNA is extracted from the collected tissue of the individual for whom an age prediction is desired. In a third step, the methylation levels of the methylation markers near the specific clock CpGs are measured. In a fourth step, a statistical prediction algorithm is applied to the methylation levels to predict the biological or chronological age. One basic approach is to form a weighted average of the clock CpGs, which is then transformed to DNAm age using a calibration function. A detailed description of the data pre-processing, data normalization, age prediction steps is provided in Example 8.

One embodiment focuses on forming a linear combination of 354 CpGs (Table 3, SEQ ID NO: 1-354), which is then transformed to an age estimate using a calibration function. The weighted average of the degree of cytosine methylation at these 354 locations is significantly correlated with age, including but not limited to, human brain tissue (frontal cortex, temporal cortex, PONS), blood tissue (whole blood, cord blood and blood cells), liver, adipose, skin, kidney, prostate, muscle, and saliva tissue. The linear combination of the 354 CpGs (which are referred to as clock CpGs) can be interpreted as an epigenetic clock. The resulting predicted age is referred to as DNA methylation (DNAm) age. In other embodiments, a linear combination of 110, 38, 15 or 6 CpGs are used (Tables 4-7 respectively), which are subsets of the 354 CpGs. In specific instances, these subsets or sub-clocks were determined by increasing the threshold of the penalty term in a penalized regression model. In further embodiments of the invention, these sequences can include either translated or untranslated 5' regulatory regions; and optionally are within 1 kilobase (5' or 3') of the specific GC loci that are identified herein.

In a further aspect of the disclosure there is provided a method for determining age of a biological sample comprising selectively measuring the methylation levels of a set of methylation markers in genomic DNA of the biological sample, said set of methylation markers comprising markers in least 6 of the genes listed in Table 3 and determining the age of the sample based on said methylation levels. In some aspects, the set of methylation markers may comprise markers in at least or at most 6, 7, 8, 9, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, or 354 of the genes listed in Table 3. In further aspects, the set of methylation markers may comprise markers in at least or at most 6, 7, 8, 9, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, or 354 of the CpG positions listed in Table 3.

In a further aspect, a method comprises selectively measuring the methylation levels of a set of methylation markers in genomic DNA of the biological sample, said set of methylation markers comprising markers in least 6 of the genes listed in Table 4 and determining the age of the sample based on said methylation levels. In some aspects, the set of methylation markers may comprise markers in at least or at most 6, 7, 8, 9, 10, 15, 20, 25, 30, 35, 40, 45, 50, 55, 60, 65, 70, 75, 80, 85, 90, 95, 100, 105 or 110 of the genes listed in Table 4. In further aspects, the set of methylation markers may comprise markers in at least or at most 6, 7, 8, 9, 10, 15, 20, 25, 30, 35, 40, 45, 50, 55, 60, 65, 70, 75, 80, 85, 90, 95, 100, 105 or 110 of the CpG positions listed in Table 4.

In yet a further aspect, a method of the disclosure comprises selectively measuring the methylation levels of a set of methylation markers in genomic DNA of the biological sample, said set of methylation markers comprising markers in least 3 of the genes listed in Table 5 and determining the age of the sample based on said methylation levels. In some aspects, the set of methylation markers may comprise markers in at least or at most 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37 or 38 of the genes listed in Table 5. In further aspects, the set of methylation markers may comprise markers in at least or at most 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37 or 38 of the CpG positions listed in Table 5.

In yet still a further aspect, a method of the disclosure comprises selectively measuring the methylation levels of a set of methylation markers in genomic DNA of the biological sample, said set of methylation markers comprising markers in least 3 of the genes listed in Table 6 and determining the age of the sample based on said methylation levels. In some aspects, the set of methylation markers may comprise markers in at least or at most 3, 4, 5, 6, 7, 8, 9, 10, 11, 12, 13, 14, 15, 16 or 17 of the genes listed in Table 6. In further aspects, the set of methylation markers may comprise markers in at least or at most 3, 4, 5, 6, 7, 8, 9, 10, 11, 12, 13, 14, 15, 16 or 17 of the CpG positions listed in Table 6.

In still a further aspect, a method of the disclosure comprises selectively measuring the methylation levels of a set of methylation markers in genomic DNA of the biological sample, said set of methylation markers comprising markers in least 2 of the genes listed in Table 7 and determining the age of the sample based on said methylation levels. In some aspects, the set of methylation markers may comprise markers in at least or at most 2, 3, 4, 5 or 6 of the genes listed in Table 7. In further aspects, the set of methylation markers may comprise markers in at least or at most 2, 3, 4, 5 or 6 of the CpG positions listed in Table 7.

In another aspect of the disclosure, a set of four methylation markers are disclosed that continuously relate to age in human blood, brain tissue, and saliva. Specifically, DNA methylation markers near the following genes: NHLRC1, GREM1, SCGN have highly significant positive correlations with age in multiple human tissues. Methylation markers near gene EDARADD have a highly significant negative correlation with age in multiple tissues. In one aspect of the disclosure, the methylation markers comprise of probes cg22736354 (SEQ ID NO: 158) near gene NHLRC1, cg21296230 near gene GREM1 (SEQ ID NO: 354), cg06493994 (SEQ ID NO: 46) near gene SCGN, and cg09809672 (SEQ ID NO: 252) near gene EDARADD. Methods for estimating age are provided which involve one to four of these markers. In these methods, biological cell or tissue sample is collected from an individual. Genomic DNA is extracted from the collected tissue and the methylation level of the methylation markers near at least one of the NHLRC1 (SEQ ID NO: 357), GREM1 (SEQ ID NO: 356), SCGN (SEQ ID NO: 358), and EDARADD (SEQ ID NO: 355) genes are measured. A statistical prediction algorithm is applied to the measured methylation levels to determine the biological or chronological age of the individual.

Embodiments of the invention include methods where observations of cytosine methylation in genomic DNA from a biological sample are used to predict the chronological age of the individual from which a sample is derived. Other embodiments of these methods comprise calculating a theoretical biological age (bio-age) of the individual based on the degree/amount of cytosine methylation observed in the sequence and then comparing the theoretical bio-age of the individual to an actual chronological age of the individual. In this way, information useful to determine a level of risk of an age-related disease in the individual is obtained. Optionally for example, the theoretical bio-age of the individual is compared to an actual chronological age to determine if the theoretical bio-age is greater than the actual chronological age; and the method further includes providing an individualized treatment to the individual to bring the theoretical bio-age closer to the actual chronological age of the individual.

DNAm age is a valuable biomarker for studying human development, aging, and cancer and can be used as a surrogate marker for evaluating rejuvenation therapies. The most salient feature of DNAm age is its applicability to a broad spectrum of tissues and cell types. DNAm age has been found to accurately predict age in various sources of DNA, including: adipose tissue/fat, blood (whole blood, cord blood, blood cells, peripheral blood mononuclear cells, B cells, T cells, monocytes), brain tissue (frontal cortex, temporal cortex, PONS), breast, buccal cells/epithelium, cartilage, cerebellum, colon, cortex (pre-frontal-, frontal-, occipital-, temporal cortex), epidermis, fibroblasts (e.g. dermal fibroblasts), gastric tissue, glial cells, head/neck tissue, kidney, lung, liver, mesenchymal stromal cells, neurons, pancreas, pons, prostate, saliva, stomach, thyroid, uterine cervix, and many other tissues/cell types. After incorporating an offset, it has also been found to perform well in heart tissue. Furthermore, DNAm age of easily accessible fluids/tissues (e.g. saliva, buccal cells, blood, skin) can serve as a surrogate marker for inaccessible tissues (e.g. brain, kidney, liver). Further, DNAm age can be used to compare the ages of different parts of the human body, e.g. to find diseased organs or tissues.

In another aspect of the present invention, a method is provided for estimating age in multiple tissues (e.g. whole blood, individual blood cells, saliva or brain tissue). In a further aspect, as shown below, easily accessible tissues (e.g. blood, saliva, buccal cells, epidermis) can be used to measure age in inaccessible tissues (e.g. brain). In one embodiment of the present invention, a method is provided for estimating of the chronological and/or biological age of an individual's human brain based on measuring DNA CpG methylation markers that are attached to the individual's DNA. Generally, human brain tissue from living individuals is not accessible and available for such measurements. However, as disclosed herein, a small set of DNA methylation markers can be measured in more accessible tissues, such as blood or saliva samples, to estimate the age-related methylation changes in the brain and other tissues. Thus, one is able to accurately predict an individual's age in the brain tissue based on blood or saliva measurements. Illustrative instances of this aspect of the disclosure include, for example, a method of predicting the age of a human by observing the methylation status of a plurality of markers such as at least 6, 17, 38, 100 markers (see, e.g. Tables 3-6) in biological sample from a human, comparing the methylation status observed in to methylation patterns observed in a population of individuals of differing ages (e.g. using a statistical prediction algorithm), and then predicting age of human from whom sample was obtained based upon the information obtained in this comparison step.

Many articles have described age-related changes in various human tissues, e.g. blood, saliva, and brain. However, these studies have never attempted to build a predictor of age in multiple tissues or cell types at the same time (e.g. combining brain and blood data). Instead, the studies have only focused on creating large lists of age-related CpG markers in various tissues for the sake of studying the biological impact of aging on individual CpGs. Currently, only three publications describe age predictors based on DNA methylation levels (Bockland et al. [23], Koch et al. [21], Hannum et al. [24]) but these publications focus on individual tissues or fluids (e.g. blood or saliva). Notably, Hannum et al. [24] found that computing a DNA methylation-based age predictor for different tissues gave basically no overlap, e.g. blood-derived predictive CpGs were different from those from other tissues. Comparison studies show that the age predictor of the present invention greatly outperforms the predictors by Bockland et al. [23] and Koch et al. [21]. A direct comparison with the predictor of Hannum et al. [24] was not possible because their predictor included additional covariates (data batch, gender and body mass index). The multi-tissue predictor provided herein only uses the clock CpGs, i.e. it does not require additional covariates.

CpGs/genes overlapping with the subclocks (110, 38, 17, and 6 CpGs shown in Tables 4, 5, 6, and 7 respectively) for Hannum/Bell include: 110/38/17/6 - IPO8 (alias: RANBP8) and NHLRC1; 110/38/17 - KLF4, SCGN, RHBDD1, and C16orf65; 110/38 - MGC16703 (alias: P2RX6) and FZD9; 38 - BRUNOL6; 110 - ABCA17P (alias: ABCA3), PIPOX, ABHD14B, EDARADD, GRP25, FLJ32110 (alias: ZNF8048) and LAG3.

In another aspect of the present invention, a very simple and cost-effective kit is provided for estimating DNAm age based on the clock CpGs. In some embodiments of the invention, the kit comprises a methylation microarray (see, e.g. U.S. Patent Application Publication No. 2006/0292585. In one embodiment, the kit is used to estimate the chronological and biological age of brain tissue or blood tissue utilizing measurements in blood or saliva. Microfluidics devices can be applied to easily accessible tissues/fluids such as blood, buccal cells, or saliva. In the invention, the kit comprises probes for detecting methylation markers consisting of each of the CpG positions of Table 3. Optionally, the kit comprises a plurality of primer sets for amplifying at least two genomic DNA sequences. In some embodiments of the invention, the kit further comprises a probe or primer used to perform a DNA fingerprinting analysis. Such kits of the invention can further include a reagent used in a genomic DNA polymerization process, a genomic DNA hybridization process, and/or a genomic DNA bisulfite conversion process. In one exemplary implementation, a kit is provided for obtaining information useful to determine the age of an individual, the kit comprising a plurality of primers or probes specific for at least one genomic DNA sequence in a biological sample, wherein the genomic DNA sequences comprises a CG loci identified in Figure 4. The invention is may also be provided in a fully developed software package or web-based program. For example, a user may access a webpage and upload their DNA methylation data. The program then emails the results, including the predicted age (DNAm age), to the user.

DNA methylation of the methylation markers (or markers close to them) can be measured using various approaches, which range from commercial array platforms (e.g. from Illumina™) to sequencing approaches of individual genes. This includes standard lab techniques or array platforms. A variety of methods for detecting methylation status or patterns have been described in, for example U.S. Pat. Nos. 6,214,556, 5,786,146, 6,017,704, 6,265,171, 6,200,756, 6,251,594, 5,912,147, 6,331,393, 6,605,432, and 6,300,071 and US Patent Application publication Nos. 20030148327, 20030148326, 20030143606, 20030082609 and 20050009059. Other array-based methods of methylation analysis are disclosed in U.S. patent application US 2005/0196792. For a review of some methylation detection methods, see, Oakeley, E. J., Pharmacology & Therapeutics 84:389-400 (1999). Available methods include, but are not limited to: reverse-phase HPLC, thin-layer chromatography, SssI methyltransferases with incorporation of labeled methyl groups, the chloracetaldehyde reaction, differentially sensitive restriction enzymes, hydrazine or permanganate treatment (m5C is cleaved by permanganate treatment but not by hydrazine treatment), sodium bisulfite, combined bisulphate-restriction analysis, and methylation sensitive single nucleotide primer extension.

The methylation levels of a subset of the DNA methylation markers disclosed herein are assayed (e.g. using an Illumina™ DNA methylation array, or using a PCR protocol involving relevant primers). To quantify the methylation level, one can follow the standard protocol described by Illumina™ to calculate the beta value of methylation, which equals the fraction of methylated cytosines in that location. The invention can also be applied to any other approach for quantifying DNA methylation at locations near the genes of the claims. DNA methylation can be quantified using many currently available assays which include, for example:

a) Molecular break light assay for DNA adenine methyltransferase activity is an assay that is based on the specificity of the restriction enzyme DpnI for fully methylated (adenine methylation) GATC sites in an oligonucleotide labeled with a fluorophore and quencher. The adenine methyltransferase methylates the oligonucleotide making it a substrate for DpnI. Cutting of the oligonucleotide by DpnI gives rise to a fluorescence increase.

b) Methylation-Specific Polymerase Chain Reaction (PCR) is based on a chemical reaction of sodium bisulfite with DNA that converts unmethylated cytosines of CpG dinucleotides to uracil or UpG, followed by traditional PCR. However, methylated cytosines will not be converted in this process, and thus primers are designed to overlap the CpG site of interest, which allows one to determine methylation status as methylated or unmethylated. The beta value can be calculated as the proportion of methylation.

c) Whole genome bisulfite sequencing, also known as BS-Seq, is a genome-wide analysis of DNA methylation. It is based on the sodium bisulfite conversion of genomic DNA, which is then sequencing on a Next-Generation Sequencing (NGS) platform. The sequences obtained are then re-aligned to the reference genome to determine methylation states of CpG dinucleotides based on mismatches resulting from the conversion of unmethylated cytosines into uracil.

d) The *HpaII* tiny fragment Enrichment by Ligation-mediated PCR (HELP) assay is based on restriction enzymes' differential ability to recognize and cleave methylated and unmethylated CpG DNA sites.

e) Methyl Sensitive Southern Blotting is similar to the HELP assay but uses Southern blotting techniques to probe gene-specific differences in methylation using restriction digests. This technique is used to evaluate local methylation near the binding site for the probe.

f) ChIP-on-chip assay is based on the ability of commercially prepared antibodies to bind to DNA methylation-associated proteins like MeCP2.

g) Restriction landmark genomic scanning is a complicated and now rarely-used assay is based upon restriction enzymes' differential recognition of methylated and unmethylated CpG sites. This assay is similar in concept to the HELP assay.

h) Methylated DNA immunoprecipitation (MeDIP) is analogous to chromatin immunoprecipitation. Immunoprecipitation is used to isolate methylated DNA fragments for input into DNA detection methods such as DNA microarrays (MeDIP-chip) or DNA sequencing (MeDIP-seq).

i) Pyrosequencing of bisulfite treated DNA is a sequencing of an amplicon made by a normal forward primer but a biatenylated reverse primer to PCR the gene of choice. The Pyrosequencer then analyses the sample by denaturing the DNA and adding one nucleotide at a time to the mix according to a sequence given by the user. If there is a mismatch, it is recorded and the percentage of DNA for which the mismatch is present is noted. This gives the user a percentage methylation per CpG island.

In certain embodiments of the invention, the genomic DNA is hybridized to a complimentary sequence (e.g. a synthetic polynucleotide sequence) that is coupled to a matrix (e.g. one disposed within a microarray). Optionally, the genomic DNA is transformed from its natural state via amplification by a polymerase chain reaction process. For example, prior to or concurrent with hybridization to an array, the sample may be amplified by a variety of mechanisms, some of which may employ PCR. See, for example, PCR Technology: Principles and Applications for DNA Amplification (Ed. H. A. Erlich, Freeman Press, NY, N.Y., 1992); PCR Protocols: A Guide to Methods and Applications (Eds. Innis, et al., Academic Press, San Diego, Calif., 1990); Mattila et al., Nucleic Acids Res. 19, 4967 (1991); Eckert et al., PCR Methods and Applications 1, 17 (1991); PCR (Eds. McPherson et al., IRL Press, Oxford); and U.S. Pat. Nos. 4,683,202, 4,683,195, 4,800,159, 4,965,188, and 5,333,675. The sample may be amplified on the array. See, for example, U.S. Pat. No. 6,300,070.

Any statistical approach can be used to relate the methylation levels to age, e.g. a transformed version of chronological age can be regressed on the CpG markers using a (penalized) linear regression model (such as elastic net regression) as described herein. Using conventional regression model/analysis tools and methodologies known in the art, a number of age prediction models are contemplated for use with specific genomic DNA samples and/or specific analysis techniques and/or specific individual populations (see, e.g., statistical package R version 2.11.1 in citation as discussed in R Development Core Team (2005) R: A language and environment for statistical computing. R Foundation for Statistical Computing, Vienna, Austria. ISBN 3-900051-07-0, URL www.R-project.org). In one embodiment, an identity transformation may be used, wherein chronological age is simply regressed on the CpGs. In other embodiments, the chronological age (the dependent variable in a penalized regression model) is transformed. In illustrative experiments, this transformation has been found to lead to an age predictor that is substantially more accurate (in relation to error) and that requires substantially fewer CpGs than one without the transformation. Additionally, one can form a weighted average of the CpGs.

In another embodiment, a linear regression model may predict age based on a weighted average of the methylation levels plus an offset. To identify the weights for the weighted average, one can use the regression coefficients of a regression model. In another embodiment, one can standardize each methylation marker so that it has a mean zero and variance. A weighted average of the standardized methylation levels is then formed where the weights are chosen to equal their correlation with age in a training data set times the standard deviation of the ages that is expected in the test data set. In one or more embodiments, the transformation of the dependent variable (i.e. chronological age) is a piecewise transformation: for ages between say 0 and 20, a logarithmic transformation is used. For ages older than 20, a linear transformation is used. Additionally, the dependent variables (CpGs) are "normalized" to a chosen gold standard (e.g. the mean methylation level in the training data or the mean methylation levels in blood tissue) using an adaptation of the BMIQ algorithm by Teschendorff. Further details are provided in Example 8. This normalization step ensures that future test data resemble those of the training data.

For example, in one training data set disclosed herein, methylation markers cg22736354 (SEQ ID NO: 158), cg21296230 (SEQ ID NO: 354), cg06493994 (SEQ ID NO: 46), and cg09809672 (SEQ ID NO: 252) near genes NHLRC1, GREM1, SCGN, and EDARADD have correlations r = -0.47, 0.80, 0.71, and 0.76, respectively (see Examples). In the training data set, the standard deviation of age was 24 and the mean value was 45. After forming this weighted average of the standardized methylation levels, the expected mean age in the test data set (e.g. 45) is added to arrive at the final prediction of the chronological and/or the biological age of the individual. While the prediction is based on the chosen tissue, it also applies to other tissues. Therefore, easily accessible tissues such as blood or saliva tissue can be used to predict the age of brain tissue or other inaccessible tissues.

In addition to the illustrative models disclosed herein, other models can, for example, customize the coefficient values (weights) for different tissues and/or cell lineages. Furthermore, in addition to tissue type, such coefficients can be weighted in data sets from different populations. For example, if a model is applied to pediatric patients only, then one set of coefficients can be used. Alternatively, if a model is applied exclusively to older people (e.g. greater than 50 years), another set of coefficients can be used. Alternatively, coefficients can be fixed, for example, when a model is broadly applied to people of ages from 10 to 100 etc. Coefficient values in various models can also reflect the specific assay that is used to measure the methylation levels (e.g. as the variance of the methylation levels of individual probes may affect the coefficient). For example, for beta values measured on Illumina™ methylation microarray platforms there can be one set of coefficients, while for other methylation measures (e.g. using sequencing technology) there can be another set of coefficients etc. Other values may also be used instead, such as M values (transformed versions of beta values). Furthermore, methylation levels may be replaced by values that adjust for the methylation levels of a background or by mean methylation levels of a set benchmark of CpGs. In practicing certain embodiments of the invention, one can collect a reference data set (e.g. of 100 individuals of varying ages) using specific technology platform(s) and tissue(s) and then design a specific multivariate linear model fit to this reference data set to estimate the coefficients (e.g. using least squares regression). The resultant multivariate model can then be used for predicting ages on test patients. In this way, different mathematical models can be adapted for analyzing methylation patterns in a wide variety of contexts.

In addition to using art accepted modeling techniques (e.g. regression analyses), embodiments of the invention can include a variety of art accepted technical processes. For example, in certain embodiments of the invention, a bisulfite conversion process is performed so that cytosine residues in the genomic DNA are transformed to uracil, while 5-methylcytosine residues in the genomic DNA are not transformed to uracil. Kits for DNA bisulfite modification are commercially available from, for example, MethylEasy™ (Human Genetic Signatures™) and CpGenome™ Modification Kit (Chemicon™). See also, WO04096825A1, which describes bisulfite modification methods and Olek et al. Nuc. Acids Res. 24:5064-6 (1994), which discloses methods of performing bisulfite treatment and subsequent amplification. Bisulfite treatment allows the methylation status of cytosines to be detected by a variety of methods. For example, any method that may be used to detect a SNP may be used, for examples, see Syvanen, Nature Rev. Gen. 2:930-942 (2001). Methods such as single base extension (SBE) may be used or hybridization of sequence specific probes similar to allele specific hybridization methods. In another aspect the Molecular Inversion Probe (MIP) assay may be used.

Furthermore, the methods provided for estimating age may involve relatively few markers. In one or more certain aspects of the disclosure, the methods involve between 1 to 4 markers. For example, DNA methylation markers near the following genes: NHLRC1 (SEQ ID NO: 357), GREM1 (SEQ ID NO: 356), SCGN (SEQ ID NO: 358) have highly significant positive correlations with age in multiple human tissues. Methylation markers near gene EDARADD (SEQ ID NO: 355) have a highly significant negative correlation with age in multiple tissues. By way of illustration, genes and corresponding Illumina™ Methylation probe IDs are provided. For example, the following probe identifiers from an Illumina™ methylation array platform denote suitable markers: i) probe cg22736354 (SEQ ID NO: 158) near gene NHLRC1, ii) probe cg21296230 (SEQ ID NO: 354) near gene GREM1, and iii) probe cg06493994 (SEQ ID NO: 46) near gene SCGN have positive correlations with age in multiple tissues; iv) probe cg09809672 (SEQ ID NO: 252) near gene EDARADD has a negative correlation with age in multiple tissues.

The methods for estimating an individual's age can be used for both diagnostic and prognostic purposes. The biomarkers for aging can be used to study the effect of medication, food compounds and/or special diets on the wellness and biological age of humans. They can also be used as biomarkers of vitality or youthfulness. For example, the biomarkers for aging can be used to determine chronological age (e.g. for forensic applications). They can also be used for determining and increasing an individual's likelihood of longevity and of retaining cognitive function during aging.

In certain embodiments the methods of the invention can be used to provide valuable information in forensic investigations (e.g. where the identity of the individual from which the DNA is derived is unknown). In one embodiment, the methods of the invention can be applied to forensic applications involving the prediction of chronological age. The methylation levels of the epigenetic markers (clock CpGs) are measured. In certain aspects of the disclosure, the methylation levels of one or more of the four methylation markers near genes EDARADD, NHLRC1, GREM1, and SCGN in blood or saliva are measured. In one aspect, probes cg22736354 (SEQ ID NO: 158) near gene NHLRC1, cg21296230 (SEQ ID NO: 354) near gene GREM1, cg06493994 (SEQ ID NO: 46) near gene SCGN, and/or cg09809672 (SEQ ID NO: 252) near gene EDARADD are used. A statistical prediction method (e.g. based on linear regression) is then applied to predict the age of the individual. The age predictive models disclosed can be applied in a variety of contexts. For instance, the ability to predict an individual's age can be used by forensic scientists to estimate a suspect's age based on a biological sample alone. In embodiments of the invention designed for forensic use, a practitioner could, for example, submit a biological sample to a lab. In the lab, DNA prepared from the sample could then be analyzed to determine the percentage of methylation at one or more of the loci identified herein. The results could be inputed in a regression model, such as those disclosed herein, to predict the age of the suspect. In certain instances, the suspect's age can be predicted to an average accuracy of 3 to 5 years.

Such embodiments of the invention can be combined with other forensic analysis procedures, for example by also performing a DNA fingerprinting analysis on the genomic DNA. DNA fingerprinting (also known as DNA profiling) using short tandem repeats (STRs) is one method for human identification in forensic sciences, finding applications in different circumstances such as determination of perpetrators of violent crime, resolving paternity, and identifying remains of missing persons or victims of mass disaster. The FBI and the forensic science community typically use 13 separate STR loci (the core CODIS loci) in routine forensic analysis. (CODIS refers to the Combined DNA Index System that was established by the FBI in 1998). Illustrative DNA fingerprinting methodologies are disclosed, for example, in U.S. Patent Nos. 7,501,253, 7,238,486, 6,929,914, 6,251,592, and 5,576,180).

In another embodiment, the methods disclosed herein can be applied to medical applications involving the prediction of the biological age. The age is predicted according to the methods described. This predicted value is interpreted as the biological age (DNA methylation age). The prediction then is contrasted with the known chronological age of the individual. If the predicted age is higher than the chronological age, it indicates that the person appears older (or more impaired or more at risk of an age related disease) than his or her peers from the same age group, i.e. shows evidence of age acceleration.

In addition, a measurement of relevant methylation patterns in genomic DNA from white blood cells or skin cells also provides a tool in routine medical screening to predict the risk of age-related diseases as well as to tailor interventions based on the epigenetic biological age instead of the chronological age. In some embodiments of the invention, one can compare the predicted age of the individual with the actual chronological age of the individual, for example as part of a diagnostic procedure for an age associated pathology (e.g. one that compares an individual's chronological age with an apparent biological age in view of their DNA methylation patterns). Such methods can be useful in clinical interventions that are predicated on an epigenetic biological age rather than an actual chronological age. In one embodiment, a biological sample can be collected in a routine health check and sent to the lab for methylation pattern analysis (e.g. as described above). If the predicted age of the patient is higher than the real age, the patient can be at an increased risk of age-related diseases, and dietary intervention, or specific drugs, could be prescribed to reduce this "genetic age". As noted above, embodiments of the invention include methods of obtaining information useful to determine a level of risk of an age-related disease in an individual (e.g. Alzheimer's disease or Parkinson's disease).

Furthermore, since DNAm age allows one to contrast the ages of various tissues/cell types from the same individual, it can be used to identify diseased tissue (e.g. cancer tissue often shows evidence of severe positive or negative age acceleration). The biomarkers for aging can also be used for determining and decreasing an individual's likelihood of developing an age-related disease, e.g. cancer, dementia. Methods are provided for diagnosing and determining the existence or likelihood of cognitive deficits in the elderly resulting from senescence or age-related disease. Accordingly, such methods allow for the determination of patients who are most likely to be at risk of age-related cognitive decline and allow these patients to be targeted for more intensive study or prophylaxis.

In a further embodiment, the methods disclosed herein can be applied to assess the efficacy of a treatment or compound (e.g. rejuvenation or curing an age-related impairment, enhancing memory function or cognition). As an example, the biomarkers for aging can be used in studying patients who, although not elderly, are afflicted by a brain disease that typically occurs in the elderly (e.g. early onset dementia). A determination is made regarding whether administration of the treatment or compound affects the predicted age. An effective treatment would lower the predicted age since the individual appears rejuvenated and younger.

An assay is provided for identifying a compound that increases memory function and/or decreases a subject's likelihood of developing an age-related cognitive decline. The assay comprises identifying a compound which counters the age-related increase or decrease of methylation in the identified markers. Age prediction methodologies are also relevant to healthcare applications. For example, significant DNA methylation differences are known to be associated with specific age-related disorders, for example in comparisons between the brains of people diagnosed with late-onset Alzheimer's disease and brains from controls. In this context, the identification of specific loci highly correlated with age can be used to enhance the understanding of aging in health and disease. In certain embodiments of the invention, age prediction methodologies can be used as part of clinical interventions tailored for patients based on their "bio-age"—a result of the interaction of genes, environment, and time—rather than their chronological age. For example, if a person's predicted age is higher than their real age, specific interventions could be designed to return the genome to a "younger" state. Age prediction methodologies can also pave the way for interventions based on specific epigenetic marks associated with disease, as occurs in certain cancer treatments.

As described in detail in the Example section below, specific age-related methylation markers have been identified and validated using further assays and additional samples. Additionally, illustrative age prediction analysis models have been designed and tested, for example by using a leave-one-out analysis where one subject from a model is systematically removed and the model is used to predict the subject's age. Since the real age of this subject is already known, such methods provide ways to validate various model designs.

## EXAMPLES

As shown in the illustrative examples below, the relationship between DNA methylation and age has been validated in 5 independent whole blood data sets, 3 brain methylation data sets and 2 saliva data sets. These findings are highly significant and have been carefully validated.

For Examples 1-4, publicly available data was used (see e.g. Gene Expression Omnibus database). Brain methylation data came from Gibbs JR et al. (2010) (Gibbs JR, van der Brug MP, Hernandez DG, Traynor BJ, Nalls MA, et al. (2010) Abundant Quantitative Trait Loci Exist for DNA Methylation and Gene Expression in Human Brain. PLoS Genet 6(5): e1000952. doi:10.1371/journal.pgen.1000952). The authors obtained frozen brain tissue from frontal cortex (FCTX), pons (PONS) and temporal cortex (TCTX) from 150 subjects (total 450 tissue samples). Using the Illumina™ 27k methylation array they assayed 27,578 CpG methylation sites in each of the brain regions. However, the authors did not study age effects. Further, they did not relate the brain methylation data to blood methylation data. The publicly available blood and saliva methylation used the same Illumina™ methylation array and are described in the following Table 1.

| Table 1. Description of public DNA methylation data sets used for the invention | | | | | | | | |
|---|---|---|---|---|---|---|---|---|
| Set No | Sample size | Tissue | Sample characteristics | Mean Age | Age Range | Methylation Assay | Reference | GSE number |
| 1 | 191 | WB | Type 1 diabetics | 44 | 24-74 | Infin 27k | Teschendorff 2010 | GSE20067 |
| 2 | 93 | WB | Healthy older women | 63 | 49-74 | Infin 27k | Rakyan 2010 | GSE20236 |
| 3 | 534 | WB | postmenopausal women from the ovarian cancer UKOPS | 66 | 49-91 | Infin 27k | Teschendorff 2010, Song 2009 | GSE19711 |
| 4 | 133 | FCTX | FCTX brain | 48 | 15-101 | Infin 27k | Gibbs 2010 | GSE15745 |
| 5 | 127 | TCTX | TCTX brain | 49 | 15-101 | Infin 27k | Gibbs 2010 | GSE15745 |
| 6 | 125 | PONS | PONS brain | 47 | 15-101 | Infin 27k | Gibbs 2010 | GSE15745 |
| 7 | 114 | CRBLM | CRBLM brain | 48 | 16-96 | Infin 27k | Gibbs 2010 | GSE15745 |
| 8 | 69 | Saliva | Saliva | 35 | 21-55 | Infin 27k | Bockland 2011 | GSE28746 |
| 9 | 168 | cord blood buffy coat | newborns, cordblood | 0 | 0-0 | Infin 27k | Adkins 2011 | GSE27317 |
| 10 | 50 | CD14+CD4+ | sorted CD4+ T-cells and CD14+ monocytes | 36 | 16-69 | Infin 27k | Rakyan 2010 | GSE20242 |
| 11 | 185 | Saliva | Saliva from alcoholics | 32 | 21-55 | Infin 27k | Liu 2010 | GSE34035 |
| (WB) Whole blood, FCTX (Frontal Cortex), TCTX (Temporal Cortex), CRBLM (Cerebellum), (NA) not available | | | | | | | | |

Table 1

For the identification of age-related methylation markers across multiple tissues, Stouffer's meta-analysis Z statistic (implemented in the metaAnalysis R function in the Weighted correlation network analysis (WGCNA) R package) was used to identify methylation markers that consistently relate to age across all data sets (see Table 2).

Table 2

| Table: P-values from a meta analysis relating age to methylation levels across multiple tissues. | | | | | | |
|---|---|---|---|---|---|---|
| **Gene Sym** | **Probe ID** | **pValueAllTissues** | **pValueBlood** | **pValueBrain** | **pValueSaliva** | **cor with age** |
| SCGN | cg06493994 | 2.05E-119 | 3.72E-23 | 2.33E-121 | 1.64E-18 | 0.76 |
| EDARADD | cg09809672 | 2.69E-87 | 3.18E-39 | 1.52E-40 | 3.50E-28 | -0.47 |
| GREM1 | cg21296230 | 4.16E-105 | 4.78E-22 | 1.71E-108 | 7.27E-16 | 0.71 |
| NHLRC1 | cg22736354 | 8.13E-146 | 3.52E-27 | 8.51E-165 | 6.50E-11 | 0.80 |

Example 1: Linear regression predictor involving only 1 methylation marker accurately predicts age in blood, brain and saliva

A univariate linear regression predictor based on a single methylation probe was examined. A single methylation probe corresponding to Illumina™ probe ID cg22736354 (SEQ ID NO: 158) (close to gene NHLRC1) was used in the univariate linear regression model. As shown in Figures 1-3, using a single cytosine marker in gene NHLRC1, the linear regression model-based prediction of age was found to correlate with the true age in brain tissue (correlation coefficient=0.88, p-value=6.8xE-126) and blood tissue (cor=0.76,p=3.6E-174). In particular, Probe ID: cg22736354 (SEQ ID NO: 158), located near the gene with gene symbol NHLRC1, had a highly significant positive correlation with age in the considered brain regions and in blood.

Example 2: A multivariate regression predictor involving 2 methylation markers accurately predicts age in blood, brain and saliva

A multivariate regression predictor based on two methylation probes was examined. Methylation probes corresponding to Illumina™ probe IDs cg09809672 (SEQ ID NO: 252, close to gene EDARADD) and cg22736354 (SEQ ID NO: 158, close to gene NHLRC1) were used in the multivariate linear regression model. As shown in Figures 4-7, using just the two cytosines near genes NHLRC1 and EDARADD, the multivariate linear regression model based prediction of age had a correlation larger than 0.90 with age in blood and brain tissue and it also correlated highly with age in saliva tissue. The median absolute difference (deviation) between predicted age and true age was 5.1 years. In particular, Probe ID: cg09809672 (SEQ ID NO: 252), located near the gene with gene symbol EDARADD, had a negative correlation with age and Probe ID: cg22736354 (SEQ ID NO: 158), located near the gene with gene symbol NHLRC1, had a positive correlation with age.

Example 3: A multivariate regression predictor involving 4 methylation markers accurately predicts age in blood, brain and saliva

A multivariate regression predictor based on four methylation probes was examined. Methylation probes corresponding to Illumina™ probe IDs cg09809672 (SEQ ID NO: 252, close to gene EDARADD), cg22736354 (SEQ ID NO: 158, close to gene NHLRC1), cg21296230 (SEQ ID NO: 354, close to gene GREM1), and cg06493994 (SEQ ID NO: 46, close to gene SCGN) were used in the multivariate linear regression model. As shown in Figures 8-11, using the four cytosines near genes EDARADD, NHLRC1, GREM1, SCGN, the multivariate linear regression model based prediction of age had a correlation larger than 0.90 with age in blood and brain tissue and that correlate with age in saliva tissue. The median absolute difference (deviation) between predicted age and true age was around 5.1 years. In particular, probe ID: cg09809672 (SEQ ID NO: 252), located near the gene with gene symbol EDARADD, had a negative correlation with age and Probe IDs: cg22736354 (SEQ ID NO: 158), cg21296230 (SEQ ID NO: 354), and cg06493994 (SEQ ID NO: 46), located near the genes with gene symbols NHLRC1, GREM1, and SCGN, respectively, had a positive correlation with age.

Example 4: Two saliva based methylation markers can be used to predict the age of brain tissue

Methylation markers near the gene EDARADD (e.g. methylation probe cg09809672, SEQ ID NO: 252) and gene SCGN (e.g. probe cg06493994, SEQ ID NO: 46) were used in predicting brain age. As shown in Figures 12-15, the predicted age in brain tissue had a correlation of 0.4 with the true age (median deviation=8.2 years). In saliva, the correlation was 0.72 and median deviation was only 4.2 years. In blood tissue, the correlation was 0.88 and median deviation was 6.1 years. Thus, the predictor is particularly well suited for predicting brain age based on saliva samples. Probe ID: cg09809672 (SEQ ID NO: 252), located near the gene with gene symbol EDARADD, had a negative correlation with age and Probe ID: cg06493994 (SEQ ID NO: 46), located near the gene with gene symbol SCGN (also known as SEGN; SECRET; setagin; DJ501N12.8) had a positive correlation with age.

Example 5: DNA methylation age of human tissues and cell types

A collection of publicly available DNA methylation data sets is used for defining and evaluating an age predictor. The demonstrated accuracy across most tissues and cell types justifies its designation as a multi-tissue age predictor. Its age prediction, referred to as DNAm age, can be used as biomarker for addressing a host of questions arising in aging research and related fields. For example, interventions used for creating induced pluripotent stem cells are shown to reset the epigenetic clock to zero.

Using 82 Illumina™ DNA methylation array data sets (n=7844) involving 51 healthy tissues and cell types, a multi-tissue predictor of age is provided which allows one to estimate the DNA methylation (DNAm) age of most tissues and cell types. DNAm age has the following properties: a) it is close to zero for embryonic and induced pluripotent stem (iPS) cells, b) it correlates with cell passage number, c) it gives rise to a highly heritable measure of age acceleration, and d) it is applicable to chimpanzee tissues. 354 clock CpGs were characterized in terms of chromatin states and tissue variance (Table 3). The application of DNAm age to 32 additional cancer DNA methylation data sets (comprised of n=5826 samples) shows that all cancer tissues exhibit significant age acceleration (on average 36.2 years). Low age acceleration of cancer tissue is associated with a high number of somatic mutations and TP53 mutations. Mutations in steroid receptors greatly accelerate DNAm age in breast cancer. The multi-tissue predictor of age has been applied to colorectal cancer, glioblastoma multiforme, AML, and cancer cell lines.

*Description of the (non-cancer) DNA methylation data sets*

A large DNA methylation data set was assembled by combining publicly available individual data sets measured on the Illumina™ 27K or Illumina™ 450K array platform (Cancer Genome Atlas (TCGA) data sets). In total, n=7844 non-cancer samples from 82 individual data sets were analyzed, which assess DNA methylation levels in 51 different tissues and cell types. Although many data sets were collected for studying certain diseases (Example 8), they largely involved healthy tissues. In particular, cancer tissues were excluded from this first large data set since it is well known that cancer has a profound effect on DNA methylation levels [6, 7, 24-26]. The Cancer Genome Atlas (TCGA) data sets involved normal adjacent tissue from cancer patients. Details on the individual data sets and data pre-processing steps are provided in Example 7 (Materials and methods) and Example 8. The first 39 data sets were used to construct ("train") the age predictor. Data sets 40-71 were used to test (validate) the age predictor. Data sets 72-82 served other purposes e.g. to estimate the DNAm age of embryonic stem and iPS cells. The criteria used for selecting the training sets are described in Example 8. Briefly, the training data were chosen i) to represent a wide spectrum of tissues/cell types, ii) to involve samples whose mean age (43 years) is similar to that in the test data, and iii) to involve a high proportion of samples (37%) measured on the Illumina™ 450K platform since many on-going studies use this recent Illumina™ platform. 21369 CpGs (measured with the Infinium type II assay), which were present on both Illumina™ platforms (Infinium 450K and 27K), were studied. There were fewer than 10 missing values across the data sets.

*The multi-tissue age predictor used for defining DNAm age*

To ensure an unbiased validation in the test data, only the training data was used to define the age predictor. As detailed in Example 7 (Materials and methods) and Example 8, a transformed version of chronological age was regressed on the CpGs using a penalized regression model (elastic net). The elastic net regression model automatically selected 354 CpGs (Table 3, Example 9). Since their weighted average (formed by the regression coefficients) amounts to an epigenetic molecular clock, the 354 CpGs are referred to as clock CpGs.

*Predictive accuracy across different tissues*

Several measures of predictive accuracy were initially considered since each measure has distinct advantages. The first, referred to as "age correlation", is the Pearson correlation coefficient between DNAm age (predicted age) and chronological age. It has the following limitations: it cannot be used for studying whether DNAm is well calibrated, it cannot be calculated in data sets whose subjects have the same chronological age (e.g. cord blood samples from newborns), and it strongly depends on the standard deviation of age (as described below). The second accuracy measure, referred to as (median) "error", is the median absolute difference between DNAm age and chronological age. Thus, a test set error of 3.6 years indicates that DNAm age differs by less than 3.6 years in 50% of subjects. The error is well suited for studying whether DNAm age is poorly calibrated. Average age acceleration, defined by the average difference between DNAm age and chronological age, can be used to determine whether the DNAm age of a given tissue is consistently higher (or lower) than expected.

According to these three accuracy measures, the multi-tissue age predictor has been found to perform remarkably well in most tissues and cell types. A high accuracy in the training data (age correlation 0.97, error=2.9 years) was demonstrated in exemplary experiments and its performance assessment (age correlation=0.96, error=3.6 years, Figure 17) in the test data is notably unbiased. Note that the age predictor performs well in heterogeneous tissues (e.g. whole blood, blood peripheral blood mononuclear cells, cerebellar samples, occipital cortex, buccal epithelium, colon, adipose, liver, lung, saliva, uterine cervix) as well as in individual cell types such as CD4 T cells and CD14 monocytes (Figure 17C) and immortalized B cells (Figure 17T).

The age predictor is particularly accurate in data sets comprised of adolescents and children, e.g. blood (Figure 17B), brain data (Figure 17F,G), and buccal epithelium (Figure 17I).

*The DNAm age of blood and brain cells*

Human blood cells have different life spans: while CD14+ monocytes (myeloid lineage) only live several weeks, CD4+ T-cells (lymphoid lineage) represent a variety of cell types that can live from months to years. An interesting question is whether blood cell types have different DNAm ages. In one experiment, it was found that DNAm age does not vary significantly across sorted blood cells from healthy male subjects. These results combined with the fact that the age predictor works well in individual cell types (Figure 17) strongly suggest that DNAm age does not reflect changes in cell type composition but rather intrinsic changes in the methylome. This conclusion is also corroborated by the finding that DNAm age is highly related to chronological age in glial cells and neurons and various brain regions.

*DNAm age and progeria*

DNAm age can be used to study whether cells from patients with accelerated aging diseases such as progeria (including Werner progeroid syndrome, Hutchinson-Gilford progeria, HGP) truly look old at an epigenetic level. An exemplary experiment has demonstrated that progeria disease status is not related to DNAm based age acceleration in Epstein-Barr-Virus transformed B cells (Figure 17T). But the study of accelerated aging effects in HGP should be repeated for vascular smooth muscle, the tissue that is most compromised in HGP.

*Tissues where DNAm age is less accurately calibrated*

In certain experiments, DNAm age was found to be less accurately calibrated (i.e. leads to a higher error) in breast tissue (Figure 17H), uterine endometrium (Figure 17S), dermal fibroblasts, skeletal muscle tissue (Figure 17P), and heart tissue (Figure 17L). The biological reasons that could explain the less accurate calibration can only be speculated. It may be possible that the higher error in breast tissue may reflect hormonal effects or cancer field effects in this normal adjacent tissue from cancer samples. Note that the lowest error (7.5 years) in breast tissue is observed in normal breast tissue, i.e. in samples from women without cancer. The menstrual cycle and concomitant increases in cell proliferation may explain the high error in uterine endometrium. Myosatellite cells may effectively rejuvenate the DNAm age of skeletal muscle tissue. Similarly, the recruitment of stem cells into cardiomyocytes for new cardiac muscle formation could explain why human heart tissue tends to have a low DNAm age. Carefully designed studies will be needed to test these hypotheses.

*The age correlation in a data set is determined by the standard deviation of age*

In the following, non-biological reasons that affect the accuracy (age correlation) of the age predictor are described. To address how well the age predictor works in individual data sets, two different approaches were used. First, the age predictor was applied to individual data sets. An obvious limitation of this approach is that it leads to biased results in the training data sets.

The second approach, referred to as leave-one-data-set-out cross validation (LOOCV) analysis, leads to unbiased estimates of the predictive accuracy for each data set. As suggested by its name, this approach estimates the DNAm age for each data set (considered as test data set) separately by fitting a separate multi-tissue age predictor to the remaining (left out) data sets.

Data sets differ greatly with respect to the median chronological age and the standard deviation (SD), which is defined as the square root of the variance of age. Some data sets only involve samples with the same age (SD=0) while others involve both young and old subjects. As expected, the SD is found to be significantly correlated (r=0.49, p=4E-5) with the corresponding LOOCV estimate of the age correlation. In contrast, the sample size of the data set has no significant relationship with the age correlation.

A host of technical artefacts could explain differences in predictive accuracy (e.g. variations in sample processing, DNA extraction, DNA storage effects, batch effects, and chip effects.

*DNAm age of multiple tissues from the same subject*

The following addresses whether solid tissues can be found whose DNAm age differs substantially from chronological age. As a first step, the mean DNAm age per tissue is compared with the corresponding mean chronological age. As expected, mean DNAm age per tissue is highly correlated (cor=0.99) with mean chronological age. But breast tissue shows evidence of significant age acceleration.

A more interesting analysis is to compare the DNAm ages of tissues collected from the same subjects. DNAm age does not change significantly across different brain regions (temporal cortex, pons, frontal cortex, cerebellum) from the same subjects. Although the limited sample sizes per tissue (mostly one sample per tissue per subject) in this illustrative experiment did not allow for rigorous testing, these data can be used to estimate the coefficient of variation of DNAm age (i.e. the standard deviation divided by the mean). Note that the coefficient of variations for the first and second adult male are relatively low (0.12 and 0.15) even though the analysis involved several tissues that were not part of the training data, e.g. jejunum, penis, pancreas, esophagus, spleen, pancreas, lymph node, diaphragm. The coefficient of variation in the adult female is relatively high (0.21) which reflects the fact that her breast tissue shows signs of substantial age acceleration.

It remains to be seen how well DNAm age performs in tissues and DNA sources that were not represented in the training data set. It is anticipated that it also performs well in several other human tissues. As expected, no significant age correlation was found in sperm. The DNAm age of sperm is significantly lower than the chronological age of the donor.

*DNAm age is applicable to chimpanzees*

It is important to study whether there are inter-primate differences when it comes to DNAm age. These studies may not only help in identifying model organisms for rejuvenating interventions but might explain differences in primate longevity. While future studies could account for sequence differences, it is straightforward to apply the DNAm age estimation algorithm to Illumina™ DNA methylation data sets 72 [27] and 73 [28]. Strikingly, the DNAm age of heart-, liver-, and kidney tissue from chimpanzees (Pan troglodytes) is aligned with that of the corresponding human tissues. Further, the DNAm age of blood samples from two extant hominid species of the genus pan (commonly referred to as chimpanzee) is highly correlated with chronological age. While DNAm age is applicable to chimpanzees, its performance appears to be diminished in gorillas, which may reflect the larger evolutionary distance.

*DNAm age of induced pluripotent stem (iPS) cells and stem cells*

The billions of cells within an individual can be organized by genealogy into a single somatic cell tree that starts from the zygote and ends with differentiated cells. Cells at the root of this tree should be young. This is indeed the case: embryonic stem cells have a DNAm age close to zero in 5 different data sets. Induced pluripotent stem (iPS) cells are a type of pluripotent stem cell artificially derived from a non-pluripotent cell (typically an adult somatic cell) by inducing a set of specific genes. Since iPS cells are similar to ES cells, it is hypothesized that the DNAm age of iPS cells should be significantly younger than that of corresponding primary cells. This hypothesis is confirmed in three independent data sets. No significant difference in DNAm age could be detected between embryonic stem (ES) cells and iPS cells.

*Effect of cell passaging on DNAm age*

Most cells lose their proliferation and differentiation potential after a limited number of cell divisions (Hayflick limit). It is hypothesized that cell passaging (also known as splitting cells) increases DNAm age. This hypothesis is confirmed in three independent data sets. A significant correlation between cell passage number and DNAm age can be also observed when restricting the analysis to iPS cells or when restricting the analysis to embryonic stem cells.

*Comparing the multi-tissue predictor with other age predictors*

The multi-tissue predictor disclosed greatly outperforms existing predictors described in other articles [21, 23]. See Example 8 for a comparison of the multi-tissue predictor versus existing predictors. While further gains in accuracy can perhaps be achieved by focusing on a single tissue and considering more CpGs, the major strength of the multi-tissue age predictor lies in its wide applicability: for most tissues it will not require any adjustments or offsets. A "shrunken" version of the multi-tissue predictor (Examples 8 and 9), based on 110 CpGs (selected from the 354 clock CpGs) has also been found to be highly accurate in the training data (cor=0.95, error=4 years) and test data (cor=0.95, error=4.2 years).

*What is known about the 354 clock CpGs?*

An Ingenuity Pathway analysis of the genes that co-locate with the 354 clock CpGs (Table 3) shows significant enrichment for cell death/survival, cellular growth/proliferation, organismal/tissue development, and cancer.

The 354 clock CpGs can be divided into two sets according to their correlation with age. The 193 positively and 160 negatively correlated CpGs get hypermethylated and hypomethylated with age, respectively. DNA methylation data measured across many different adult and fetal tissues is used to study the relationship between tissue variance and age effects. While the DNA methylation levels of the 193 positively related CpGs vary less across different tissues, those of the 160 negatively related CpGs vary more across tissues than the remaining CpGs on the Illumina™ 27K array. To estimate "pure" age effects, a meta-analysis method was used that implicitly conditions on data set, i.e. it removes the confounding effects due to data set and tissue type. The clock CpGs include those with the most significant meta-analysis p-value for age irrespective of whether the meta-analysis p-value was calculated using only training data sets or all data sets. While positively related markers don't show a significant relationship with CpG island status, negatively related markers tend to be over-represented in CpG shores (p=9.3E-6).

Significant differences between positive and negative markers exist when it comes to Polycomb-group protein binding: positively related CpGs are over-represented near Polycomb-group target genes (reflecting results from [10, 14]) while negative CpGs show no significant relationship.

*Chromatin state analysis*

Chromatin state profiling has emerged as a powerful means of genome annotation and detection of regulatory activity. It provides a systematic means of detecting cis-regulatory elements (given the central role of chromatin in mediating regulatory signals and controlling DNA access) and can be used for characterizing non-coding portions of the genome, which contribute to cellular phenotypes [29]. While individual histone modifications are associated with regulator binding, transcriptional initiation, enhancer activity, combinations of chromatin modifications can provide even more precise insight into chromatin state [29]. Ernst et al (2011) distinguish six broad classes of chromatin states, referred to as promoter, enhancer, insulator, transcribed, repressed, and inactive states. Within them, active, weak and poised promoters (states 1-3) differ in expression levels, while strong and weak enhancers (states 4-7) differ in expression of proximal genes. The 193 positively related CpGs are more likely to be in poised promoters (chromatin state 3 regions) while the 160 negatively related CpGs are more likely to be either in weak promoters (chromatin state 2) or strong enhancers (chromatin state 4).

*Age acceleration is highly heritable*

Several authors have found that DNA methylation levels are under genetic control [24, 26, 30-32]. Since many age-related diseases are heritable, it is interesting to study to whether age acceleration (here defined as difference between DNAm age and chronological age) is heritable as well. The broad sense heritability of age acceleration is estimated using Falconer's formula, $H^2=2(cor(MZ)-cor(DZ))$, in two twin data sets that included both monozygotic (MZ) and dizygotic (DZ) twins.

An illustrative experiment estimating the heritability of age acceleration found that the broad sense heritability of age acceleration was 100% in newborns and 39% in older subjects, which suggests that non-genetic factors become more relevant later in life.

*Aging effects on gene expression (messenger RNA) levels*

Since DNA methylation is an important epigenetic mechanism for regulating gene expression levels (messenger RNA abundance), it is natural to wonder how age-related DNAm changes relate to those observed in gene expression levels. It has been found that there is very little overlap. Further, age effects on DNAm levels have not been found to affect genes known to be differentially expressed between naive CD8 T cells and CD8 memory cells. These non-significant results reflect the fact that the relationship between DNAm levels and expression levels is complex [33, 34].

*Age effects on individual CpGs*

In this example, for each CpG, the median DNAm level in subjects younger than 35 and in subjects older than 55 is examined (Example 9). The age-related change in beta values is typically small (the average absolute difference across the 354 CpGs is only 0.032). The weak age effect on individual clock CpGs can also be observed in a heat map that visualizes how the DNAm levels change across subjects. Few vertical bands in the heat map suggest that the clock CpGs are relatively robust against tissue and data set effects.

*The changing ticking rate of the epigenetic clock*

The linear combination of the 354 clock CpGs (resulting from the regression coefficients) varies greatly across ages. There is a logarithmic dependence until adulthood which slows to a linear dependence later in life (see formula in Example 8). The rate of change is interpreted as the ticking rate of the epigenetic clock. Using this terminology, it has been found that organismal growth (and concomitant cell division) leads to a high ticking rate which slows down to a constant ticking rate (linear dependence) after adulthood.

*DNAm age does not measure mitotic age or cellular senescence*

Since epigenetic somatic errors in somatic replications appear to be readily detected as age-related changes in methylation [35, 36], it is a plausible hypothesis that DNAm age measures the number of somatic cell replications. In other words, that it measures mitotic age (which assigns a cell copy number to every cell) [35, 37]. While DNAm age is correlated with cell passage number and the clock ticking rate is highest during organismal growth, it is clearly different from mitotic age since it tracks chronological age in non-proliferative tissue (e.g. brain tissue) and assigns similar ages to both short and long lived blood cells.

One explanation is that DNAm age is a marker of cellular senescence. This turns out to be wrong as can be seen from the fact that DNAm age is highly related to chronological age in immortal, non-senescent cells, e.g. immortalized B cells (Figure 17T). Further, DNAm age and cell passage number are highly correlated in ES cells which are also immortal [38].

<u>Example 6: Model: DNAm age measures the work done by an epigenetic maintenance system</u>

It is proposed that DNAm age measures the cumulative work done by a particular kind of epigenetic maintenance system (EMS), which helps maintain epigenetic stability. While epigenetic stability is related to genomic stability, it is useful to distinguish these two concepts. If the EMS model of DNAm age is correct then this particular kind of EMS appears to be inactive in the perfectly young ES cells. Maintenance methyltransferases are likely to play an important role. In physics, "work" is defined by the integral of power over time. Using this terminology, it is hypothesized that the power (defined as rate of change of the energy spent by this EMS) corresponds to the tick rate of the epigenetic clock. This model would explain the high tick rate during organismal development since a high power is required to maintain epigenetic stability during this stressful time. At the end of development, a constant amount of power is sufficient to maintain stability leading to a constant tick rate.

If this EMS model of DNAm age is correct then DNAm age should be accelerated by many perturbations that affect epigenetic stability. Further, age acceleration should have some beneficial effects given the protective role of the EMS. In particular, the EMS model of DNAm age entails the following testable predictions. First, cancer tissue should show signs of positive or negative accelerated age, reflecting the actions of the EMS. Second, many mitogens, genomic aberrations, and oncogenes, which trigger the response of the EMS, should be associated with accelerated DNAm age. Third, high age acceleration of cancer tissue should be associated with fewer somatic mutations given the protective role of the EMS. Fourth, mutations in TP53 should be associated with a lower age acceleration of cancer tissue if one further assumes that p53 signaling helps trigger the EMS. All of these model predictions turn out to be true as will be shown in the following cancer applications.

*DNAm age of cancer tissue versus tumor morphology*

A large collection of cancer data sets was assembled comprising n=5826 cancer samples from 32 individual cancer data sets (Example 10). Details on the cancer data sets can be found in Example 8. While some cancer tissues show relatively large correlations between DNAm age and patient age, the correlation between DNAm age and chronological age tends to be weak. Some cancer types exhibit increased age acceleration while others exhibit negative age acceleration. Tumor morphology (grade and stage) has only a weak relationship with age acceleration in most cancers: only 4 out of 33 hypothesis tests led to a nominally ($p<0.05$) significant result. Only the negative correlation between stage and age acceleration in thyroid cancer remains significant after applying a Bonferroni correction.

*Cancer tissues with high age acceleration exhibit fewer somatic mutations*

Strikingly, the number of mutations per cancer sample tends to be inversely correlated with age acceleration, which may reflect that DNAm age acceleration results from processes that promote genome stability. Specifically, a significant negative relationship between age acceleration and the number of somatic mutations can be observed in the following seven affected tissues/cancers: bone marrow (AML data from TCGA), breast carcinoma (BRCA data), kidney renal cell carcinoma (KIRC), kidney renal papillary cell carcinoma (KIRP), ovarian cancer (OVAR), prostate (PRAD), and thyroid (THCA). Similar results can also be observed in several breast cancer types.

*TP53 mutations are associated with lower age acceleration*

Strikingly, TP53 was among the top 2 most significant genes in 4 out of the 13 cancer data sets whose mutation has the strongest effect on age acceleration. Further, TP53 mutation is associated with significantly lower age acceleration in five different cancer types including AML, breast cancer, ovarian cancer, and uterine corpus endometrioid. Further, marginally significant result can be observed in lung squamous cell carcinoma and colorectal cancer (below). Only one cancer type (GBM) was found where mutations in TP53 are associated with a nominally significant increased age acceleration. Overall, these results suggest that p53 signaling can trigger processes that accelerate DNAm age.

*Somatic mutations in steroid receptors accelerate DNAm age in breast cancer*

In the following, DNAm age changes across different breast cancer types are shown. Somatic mutations in steroid receptors have a pronounced effect on DNAm age in breast cancer samples: samples with a mutated estrogen receptor (ER) or mutated progesterone receptor (PR) exhibit a much higher age acceleration than ER- or PR-samples in four independent data sets. In contrast, HER2/neu amplification has no significant relationship with age acceleration. Age acceleration differs greatly across different breast cancer types: Luminal A tumors (typically ER+ or PR+, HER2-, low Ki67), show the highest positive age acceleration. Luminal B tumors (typically ER+ or PR+, HER2+ or HER2- with high Ki67) show a similar effect. The lowest age acceleration can be observed for basal-like tumors (often triple negative ER-, PR-, HER2-) and HER2 type tumors (typically HER2+,ER-,PR-).

*Proto-oncogenes affect DNAm age in colorectal cancer*

Colorectal cancer samples with a BRAF (V600E) mutation are associated with an increased age acceleration whereas samples with a K-RAS mutation have a decreased age acceleration. Echoing previous results, TP53 mutations appear to be associated with decreased age acceleration. Promoter hypermethylation of the mismatch repair gene MLH1 leads to the most significant increase in age acceleration, which supports the EMS model of DNAm age. The CpG island methylator phenotype, defined by exceptionally high cancer-specific DNA hypermethylation [39], is also significantly associated with age acceleration, which may reflect its association with MLH1 hypermethylation and BRAF mutations.

*DNAm age in glioblastoma multiforme (GBM)*

In general, the CpG island methylator phenotype and age acceleration measure different properties as can be seen in glioblastoma multiforme.

Interestingly, age acceleration in GBM samples is highly significantly associated with certain mutations in H3F3A, which encodes the replication-independent histone variant H3.3. These mutations are single-nucleotide variants (SNV) changing lysine 27 to methionine (K27M) or changing glycine 34 to arginine (G34R) [40]. The fact that GBMs with a G34R mutation in H3F3A have a much higher age acceleration than those with a K27M mutation makes sense since each H3F3A mutation defines an epigenetic subgroup of GBM with a distinct global methylation pattern and acts through a different set of genes [40]. Lysine 27 is a critical residue of histone 3 variants, and methylation at this position (H3K27me), which may be mimicked by the terminal $CH_3$ of methionine substituted at this residue [40], is commonly associated with transcriptional repression [41] while H3K36 methylation or acetylation typically promotes gene transcription [42]. G34-mutant cells exhibit increased RNA polymerase II binding, increased gene expression, most notably that of the oncogene MYCN [43]. Both H3F3A mutations are mutually exclusive with IDH1 mutations, which characterize a third mutation-defined subgroup [44]. Age acceleration in GBM samples is also associated with the following genomic aberrations: TP53 mutation, ATRX mutation, chromosome 7 gain, chromosome 10 loss, CDKN2A del, and EGFR amplification. Reflecting these results for individual markers, age acceleration varies significantly across the GBM subtypes defined in [44].

*DNAm age of cancer cell lines.*

Using seven publicly available cell line data sets (Example 10), the DNAm age of 59 different cancer cell lines (from bladder, breast, gliomas, head/neck, leukemia, and osteosarcoma) was estimated. Across all cell lines, it was found that DNAm age does not have a significant correlation with the chronological age of the patient from whom the cancer cell line was derived. However, a marginally significant age correlation can be observed across osteosarcoma cell lines (cor=0.41, p=0.08). Overall, DNAm age acceleration varies greatly across the cancer lines (Example 11): the highest values can be observed for AML cell lines (KG1A: 182 years, HL-60: 177 years); the lowest values for head/neck squamous cell carcinoma cell line (UPCI SCC47: 6 years) and two breast cancer cell lines (SK-BR-3: 8 years, MDA-MB-468: 11 years).

*Conclusions*

Through the generosity of hundreds of researchers, an unprecedented collection of DNA methylation data from healthy tissues, cancer tissues, and cancer cell lines were analyzed. The healthy tissue data allowed for the development of a multi-tissue predictor of age (mathematical details are provided in Example 8). Relevant software can be accessed from [45]. A brief software tutorial is also presented in Example 8. The basic approach of the multi-tissue predictor of age is to form a weighted average of 354 clock CpGs (Table 3), which is then transformed to DNAm age using a calibration function. The calibration function reveals that the epigenetic clock has a high tick rate until adulthood after which it slows to a constant tick rate.

It is proposed that DNAm age measures the cumulative work done by an epigenetic maintenance system. This novel epigenetic clock can be used to address a host of questions in developmental biology, cancer-, and aging research. This EMS model of DNAm age leads to several testable model predictions which have been validated using cancer data. But irrespective of the validity of the EMS model, the findings in cancer are interesting in their own right. Overall, high age acceleration is associated with fewer somatic mutations in cancer tissue. Mutations in TP53 are associated with lower DNAm age. To provide a glimpse of how DNAm age can inform cancer research, DNAm age has been related to several widely used genomic aberrations in breast cancer, colorectal cancer, glioblastoma multiforme, and acute myeloid leukemia.

DNAm age is a promising marker for studying human development, aging, and cancer. It may become a useful surrogate marker for evaluating rejuvenation therapies. The most salient feature of DNAm age is its applicability to a broad spectrum of tissues and cell types. Since it allows one to contrast the ages of different tissues from the same subject, it can be used to identify tissues that show evidence of accelerated age due to disease (e.g. cancer). It is likely that the DNAm age of easily accessible fluids/tissues (e.g. saliva, buccal cells, blood, skin) can serve as surrogate marker for inaccessible tissues (e.g. brain, kidney, liver). It is noteworthy that DNAm age is applicable to chimpanzee tissues. Given the high heritability of age acceleration in young subjects, it is expected that age acceleration will mainly be a relevant measure in older subjects. Using a relatively small data set, no evidence was found that a premature aging disease (progeria) is associated with accelerated DNAm age (Figure 17T). Example 8, further describes if DNAm age fulfills the biomarker criteria developed by the American Federation for Aging Research.

Future research will need to clarify whether DNAm age is only a marker of aging or relates to an effector of aging. In conclusion, the epigenetic clock described here is likely to become a valuable addition to the telomere clock.

Example 7: Materials and methods

*Definition of DNAm age using a penalized regression model*

Using the training data sets, a penalized regression model (implemented in the R package glmnet [46]) is used to regress a log transformed version of chronological age on 21369 CpG probes which a) were present both on the Illumina™ 450K and 27K platform and b) had fewer than 10 missing values. The alpha parameter of glmnet was chosen to 0.5 (elastic net regression) and the lambda value was chosen using cross validation on the training data (lambda=0.0226). DNAm age was defined as predicted age. Mathematical details are provided in Example 8.

*Short description of the healthy tissue data sets*

All data are publicly available. Many data sets involve normal adjacent tissue from The Cancer Genome Data Base (TCGA). Details on the individual data sets can be found in Example 8. Briefly, relevant citations include: Data sets 1 and 2 (whole blood samples from a Dutch population) were generated by Roel Ophoff [14]. Data set 3 (whole blood) consists of whole blood samples from a recent large scale study of healthy individuals [24]. The authors used these and other data to estimate human aging rates and developed a highly accurate predictor of age based on blood data. Data set 4 leukocyte samples from healthy male children from Children's Hospital Boston [47]. Data set 5 peripheral blood leukocytes samples [48]. Data set 6 cord blood samples from newborns [30]. Data set 7 cerebellum samples were provided by C. Liu and C. Chen (GEO identifier GSE38873). Data set 8, 9, 10, 13 cerebellum, frontal cortex, pons, temporal cortex samples obtained from the same subjects [49]. Data set 11 prefrontal cortex samples from healthy controls [22]. Data set 12 neuron and glial cell samples from [50]). Data set 14 normal breast tissue samples [51]. Data set 15 buccal cells involved 109 fifteen-year-old adolescents from a longitudinal study of child development [52]. Data set 16 buccal cells from 8 different subjects [15]). Data set 17 buccal cells from monozygotic (MZ) and dizygotic (DZ) twin pairs from the Peri/postnatal Epigenetic Twins Study (PETS) cohort [53]. Data set 18 cartilage (chondrocyte) samples from [54]. Data set 19 normal adjacent colon tissue from TCGA. Data set 20 colon mucosa samples from [55]. Data set 21 dermal fibroblast samples from [21]. Data set 22 epidermis samples from [56]. Data set 23 gastric tissue samples from [57]. Data set 24 head/neck normal adjacent tissue samples from the TCGA data base (HNSC data). Data set 25 heart tissue samples from [58]. Data set 26 normal adjacent renal papillary tissue from TCGA (KIRP data). Data sets 27 normal adjacent tissue from TCGA (KIRC data). Data set 28 normal adjacent liver samples from [59]. Data set 29 normal adjacent lung tissue from TCGA data base (LUSC data). Data set 30 normal adjacent lung tissue samples from TCGA (LUAD data). Data set 31 from TCGA (LUSC). Data set 32 mesenchymal stromal cells isolated from bone marrow [60]. Data set 33 placenta samples from mothers of monozygotic and dizygotic twins [61]. Data set 34 prostate samples from [62]. Data set 35 normal adjacent prostate tissue from TCGA (PRAD data). Data set 36 male saliva samples from [63]. Data set 37 male saliva samples from [23]. Data set 38 stomach from TCGA (STAD data). Data set 39 thyroid TCGA (THCA data). Data set 40 WB from type 1 diabetics from [10, 64]. Data set 41 WB from [15]. Data sets 42 and 43 involve whole blood samples from women with ovarian cancer and healthy controls, respectively. These are the samples from the United Kingdom Ovarian Cancer Population Study [10, 64]. Data set 44 WB from [65]. Data set 45 leukocytes from healthy children of the Simons Simple Collection [47]. Data set 46 peripheral blood mononuclear cells from [66]. Data set 47 peripheral blood mononuclear cells from [67]. Data set 48 cord blood samples from newborns provided by N Turan and C Sapienza (GEO GSE36812). Data set 49 cord blood mononuclear cells from [68]. Data set 50 cord blood mononuclear cells from [61]. Data set 51 CD4 T cells from infants [69]. Data set 52 CD4+T cells and CD14+monocytes from [15]. Data set 53 immortalized B cells and other cells from progeria, Werner syndrome patients, and controls [70]. Data set 54 and 55 are brain samples from [71]. Data set 56 and 57 breast tissue from TCGA (27K and 450K platform, respectively). Data set 58 buccal cells from [72]. Data set 59 colon from TCGA (COAD data). Data set 60 fat (adipose) tissue from [73]. Data set 61 human heart tissue from [27]. Data set 62 kidney (normal adjacent) tissue from TCGA (KIRC). Data set 63 liver (normal adjacent tissue) from TCGA data base (LIHC data). Data set 64 lung from TCGA. Data set 65 muscle tissue from [73]. Data set 66 muscle tissue from [74]. Data set 67 placenta samples from [75]. Data set 68 female saliva samples [63]. Data set 69 uterine cervix samples from [51, 76]. Data set 70 uterine endometrium (normal adjacent) tissue from TCGA (UCEC data). Data set 71 various human tissues from the ENCODE/HAIB Project (GEO GSE40700). Data set 72 chimpanzees and human tissues from [27]. Data set 73 great ape blood samples from [28]. Data set 74 sperm samples from [77]. Data set 75 sperm samples from [78]. Data set 76 vascular endothelial cells from human umbilical cords from [61]. Data sets 77 and 78 (special cell types) involved human embryonic stem cells, iPS cells, and somatic cell samples measured on the Illumina™ 27K array and Illumina™ 450K array, respectively [79]. Data set 79 reprogrammed mesenchymal stromal cells from human bone marrow (iP-MSC), initial MSC, and embryonic stem cells [80]. Data set 80 human ES cells and normal primary tissue from [81]. Data set 81 human ES cells from [82]. Data set 82 blood cell type data from [83].

*Description of the cancer data sets*

All data are publicly available as can be seen from the column that reports GSE identifiers from the Gene Expression Omnibus (GEO) database and other online resources. Most cancer data sets came from the TCGA data base. Data set 3 glioblastoma multiforme from [44]. Data set 4 breast cancer from [84]. Data set 5 breast cancer from [85]. Data set 6 breast cancer from [51]. Data set 10 colorectal cancer from [39]. Data set 23 prostate cancer from [62]. Data set 30 urothelial carcinoma from [86]. More details of the cancer tissue and cancer cell line data sets can be found in Examples 8 and 10.

*DNA methylation profiling and normalization steps*

All of the public Illumina™ DNA data were generated by following the standard protocol of Illumina™ methylation assays, which quantifies DNA methylation levels by the $\beta$ value. A detailed description of the pre-processing and data normalization steps is provided in Example 8.

*Meta analysis for measuring pure age effects (irrespective of tissue type)*

The *metaAnalysis* R function in the WGCNA R package [87] is used to measure pure age effects as detailed in Example 8.

*Analysis of variance for measuring tissue variation*

To measure tissue effects in the training data, analysis of variance (ANOVA) is used to calculate an F statistic as follows. First, a multivariate regression model was used to regress each CpG (dependent variable) on age and tissue type. The analysis adjusted for age since the different data sets have very different mean ages. Next, ANOVA based on the multivariate regression model was used to calculate an F statistic, *F.tissueTraining*, for measuring the tissue effect in the training data. This F statistic measures the tissue effect after adjusting for age in the training data sets. The F statistic was not translated into a corresponding p-value since the latter turned out to be extremely significant for most CpGs. *F.tissueTraining* is shown to be highly correlated with an independent measure of tissue variance (defined using adult somatic tissues from data set 77).

*Characterizing the CpGs using sequence properties*

Occupancy counts for Polycomb-group target (PCGT) genes was studied since they have an increased chance of becoming methylated with age compared to non-targets [10]. Toward this end, the occupancy counts of Suz12, Eed, and H3K27me3 published in [88] were used. To obtain the protein binding site occupancy throughout the entire nonrepeat portion of the human genome, Lee et al. 2006 isolated DNA sequences bound to a particular protein of interest (for example, Polycomb-group protein SUZ12) by immunoprecipitating that protein (chromatin immunoprecipitation) and subsequently hybridizing the resulting fragments to a DNA microarray. More details on the chromatin state data from [29] can be found in Example 8.

ABBREVIATIONS

AML-acute myeloid leukemia (AML),
BLCA-bladder urothelial carcinoma,
CBMC-cord blood mononuclear cell
CESC-cervical squamous cell carcinoma and endocervical adenocarcinoma
COAD-colon adenocarcinoma
CpG: Cytosine phospate Guanin

ES-embryonic stem
EMS-epigenetic maintenance system
GBM-glioblastoma multiforme
GEO-Gene Expression Omnibus data base
HNSC-head/neck squamous cell carcinoma
HUVEC cell- human umbilical vascular endothelial cells
iPS- induced pluripotent cell
KIRC-kidney renal clear cell carcinoma
KIRP-kidney renal papillary cell carcinoma
LIHC-liver hepatocellular carcinoma
LOO-leave one data set out
MSC-mesenchymal stromal cell
OVAR- ovarian serous cystadenocarcinoma
PBMC- peripheral blood mononuclear cell
PRAD-prostate adenocarcinoma
READ-rectum adenocarcinoma
SARC-sarcoma
TCGA-The Cancer Genome Atlas
THCA-thyroid carcinoma
SCM-skin cutaneous melanoma
UCEC-uterine corpus endometrioid carcinoma
WB-whole blood

Example 8: Materials and Methods Supplement

(Note: This example references an additional number of different publications as indicated throughout by reference numbers enclosed in braces, e.g., {x}. A list of these different publications ordered according to these reference numbers can be found in the section below entitled "*Example 8 References*".)

The following reasons may explain the remarkable accuracy of the age predictor in the test data sets. First, measurements from Illumina™ DNA methylation arrays (Methods) are known to be less affected by normalization issues than those from gene expression (mRNA) arrays and even non-normalized beta-values (Methods) turn out to be highly correlated with corresponding measures found using pyrosequencing {1-3}. Second, the penalized regression model automatically selected CpGs that are relatively robust since it was trained on data sets from different labs and platforms. Third, the large number of data sets helped average out spurious results and artifacts. Fourth, age has a profound effect on the DNAm levels of tens of thousands of CpGs as shown by many authors {4-13}.

The results of this article do not contradict previous studies that have noted age-related DNA methylation changes which occur in a tissue specific manner, e.g. {14, 15}. Instead, the results of this article demonstrate that one can use a couple of hundred CpGs for forming an age predictor that a) performs remarkably well across a broad spectrum of human tissues and b) the resulting DNAm age estimate is biologically meaningful.

*Description of the healthy tissue and cell line data sets*

Data sets 1 and 2 (whole blood samples from a Dutch population) are comprised of schizophrenics and healthy control subjects measured on the Illumina™ 27K and 450K array platform, respectively. These data from Dr. Roel Ophoff's lab were formerly used to find co-methylation modules related to age {13}. The current study has a different aim, namely the development of an age predictor based on methylation levels. Since schizophrenia status had a negligible effect on age relationships {13}, it was ignored in this analysis. Further, it turned out that schizophrenia status was not related to DNAm age. GEO identifier of the data is GSE41037.

Data set 3 (whole blood) consists of whole blood samples from a recent large scale study of healthy individuals {16}. The authors used these data (and additional data) to estimate human aging rates and developed a highly accurate predictor of age based on blood data.

Data set 4 (leukocytes from healthy male children from Children's Hospital Boston) consists of 72 peripheral blood leukocyte samples from healthy males (mean age 5, range 1-16) {17}.

Data set 5 (peripheral blood leukocytes) from a DNAm study of Crohn's disease and ulcerative colitis {18}. Illumina™ 450K were used on 48 samples of peripheral blood leukocyte (PBL) DNA from discordant MZ twin pairs (CD: 3; UC: 3) and treatment-naive pediatric cases of IBD (CD: 14; UC: 8), as well as controls (n = 14). I ignored disease status in the analysis. I did not find significant evidence that disease status affects DNAm age in this moderately sized data set.

Data set 6 (cord blood from newborns) is comprised of cord blood samples from 216 subjects (of age zero) {19}.

Data set 7 (cerebellum) is comprised of postmortem cerebellum brains. The data were provided by C. Liu and C. Chen (GEO identifier GSE38873).

Data set 8, 9, 10, 13 (cerebellum, frontal cortex, pons, temporal cortex) consist of brain tissue samples obtained from the same subjects whose mean age was 49 (range 15-101) {20}. These subjects, who had donated their brains for research, were of non-Hispanic, Caucasian ethnicity, and none had a clinical history of neurological or cerebrovascular disease, or a diagnosis of cognitive impairment during life. Demographics, tissue source and cause of death for each subject are reported in {20}. Unbiased removal of potential outliers (as described in the section on sample pre-processing) reduced the number of retained samples.

Data set 11 (prefrontal cortex from healthy controls) consists of 108 samples (mean age 26, ranging from samples before birth up to age 84) {21}. These post-mortem human brains from non-psychiatric controls were collected at the Clinical Brain Disorders Branch (National Institute of Mental Health). The DNAm data are publicly available from the webpage of the standalone package BrainCloudMethyl, which can be downloaded from the following URL http://braincloud.jhmi.edu/Methylation32/BrainCloudMethyl.htm

Data set 12 (neuron and glial cells) from {22}. The authors developed a cell epigenotype specific model for the correction of brain cellular heterogeneity bias and applied it to study age, brain region and major depression. After performing fluorescence activated cell sorting (FACS) of neuronal nuclei in post mortem frontal cortex 58 samples (29 major depression and 29 matched control samples) followed by Illumina™ HM450 microarray based DNAm profiling, the authors characterized the extent of neuron and glia specific DNAm variation independent of disease status and identified significant cell type specific epigenetic variation at 51% of loci. I ignored disease status in the analysis. I found no evidence that disease status accelerated age in this data set.

Data set 14 (breast) consists of normal breast tissue from 23 females (mean age 48, range 19-75) downloaded from GEO {23}.

Data set 15 (buccal cells) involved 109 fifteen-year-old adolescents from a longitudinal study of child development {24}. While the authors found that DNA derived from buccal epithelial cells showed differential methylation among adolescents whose parents reported high levels of stress during their children's early lives, parental stress was ignored. All samples have the same chronological age (15 years).

Data set 16 (buccal cells) involved 8 different subjects. Rakyan et al (2010) confirmed that these buccal cell preparations contained very little, if any, leukocyte contamination, hence showing that the measured methylation profiles were predominantly from buccal cells {25}.

Data set 17 (buccal cells) from {26}. The authors applied the Illumina™ 450K platform to buccal swabs from 10 monozygotic (MZ) and 5 dizygotic (DZ) twin pairs from the Peri/postnatal Epigenetic Twins Study (PETS) cohort. In this longitudinal study, DNAm profiles were generated at birth (age 0) and at age 1.5 years (18 months).

Data set 18 (cartilage, chondrocytes) from {27}. The authors analyzed human articular chondrocytes from osteoarthritic patients and healthy cartilage samples. I did not find a relationship between disease status and accelerated DNAm age.

Data sets 19 (colon, normal tissue) consists of samples downloaded from TCGA data base measured on the Illumina™ 27K array.

Data set 20 (colon mucosa) from {28}. Crohn's disease, ulcerative colitis, and normal colon mucosa samples were measured on the Illumina™ Infinium HumanMethylation450 BeadChip v1.1. Samples came from 9 Crohn's disease affected, 5 ulcerative colitis affected, and 10 normal individuals. I did not detect a significant relationship between disease status and DNAm age acceleration.

Data set 21 (dermal fibroblasts) consists of 14 female fibroblast samples (mean age 32, range 6-73). The samples came from different locations on the human body (5 abdomen, 2 arm, 2 breast, 3 ear, and 2 leg samples) {2}. The single blepharoblast sample was removed from this data set since hierarchical clustering (based on the Euclidean distance, single linkage) indicated that it was an outlier.

Data set 22 (epidermis) came from a study that evaluated the epigenetic effects of aging and chronic sun exposure {29}. I used the 10 epidermal samples collected using suction blistering.

Data set 23 (gastric tissue) from {30}. The Illumina™ HumanMethylation27 BeadChip was used to obtain DNAm profiles across 27,578 CpGs in 203 gastric tumors and 94 matched non-malignant gastric samples. I focused on matched control samples.

Data set 24 (head/neck normal adjacent tissues) measured on the Illumina™ 450K platform from the TCGA data base (HNSC data).

Data set 25 (heart tissue) {31}. The authors generated DNAm profiles from human left ventricular myocardium DNA in order to study alterations in cardiac DNAm in human dilated cardiomyopathy (DCM). There were n = 8 controls (patients after heart transplantation) and n = 9 patients with idiopathic DCM. I ignored disease status in the analysis. I could find no significant evidence that disease status affects DNAm age in this small data set.

Data sets 26 (renal papillary, normal tissue) consists of 44 samples (mean age 66) downloaded from TCGA data base (KIRP) measured on the Illumina™ 450K array.

Data sets 27 (adjacent normal tissue, kidney measured on the Illumina™ 450K array) from TCGA (Kidney Clear Cell Renal Carcinoma, KIRC).

Data set 28 (liver) consists of normal adjacent tissue samples from Taiwanese hepatocellular carcinoma subjects {32}. The data were downloaded from GEO (GSE37988).

Data set 29 (lung squamous cells from normal adjacent tissue) consists of samples downloaded from TCGA data base (normal from LUSC) that were measured on the Illumina™ 27K array.

Data set 30 (lung normal adjacent lung tissue, Illumina™ 27K) from the Cancer Genome Atlas (TCGA) data base (http://tcga-data.nci.nih.gov/), LUAD

Data sets 31 (lung squamous cells from normal adjacent tissue measured on the Illumina™ 450K) from the TCGA data base (normal samples from LUSC).

Data set 32 (mesenchymal stromal cells from bone marrow) consists of 16 female samples (mean age 53, range 21-85) {33}. The MSC from human bone marrow were either isolated from bone marrow aspirates or from the caput femoris upon hip fracture of elderly donors {33}. Due to sample size constraints, cell passage status (reflecting short versus long term culture) was ignored.

Data set 33 (placenta) from mothers of monozygotic and dizygotic twins {34}. Since placenta only develops during pregnancy, its chronological age was set to zero.

Data set 34 (prostate) consists of 69 normal prostate samples (mean age 61) {35}.

Data set 35 (prostate, normal adjacent tissue) measured on the Illumina™ 450K platform from the TCGA data base (PRAD data).

Data set 36 (saliva from alcoholic males) is from {36} as data set 68, but involves 131 male samples (again with mean age 32, range 21-55). Thus, I split the original data by gender.

Data set 37 (saliva from healthy men) involved 69 healthy male samples (mean age 35, range 21-55). We used these twin pairs and triplets to develop a saliva based predictor of age {3}. Since all twins were monozygotic, I could not use these data to estimate heritability with Falconer's formula.

Data sets 38 (stomach normal adjacent tissue measured on the Illumina™ 27K array) consists of 41 samples (mean age 69) downloaded from TCGA data base (STAD data).

Data set 39 (thyroid, normal adjacent tissue) measured on the Illumina™ 450K platform from the TCGA data base (THCA data).

Data set 40 (WB from type 1 diabetics) consists of samples from 191 subjects (mean age 44, range 24-74) {12, 37}. Since all subjects had type 1 diabetes, disease status was ignored. These data were downloaded from GEO (GSE20067).

Data set 41 (WB from healthy females) consists of 93 whole blood samples from women whose mean age was 63 (range 49-74) {25}. The samples were collected from different healthy females (both twin pairs and singletons).

Data set 42 (WB from postmenopausal women) consists of 262 whole blood samples from women with ovarian cancer (mean age 66 , range 49-91). These are the cases from the UKOPS data (see data set 43). These samples were used since ovarian cancer did not have a global effect on blood methylation levels {12, 37}.

Data set 43 (WB from healthy postmenopausal women) consists of 269 whole blood samples from women with a mean of 65 (range 52-78) {12, 37}. While the data come from the United Kingdom Ovarian Cancer Population Study (UKOPS), it is important to emphasize that the samples come from healthy age matched controls of ovarian cancer patients. The data were downloaded from GEO (GSE19711).

Data set 44 (WB from rheumatoid arthritis) from a differential DNAm study of rheumatoid arthritis {38}. The authors found DNAm could serve as an intermediary of genetic risk in rheumatoid arthritis. I ignored disease status in the analysis. I did find that the whole blood of rheumatoid arthritis patients showed evidence of negative age acceleration compared to controls. While the large sample size led to a statistically significant (p=0.0049) finding, the effect size (age difference of 1.2 years) appears to be negligible.

Data set 45 (leukocytes from healthy children of the Simons Simple Collection) consists of peripheral blood leukocyte samples from 386 healthy (mostly male) subjects (mean age 10, range 3-17). These are healthy siblings of subjects with autism spectrum disorder (ASD) {17}.

Data set 46 (peripheral blood mononuclear cells from newborns and nonagenarians) {39} can be downloaded from GEO GSE30870.

Data set 47 (peripheral blood mononuclear cells) collected from a community-based cohort stratified for early-life socioeconomic status {40}. The data were downloaded from GEO (GSE37008). The authors found that psychosocial factors, such as perceived stress, and cortisol output were associated with DNAm patterns, as was early-life socioeconomic status. But none of these factors turned out to be related to DNAm age which justified that these covariates were ignored in this study.

Data set 48 (cord blood samples from newborns) comes from a study that related DNAm data to birth weight. Incidentally, DNAm age did not appear to be correlated with birth weight. No citation appears to be available for these data that were submitted to GEO (GSE36812) by N Turan and C Sapienza.

Data set 49 (cord blood mononuclear cells) comes from a study that investigated the effects of periconceptional maternal micronutrient supplementation on infant blood methylation patterns from offspring of Gambian women enrolled into a randomized, double blind controlled trial {41}. No significant relationship between DNAm age and micronutrient supplementation status could be observed.

Data set 50 (cord blood mononuclear cells) is from monozygotic and dizygotic twins {34} but twin status was ignored in our analysis.

Data set 51 (CD4 T cells from infants) consisted of sorted CD4+ T cell samples. The authors used the data to investigate the dynamics and relationship between DNAm and gene expression during early T-cell development{42}. The mononuclear cells were collected from 24 infants at birth (n=12) and resampled at 12 months (n=12). CD4+ cells were purified and the DNA analyzed using Illumina™ Inf450K arrays. The data were downloaded from GEO (GSE34639).

Data set 52 (CD4+T cells and CD14+monocytes) consisted of sorted CD4+ T-cells and CD14+ monocytes from blood of an independent cohort of 25 healthy subjects {25}.

Data set 53 (immortalized B cells) and other cells from progeria and Werner syndrome patients and controls {43}. The Hutchinson-Gilford Progeria Syndrome (HGP) and Werner Syndrome are two premature aging diseases showing features of common aging. Mutations in LMNA and WRN genes are associated to disease onset; however for a subset of patients the underlying causative mechanisms remains elusive. In this study, the authors aimed to evaluate the role of epigenetic alteration on premature aging diseases by performing genome-wide DNAm profiling of HGP and WS patients. The authors analyzed Epstein-Bar virus (EBV) immortalized B cells, naive B-cells, and peripheral blood mononuclear cells. The authors found aberrant DNAm profiles in the premature aging disorders Hutchinson-Gilford Progeria and Werner syndrome {43}. In this relatively small data set, I found no evidence that these premature aging diseases accelerate DNAm age in immortalized B cells. Future studies could evaluate whether premature aging diseases are associated with accelerated DNAm age in other tissues or cell types. Interestingly, chronological age continued to be highly correlated with DNAm age in these immortalized B cells which suggests that immortalization via EBV does not have a major effect on DNAm age.

Data set 54 (cerebellar samples) and data set 55 (occipital cortex samples) from autism cases and controls {44}. The authors collected idiopathic autistic and control cerebellar and BA19 (occipital) brain tissues. Here we ignored autism disease status. Incidentally, we could not detect an association between autism status and DNAm age.

Data set 56 (breast, normal adjacent tissue, Illumina™ 450K) consists of normal breast tissue samples from 90 female breast cancer cases (mean age 57, range 28-90) from TCGA, but unlike data set 57 these samples were assayed on the Illumina™ 450K platform.

Data set 57 (breast, normal adjacent tissue, Illumina™ 27K) consists of normal breast tissue samples from 27 female breast cancer cases (mean age 55, range 35-88) from the Cancer Genome Atlas (TCGA) data base (http://tcga-data.nci.nih.gov/).

Data set 58 (buccal cells) from {45}. The authors performed a longitudinal study of DNA methylation at birth and age 18 months in DNA from buccal swabs from 10 monozygotic (MZ) and 5 dizygotic (DZ) twin pairs from the Peri/postnatal Epigenetic Twins Study (PETS) cohort.

Data sets 59 (colon) normal adjacent tissue measured on the Illumina™ 450K array, downloaded from TCGA (COAD data).

Data set 60 (adipose) from monozygotic Twins Discordant for Type 2 Diabetes. {46}. Monozygotic twins discordant for type 2 diabetes constitute an ideal model to study environmental contributions to type 2 diabetic traits. The authors aimed to examine whether global DNAm differences exist in major glucose metabolic tissues from twelve 53-80 year-old monozygotic discordant twin pairs. DNAm was measured by the Illumina™ HumanMethylation27 BeadChip in 22 (11 pairs) skeletal muscle and 10 (5 pairs) subcutaneous adipose tissue biopsies. Diabetes status was ignored in my analysis. I could find no significant evidence that disease status affects DNAm age in this small data set.

Data set 61 (heart tissue) consists of only 6 human male samples (mean age 61, range 55-71) {47}. Clearly, larger sample sizes will be needed to evaluate this tissue.

Data set 62 (kidney) normal adjacent tissue from clear cell renal carcinoma consists of samples downloaded from the TCGA data base (KIRC) that were measured on the Illumina™ 27K platform.

Data set 63 (liver normal adjacent tissues) measured on the Illumina™ 450K platform from the TCGA data base (LIHC data).

Data sets 64 (lung, normal adjacent tissue) measured on the Illumina™ 450K arrays. The data consists of samples downloaded from TCGA data base (normal from LUAD).

Data set 65 (muscle) from monozygotic Twins Discordant for Type 2 Diabetes {46}. Monozygotic twins discordant for type 2 diabetes constitute an ideal model to study environmental contributions to type 2 diabetic traits. The authors aimed to examine whether global DNAm differences exist in major glucose metabolic tissues from twelve 53-80 year-old monozygotic discordant twin pairs. DNAm was measured by the Illumina™ HumanMethylation27 BeadChip in 22 (11 pairs) skeletal muscle and 10 (5 pairs) subcutaneous adipose tissue biopsies. Diabetes status was ignored in my analysis. I could find no significant evidence that disease status affects DNAm age in this small data set.

Data set 66 (muscle) tissue from healthy men who were 24 years old. These data came from an epigenetic analysis of healthy young men following a control and high-fat overfeeding diet {48}. These data came from a randomized cross-over design, where all subjects received both treatments (control and high-fat overfeeding diet). Biopsies were obtained from 23 different individuals amounting to 22 samples following the control diet and 22 samples following the high-fat overfeeding diet (paired n=21). The resulting 44 samples were analyzed using the Illumina™ 27K platform. Diet status was ignored in my analysis. I could find no significant evidence that diet affects DNAm age in this relatively small data set.

Data set 67 (placenta) from {49}. DNA from 20 third trimester early onset preeclampsia placentas and 20 gestational age matched controls.

Data sets 68 (saliva) from alcoholic females involved 52 samples (mean age 32, range 21-55) {36}.

Data set 69 (uterine cervix) involved cytologically normal cells from the uterine cervix of 152 women {23, 50}.

Data set 70 (uterine endometrium normal adjacent tissue) measured on the Illumina™ 450K platform from the TCGA data base (UCEC data).

Data set 71 (various human tissues)from the ENCODE/HAIB Project. These Illumina™ 27K data were downloaded from GEO GSE40700.

Data set 72 (chimpanzees and humans) from {47} The authors used the Illumina™ 27K array to compare DNAm profiles in the following human and chimpanzee tissue samples: 6 human livers, 6 human kidneys, 6 human heart, 6 chimpanzee livers, 6 chimpanzee kidneys, and 6 chimpanzee hearts.

Data set 73 (ape blood) from {51}. The authors applied the Illumina™ 450K arrays to blood derived DNA from humans, chimpanzees, bonobos, gorillas and orangutans. Since ages were not available for humans and orangutans, I focused on chimpanzees, bonobos, gorillas for whom ages were available.

Data set 74 (sperm) from {52}. The authors performed a genome-wide analysis of sperm DNA isolated from 21 men with a range of semen parameters presenting to a tertiary male reproductive health clinic. DNAm was measured with the Illumina™ Infinium array at 27,000 CpG loci.

Data set 75 (sperm) from {53}. The authors applied the 450K platform to DNA derived from 26 normal sperm samples.

Data set 76 (vascular endothelial cells from human umbilical cords) from monozygotic and dizygotic twins {34}.

Data sets 77 and 78(special cell types) involved human embryonic stem cells, iPS cells, and somatic cell samples measured on the Illumina™ 27K array and Illumina™ 450K array, respectively {54}. Although no specific age information was available, these two valuable data sets could be used a) to compare adult somatic tissues versus fetal somatic tissues, b) to compare the DNAm ages of different tissues from the same individual (Figure 3), c) to assess the variance of methylation probes across adult somatic tissues and fetal somatic tissues, d) to study how the DNAm age of iPS cells compares to that of somatic primary tissue and primary cell lines (Figure 6), e) to evaluate how cell passaging effects DNAm age (Figure 6). Data set 78 contained multiple tissue samples from two adults. For data set 78, the following tissues and sample sizes were available: Adipose (n=2 samples), Adrenal (n=4), Aorta (2), Bladder (2), Blood (2), Brain (3), Breast (1), Colon (1), Diaphragm (2), Duodenum (1), human embryonic stem (ES) cells (118), Gallbladder (1), Heart (2), iPS (46), Kidney (2), Liver (1), Lung (4), Lymph Node (2), Ovary (2), Pancreas (2), Prostate (1), Skeletal Muscle (2), Skin (1), Small Intestine (1), Somatic Primary Cell Line (49), Spleen (3), Stomach (4), Tongue (1) Ureter (2). For data set 52, the following sample sizes were available {54} Adipose (2), Adrenal (5), Bladder (2), Blood (2), Brain (5), ES (19), Heart (5), iPSC (29), Kidney (5), Liver (4), Lung (7), Lymph Node (2), Pancreas (2), Skeletal Muscle (2), Somatic Primary Cell Line (22), Spleen (5), Stomach (6), Thymus (2), Tongue (2), Ureter (2).

Data set 79 (reprogrammed mesenchymal stromal cells from human bone marrow (iP-MSC), initial MSC, and embryonic stem cells) {55}. The authors reprogrammed mesenchymal stromal cells from human bone marrow (iP-MSC) and compared their DNAm profiles with initial MSC and embryonic stem cells (ESCs) using the Illumina™ 450K array. The data were downloaded from GEO (GSE37066).

Data set 80 (hESC and normal primary tissue) from {56}. The authors extracted DNA from the following well-characterized human embryonic stem cell (hESC)lines: SHEF-1, SHEF-4, SHEF-5, SHEF-7, H7, H14, H14S9, H7S14, HS181 and 13. The authors used DNA from human normal primary tissues provided by Biochain (Hayward, CA, USA).

Data set 81 (hESC) from {57}.DNA derived from H9, H13C, SHEF2 hESC cultured in two different media. The medium was not significantly related with DNAm age estimate.

Data set 82 (blood cell type data) {58} Six healthy male blood donors, age 38 $\pm$ 13.6 years, were included in the study. From each individual, global DNAm levels were analyzed in whole blood, peripheral blood mononuclear cells (PBMC) and granulocytes as well as for seven isolated cell populations (CD4+ T cells, CD8+ T cells, CD56+ NK cells, CD19+ B cells, CD14+ monocytes, neutrophils, and eosinophils), n=60 samples analyzed in total. The data were downloaded from GEO (GSE35069).

*Criteria guiding the choice of the training sets*

The choice of training data sets was guided by the following criteria: First, the training data should represent a wide spectrum of tissues and cell types. In this example, the training data involved blood (whole blood, cord blood, PBMCs), brain (cerebellum, frontal cortex, pons, prefrontal cortex, temporal cortex, neurons and glial cells), breast, buccal epithelium, cartilage, colon, dermal fibroblasts, epidermis, gastric tissue, head/neck tissue, heart, kidney, liver, lung, mesenchymal stromal cells, prostate, saliva, stomach, thyroid, etc.

Second, the individual training sets (that make up the combined training set) should have a similar age distribution. The training data should contain a high proportion of samples (37%) measured on the Illumina™ 450K platform since many on-going studies use this recent Illumina™ platform. Incidentally, 34% of test set samples were measured on the 450K platform. Here I only studied 21369 probes measured with the Infinium type II assay which satisfied the following criteria: a) they were present on both Illumina™ platforms (Infinium 450K and 27K) and b) had fewer than 10 missing values.

*Description of the cancer data sets*

Data set 3 (glioblastoma multiforme, GBM) measured on the Illumina™ 450K array from {59} (GEO identifier GSE36278).

Data set 4 (breast cancer) measured on the Illumina™ 27K array from {60} (GEO identifier GSE31979).

Data set 5 (breast cancer) measured on the Illumina™ 27K array from {61}(GEO identifier GSE20712).

Data set 6 (breast cancer) measured on the Illumina™ 27K array from {23} (GEO identifier GSE33510).

Data set 10 (colorectal cancer) measured on the Illumina™ 27K array from {62} (GEO identifier GSE25062).

Data set 23 (prostate cancer) measured on the Illumina™ 27K array from {35} (GEO identifier GSE26126).

Data set 30 (urothelial carcinoma) measured on the Illumina™ 27L array from {63}.

All other cancer data sets came from the TCGA data base. In particular, acute myeloid leukemia (AML), bladder urothelial carcinoma (BLCA), cervical squamous cell carcinoma and endocervical adenocarcinoma (CESC), colon adenocarcinoma (COAD), head/neck squamous cell carcinoma (HNSC), liver hepatocellular carcinoma (LIHC), kidney renal clear cell carcinoma (KIRC), kidney renal papillary cell carcinoma (KIRP), liver ovarian serous cystadenocarcinoma (OVAR), prostate adenocarcinoma (PRAD), rectum adenocarcinoma (READ), sarcoma (SARC), thyroid carcinoma (THCA), skin cutaneous melanoma (SKCM), uterine corpus endometrioid carcinoma (UCEC).

*DNAm profiling and pre-processing steps*

Full experimental methods and detailed descriptions of these public data sets can be found in the original references. The following briefly summarizes the main steps. Methylation analysis was performed either using the Illumina™ Infinium Human Methylation27 BeadChip {64} or the Illumina™ Infinium HumanMethylation450 BeadChip. The Illumina™ HumanMethylation27 BeadChips measures bisulfite-conversion-based, single-CpG resolution DNAm levels at 27,578 different CpG sites within 5' promoter regions of 14,475 well-annotated genes in the human genome. Data from the two platforms were merged by focusing on the roughly 26k CpG sites that are present on both platforms. The HumanMethylation27 BeadChip mainly represents specific CpG that are located near gene promoter regions.

All of the public data were generated by following the standard protocol of Illumina™ methylation assays, which quantifies DNAm levels by the $\beta$ value using the ratio of intensities between methylated (signal A) and un-methylated (signal B) alleles. Specifically, the $\beta$ value was calculated from the intensity of the methylated (M corresponding to signal A) and un-methylated (U corresponding to signal B) alleles, as the ratio of fluorescent signals $\beta = Max(M,0)/[Max(M,0)+Max(U,0)+100]$. Thus, $\beta$ values range from 0 (completely un-methylated) to 1 (completely methylated) {65}.

The mean inter-array correlation was used to measure how similar (correlated) a given sample is compared to the remaining samples of the data set. To ensure high quality data without technical artifacts, non-cancer samples were only used if their mean inter-array correlation was larger than 0.90 and if their maximum DNAm level (across all probes) was larger than 0.96. This filtering step was *not* applied to the cancer samples since it is well known that cancer greatly affects the DNAm levels. It is worth mentioning that my results would barely change if all samples had been used.

*Normalization methods for the DNA methylation data*

I carried out several normalization steps to ensure that these data are comparable. While quantile normalization is often used in gene expression studies, it is less frequently used in DNAm studies. Before explaining my unbiased normalization strategy, I briefly provide some background. The Illumina™ 450K platforms uses 2 different chemical assays. The Infinium I and Infinium II assays for the assessment of the DNAm status of more than 480,000 cytosines distributed over the whole genome. The older Illumina™ 27K platform only uses the Infinium II assays. Several authors have noted that the data generated by the two chemical assays used by the 450K platform are not entirely compatible {66}. Dedeurwaerder et al (2011) showed that their correction technique called 'peak-based correction', which rescales type II probes on the basis of type I probes greatly improved the signal in Illumina™ Inf450K data. Similarly, Maksimovic et al (2012) showed that their subset-quantile within array normalization (SWAN) substantially improves the results for the Illumina™ 450K platform {67}. Unfortunately, I could not adopt the SWAN normalization here since it requires idat input files, which were not available for many of the data sets.

Teschendorff et al (2012) developed a model-based intra-array normalization strategy for the 450K platform, called BMIQ (Beta MIxture Quantile dilation), which adjusts beta-values of type II probes into a statistical distribution characteristic of type I probes{68}.

My own studies support the claim of these authors that normalizing type II probes so that they correspond to type I probes is a very useful pre-processing step for any study using the Illumina™ 450K platform. I could not adopt these techniques directly since my study only involves type II probes from the 27K platform. About 26000 CpGs from the 27K platform are also represented on the 450K platform and have the same probe identifier. Therefore, it is straightforward to merge data from the two platforms as long as one restricts attention to these overlapping probes. The age predictor was trained on the roughly 21368 type II probes that a) are shared between the Illumina™ 27K and the 450K platforms and b) had <= 10 missing values across the training data. However, I adopted the idea underlying these articles as follows. Instead of using type I probes as gold standard for rescaling type II probes, I created another gold standard by forming the mean DNAm value in the largest single study of this article (data set 1, i.e. whole blood samples from {13}). Next, I adapted the BMIQ R function from Teschendorff et al (2013) {68} so that it would rescale the overlapping 21k probes of each array so that their distribution matched that of the new gold standard. My empirical studies showed that this pre-processing step improved the accuracy of the resulting age predictor especially when it comes to the median error. Even though only the 21k CpGs that overlap between the Illumina™ 27K and 450K array used in this illustrative example, it can be applied to any set of CpGs (e.g. all CpGs on the 450K array).

*Explicit details on the definition of DNAm age*

Based on the training set data, I found that it is advantageous to transform age before carrying out an elastic net regression analysis. Toward this end, I used the following novel function F for transforming age (though it is contemplated that other transformations may also possibly be used):

$$F(age)=log(age+1)-log(adult.age+1) \text{ if } age<= adult.age.$$

$$F(age)=(age-adult.age)/(adult.age+1) \text{ if } age> adult.age.$$

The parameter adult.age was set to 20 for humans (different values can also be chosen) and 15 for chimpanzees. Note that F satisfies the following desirable properties: it

i) is a continuous, monotonically increasing function (which can be inverted),
ii) has a logarithmic dependence on age until adulthood (here set at 20 years),
iii) has a linear dependence on age after adulthood (here set to 20),
iv) is defined for negative ages (i.e. prenatal samples) by adding 1 (year) to age in the logarithm,
v) it has a continuous first derivative (slope function). In particular the slope at age=adult.age is given by 1/(adult.age+1).

The function F is visualized by a red line. As expected, the red line passes through the weighted average of the CpGs (i.e. the linear part of the regression model). The inverse of the function F, denoted by inverse.F, is used to transform the linear part of the regression model into DNAm age.

An elastic net regression model (implemented in the glmnet R function) was used to regress a transformed version of age on the roughly 21k beta values in the training data. The elastic net regression results in a linear regression model whose coefficients bo, $b_1$, ..., $b_{354}$ relate to transformed age as follows

$$F(chronological\ age) = b_0 + b_1CpG_1 + ... + b_{354}CpG_{354} + \text{error}$$

The coefficient values can be found in Example 9. Based, on the coefficient values from the regression model, DNAmAge is estimated as follows

$$DNAmAge = inverse.F(b_0 + b_1CpG_1 + ... + b_{354}CpG_{354})$$

Thus, the regression model can be used to predict to transformed age value by simply plugging the beta values of the selected CpGs into the formula. The linear part, (i.e. the weighted average of the selected CpGs) is visualized as a red line.

The glmnet function requires the user to specify two parameters (alpha and beta). Since I used an elastic net predictor, alpha was set to 0.5. But the lambda value of 0.02255706 was chosen by applying a 10 fold cross validation to the training data (via the R function cv.glmnet).

The following R code provides details on the analysis.

```
library(glmnet)
# use 10 fold cross validation to estimate the lambda parameter
# in the training data
glmnet.Training.CV = cv.glmnet(datMethTraining, F(Age), nfolds=10,alpha=alpha,family="gaussian")
# The definition of the lambda parameter:

    lambda.glmnet.Training = glmnet. Training. CV$lambda.min
    # Fit the elastic net predictor to the training data
    glmnet.Training = glmnet(datMethTraining, F(Age), family="gaussian", alpha=0.5, nlambda=100)
    # Arrive at an estimate of of DNAmAge
    DNAmAgeBasedOnTraining=inverse. F(predict(glmnet. Training,datMeth,type="r
    esponse" ,s=lambda.glmnet.Training))
```

*Chromatin state data used*

While specific histone modifications correlate with regulator binding, transcriptional initiation and elongation, enhancer activity and repression, combinations of chromatin modifications can provide even more precise insight into chromatin state {69}. Here I used the chromatin state data from {69}. The authors profiled nine human cell types, including common lines designated by the ENCODE consortium and primary cell types. These consisted of embryonic stem cells (HI ES), erythrocytic leukemia cells (K562), B-lymphoblastoid cells (GM12878), hepatocellular carcinoma cells (HepG2), umbilical vein endothelial cells (HUVEC), skeletal muscle myoblasts (HSMM), normal lung fibroblasts (NHLF), normal epidermal keratinocytes (NHEK), and mammary epithelial cells (HMEC).

Ernst et al (2011) distinguish six broad classes of chromatin states, referred to as promoter, enhancer, insulator, transcribed, repressed, and inactive states. Within them, active, weak and poised promoters (states 1-3) differ in expression levels, strong and weak candidate enhancers (states 4-7) differ in expression of proximal genes, and strongly and weakly transcribed regions (states 9-11) also differ in their positional enrichments along transcripts. Similarly, Polycomb-repressed regions (state 12) differ from heterochromatic and repetitive states (states 13-15), which are also enriched for H3K9me3. It will be interesting to map the 354 clock CpGs to the states of individual cell lines. Since the number of profiled cell lines keeps expanding and warrants a comprehensive analysis, reporting results for individual cell lines is beyond the scope of this article. Instead, I provide a broad overview by averaging the results across the 9 cell lines mentioned by Ernst 2011. Specifically, the y-axis reports the mean number of cell lines (out of 9 cell lines) for which the CpGs were in the chromatin state mentioned in the title.

*Comparing the multi-tissue predictor with other age predictors*

Several recent publications describe age predictors based on DNA methylation levels {2, 3, 16}. Hannum et al (2012) found that computing a DNAm based age predictor for different tissues gave basically no overlap, e.g. blood-derived predictive CpGs were different from those from other tissues {16}. This suggests that an optimal age predictor for one tissue may be sub-optimal for another. I don't disagree with these results. Instead, I show that one can build a multi-tissue age predictor which can be used for addressing a wide range of questions arising in aging research. While slight gains in accuracy can probably be achieved by focusing on a single tissue and considering more CpGs, the major strength of the proposed multi-tissue age predictor lies in its wide applicability: for most tissues it will not require any adjustments or offsets. The proposed multi-tissue age predictor greatly outperforms the predictors by {2, 3} as detailed below. I could not directly evaluate the predictor by {16} since a) only seven out of its 71 CpGs are represented on the Illumina™ 27K platform, b) it included gender and body mass index as covariates. However, I was able to evaluate the performance of a sparse version of the published predictor by using the seven overlapping CpGs that could be found on both Illumina™ platforms. In the following, I provide more details. To provide an unbiased comparison, I constructed each predictor in an analogous fashion in the training data, i.e. its coefficient values were estimated using the same penalized regression approach. Thus, the predictors only differed with respect to the sets of CpGs that were considered in the penalized regression model. While this does not allow me to assess the performance of the published predictors directly, it provides a completely unbiased comparison of the age predictors. Using the coefficient values from the respective publications would have biased the comparison against them since most were constructed on significantly smaller training data sets (often involving a single tissue) or using a single Illumina™ platform.

I evaluated the performance of each age predictor a) across the training data sets and b) across the test data sets. Since I constructed each predictor using the training data sets, the estimated accuracy in the training set is overly optimistic. I also defined a "shrunken" version of my multi-tissue age predictor, which only involves a subset of 110 CpGs from the 354 CpGs. As indicated by its name, the shrunken predictor is defined by using a more stringent shrinkage parameter (50 times that of the original model) in the penalized regression model. The shrunken predictor is highly accurate in the training data (cor=0.95, error=4 years) and test data (cor=0.95, error=4.2 years). Coefficient values of the multi-tissue predictor and its shrunken version can be found in Example 9. I find that my multi-tissue age predictor greatly outperforms the predictors by {2, 3}. Even when I use the same penalized regression approach for re-training their CpGs, both predictors lead to high errors in training and test data (>14 years) and much lower age correlations (<=0.56). Hannum et al (2012) proposed an age predictor based on 71 CpGs {16}. The authors built a predictive model of aging using a penalized regression method (elastic net) but it differs from the current analysis in the following aspects. First, the aging model from {16} was trained on whole blood, which is a noteworthy advantage when it comes to the design of practical diagnostics and for testing blood samples collected from other studies. Second, it also included clinical parameters such as gender and body mass index as covariates. Third, it is based on CpGs from the Illumina™ 450K arrays while my predictor only involves CpGs from the Illumina™ 27K array. Since only seven of the 71 CpG markers from {16} can be found on the Illumina™ 27K array, I could not carry out a direct comparison across the many tissues considered here. Instead, I was only able to evaluate the performance of a very sparse version of the published predictor by using the seven overlapping CpGs (cg04474832, cg05442902, cg06493994, cg09809672, cg19722847, cg21296230, cg22736354) that could be found on both Illumina™ platforms. The resulting sparse version performs well in the training data (age cor=0.82, error=8.0 years) and in the test data (cor=0.86, error=8.0 years).

In conclusion, a sparse version of the predictor from {16} (based on 7 CpGs) works best among predictors with fewer than 10 CpGs. The proposed multi-tissue predictor suggests that a couple of hundred CpGs will be needed to accurately predicted age across multiple tissue types and the two Illumina™ platforms.

*Meta analysis for finding age-related CpGs*

To measure pure age effects in the marginal analysis, I used the *metaAnalysis* R function in the WGCNA R package {70}. This function allowed to calculate two p-values: *pValueHighScale* and *pValueLowScale* for finding consistently positively and negatively age related CpGs, respectively. Thus, CpGs with a low *pValueHighScale* have a consistently high age correlation in the individual data sets. Since this meta analysis method conditions on the data sets, the p-values are not confounded by data set or tissue. I used the signed logarithm (base 10) of the meta analysis p-value in scatter plots. The sign was chosen so that CpGs with positive (negative) age correlations lead to positive (negative) log p-values. It is shown that the meta analysis p-value based on the training data sets is highly correlated with a corresponding meta analysis p-value calculated using all training and test sets. The high correlation shows that little information is lost by focusing on the training data. The most significant age-related CpGs found in all data can already be found using the training data alone.

*Variation of age related CpGs across somatic tissues*

Since the age predictor performs well across a wide spectrum of tissues, I hypothesized that many of the 354 CpGs used for estimating DNAm age vary little across tissues and that many of them correlate highly with age.

To test this hypothesis, I first defined three different measures of tissue variance. The first measure of tissue variance used analysis of variance (ANOVA) across the training data sets. Toward this end, I used a multivariate regression model to regress each CpG (dependent variable) on age and tissue type. The regression model included age as covariate since the analysis needed to adjust for the fact that different data sets had different age distributions. ANOVA allowed me to calculate an F statistic for tissue effect which takes on a large value for CpGs that vary greatly across the different training set tissues. The second and third measure of tissue variance were defined using the adult somatic tissues and the fetal somatic tissues, respectively, from {54} (data set 77). As an aside, I mention that the mean DNAm age (predicted age) of fetal somatic tissues is close to zero, i.e. it is much lower than that of adult somatic tissues in this data set, which again validates the age predictor. The adult- and the fetal measure of tissue variance of each CpGs is defined by its variance across the adult and somatic tissue samples from {54}, respectively. I find that the adult and the fetal tissue variance measures are highly correlated (cor=0.8) which indicates that these measures are robustly defined and change little with age. Since the data from Nazor et al (data set 77) were not part of the training data, these measures could be used to validate the F-statistic measure of tissue variance. I find a high correlation between the adult measure of tissue variance and the F statistic (cor=0.73) which shows that these measures of tissue variance are highly reproducible. I also defined a stringent measure of age variation for each CpG using a meta analysis approach. The meta analysis calculated age correlations in each training data set separately and next aggregated the correlation test p-values resulting in a meta analysis p-value. Different from the construction of the age predictor, the meta analysis approach explicitly conditioned on each data set. Thus, a CpG has a significant meta analysis p-value if it consistently correlates with age irrespective of tissue type, data set effect, or Illumina™ platform version. It did not really matter that I calculated the meta analysis p-value using the training data alone since the resulting p-value is highly correlated (cor=0.97) with the analogous p-value that results from using all data sets.

To address the question how the tissue variation of a CpG relates to its age variation, I plotted tissue variance versus age variance. Using the ANOVA F statistic for tissue effect, I find the that CpGs with high positive or negative age correlations do not vary much across the somatic adult tissues. A completely analogous result can be observed when using the somatic variance measures involving the adult and fetal tissues from Nazor et al (data 77). CpGs that vary little across tissues appear to be more susceptible to aging effects. Conversely, CpGs that vary greatly across tissues are less affected by aging effects which might reflect that they are actively protected against aging effects.

*Studying age effects using gene expression data*

The publicly available microarray data sets involved mainly healthy individuals (in particular no cancer samples were considered).

To estimate the age effect on gene expression levels, I analyzed multiple independent publicly available microarray data sets. Blood microarray data sets involving mainly healthy control individuals (referred to as SAFHS {71}, Chaussabel {72} and NOWAC {73} data) and the CD8 T cell microarray data Cao {74}. To assess whether a gene was differentially expressed between naive CD8+ T cells and antigen exposed CD8+ T cells, I used the data from Willinger et al {75, 76}).In the following I provide more details.

The data from a study of post-menopausal women (the NOWAC data).In my largest data set, the San Antonio Family Heart Study (SAFHS) data set, individuals were ascertained from probands meeting two criteria: 1) having a living spouse and 2) having six first-degree relatives 16 years or older in the San Antonio area - excluding parents. While this data set was used to study cardiovascular phenotypes, the data was obtained without selection bias towards these traits, and therefore can be considered a random sampling.

I obtained the San Antonio Family Heart Study (SAFHS) blood data set, which was previously analyzed by Goring, et al {71}. This data set was derived from lymphocytes; RNA was hybridized to Illumina™ Sentrix Human Whole Genome (WG-6) Series I BeadChips with probe sets corresponding to 18,544 genes. Quantile normalization was applied to the raw data. This data set consisted of 1,084 samples: 452 males and 632 females between ages 15 and 94 after outlier removal. Specifically, outlier detection and removal was performed using an iterative process of removing outliers with average interarray correlation (IAC) $\leq$ 2 SD below the mean until visual inspection of the cluster dendrogram and plot of the mean IAC revealed no further outliers. This analysis was completely unbiased and agnostic to chronological age. Toward this end, I used our recently developed sampleNetwork R function described in {77}

The Chaussabel data set was originally published by Pankla, et al. {72} and was used to study melioidosis. 67 whole blood samples were hybridized to Illumina™ Sentrix Human-6 V2 BeadChip arrays with 12,483 genes. Background subtraction and average normalization was performed using Illumina™ BeadStudio version 2 software, and standard normalization for one-color array data was performed using Gene-Spring GX7.3 software (Agilent Technologies) by the original authors. This data set consisted of 35 men and 32 women between the ages of 18 and 74. I also used healthy postmenopausal women from the Norwegian Women and Cancer (NOWAC)study {73}. The whole blood data were measured using AB Human Genome Survey Microarray V2.0 with 16,753 genes. For sets of technical replicates, arrays with the least number of probes with a S/N > 3 were excluded. Arrays with less than 40% of probes with a S/N $\geq$ 3 were removed. Probes with an S/N > 3 in less than 50% of samples were excluded. Log (base 2) transformation, quantile normalization and imputation was performed. I furthermore excluded samples using an iterative process of removing samples with average interarray correlation <2 SD ultimately resulting in 245 samples. Age ranges of {48,53), {53,58) and {58,63} were given, and I used for the analysis corresponding ages of 50, 55 and 60.

In the CD8+ T cell data set from Cao, et al. {74} Affymetrix HG-U133A_2 Gene Arrays were used to explore the expression profiles of three male and six female donors whose ages ranged from 23 to 81. Microarray Suite Version 5.0 (MAS 5.0; Affymetrix) was used to quantify the expression levels of 12,483 genes. In the CD8+ T cell data set from Willinger et al {75, 76}, Affymetrix HG-U133 plus 2.0 arrays (log transformed MAS5 data) were used to explore the expression profiles of human CD8+ naive T cells (TN), central memory (TCM), effector memory (TEM), and effector memory RA (TEMRA) CD8+ T cells. TN can be regarded as peripheral stem cells, while TEM and TEMRA are differentiated cells with effector function. For each T cell type, the original data set contained 4 replicates (i.e. there were 16 arrays). Since one of the central memory samples had very low interarray correlation with the other samples, I removed this potential outlier from the analysis. A Student t-test of differential expression was used to compare expression levels in naive CD8+ cells versus the memory T cells.

The first brain data set was previously analyzed by Lu, et al. {78}. 30 frontal lobe samples were hybridized to Affymetrix HG-U95Av2 oligonucleotide arrays with 8,760 genes. Arrays were normalized by Lu, et al. using dChip V1.3 software, and after using the aforementioned iterative process of removing samples with average interarray correlation <2 SD below the mean I obtained 25 samples. This data set consisted of 16 men and 9 women between ages 26 and 91.

The second cortical brain data set was previously analyzed by Myers, et al. {79}. The Illumina™ HumanRef-8 Expression BeadChip was utilized, and expression profiles were rank-invariant normalized using Illumina™ BeadStudio software. I utilized a iterative normalization process and removed 25 samples for a total of 168 samples and 19,880 genes. This data set consisted of 92 men and 76 women between ages 65 and 100. The third cortical brain data set was previously analyzed by Oldham, et al. {80}.Affymetrix HG-U95Av2 microarrays were used. Quantile normalization was utilized. Ultimately I identified 7763 genes in 67 individuals. This data set consisted of 48 men and 19 women between ages 22 and 81. The kidney data sets were previously analyzed by Rodwell, et al. {81}. I utilized data from HG-U133A high-density oligonucleotide arrays; Rodwell, et al. normalized data using the dChip program according to the stable invariant set, and I further processed using the normalization and iterative outlier removal process. These normalization and outlier detection procedures resulted in 63 kidney cortex samples and 52 kidney medulla samples. There were 12,606 genes in both data sets. The kidney cortex data set consisted of 35 men and 26 women between ages 27 and 87, and the kidney medulla data set consisted of 29 men and 23 women between ages 29 and 92.

The muscle data set was previously analyzed by Zahn, et al. {82}. 81 samples were hybridized to Affymetrix HG-U133 2.0 Plus high-density oligonucleotide arrays. The authors used the DChip program to normalize the data. I omitted 10 samples using the iterative normalization and outlier removal process, resulting in 71 samples and 19,621 genes. This data set consisted of 39 men and 32 women between ages 16 and 89.

*Meta analysis applied to gene expression data*

In the following, I describe how I obtained the Pearson correlation coefficient, the corresponding t-test statistic Z in each data set, the metaZ statistics summarizing correlation test statistics across multiple data, a corresponding empirical p-value (pMetaZ). I denote by $r_s$ the Pearson correlation coefficient (e.g. between age and the gene expression profile) in the s-th data set. The Student t-test statistic for testing whether the correlation is different from zero is given by $Z_s = \frac{\sqrt{m_s - 2} \cdot r_s}{\sqrt{1 - r_s^2}}$ where $m_s$ denotes the number of observations (i.e. microarrays, individuals) in the s-th data set. This Z statistic is equivalent to the Wald test statistic resulting from a univariate regression model where age is regressed on the gene expression profile. To combine multiple correlation test statistics across the data sets, I used the metaZ statistic $metaZ = \frac{\sum_{s=1}^{no.dataSets} w_s Z_s}{\sqrt{\sum_{s=1}^{no.dataSets} (w_s)^2}}$ where $w_s$ denotes a weight associated with the s-th data set. All data sets received a weight of $w_s$=1 but the weight had a negligible effect. Under the null hypothesis of zero correlation, metaZ follows an approximate normal distribution under weak assumptions, which will be outlined in the following. First, metaZ follows approximately a standard normal distribution if each individual $Z_s$ follows approximately a standard normal distribution since the data sets are independent. Second, even if individual Z statistics do not follow a normal distribution, one can invoke the central limit theorem if many independent data sets are being considered.

*Names of the genes whose mutations are associated with age acceleration*

Mutations in the following genes either increase or decrease DNAm age.

AKAP9-A kinase (PRKA) anchor protein (yotiao) 9
CHD7-chromodomain helicase DNA binding protein 7 [Homo sapiens]
CTNND2-catenin (cadherin-associated protein), delta 2

DMBT1—deleted in malignant brain tumors 1
DSG3-desmoglein 3
FAM123C-family with sequence similarity 123C
FAT4-FAT atypical cadherin 4
GATA3-GATA binding protein 3
KCNB1—potassium voltage-gated channel, Shab-related subfamily, member 1
LEPR-leptin receptor
MACF1—microtubule-actin crosslinking factor 1
MB21D1—Mab-21 domain containing 1
MGAM-maltase-glucoamylase (alpha-glucosidase)
MUC17—mucin 17, cell surface associated
MYH7-myosin, heavy chain 7, cardiac muscle, beta
RELN-reelin
THOC2—THO complex 2
TMEM132D-transmembrane protein 132D
TTN—titin
TP53-tumor protein p53
U2AF1—U2 small nuclear RNA auxiliary factor 1

*Is DNAm age a biomarker of aging?*

The American Federation for Aging Research proposed the following criteria for a biomarker of aging (reviewed in {83-85}):

1. It must predict the rate of aging.
2. It must monitor a basic process that underlies the aging process, not the effects of disease.
3. It must be able to be tested repeatedly without harming the person.
4. It must be something that works in humans and in laboratory animals.

I will address these criteria in reverse order. DNAm age probably meets criterion 4 if chimpanzees are acceptable as lab animals (given my results in Figure 4). There is a good chance that it meets criterion 3 (given my results in blood, saliva, buccal cells, skin) and criterion 2 (see my EMS model of DNAm age and the vast literature on aging effects on DNA methylation levels). Large cohort studies will be very valuable for addressing criterion 1. These studies need to test whether a measure of DNAm based age acceleration will, in the absence of disease, better predict functional capability than chronological age {86}.

*Example 8 References*

1. Koch CM, Suschek CV, Lin Q, Bork S, Goergens M, Joussen S, Pallua N, Ho AD, Zenke M, Wagner W: Specific Age-Associated DNA Methylation Changes in Human Dermal Fibroblasts. PLoS ONE 2011, 6:e16679.
2. Koch C, Wagner W: Epigenetic-aging-signature to determine age in different tissues. Aging 2011, 3:1018-1027.
3. Bocklandt S, Lin W, Sehl ME, Sanchez FJ, Sinsheimer JS, Horvath S, Vilain E: Epigenetic predictor of age. PLoS ONE 2011, 6:e14821.
4. Esteller M: Epigenetic lesions causing genetic lesions in human cancer: promoter hypermethylation of DNA repair genes. European Journal of Cancer 2000, 36:2294-2300.
5. Ushijima T: Detection and interpretation of altered methylation patterns in cancer cells. Nat Rev Cancer 2005, 5:223-231.
6. So K, Tamura G, Honda T, Homma N, Waki T, Togawa N, Nishizuka S, Motoyama T: Multiple tumor suppressor genes are increasingly methylated with age in non-neoplastic gastric epithelia. Cancer Science 2006, 97:1155-1158.
7. Fraga MF, Esteller M: Epigenetics and aging: the targets and the marks. Trends in Genetics 2007, 23:413-418.
8. Fraga MF, Agrelo R, Esteller M: Cross-Talk between Aging and Cancer. Annals of the New York Academy of Sciences 2007, 1100:60-74.
9. Bjornsson HT, Sigurdsson MI, Fallin MD, Irizarry RA, Aspelund T, Cui H, Yu W, Rongione MA, Ekström TJ, Harris TB, et al: Intra-individual Change Over Time in DNA Methylation With Familial Clustering. JAMA: The Journal of the American Medical Association 2008, 299:2877-2883.
10. Christensen B, Houseman E, Marsit C, Zheng S, Wrensch M, Wiemels J, Nelson H, Karagas M, Padbury J, Bueno R, et al: Aging and Environmental Exposures Alter Tissue-Specific DNA Methylation Dependent upon CpG Island Context. PLoS Genet 2009, 5:e1000602.

11. Rodriguez-Rodero S, Fernández-Morera J, Fernandez A, Menéndez-Torre E, Fraga M: Epigenetic regulation of aging. Discov Med 2010, 10:225-233.

12. Teschendorff AE, Menon U, Gentry-Maharaj A, Ramus SJ, Weisenberger DJ, Shen H, Campan M, Noushmehr H, Bell CG, Maxwell AP, et al: Age-dependent DNA methylation of genes that are suppressed in stem cells is a hallmark of cancer. Genome Res 2010, 20:440-446.

13. Horvath S, Zhang Y, Langfelder P, Kahn R, Boks M, van Eijk K, van den Berg L, Ophoff RA: Aging effects on DNA methylation modules in human brain and blood tissue. Genome Biology 2012, 13.

14. Issa J-PJ, Ottaviano YL, Celano P, Hamilton SR, Davidson NE, Baylin SB: Methylation of the oestrogen receptor CpG island links ageing and neoplasia in human colon. Nat Genet 1994, 7:536-540.

15. Maegawa S, Hinkal G, Kim HS, Shen L, Zhang L, Zhang J, Zhang N, Liang S, Donehower LA, Issa J-PJ: Widespread and tissue specific age-related DNA methylation changes in mice. Genome Res 2010, 20:332-340.

16. Hannum G, Guinney J, Zhao L, Zhang L, Hughes G, Sadda S, Klotzle B, Bibikova M, Fan J-B, Gao Y, et al: Genome-wide Methylation Profiles Reveal Quantitative Views of Human Aging Rates. Molecular cell 2012.

17. Alisch RS, Barwick BG, Chopra P, Myrick LK, Satten GA, Conneely KN, Warren ST: Age-associated DNA methylation in pediatric populations. Genome Res 2012, 22:623-632.

18. Harris R, Nagy-Szakal D, Pedersen N, Opekun A, Bronsky J, Munkholm P, Jespersgaard C, Andersen P, Melegh B, Ferry G, et al: Genome-wide peripheral blood leukocyte DNA methylation microarrays identified a single association with inflammatory bowel diseases Inflamm Bowel Dis 2012, 18:2334-2341.

19. Adkins RM, Krushkal J, Tylavsky FA, Thomas F: Racial differences in gene-specific DNA methylation levels are present at birth. Birth Defects Research Part A: Clinical and Molecular Teratology 2011, 91:728-736.

20. Gibbs JR, van der Brug MP, Hernandez DG, Traynor BJ, Nalls MA, Lai S-L, Arepalli S, Dillman A, Rafferty IP, Troncoso J, et al: Abundant Quantitative Trait Loci Exist for DNA Methylation and Gene Expression in Human Brain. PLoS Genet 2010, 6:e1000952.

21. Numata S, Ye T, Hyde Thomas M, Guitart-Navarro X, Tao R, Wininger M, Colantuoni C, Weinberger Daniel R, Kleinman Joel E, Lipska Barbara K: DNA Methylation Signatures in Development and Aging of the Human Prefrontal Cortex. The American Journal of Human Genetics 2012, 90:260-272.

22. Guintivano J, Aryee MJ, Kaminsky ZA: A cell epigenotype specific model for the correction of brain cellular heterogeneity bias and its application to age, brain region and major depression. Epigenetics 2013, 8:290-302.

23. Zhuang J, Jones A, Lee S-H, Ng E, Fiegl H, Zikan M, Cibula D, Sargent A, Salvesen HB, Jacobs IJ, et al: The Dynamics and Prognostic Potential of DNA Methylation Changes at Stem Cell Gene Loci in Women's Cancer. PLoS Genet 2012, 8:e1002517.

24. Essex MJ, Thomas Boyce W, Hertzman C, Lam LL, Armstrong JM, Neumann SMA, Kobor MS: Epigenetic Vestiges of Early Developmental Adversity: Childhood Stress Exposure and DNA Methylation in Adolescence. Child Development 2011, 84:58-75.

25. Rakyan VK, Down TA, Maslau S, Andrew T, Yang TP, Beyan H, Whittaker P, McCann OT, Finer S, Valdes AM, et al: Human aging-associated DNA hypermethylation occurs preferentially at bivalent chromatin domains. Genome Res 2010, 20:434-439.

26. Martino DJ, Tulic MK, Gordon L, Hodder M, Richman T, Metcalfe J, Prescott SL, Saffery R: Evidence for age-related and individual-specific changes in DNA methylation profile of mononuclear cells during early immune development in humans. Epigenetics: official journal of the DNA Methylation Society 2011, 6.

27. Fernández-Tajes J, Soto-Hermida A, Vázquez-Mosquera ME, Cortés-Pereira E, Mosquera A, Fernández-Moreno M, Oreiro N, Fernández-López C, Fernández JL, Rego-Pérez I, Blanco FJ: Genome-wide DNA methylation analysis of articular chondrocytes reveals a cluster of osteoarthritic patients. Annals of the Rheumatic Diseases 2013:PMID: 23505229.

28. Harris RA, Nagy-Szakal D, Kellermayer R: Human metastable epiallele candidates link to common disorders. Epigenetics 2013, 8:157-163.

29. Grönniger E, Weber B, Heil O, Peters N, Stab F, Wenck H, Korn B, Winnefeld M, Lyko F: Aging and Chronic Sun Exposure Cause Distinct Epigenetic Changes in Human Skin. PLoS Genet 2010, 6:e1000971.

30. Zouridis H, Deng N, Ivanova T, Zhu Y, Wong B, Huang D, Wu YH, Wu Y, Tan IB, Liem N, et al: Methylation Subtypes and Large-Scale Epigenetic Alterations in Gastric Cancer. Science Translational Medicine 2012, 4:156ra140.

31. Haas J, Frese KS, Park YJ, Keller A, Vogel B, Lindroth AM, Weichenhan D, Franke J, Fischer S, Bauer A, et al: Alterations in cardiac DNA methylation in human dilated cardiomyopathy. EMBO Molecular Medicine 2013, 5:413-429.

32. Shen J, Wang S, Zhang Y-J, Kappil M, Wu H-C, Kibriya MG, Wang Q, Jasmine F, Ahsan H, Lee P-H, et al: Genome-wide DNA methylation profiles in hepatocellular carcinoma. Hepatology 2012, 55:1799-1808.

33. Bork S, Pfister S, Witt H, Horn P, Korn, B, Ho A, Wagner W: DNA methylation pattern changes upon long-term culture and aging of human mesenchymal stromal cells. Aging Cell 2010, 9:54-63.

34. Gordon L, Joo JE, Powell JE, Ollikainen M, Novakovic B, Li X, Andronikos R, Cruickshank MN, Conneely KN, Smith AK, et al: Neonatal DNA methylation profile in human twins is specified by a complex interplay between intrauterine environmental and genetic factors, subject to tissue-specific influence. Genome Res 2012, 22:1395-1406.

35. Kobayashi Y, Absher DM, Gulzar ZG, Young SR, McKenney JK, Peehl DM, Brooks JD, Myers RM, Sherlock G: DNA methylation profiling reveals novel biomarkers and important roles for DNA methyltransferases in prostate cancer. Genome Res 2011, 21:1017-1027.

36. Liu J, Morgan M, Hutchison K, Calhoun VD: A Study of the Influence of Sex on Genome Wide Methylation. PLoS ONE 2010, 5:e10028.

37. Song H, Ramus SJ, Tyrer J, Bolton KL, Gentry-Maharaj A, Wozniak E, Anton-Culver H, Chang-Claude J, Cramer DW, DiCioccio R, et al: A genome-wide association study identifies a new ovarian cancer susceptibility locus on 9p22.2. Nat Genet 2009, 41:996-1000.

38. Liu Y, Aryee MJ, Padyukov L, Fallin MD, Hesselberg E, Runarsson A, Reinius L, Acevedo N, Taub M, Ronninger M, et al: Epigenome-wide association data implicate DNA methylation as an intermediary of genetic risk in rheumatoid arthritis. Nat Biotech 2013, 31:142-147.

39. Heyn H, Li N, Ferreira HJ, Moran S, Pisano DG, Gomez A, Diez J, Sanchez-Mut JV, Setien F, Carmona FJ, et al: Distinct DNA methylomes of newborns and centenarians. Proceedings of the National Academy of Sciences 2012, 109:10522-10527.

40. Lam LL, Emberly E, Fraser HB, Neumann SM, Chen E, Miller GE, Kobor MS: Factors underlying variable DNA methylation in a human community cohort. Proceedings of the National Academy of Sciences 2012, 109:17253-17260.

41. Khulan B, Cooper WN, Skinner BM, Bauer J, Owens S, Prentice AM, Belteki G, Constancia M, Dunger D, Affara NA: Periconceptional maternal micronutrient supplementation is associated with widespread gender related changes in the epigenome: a study of a unique resource in the Gambia. Human Molecular Genetics 2012, 21:2086-2101.

42. Martino D, Maksimovic J, Joo JH, Prescott SL, Saffery R: Genome-scale profiling reveals a subset of genes regulated by DNA methylation that program somatic T-cell phenotypes in humans. Genes Immun 2012, 13:388-398.

43. Heyn H, Moran S, Esteller M: Aberrant DNA methylation profiles in the premature aging disorders Hutchinson-Gilford Progeria and Werner syndrome. Epigenetics 2013, 8:28-33.

44. Ginsberg MR, Rubin RA, Falcone T, Ting AH, Natowicz MR: Brain Transcriptional and Epigenetic Associations with Autism. PLoS ONE 2012, 7:e44736.

45. Martino D, Loke Y, Gordon L, Ollikainen M, Cruickshank M, Saffery R, Craig J: Longitudinal, genome-scale analysis of DNA methylation in twins from birth to 18 months of age reveals rapid epigenetic change in early life and pair-specific effects of discordance. Genome Biology 2013, 14:R42.

46. Ribel-Madsen R, Fraga MF, Jacobsen S, Bork-Jensen J, Lara E, Calvanese V, Fernandez AF, Friedrichsen M, Vind BF, Højlund K, et al: Genome-Wide Analysis of DNA Methylation Differences in Muscle and Fat from Monozygotic Twins Discordant for Type 2 Diabetes. PLoS ONE 2012, 7:e51302.

47. Pai AA, Bell JT, Marioni JC, Pritchard JK, Gilad Y: A Genome-Wide Study of DNA Methylation Patterns and Gene Expression Levels in Multiple Human and Chimpanzee Tissues. PLoS Genet 2011, 7:e1001316.

48. Jacobsen SC, Brøns C, Bork-Jensen J, Ribel-Madsen R, Yang B, Lara E, Hall E, Calvanese V, Nilsson E, Jorgensen SW, et al: Effects of short-term high-fat overfeeding on genome-wide DNA methylation in the skeletal muscle of healthy young men. Diabetologia 2012, 55:3341-3349.

49. Blair JD, Yuen RKC, Lim BK, McFadden DE, von Dadelszen P, Robinson WP Widespread DNA hypomethylation at gene enhancer regions in placentas associated with early-onset pre-eclampsia. Molecular Human Reproduction 2013.

50. Teschendorff A, Jones A, Fiegl H, Sargent A, Zhuang J, Kitchener H, Widschwendter M: Epigenetic variability in cells of normal cytology is associated with the risk of future morphological transformation. Genome Medicine 2012, 4:24.

51. Hernando-Herraez I, Prado-Martinez J, Garg P, Fernandez-Callejo M, Heyn H, Hvilsom C, Navarro A, Esteller M, Sharp A, Marques-Bonet T: Dynamics of DNA Methylation in Recent Human and Great Apes Evolution. PLoS Genet 2013, In Press.

52. Pacheco SE, Houseman EA, Christensen BC, Marsit CJ, Kelsey KT, Sigman M, Boekelheide K: Integrative DNA Methylation and Gene Expression Analyses Identify DNA Packaging and Epigenetic Regulatory Genes Associated with Low Motility Sperm. PLoS ONE 2011, 6:e20280.

53. Krausz C, Sandoval J, Sayols S, Chianese C, Giachini C, Heyn H, Esteller M: Novel Insights into DNA Methylation Features in Spermatozoa: Stability and Peculiarities. PLoS ONE 2012, 7:e44479.

54. Nazor Kristopher L, Altun G, Lynch C, Tran H, Harness Julie V, Slavin I, Garitaonandia I, Müller F-J, Wang Y-C, Boscolo Francesca S, et al: Recurrent Variations in DNA Methylation in Human Pluripotent Stem Cells and Their Differentiated Derivatives. Cell stem cell 2012, 10:620-634.
55. Shao K, Koch C, Gupta MK, Lin Q, Lenz M, Laufs S, Denecke B, Schmidt M, Linke M, Hennies HC, et al: Induced Pluripotent Mesenchymal Stromal Cell Clones Retain Donor-derived Differences in DNA Methylation Profiles. Mol Ther 2012.
56. Calvanese V, Fernández AF, Urdinguio RG, Suárez-Alvarez B, Mangas C, Perez-Garcia V, Bueno C, Montes R, Ramos-Mejia V, Martinez-Camblor P, et al: A promoter DNA demethylation landscape of human hematopoietic differentiation. Nucleic Acids Research 2012, 40:116-131.
57. Ramos-Mejia V, Fernandez A, Ayllon V, Real P, Bueno C, Anderson P, Martin F, Fraga M, Menendez P: Maintenance of human embryonic stem cells in mesenchymal stem cell-conditioned media augments hematopoietic specification. Stem Cells Dev 2012, 21:1549-1558.
58. Reinius LE, Acevedo N, Joerink M, Pershagen G, Dahlén S-E, Greco D, Söderhäll C, Scheynius A, Kere J: Differential DNA Methylation in Purified Human Blood Cells: Implications for Cell Lineage and Studies on Disease Susceptibility. PLoS ONE 2012, 7:e41361.
59. Sturm D, Witt H, Hovestadt V, Khuong-Quang D-A, Jones David TW, Konermann C, Pfaff E, Tönjes M, Sill M, Bender S, et al: Hotspot Mutations in H3F3A and IDH1 Define Distinct Epigenetic and Biological Subgroups of Glioblastoma. Cancer Cell 2012, 22:425-437.
60. Fackler MJ, Umbricht CB, Williams D, Argani P, Cruz L-A, Merino VF, Teo WW, Zhang Z, Huang P, Visvananthan K, et al: Genome-wide Methylation Analysis Identifies Genes Specific to Breast Cancer Hormone Receptor Status and Risk of Recurrence. Cancer Research 2011, 71:6195-6207.
61. Dedeurwaerder S, Desmedt C, Calonne E, Singhal SK, Haibe-Kains B, Defrance M, Michiels S, Volkmar M, Deplus R, Luciani J, et al: DNA methylation profiling reveals a predominant immune component in breast cancers. EMBO Molecular Medicine 2011, 3:726-741.
62. Hinoue T, Weisenberger DJ, Lange CPE, Shen H, Byun H-M, Van Den Berg D, Malik S, Pan F, Noushmehr H, van Dijk CM, et al: Genome-scale analysis of aberrant DNA methylation in colorectal cancer. Genome Res 2012, 22:271-282.
63. Lauss M, Aine M, Sjödahl G, Veerla S, Patschan O, Gudjonsson S, Chebil G, Lövgren K, Fernö M, Månsson W, et al: DNA methylation analyses of urothelial carcinoma reveal distinct epigenetic subtypes and an association between gene copy number and methylation status. Epigenetics 2012, 7:858-867.
64. Weisenberger D, den Berg D, Pan F, Berman B, Laird P: Comprehensive DNA methylation analysis on the Illumina Infinium assay platform. Technical report Illumina, Inc, San Diego 2008.
65. Dunning M, Barbosa-Morais N, Lynch A, Tavare S, Ritchie M: Statistical issues in the analysis of Illumina data. BMC Bioinformatics 2008, 9:85.
66. Dedeurwaerder S, Defrance M, Calonne E, Denis H, Sotiriou C, Fuks F: Evaluation of the Infinium Methylation 450K technology. Epigenomics 2011, 3:771-784.
67. Maksimovic J, Gordon L, Oshlack A: SWAN: Subset-quantile Within Array Normalization for Illumina Infinium HumanMethylation450 BeadChips. Genome Biology 2012, 13:R44.
68. Teschendorff AE, Marabita F, Lechner M, Bartlett T, Tegner J, Gomez-Cabrero D, Beck S: A beta-mixture quantile normalization method for correcting probe design bias in Illumina Infinium 450 k DNA methylation data. Bioinformatics 2013, 29:189-196.
69. Ernst J, Kheradpour P, Mikkelsen TS, Shoresh N, Ward LD, Epstein CB, Zhang X, Wang L, Issner R, Coyne M, et al: Mapping and analysis of chromatin state dynamics in nine human cell types. Nature 2011, 473:43-49.
70. Langfelder P, Mischel PS, Horvath S: When is hub gene selection better than standard meta-analysis? PLoS ONE 2013, 8:e61505.
71. Göring H, Curran J, Johnson M, Dyer T, Charlesworth J, Cole S, Jowett J, Abraham L, Rainwater D, Comuzzie A, et al: Discovery of expression QTLs using large-scale transcriptional profiling in human lymphocytes. Nat Genet 2007, 39:1208-1216.
72. Pankla R, Buddhisa S, Berry M, Blankenship DM, Bancroft GJ, Banchereau J, Lertmemongkolchai G, Chaussabel D: Genomic transcriptional profiling identifies a candidate blood biomarker signature for the diagnosis of septicemic melioidosis. Genome Biol 2009, 10:R127.
73. Dumeaux V, Olsen KS, Nuel G, Paulssen RH, B√∏rresen-Dale A-L, Lund E: Deciphering normal blood gene expression variation--the NOWAC postgenome study. PLoS Genet, 6:e1000873.
74. Cao J-N, Gollapudi S, Sharman EH, Jia Z, Gupta S: Age-related alterations of gene expression patterns in human CD8+ T cells. Aging Cell 2010, 9:19-31.
75. Willinger T, Freeman T, Hasegawa H, McMichael AJ, Callan MFC: Molecular Signatures Distinguish Human Central Memory from Effector Memory CD8 T Cell Subsets. The Journal of Immunology 2005, 175:5895-5903.
76. Willinger T, Freeman T, Herbert M, Hasegawa H, McMichael AJ, Callan MFC: Human Naive CD8 T Cells Down-Regulate Expression of the WNT Pathway Transcription Factors Lymphoid Enhancer Binding Factor 1 and Transcription Factor 7 (T Cell Factor-1) following Antigen Encounter In Vitro and In Vivo. The Journal of Immunology 2006, 176:1439-1446.
77. Oldham M, Langfelder P, Horvath S: Network methods for describing sample relationships in genomic datasets: application to Huntington's disease. BMC Syst Biol 2012, 6:63.
78. Lu T, Pan Y, Kao S-Y, Li C, Kohane I, Chan J, Yankner BA: Gene regulation and DNA damage in the ageing human brain. Nature 2004, 429:883-891.
79. Myers AJ, Gibbs JR, Webster JA, Rohrer K, Zhao A, Marlowe L, Kaleem M, Leung D, Bryden L, Nath P, et al: A survey of genetic human cortical gene expression. Nat Genet 2007, 39:1494-1499.
80. Oldham M, Konopka G, Iwamoto K, Langfelder P, Kato T, Horvath S, Geschwind D: Functional organization of the transcriptome in human brain.. Nature Neuroscience 2008, 11:1271-1282.
81. Rodwell GE, Sonu R, Zahn JM, Lund J, Wilhelmy J, Wang L, Xiao W, Mindrinos M, Crane E, Segal E, et al: A transcriptional profile of aging in the human kidney. PLoS Biol 2004, 2:e427.
82. Zahn J, Sonu R, Vogel H, Crane E, Mazan-Mamczarz K, Rabkin R, Davis R, Becker K, Owen A, Kim S: Transcriptional profiling of aging in human muscle reveals a common aging signature. PLoS Genet 2006, 2:e115.
83. Warner HR: The Future of Aging Interventions. The Journals of Gerontology Series A: Biological Sciences and Medical Sciences 2004, 59:B692-B696.
84. Johnson T: Recent results: Biomarkers of aging. Experimental Gerontology 2006, 41:1243-1246.
85. Mather KA, Jorm AF, Parslow RA, Christensen H: Is Telomere Length a Biomarker of Aging? A Review. The Journals of Gerontology Series A: Biological Sciences and Medical Sciences 2011, 66A: 202-213.
86. Baker G, Sprott R: Biomarkers of aging. Exp Gerontol 1988, 23:223-239.

Example 9: Coefficient values for the DNAm age predictor

This example provides information on the multi-tissue age predictor defined using the training set data. The multi-tissue age predictor uses 354 CpGs of which 193 and 160 have positive and negative correlations with age, respectively. The table also represents the coefficient values for the shrunken new predictor that is based on a subset of 110 CpGs (a subset of the 354 CpGs). Although this information is sufficient for predicting age, the software posted on [45] is recommended. The table reports a host of additional information for each CpG including its variance, minimum value, maximum value, and median value across all training and test data. Further, it reports the median beta value in subjects younger than 35 and in subjects older than 55.

Example 10: Description of cancer data sets

This example describes 32 publicly available cancer tissue data sets and 7 cancer cell line data sets. Column 1 reports the data number and corresponding color code. Other columns report the affected tissue, Illumina™ platform, sample size n, proportion of females, median age, age range (minimum and maximum age), relevant citation (TCGA or first author with publication year), and public availability. None of these data sets were used in the construction of estimator of DNAm age. The table also reports the age correlation, cor(Age,DNAmage), median error, and median age acceleration. The epigenetic clock was applied to many different cancer types and cancer data sets. The last columns of Example 10 show that DNAm age has only a weak relationship with chronological age in cancer tissue.

Example 11: Cancer lines and DNAm age

This example reports the DNAm age and age acceleration for 59 cancer cell lines. The epigenetic clock was applied to many different cancer cell lines. It turns out that the DNAm age changes greatly across cell lines.

CONCLUSION

This concludes the description of the preferred embodiment of the present invention. The foregoing description of one or more embodiments of the invention has been presented for the purposes of illustration and description. It is not intended to be exhaustive or to limit the invention to the precise form disclosed. Many modifications and variations are possible in light of the above teaching. It is intended that the scope of the invention be limited not by this detailed description, but rather by the claims appended hereto.

REFERENCES

1. Oberdoerffer P, Sinclair DA: The role of nuclear architecture in genomic instability and ageing. Nat Rev Mol Cell Biol 2007, 8:692-702.

2. Campisi J, Vijg J: Does Damage to DNA and Other Macromolecules Play a Role in Aging? If So, How? The Journals of Gerontology Series A: Biological Sciences and Medical Sciences 2009, 64A: 175-178.

3. Berdyshev G, Korotaev G, Boiarskikh G, Vaniushin B: Nucleotide composition of DNA and RNA from somatic tissues of humpback and its changes during spawning. Biokhimiia 1967, 31:88-993.

4. Vanyushin B, Nemirovsky L, Klimenko V, Vasiliev V, Belozersky A: The 5 mehylcytosine in DNA of rats. Tissue and age specificity and the changes induced by hydrocortisone and other agents. Gerontologia 1973, 19:138-152.

5. Wilson V, Smith R, Ma S, Cutler R: Genomic 5-methyldeoxycytidine decreases with age. J Biol Chem 1987, 262:9948-9951.

6. Fraga MF, Agrelo R, Esteller M: Cross-Talk between Aging and Cancer. Annals of the New York Academy of Sciences 2007, 1100:60-74.

7. Fraga MF, Esteller M: Epigenetics and aging: the targets and the marks. Trends in Genetics 2007, 23:413-418.

8. Christensen B, Houseman E, Marsit C, Zheng S, Wrensch M, Wiemels J, Nelson H, Karagas M, Padbury J, Bueno R, et al: Aging and Environmental Exposures Alter Tissue-Specific DNA Methylation Dependent upon CpG Island Context. PLoS Genet 2009, 5:e1000602.

9. Bollati V, Schwartz J, Wright R, Litonjua A, Tarantini L, Suh H, Sparrow D, Vokonas P, Baccarelli A: Decline in genomic DNA methylation through aging in a cohort of elderly subjects. Mechanisms of Ageing and Development 2009, 130:234-239.

10. Teschendorff AE, Menon U, Gentry-Maharaj A, Ramus SJ, Weisenberger DJ, Shen H, Campan M, Noushmehr H, Bell CG, Maxwell AP, et al: Age-dependent DNA methylation of genes that are suppressed in stem cells is a hallmark of cancer. Genome Res 2010, 20:440-446.

11. Mugatroyd C, Wu Y, Bockmühl Y, Spengler D: The Janus face of DNA methylation in aging. AGING 2010, 2.

12. Rodriguez-Rodero S, Fernández-Morera J, Fernandez A, Menéndez-Torre E, Fraga M: Epigenetic regulation of aging. Discov Med 2010, 10:225-233.

13. Bell JT, Tsai P-C, Yang T-P, Pidsley R, Nisbet J, Glass D, Mangino M, Zhai G, Zhang F, Valdes A, et al: Epigenome-Wide Scans Identify Differentially Methylated Regions for Age and Age-Related Phenotypes in a Healthy Ageing Population. PLoS Genet 2012, 8:e1002629.

14. Horvath S, Zhang Y, Langfelder P, Kahn R, Boks M, van Eijk K, van den Berg L, Ophoff RA: Aging effects on DNA methylation modules in human brain and blood tissue. Genome Biology 2012, 13.

15. Rakyan VK, Down TA, Maslau S, Andrew T, Yang TP, Beyan H, Whittaker P, McCann OT, Finer S, Valdes AM, et al: Human aging-associated DNA hypermethylation occurs preferentially at bivalent chromatin domains. Genome Res 2010, 20:434-439.

16. Bernstein BE, Stamatoyannopoulos JA, Costello JF, Ren B, Milosavljevic A, Meissner A, Kellis M, Marra MA, Beaudet AL, Ecker JR, et al: The NIH Roadmap Epigenomics Mapping Consortium. Nat Biotech 2010, 28:1045-1048.

17. Illingworth R, Kerr A, DeSousa D, Jorgensen H, Ellis P, Stalker J, Jackson D, Clee C, Plumb R, Rogers J, et al: A Novel CpG Island Set Identifies Tissue-Specific Methylation at Developmental Gene Loci. PLoS Biol 2008, 6:e22.

18. Li Y, Zhu J, Tian G, Li N, Li Q, Ye M, Zheng H, Yu J, Wu H, Sun J, et al: The DNA Methylome of Human Peripheral Blood Mononuclear Cells. PLoS Biol 2010, 8:e1000533.

19. Thompson RF, Atzmon G, Gheorghe C, Liang HQ, Lowes C, Greally JM, Barzilai N: Tissue-specific dysregulation of DNA methylation in aging. Aging Cell 2010, 9:506-518.

20. Hernandez DG, Nalls MA, Gibbs JR, Arepalli S, van der Brug M, Chong S, Moore M, Longo DL, Cookson MR, Traynor BJ, Singleton AB: Distinct DNA methylation changes highly correlated with chronological age in the human brain. Human Molecular Genetics 2011, 20:1164-1172.

21. Koch C, Wagner W: Epigenetic-aging-signature to determine age in different tissues. Aging 2011, 3:1018-1027.

22. Numata S, Ye T, Hyde Thomas M, Guitart-Navarro X, Tao R, Wininger M, Colantuoni C, Weinberger Daniel R, Kleinman Joel E, Lipska Barbara K: DNA Methylation Signatures in Development and Aging of the Human Prefrontal Cortex. The American Journal of Human Genetics 2012, 90:260-272.

23. Bocklandt S, Lin W, Sehl ME, Sanchez FJ, Sinsheimer JS, Horvath S, Vilain E: Epigenetic predictor of age. PLoS ONE 2011, 6:e148215.

24. Hannum G, Guinney J, Zhao L, Zhang L, Hughes G, Sadda S, Klotzle B, Bibikova M, Fan J-B, Gao Y, et al: Genome-wide Methylation Profiles Reveal Quantitative Views of Human Aging Rates. Molecular cell 2012.

25. Laird PW: The power and the promise of DNA methylation markers. Nat Rev Cancer 2003, 3:253-266.

26. Bjornsson HT, Sigurdsson MI, Fallin MD, Irizarry RA, Aspelund T, Cui H, Yu W, Rongione MA, Ekström TJ, Harris TB, et al: Intra-individual Change Over Time in DNA Methylation With Familial Clustering. JAMA: The Journal of the American Medical Association 2008, 299:2877-2883.

27. Pai AA, Bell JT, Marioni JC, Pritchard JK, Gilad Y: A Genome-Wide Study of DNA Methylation Patterns and Gene Expression Levels in Multiple Human and Chimpanzee Tissues. PLoS Genet 2011, 7:e1001316.

28. Hernando-Herraez I, Prado-Martinez J, Garg P, Fernandez-Callejo M, Heyn H, Hvilsom C, Navarro A, Esteller M, Sharp A, Marques-Bonet T: Dynamics of DNA Methylation in Recent Human and Great Apes Evolution. PLoS Genet 2013, In Press.

29. Ernst J, Kheradpour P, Mikkelsen TS, Shoresh N, Ward LD, Epstein CB, Zhang X, Wang L, Issner R, Coyne M, et al: Mapping and analysis of chromatin state dynamics in nine human cell types. Nature 2011, 473:43-49.

30. Adkins RM, Krushkal J, Tylavsky FA, Thomas F: Racial differences in gene-specific DNA methylation levels are present at birth. Birth Defects Research Part A: Clinical and Molecular Teratology 2011, 91:728-736.

31. Bell J, Pai A, Pickrell J, Gaffney D, Pique-Regi R, Degner J, Gilad Y, Pritchard J: DNA methylation patterns associate with genetic and gene expression variation in HapMap cell lines. Genome Biology 2011, 12:R10.

32. Fraser H, Lam L, Neumann S, Kobor M: Population-specificity of human DNA methylation. Genome Biology 2012, 13:R8.

33. van Eijk K, de Jong S, Boks M, Langeveld T, Colas F, Veldink J, de Kovel C, Janson E, Strengman E, Langfelder P, et al: Genetic Analysis of DNA Methylation and Gene Expression Levels in Whole Blood of Healthy Human Subjects. BMC Genomics 2012, 13:636.

34. Jones M, Fejes A, Kobor M: DNA methylation, genotype and gene expression: who is driving and who is along for the ride? Genome Biology 2013, 14:126.

35. Shibata D, Tavaré S: Counting Divisions in a Human Somatic Cell Tree: How, What and Why. Cell Cycle 2006, 5:610-614.

36. Richardson B: Impact of aging on DNA methylation. Ageing Research Reviews 2003, 2:245-261.

37. Kim JY, Tavaré S, Shibata D: Counting human somatic cell replications: Methylation mirrors endometrial stem cell divisions. Proceedings of the National Academy of Sciences of the United States of America 2005, 102:17739-17744.

38. Thomson JA, Itskovitz-Eldor J, Shapiro SS, Waknitz MA, Swiergiel JJ, Marshall VS, Jones JM: Embryonic Stem Cell Lines Derived from Human Blastocysts. Science 1998, 282:1145-1147.

39. Hinoue T, Weisenberger DJ, Lange CPE, Shen H, Byun H-M, Van Den Berg D, Malik S, Pan F, Noushmehr H, van Dijk CM, et al: Genome-scale analysis of aberrant DNA methylation in colorectal cancer. Genome Res 2012, 22:271-282.

40. Schwartzentruber J, Korshunov A, Liu X-Y, Jones DTW, Pfaff E, Jacob K, Sturm D, Fontebasso AM, Quang D-AK, Tonjes M, et al: Driver mutations in histone H3.3 and chromatin remodelling genes in paediatric glioblastoma. Nature 2012, 482:226-231.

41. Bernstein BE, Mikkelsen TS, Xie X, Kamal M, Huebert DJ, Cuff J, Fry B, Meissner A, Wernig M, Plath K, et al: A Bivalent Chromatin Structure Marks Key Developmental Genes in Embryonic Stem Cells. Cell 2006, 125:315-326.

42. Kolasinska-Zwierz P, Down T, Latorre I, Liu T, Liu XS, Ahringer J: Differential chromatin marking of introns and expressed exons by H3K36me3. Nat Genet 2009, 41:376-381.

43. Bjerke L, Mackay A, Nandhabalan M, Burford A, Jury A, Popov S, Bax DA, Carvalho D, Taylor KR, Vinci M, et al: Histone H3.3 Mutations Drive Pediatric Glioblastoma through Upregulation of MYCN. Cancer Discovery 2013.

44. Sturm D, Witt H, Hovestadt V, Khuong-Quang D-A, Jones David TW, Konermann C, Pfaff E, Tönjes M, Sill M, Bender S, et al: Hotspot Mutations in H3F3A and IDH1 Define Distinct Epigenetic and Biological Subgroups of Glioblastoma. Cancer Cell 2012, 22:425-437.

45. Webpage: http://labs.genetics.ucla.edu/horvath/dnamage

46. Friedman J, Hastie T, Tibshirani R: Regularization Paths for Generalized Linear Models via Coordinate Descent. Journal of Statistical Software 2010, 33:1-22.

47. Alisch RS, Barwick BG, Chopra P, Myrick LK, Satten GA, Conneely KN, Warren ST: Age-associated DNA methylation in pediatric populations. Genome Res 2012, 22:623-632.

48. Harris R, Nagy-Szakal D, Pedersen N, Opekun A, Bronsky J, Munkholm P, Jespersgaard C, Andersen P, Melegh B, Ferry G, et al: Genome-wide peripheral blood leukocyte DNA methylation microarrays identified a single association with inflammatory bowel diseases Inflamm Bowel Dis 2012, 18:2334-2341.

49. Gibbs JR, van der Brug MP, Hernandez DG, Traynor BJ, Nalls MA, Lai S-L, Arepalli S, Dillman A, Rafferty IP, Troncoso J, et al: Abundant Quantitative Trait Loci Exist for DNA Methylation and Gene Expression in Human Brain. PLoS Genet 2010, 6:e1000952.

50. Guintivano J, Aryee MJ, Kaminsky ZA: A cell epigenotype specific model for the correction of brain cellular heterogeneity bias and its application to age, brain region and major depression. Epigenetics 2013, 8:290-302.

51. Zhuang J, Jones A, Lee S-H, Ng E, Fiegl H, Zikan M, Cibula D, Sargent A, Salvesen HB, Jacobs IJ, et al: The Dynamics and Prognostic Potential of DNA Methylation Changes at Stem Cell Gene Loci in Women's Cancer. PLoS Genet 2012, 8:e1002517.

52. Essex MJ, Thomas Boyce W, Hertzman C, Lam LL, Armstrong JM, Neumann SMA, Kobor MS: Epigenetic Vestiges of Early Developmental Adversity: Childhood Stress Exposure and DNA Methylation in Adolescence. Child Development 2011, 84:58-75.

53. Martino DJ, Tulic MK, Gordon L, Hodder M, Richman T, Metcalfe J, Prescott SL, Saffery R: Evidence for age-related and individual-specific changes in DNA methylation profile of mononuclear cells during early immune development in humans. Epigenetics: official journal of the DNA Methylation Society 2011, 6.

54. Fernández-Tajes J, Soto-Hermida A, Vázquez-Mosquera ME, Cortés-Pereira E, Mosquera A, Fernández-Moreno M, Oreiro N, Fernández-López C, Fernández JL, Rego-Pérez I, Blanco FJ: Genome-wide DNA methylation analysis of articular chondrocytes reveals a cluster of osteoarthritic patients. Annals of the Rheumatic Diseases 2013:PMID: 23505229.

55. Harris RA, Nagy-Szakal D, Kellermayer R: Human metastable epiallele candidates link to common disorders. Epigenetics 2013, 8:157-163.

56. Grönniger E, Weber B, Heil O, Peters N, Stab F, Wenck H, Korn B, Winnefeld M, Lyko F: Aging and Chronic Sun Exposure Cause Distinct Epigenetic Changes in Human Skin. PLoS Genet 2010, 6:e1000971.

57. Zouridis H, Deng N, Ivanova T, Zhu Y, Wong B, Huang D, Wu YH, Wu Y, Tan IB, Liem N, et al: Methylation Subtypes and Large-Scale Epigenetic Alterations in Gastric Cancer. Science Translational Medicine 2012, 4:156ra140.

58. Haas J, Frese KS, Park YJ, Keller A, Vogel B, Lindroth AM, Weichenhan D, Franke J, Fischer S, Bauer A, et al: Alterations in cardiac DNA methylation in human dilated cardiomyopathy. EMBO Molecular Medicine 2013, 5:413-429.

59. Shen J, Wang S, Zhang Y-J, Kappil M, Wu H-C, Kibriya MG, Wang Q, Jasmine F, Ahsan H, Lee P-H, et al: Genome-wide DNA methylation profiles in hepatocellular carcinoma. Hepatology 2012, 55:1799-1808.

60. Bork S, Pfister S, Witt H, Horn P, Korn, B, Ho A, Wagner W: DNA methylation pattern changes upon long-term culture and aging of human mesenchymal stromal cells. Aging Cell 2010, 9:54-63.

61. Gordon L, Joo JE, Powell JE, Ollikainen M, Novakovic B, Li X, Andronikos R, Cruickshank MN, Conneely KN, Smith AK, et al: Neonatal DNA methylation profile in human twins is specified by a complex interplay between intrauterine environmental and genetic factors, subject to tissue-specific influence. Genome Res 2012, 22:1395-1406.

62. Kobayashi Y, Absher DM, Gulzar ZG, Young SR, McKenney JK, Peehl DM, Brooks JD, Myers RM, Sherlock G: DNA methylation profiling reveals novel biomarkers and important roles for DNA methyltransferases in prostate cancer. Genome Res 2011, 21:1017-1027.

63. Liu J, Morgan M, Hutchison K, Calhoun VD: A Study of the Influence of Sex on Genome Wide Methylation. PLoS ONE 2010, 5:e10028.

64. Song H, Ramus SJ, Tyrer J, Bolton KL, Gentry-Maharaj A, Wozniak E, Anton-Culver H, Chang-Claude J, Cramer DW, DiCioccio R, et al: A genome-wide association study identifies a new ovarian cancer susceptibility locus on 9p22.2. Nat Genet 2009, 41:996-1000.

65. Liu Y, Aryee MJ, Padyukov L, Fallin MD, Hesselberg E, Runarsson A, Reinius L, Acevedo N, Taub M, Ronninger M, et al: Epigenome-wide association data implicate DNA methylation as an intermediary of genetic risk in rheumatoid arthritis. Nat Biotech 2013, 31:142-147.

66. Heyn H, Li N, Ferreira HJ, Moran S, Pisano DG, Gomez A, Diez J, Sanchez-Mut JV, Setien F, Carmona FJ, et al: Distinct DNA methylomes of newborns and centenarians. Proceedings of the National Academy of Sciences 2012, 109:10522-10527.

67. Lam LL, Emberly E, Fraser HB, Neumann SM, Chen E, Miller GE, Kobor MS: Factors underlying variable DNA methylation in a human community cohort. Proceedings of the National Academy of Sciences 2012, 109:17253-17260.

68. Khulan B, Cooper WN, Skinner BM, Bauer J, Owens S, Prentice AM, Belteki G, Constancia M, Dunger D, Affara NA: Periconceptional maternal micronutrient supplementation is associated with widespread gender related changes in the epigenome: a study of a unique resource in the Gambia. Human Molecular Genetics 2012, 21:2086-2101.

69. Martino D, Maksimovic J, Joo JH, Prescott SL, Saffery R: Genome-scale profiling reveals a subset of genes regulated by DNA methylation that program somatic T-cell phenotypes in humans. Genes Immun 2012, 13:388-398.

70. Heyn H, Moran S, Esteller M: Aberrant DNA methylation profiles in the premature aging disorders Hutchinson-Gilford Progeria and Werner syndrome. Epigenetics 2013, 8:28-33.

71. Ginsberg MR, Rubin RA, Falcone T, Ting AH, Natowicz MR: Brain Transcriptional and Epigenetic Associations with Autism. PLoS ONE 2012, 7:e44736.

72. Martino D, Loke Y, Gordon L, Ollikainen M, Cruickshank M, Saffery R, Craig J: Longitudinal, genome-scale analysis of DNA methylation in twins from birth to 18 months of age reveals rapid epigenetic change in early life and pair-specific effects of discordance. Genome Biology 2013, 14:R42.

73. Ribel-Madsen R, Fraga MF, Jacobsen S, Bork-Jensen J, Lara E, Calvanese V, Fernandez AF, Friedrichsen M, Vind BF, Højlund K, et al: Genome-Wide Analysis of DNA Methylation Differences in Muscle and Fat from Monozygotic Twins Discordant for Type 2 Diabetes. PLoS ONE 2012, 7:e51302.

74. Jacobsen SC, Brøns C, Bork-Jensen J, Ribel-Madsen R, Yang B, Lara E, Hall E, Calvanese V, Nilsson E, Jorgensen SW, et al: Effects of short-term high-fat overfeeding on genome-wide DNA methylation in the skeletal muscle of healthy young men. Diabetologia 2012, 55:3341-3349.

75. Blair JD, Yuen RKC, Lim BK, McFadden DE, von Dadelszen P, Robinson WP Widespread DNA hypomethylation at gene enhancer regions in placentas associated with early-onset pre-eclampsia. Molecular Human Reproduction 2013.

76. Teschendorff A, Jones A, Fiegl H, Sargent A, Zhuang J, Kitchener H, Widschwendter M: Epigenetic variability in cells of normal cytology is associated with the risk of future morphological transformation. Genome Medicine 2012, 4:24.

77. Pacheco SE, Houseman EA, Christensen BC, Marsit CJ, Kelsey KT, Sigman M, Boekelheide K: Integrative DNA Methylation and Gene Expression Analyses Identify DNA Packaging and Epigenetic Regulatory Genes Associated with Low Motility Sperm. PLoS ONE 2011, 6:e20280.

78. Krausz C, Sandoval J, Sayols S, Chianese C, Giachini C, Heyn H, Esteller M: Novel Insights into DNA Methylation Features in Spermatozoa: Stability and Peculiarities. PLoS ONE 2012, 7:e44479.

79. Nazor Kristopher L, Altun G, Lynch C, Tran H, Harness Julie V, Slavin I, Garitaonandia I, Müller F-J, Wang Y-C, Boscolo Francesca S, et al: Recurrent Variations in DNA Methylation in Human Pluripotent Stem Cells and Their Differentiated Derivatives. Cell stem cell 2012, 10:620-634.

80. Shao K, Koch C, Gupta MK, Lin Q, Lenz M, Laufs S, Denecke B, Schmidt M, Linke M, Hennies HC, et al: Induced Pluripotent Mesenchymal Stromal Cell Clones Retain Donor-derived Differences in DNA Methylation Profiles. Mol Ther 2012.

81. Calvanese V, Fernández AF, Urdinguio RG, Suárez-Alvarez B, Mangas C, Perez-Garcia V, Bueno C, Montes R, Ramos-Mejia V, Martinez-Camblor P, et al: A promoter DNA demethylation landscape of human hematopoietic differentiation. Nucleic Acids Research 2012, 40:116-131.

82. Ramos-Mejia V, Fernandez A, Ayllon V, Real P, Bueno C, Anderson P, Martin F, Fraga M, Menendez P: Maintenance of human embryonic stem cells in mesenchymal stem cell-conditioned media augments hematopoietic specification. Stem Cells Dev 2012, 21:1549-1558.

83. Reinius LE, Acevedo N, Joerink M, Pershagen G, Dahlén S-E, Greco D, Söderhäll C, Scheynius A, Kere J: Differential DNA Methylation in Purified Human Blood Cells: Implications for Cell Lineage and Studies on Disease Susceptibility. PLoS ONE 2012, 7:e41361.

84. Fackler MJ, Umbricht CB, Williams D, Argani P, Cruz L-A, Merino VF, Teo WW, Zhang Z, Huang P, Visvananthan K, et al: Genome-wide Methylation Analysis Identifies Genes Specific to Breast Cancer Hormone Receptor Status and Risk of Recurrence. Cancer Research 2011, 71:6195-6207.

85. Dedeurwaerder S, Desmedt C, Calonne E, Singhal SK, Haibe-Kains B, Defrance M, Michiels S, Volkmar M, Deplus R, Luciani J, et al: DNA methylation profiling reveals a predominant immune component in breast cancers. EMBO Molecular Medicine 2011, 3:726-741.

86. Lauss M, Aine M, Sjödahl G, Veerla S, Patschan O, Gudjonsson S, Chebil G, Lövgren K, Fernö M, Månsson W, et al: DNA methylation analyses of urothelial carcinoma reveal distinct epigenetic subtypes and an association between gene copy number and methylation status. Epigenetics 2012, 7:858-867.

87. Langfelder P, Mischel PS, Horvath S: When is hub gene selection better than standard meta-analysis? PLoS ONE 2013, 8:e61505.

88. Lee TI, Jenner RG, Boyer LA, Guenther MG, Levine SS, Kumar RM, Chevalier B, Johnstone SE, Cole MF, Isono K-i, et al: Control of Developmental Regulators by Polycomb in Human Embryonic Stem Cells. Cell 2006, 125:301-313.

89. Miller JA, Cai C, Langfelder P, Geschwind DH, Kurian SM, Salomon DR, Horvath S: Strategies for aggregating gene expression data: The collapseRows R function. BMC Bioinformatics 2011, 12:322.

90. Teschendorff AE, Menon U, Gentry-Maharaj A, Ramus SJ et al. Age-dependent DNA methylation of genes that are suppressed in stem cells is a hallmark of cancer. Genome Res 2010 Apr;20(4):440-6. PMID: 20219944

91. Rakyan VK, Down TA, Maslau S, Andrew T et al. Human aging-associated DNA hypermethylation occurs preferentially at bivalent chromatin domains. Genome Res 2010 Apr;20(4):434-9. PMID: 20219945

92. Gibbs JR, van der Brug MP, Hernandez DG, Traynor BJ, Nalls MA, Lai SL, Arepalli S, Dillman A, Rafferty IP, Troncoso J, Johnson R, Zielke HR, Ferrucci L, Longo DL, Cookson MR, Singleton AB. Abundant quantitative trait loci exist for DNA methylation and gene expression in human brain. PLoS Genet. 2010 May 13;6(5):e1000952.

93. Bocklandt S, Lin W, Sehl ME, Sanchez FJ, Sinsheimer JS, et al. 2011 Epigenetic Predictor of Age. PLoS ONE 6(6): e14821

94. Pacheco SE, Houseman EA, Christensen BC, Marsit CJ et al. Integrative DNA methylation and gene expression analyses identify DNA packaging and epigenetic regulatory genes associated with low motility sperm. PLoS One 2011;6(6):e20280. PMID: 21674046

95. Song H, Ramus SJ, Tyrer J, Bolton KL, Gentry-Maharaj A, Wozniak E,Anton-Culver H, Chang-Claude J, Cramer DW, DiCioccio R, et al. 2009. A genome-wide association study identifies a new ovarian cancersusceptibility locus on 9p22.2. Nat Genet 41: 996-1000
96. Adkins RM, Thomas F, Tylavsky FA, Julia Krushkal (2011) Parental ages and levels of DNA methylation in the newborn are correlated. BMC Med Genet. 2011; 12: 47.
97. Liu J, Morgan M, Hutchison K, Calhoun VD. A study of the influence of sex on genome wide methylation. PLoS One 2010 Apr 6;5(4):e10028. PMID: 20386599
98. Adkins, RM, Krushkal, J, Tylavsky, FA and Thomas, F (2011), Racial differences in gene-specific DNA methylation levels are present at birth. Birth Defects Research Part A: Clinical and Molecular Teratology, 91: 728-736. doi: 10.1002/bdra.20770
99. Teschendorff AE, Menon U, Gentry-Maharaj A, Ramus SJ et al. Age-dependent DNA methylation of genes that are suppressed in stem cells is a hallmark of cancer. Genome Res 2010 Apr;20(4):440-6. PMID: 20219944
100. Rakyan VK, Down TA, Maslau S, Andrew T et al. Human aging-associated DNA hypermethylation occurs preferentially at bivalent chromatin domains. Genome Res 2010 Apr;20(4):434-9. PMID: 20219945"
101. Bocklandt S, Lin W, Sehl ME, Sanchez FJ, Sinsheimer JS, Horvath S, Vilain E (2011) Epigenetic Predictor of Age. PLoS ONE 6(6): e14821

TABLE 3: Listing of 354 CpGs Set

This Table provides sequence and methylation residue information (in brackets) for the 354 clock CpGs of the present invention. Further explanations of these sequences can be found, for example, on the Illumina™ website, under Technical Note: Epigenetics

- CpG Loci Identification (Search: "res.illumina.com/documents/products/technotes/technote_cpg_loci_identification.pdf"). Briefly, these 354 CpGs correspond to Illumina probes specified by so called Cluster CG numbers (see Table 1 in the Illumina™ Techical Notes). For convenience, the genomic coordinates of these clock CpGs and the gene names are also provided.

| SEQ ID NO. | Probe | Sequence with the CpG site marked with [] | Chro moso me | Position | Gene |
|---|---|---|---|---|---|
| 1 | cg00075967 | GGTGTGGCCAGGAGCCACCCCCACCCC CGCACCTGACTTCACACACATACCTGC CTTCAG[CG]CCTGCCCCAGAGCTCCCA AGCCCCTGCCCGCCACATCTGCAGTGC CGCACACAGACAGGA | 15 | 74495354 | STRA6 |
| 2 | cg00374717 | AAACCTTACAGAAACATGAAGCCCTCA ACCATCTGCTACTCAGTTATTCGGGGC TGACGG[CG]GCTTCTAGAACATCCAGG TGTTCTGCAGATGCGAGAACTCATCCT GTAGTCACCAGATGG | 17 | 66303145 | ARSG |
| 3 | cg00864867 | AGTACAAGACCGTATTATTTGAGAGAA AGTCTCGAACGCTGCTGGCTAAGGGGA AAAGTG[CG]ATAACTTGTGATGATTCA GGGAATGACTAGACAGGATGGGAAAA TACCCACGTGTCTCTT | 12 | 80085268 | PAWR |
| 4 | cg00945507 | TGGGATTACAGACGTGAGCCACCGCGC CCGGCCATGTTTCCTTTTAGCAATGGA GCATAA[CG]GGATCTGAGGAACAATAT AACTCAGGAAGAGCTGATGGAACATTA AGACGTGTTACAACT | 7 | 54827677 | SEC61G |
| 5 | cg01027739 | CCTTAACTGTAGCTAAGCTTCCACTCTT AAGTATCAATTAAGCTTCTCTGTTCAGT CCAG[CG]TTTAGGGCGCCTACTGCGCG CCCCGCCCCACACACTTTTGACAAAAA GGTCGCCTGCTCT | 9 | 131842738 | DOLPP1 |
| 6 | cg01353448 | GCCCAGCCTCGGTGAGCACACACGCCC TCCCTGTCTCTCGCCTTCGCTTCCCTGC ATCTG[CG]CTGATTGGTAAGTGCTTCA GATTTTTACTCCAAGAACTTTTGTGGTG AGAAAAGCAAGTT | 7 | 31726912 | C7orf16 |
| 7 | cg01584473 | CAGGGACCAAAGGTCTCTGGCACCCAT TTATTTATCAGTTTCCTTCTCTGAGGCT CATTT[CG]CCAGCTCCTCTGGGGGTGA CAGGCAAGTGAGACGTGCTCAGAGCTC CGATGCCAAGGCCA | 7 | 100663367 | MUC17 |

(continued)

| SEQ ID NO. | Probe | Sequence with the CpG site marked with [] | Chro moso me | Position | Gene |
|---|---|---|---|---|---|
| 8 | cg01644850 | ACAGCACCTCAGAATACAAGTTCGCAG AGGTCAAAGCAGTGGACACACTCCGA AGAGCTC[CG]TGGAGTTTTGGAAACTA CATTATCCAGAGTGCAGAGCGCAAAAC GGCGGCGGAGTTGAGC | 19 | 58193231 | ZNF551 |
| 9 | cg01656216 | CATGTGCATAATACTGTGGAAATTAGT AAACAGTCACAAACAAGTGATTCATAT TCAGGG[CG]CAGCCTTTTTGACAGGAA AACAGTAATCAAGAGTTTGGGATTTGA AGATTTTTAAAAGGA | 10 | 31273710 | ZNF438 |
| 10 | cg01873645 | TTGGTTTTCTTTCCCCTCATCCTTTTGC CTGCTCCCGGCGAGGGGTGGCTTTGAT TTCGG[CG]ATGAGCTCCCAGAAAGGCA ACGTGGCTCGTTCCAGACCTCAGAAGC ACCAGAATACGTTT | 9 | 74526649 | FAM108B 1; C9orf85 |
| 11 | cg01968178 | CTGCAGCGGCCCCGTTTGCAGGGCAGG GACCCGGGTGCTGCCCCACCCTCAGCG TTCCAG[CG]GAGAAACTGAAGTCCGAA CCTGAACCTCGGGAATCTGTCTGCACC TGTCTAGGTGGGATG | 2 | 86565038 | REEP1 |
| 12 | cg02085507 | CTGGGGGAGGGAAGGCAGGATGCGGT GCGGGAGTTAATGGACCTGGCCTTGGC GAAGGCG[CG]TCCTGGGTTGGATCGAA ACCCTCTCATCCGCCCTGTGGCCGGAG GGACCAGACCATTAGT | 19 | 6739192 | TRIP10 |
| 13 | cg02154074 | TGGGGAACGCGAGTGGGGACAGGGGG GCCTTCAGCTGGGCCCCAGGGAACCGC CCCGTGG[CG]CTCTCGGCCTCGCTCTC ACTCACGGTGCTACAGGTGGTAAGCAA ATTGACTATGTTGTGG | 2 | 74756234 | HTRA2; AUP1 |

(continued)

| SEQ ID NO. | Probe | Sequence with the CpG site marked with [] | Chro moso me | Position | Gene |
|---|---|---|---|---|---|
| 14 | cg02217159 | TATTTCCGATGACCTACATCTCAGGGA CGCAGTAGGATGTTCATTGATAAACAA ATAAAG[CG]GCTCGAAGAAATATTGTG CAGAGACATGATTGAGGTGTACAATCA TTAGGATATTGAATT | 6 | 62996697 | KHDRBS2 |
| 15 | cg02331561 | CAGCGGCGGTAGCCGAGCGAGGGCGC GGTGGCCTCTGACAGGAATGACTCTGC GCACGTG[CG]TTTCGCAGCAGTGGAAG TCTTCACACCCGGAAACTCGACTTTGG CCGTTTCTCCATTTCT | 16 | 2391081 | ABCA17P; ABCA3 |
| 16 | cg02332492 | CGGGGCAGCTGTCAGTGAAGCTCTACG GTATGTGGGGGCCAGCCTCTGTGACCA GGCAGG[CG]CTCAAGCTCTGCACACTC ACTGGGCCACCCCGAGGGGCTGGGTG AGCCCATGGGGACACA | 9 | 139840678 | C8G |
| 17 | cg02364642 | GGGTCGCTGTGCCTGTCCCCGTGTGAT CCGAAAAGTGCTGGCAAAATGCGGCTG CTGCTT[CG]CCCGGGGGGGACGTGGTG AGTGCCAGGTCGAGAGGGTCCAGTGTT GAGTGGGGGGCGGGC | 12 | 58005758 | GEFT |
| 18 | cg02388150 | AACCTATGAAAATAAACAAAAGCTGCT CCAAGCATTCTCTCGGCCTTTCTGAACT TTCTA[CG]CTTTGGGTTTTTGTTTTTTCC TCCCGTCTCAGAGGTTAAAAACTTCGA TAGGGACTCGGA | 8 | 41165699 | SFRP1 |
| 19 | cg02479575 | GAGGGACAGCTCTCCACCGACCGAAG GAGGAGAATGCTATTTATTTCAGCACC AAATATC[CG]GACAGCGCCTCTCGGGA GGTCCGAGAAGAGAACCGCGATCTGTT TCAGCACCGGGGCTCA | 19 | 4769653 | MIR7-3; C19orf30 |

(continued)

| SEQ ID NO. | Probe | Sequence with the CpG site marked with [] | Chro moso me | Position | Gene |
|---|---|---|---|---|---|
| 20 | cg02489552 | CTCCTCCCCCCACCTCTGGAATTCCACCTCCCTTGTTGCGCCCATCGCTATGGTGACGGG[CG]CTCTCAGTACACTGTCTCTACAGGCCAGGAAAGAGTTGTGTGTCTTTGGGGTCCCTTCCG | 19 | 15121531 | CCDC105 |
| 21 | cg02580606 | AACCTAAATTTTGGGAGCACCTACTCTGCATGAAGCACTGTGCTCCATGCCTGTGCACAG[CG]TGACTCTGTCATTGGTGATGGGTCCTGCTTGCTGAGCCTCCACTGTGCACCAGGCACAGT | 17 | 39526726 | KRT33B |
| 22 | cg02654291 | GCCTCGAAGAGCATTATGGCCGTAGATCTGGGTGCTGAGGACTGAGCCACCCCCAGACTG[CG]ACATGGGCGGCGGTGCCTCCTTCCCCAAGCCCCAGGGAGTGTTTTTTTGTTTGTTTTGTT | 9 | 86572014 | C9orf64 |
| 23 | cg02827112 | AATTGTTGCGGCCTAACAATGAAGCGCAGCCATAACAGTCCTGAGCCACTGGCATGTTTG[CG]GGCCCTTTATTGCCTTGGGAATAAACTGCTGTGGCATTGTATCGTATATTGTTTTCATGG | 4 | 95129403 | SMARCA D1 |
| 24 | cg02972551 | ACCCTTTCCTGTGAGATTCTTCCGCCAAGTGGAAGGCTCATCTTCGGTCGACAGCCTACG[CG]GTTGAAGAACAATCCAGTAGGCACTTATAGCTCAGGGTCTCGCCATTCAGTCTTATCTAT | 2 | 86668068 | KDM3A |
| 25 | cg03103192 | AAGCTAGAAGTAAGAAGTACTGAAATTTTAGTTACAAGTTTCATACAGGTAAACCCAAGG[CG]CTACAAATGAAGAATTAAAGGAATGAAAGGCGAAAGAATAAAGGGGCCAAAGAGGTGATC | 4 | 52917271 | SPATA18 |

(continued)

| SEQ ID NO. | Probe | Sequence with the CpG site marked with [] | Chro moso me | Position | Gene |
|---|---|---|---|---|---|
| 26 | cg03167275 | GCCTGGACGGTGTTAGTCTCCTGGAAGCAGCTCGCCCAGGCAGGAGCTGCTAACCAGACG[CG]CATTGTGAAGGAGACCGTGGAAAATCAAAAGTGGGTTCCTGCAAAAATGTAGCATTGGTT | 21 | 18886093 | CXADR |
| 27 | cg03270204 | AAGAGAGTGGGCCCGCCTTCAGGGTCTGGGGCCTTCCAGGTTGGGTCGTAGGGGCGGGAG[CG]CACAGGCTGCGAGAGAGGAGCAAAGGTTGGTGGAGGGAGAAGAGCAGTCTGGGGCCTGGC | 6 | 30851638 | DDR1 |
| 28 | cg03565323 | TTTCCTAGAGGAAGAATGGGCAGGGAAGATGTGGGTCTAAAGGCAGAAAGACTTAATGTG[CG]GTTTCGGGCTTTACTGTGCATACATACTAACTGTGAAAGGTTTTCACTTCCTCCTCAGGA | 17 | 16472866 | ZNF287 |
| 29 | cg03588357 | GCCAGCGCGCACGCAGATGGCGGGGTGGCCTGGGGAGGTCTTCGGGTCCCTTCCTGGGAA[CG]CAGGGCCAAGTTGTGCTCCGATTCCACGCCCCCCCCACCCACGTCGGGCACACGCAGCCC | 14 | 91720173 | GPR68 |
| 30 | cg03760483 | ACAGCCGGCTCTACCGCTCTGCTCGCAGGTTTGGGCTAGTCTGGGGCGGGGACTTGGGAG[CG]CCTAAAACTTGCGAGGAGGGCGGGGCCGCAGACCGGTCCTTTAAAGGTTGGAAGTGGCCC | 17 | 6899297 | ALOX12 |
| 31 | cg04084157 | AGGGTGCCTGCCTCTCCCGGCCTGCGCCTGCGCGCTGGGGCCTTCGGCTGAAGGGGTGTG[CG]CTAGCGGAGCTCCGGGAAATGAATGAATGAATGAATGAATGAAATGCTGAAGCGGGCAGG | 7 | 100809049 | VGF |

(continued)

| SEQ ID NO. | Probe | Sequence with the CpG site marked with [] | Chro moso me | Position | Gene |
|---|---|---|---|---|---|
| 32 | cg04126866 | CTCCACCAACAGGAGCTCCTTGAGGCGAGGCACAGTGTCTTCTGTGTCCCTGGAGCCAAG[CG]CATGGCTCAGCCCAGGTCACGTGTCCAGTGAATGGGTGGCATCTGAGCCTCCTGCACCTG | 10 | 85932763 | C10orf99 |
| 33 | cg04528819 | GCAGCCCGGGAAGGGGCATTGGTGGCGCTTGGCAGCAGGTGTGACAGACCTCCTCCGGGG[CG]CCTGATCCGCGGCGGGGGCGGGGCCTGCCCCTAGGGCCCCTCCAGAGAACCCACCAGAGG | 7 | 130418315 | KLF14 |
| 34 | cg04836038 | CTCTGCGGGGACAGAGGTCTCAGGAAAGTAGCCTTTATTTATGTGGCACCGATCGGAACC[CG]CGGCCGGCCAGGCGGACCTGGACGGAGCGTCCCTGCTCGGAACCTGGCGCGGGGCGCCGC | 13 | 99739382 | DOCK9 |
| 35 | cg05250458 | TTAATTGGCTTGTGCCTCTTATTTTACTCTAATGCAATGAATAAAGACAGTCCCAGCCTT[CG]CCCTAAGGGAGCAGGAGCACCTGCGATGCCCCGTTCCCAAGTCCTCAGGGCGAATCCGCC | 19 | 9473565 | ZNF177 |
| 36 | cg05294243 | GATGTCTCCAGGCACCCCCGACCTGGGCTTGGCCCTCTGCTTGGGGCGGAGCTTCCAGGA[CG]TGCTGGGACCTAGGTCTGACCCCGCCCAAGGCAGAGTTGAACCCACTGTGAACTTTCAGG | 19 | 51569106 | KLK13 |
| 37 | cg05365729 | ACATAATACACGCTCAATTAAAGCTGCCGAATGAAAGTGTTCAGAAACTTGCACCCATCT[CG]CCTGGGTTTCACCTCCCTTTTCCTGTAGGGGGAAAACCGATCCTGAACCAGTAAATAAAC | 8 | 23262073 | LOXL2 |

(continued)

| SEQ ID NO. | Probe | Sequence with the CpG site marked with [] | Chro moso me | Position | Gene |
|---|---|---|---|---|---|
| 38 | cg05675373 | AAGGAGGAGATGGCCAAGGGCGAGGCGTCGGAGAAGATCATCATCAACGTGGGCGGCACG[CG]ACATGAGACCTACCGCAGCACCCTGCGCACCCTACCGGGAACCCGCCTCGCCTGGCTGGC | 1 | 110754257 | KCNC4 |
| 39 | cg05755779 | CCTGGTACTATTTCTTTTGCAAATTCAGAGTCTGGGTCTGGATATTGATAGCCGTCCTAC[CG]CTGAAGTCTGTGCCACACACACAATTTCACCAGGACCCAAAGGTGAGGAAAGAAAACCAC | 8 | 120079625 | COLEC10 |
| 40 | cg05921699 | AAGAATTCCAGTAAAGAGCTGATCATGGTTCTCACTCCTTGAATACCAGGAACACCATCT[CG]TATCACATAATGAGACAGGGAGACATTCTGGTCCTCATCTCACAGATGAAAAATGTCAAG | 19 | 42380725 | CD79A |
| 41 | cg05960024 | CAAGGAAAGTAGCAGATCATTACCCAAGTATTTTTATAATTCCTTGTCCTATGCTTCCAC[CG]GTACACTGCAAATTCCACCCAACCATGATTAAGGGAAAAGAAACAAAGATAGCATACCTT | 4 | 56376020 | CLOCK |
| 42 | cg06121469 | CCAGTCCCACTCTGCTTAACTGCTCTGGCATGCTTGAAGGCCTAGCTTAGCGTAGCAGGC[CG]TTGCAGCCGTTCTCGCTCTGTGGCATTGCTCTTTGCCTTCTTGGTCCAGCTGCCTCCAGC | 15 | 44956098 | SPG11 |
| 43 | cg06144905 | CTGACCTCACCACCCACCAGGGAGGTGGGTCTTATTCTGGGCATCGTGCCAAGTTCTTAG[CG]GGGCCCTCTAGAATCTCTAAAGCAAATCAGGCTGAAGAGGGGAAAACCAGCAGGGGGAGG | 17 | 27369780 | PIPOX |

(continued)

| SEQ ID NO. | Probe | Sequence with the CpG site marked with [] | Chro moso me | Position | Gene |
|---|---|---|---|---|---|
| 44 | cg06361108 | GGTCAGCGTTCCGCGGGGGAGACTTCCCAGCGTCAGCTCCGACCTCCTCTTTCTCTACCA[CG]ATCCCGGCCAGCATCCCCGCCCAGCAGCGGCTCAGCCACAAACCCAAGGGTCTCCACCTG | 16 | 2478781 | CCNF |
| 45 | cg06462291 | TCTCTCCGCATTAATGGCCTCTGGCAGTCTAATTAATGGCAGTCTGGACCTCCCCTGGAT[CG]TGGGGCCCCTCTGAGACGTCCCCGATCCCCAGCTTAAATTTATCCAGGAGGACCTGTGAG | 12 | 104235479 | NT5DC3 |
| 46 | cg06493994 | GGAGAGCAAGTCAAGAAATACGGTGAAGGAGTCCTTCCCAAAGTTGTCTAGGTCCTTCCG[CG]CCGGTGCCTGGTCTTCGTCGTCAACACCATGGACAGCTCCCGGGAACCGACTCTGGGGCG | 6 | 25652602 | SCGN |
| 47 | cg06557358 | AGCATCGAGACAGCGGGCGAACGGGCGTCCGGGGACAGGGTGGGGGCGGCGGGGAGGAGG[CG]TCGGAGACTCTGAACCCCAGAAAAGTTCAAGGTTTGTGCAGGTTCCCCCAGGGAAGGCGA | 17 | 32907002 | TMEM132 E; C17orf102 |
| 48 | cg06738602 | ACTTCATTGTTTGGTGAGTTGCTTTGCTTTGCTCGTTGCCCCGATCTTCTGTGTATTCTG[CG]CAGACCCCGCAAGTGCTCCTGCACTCCCTCCCAGCCCTCTGCTGGGGCTTAACGCTTCCC | 14 | 52780634 | PTGER2 |
| 49 | cg06810647 | TGCCGCGGGGGAGAGGAACCCCTCGCCCCAGCCGGGCTCCACCCTAGCTCACCCATCCCG[CG]GCCTACACTGAGGCTCTCAATTTGGGTGGCACTTATGGGGCATGTGTCCCCTCTCTCCTT | 16 | 1665094 | CRAMP 1L |

(continued)

| SEQ ID NO. | Probe | Sequence with the CpG site marked with [] | Chro moso me | Position | Gene |
|---|---|---|---|---|---|
| 50 | cg06952310 | TGGCATGGGCTAGAGAATAAAATGAGAATAGATTTTAAAAGGTCTTTGAACAGTCAAAAG[CG]AACAGGATACCTAAGAGGTTATTTTTAGTCATTGTCAGCAGAAGCTGGAGATTCCCGCCT | 19 | 19327990 | NCAN |
| 51 | cg06993413 | GAGGCGCGGGGTGGAGACTGGGCCGAGCAGGGGATAGAGATGAACTCCAGAAAGGAACAG[CG]ACTTGCTGAAAGTCACAGGGCAAAATGTGGCGCGTCTGTAGTCAATAAATAATATATATT | 15 | 65810204 | DPP8 |
| 52 | cg07285276 | GGCCTCAGGTCTTTCTCCCAAATAGCAGAGAACTCAAATGAAGAGTCATTTCATTCCCAG[CG]GTTTGGGCAGCTCATGGGATGACAGGCAACTTTTTCCTTTTTTTAAAAAAAGAGGCCCAG | 9 | 134613015 | RAPGEF1 |
| 53 | cg07291563 | CGCTACGCGAAGGGGAGGAGCTGGTCATGGACGAGGAGGCCTATGTGCTCTACCACCGAG[CG]CAGACTGGTAGGGCTGAGTCCGGACTCCAGGGTCCTGAGGTGGCTGATCCCGAGCCTTTA | 19 | 48949441 | GRWD1 |
| 54 | cg07337598 | GGCTGTGTTTAGACCTGAGGGAGCCAGCTGTGAGGCTGGAGCAGTTGCTGCATGGCGGGG[CG]GGGGCTCCACAGGGCTGTTCACCTGCTGCTCTGTGCAGAGACAGCCTCAAGTCCAGCTGC | 1 | 150953943 | ANXA9 |
| 55 | cg07455279 | GGTAACAGAGCACTGTGAGAGCCCGCAGAAAGCTCCTAACCCATCTGGGATGAGACCTAG[CG]CTTCCAGGACGAGCCGATGTTGAGCTGAGACCTCGAAGGACAGGTTAGTCATTCACCTTC | 19 | 54605703 | NDUFA3 |
| 56 | cg07595943 | CTTCGGCTTCTCAGGGCGCTGACGACG | 16 | 84224901 | ADAD2 |

(continued)

| SEQ ID NO. | Probe | Sequence with the CpG site marked with [] | Chro moso me | Position | Gene |
|---|---|---|---|---|---|
| | | ACGGCAGTCGTAGGAAGCCCCGCCTGGCTGCAT[CG]TTGCAGATCAGCCCCCAGCCCCGCCCCTGGCGACCGCTACCCGCCCAGGCCCAAAGTGCC | | | |
| 57 | cg08030082 | GGCGAGGGTGAAGTTACCTGCGTGCGTGCTGGGGCTGGCATCTGCCTGGTTCGCATTTGG[CG]GTAAATATCACCGTCTGCACACGGGGAGGCCTCCGATTTCCCCATTGTTTGGAAACTGTG | 2 | 25391839 | POMC |
| 58 | cg08090772 | TCTTACTCCGTGGGAAAATGGCCCTGAGCCCGACTGGCTTGAGGCTTAGACAGGTGACCC[CG]CGAAGCGGGTGGGCAGGCGCGGCCGAGGGGCGGGAGGCGGGCAGCCTCCGTGATTGGCCG | 8 | 67344640 | ADHFE1 |
| 59 | cg08124722 | CTTCCAGCAGAATTTGGGATCAGGGTGATCAAAGACAGGAGGCTTCTGGGGATGGGTGTG[CG]GGCTGTTTCCAGATACCGGGAGACCCAGAATCTGGTCTGTGGAAGCCCAGCTTCCAGAAA | 17 | 32597714 | CCL7 |
| 60 | cg08251036 | ATCTTGTTCACTGTTCAGTCACCAGGGCCTGATGGCCGCTCATGCTCAATATAGACTTGG[CG]CGGAGCGGAGTGGAGGAAGGAAAGAGGGCAGGTGCTAGTTGGCTGGCCTGCAGTTAGAAG | 2 | 135008923 | |
| 61 | cg08370996 | CCCTCCCGCGCCCCCCTTTTTAGCATATTTGATCACTTTGATTCTCTGTTCTTTTCTCTC[CG]CGGTGTGTGTGTGCGTGCGCGCGTGTGTGTTTTCTTCTTCTCCTCCTCCTCTCCCCGAGT | 15 | 96874031 | NR2F2 |
| 62 | cg08413469 | GCTGCGTCCTGGGGCTCCAGTAGCTGGCGCGGGCTGGGGTGGGCTGGGCTGGCCTGGGAC[CG]CCTCGATGGGACAGGCTCGGGTTTCCCTGGCGCTGTTTCTCCCTCCTGCGGTCTACGGCG | 1 | 68962940 | DEPDC1 |

(continued)

| SEQ ID NO. | Probe | Sequence with the CpG site marked with [] | Chro moso me | Position | Gene |
|---|---|---|---|---|---|
| 63 | cg08434234 | AGGTGCCCAACTCCGCGGAAGCGCCCC TTGCTGGGTAGAAGAGTGGGTCTCCCG CCGCGG[CG]CACCTGTCTCGGCTGCCG GCTCCCCGCACCTACCTGTACGAGACC TGCTTCCGGAAAGTT | 7 | 137531173 | DGKI |
| 64 | cg08771731 | TGAAAGCGATCCAAACACAGCCAGAG GGCGCCAAAATGCCGCAAATAAAAGTT CCAAAGG[CG]TCAACTGGCTTTTGCGG GAAGGTAAAATTGGCTTTTGTGTAATC AAAGAGCTACCGTTGT | 5 | 17216434 | LOC28569 6; BASP1 |
| 65 | cg08965235 | ACCCACGCGGAAGCCGGAGCCCGTGA GCGTGTCTGTGCTGTGGCCGTTCTCTCC GATGAG[CG]TCATGTTGGAGCCCTGCT GACAACTGTCCCGACACTGGCCCTTGA GACAGGTCCGCTTGC | 11 | 65325158 | LTBP3 |
| 66 | cg09019938 | CTGGAGTTGGATCAGAAGGACGAACTG ATCCAGAAGCTGCAGAACGAGCTGGA CAAGTAC[CG]CTCGGTGATCCGACCAG CCACCCAGCAGGCGCAGAAGCAGAGC GCGAGCACCTTGCAGGG | 10 | 52834498 | PRKG1 |
| 67 | cg09118625 | GCAGGGCGGGCAGAAGCCGCAACCGC TTCAGCAGCTTCTGTTCCTTGGAGCCA AAGCTGG[CG]TTACCCATCGTTGGGAT TCGGAGGGGAGATACGTGCACAAGTTC TCCCACACTTAGCTGG | 1 | 68512971 | DIRAS3 |
| 68 | cg09191327 | GCTCCGTGCTCCCGGCTGAGGCCCTGG TGCTCAAGACCGGGCTGAAGGCGCCG GGACTGG[CG]CTGGCCGAGGTTATCAC CTCCGACATCCTGCACAGCTTCCTGTA CGGCCGCTGGCGCAAC | 9 | 133540108 | PRDM12 |

(continued)

| SEQ ID NO. | Probe | Sequence with the CpG site marked with [] | Chro moso me | Position | Gene |
|---|---|---|---|---|---|
| 69 | cg09418283 | GGAGCTTGTAGGGGACGAGGCGTAGG GCTGGGATCCGGCTCCCAGGTGTGCCG AAGCTGG[CG]CGCGCTCTTCCGCCGCG CGGAAAGTGCCGCGGCAAACTCGCGG TGCGGAGCTCCAGGCAA | 12 | 80084718 | PAWR |
| 70 | cg09509673 | CCACAACCCCAGCCTCACACCACCAGC CCATTTATCTGGAGGACCCCTAGTCTG AGACAG[CG]CCAAGAATCCTGAATAA GCCATAGGATGGCAGAGGCCCATTGCC AGGTGGGGAATCCCAT | 17 | 40833697 | CCR10; CNTNAP1 |
| 71 | cg09785172 | GGCTCTTCAGCAGCGAGTGCAGATTGC TCCCCCGCGGCCGCAGATCTCCCGTTT GCGCCG[CG]TTCAGCTGCTCCCGAACA ACTTTTCTGCCGGCCCAGAGGCCCCAG GGCGTCGCAGCGCCG | 4 | 6271658 | WFS1 |
| 72 | cg09869858 | GTTGGATCTGACAATCCCTTCCAGGTT CTCAGACTTTAATCTCGAGTTTTCCTGC CCATG[CG]CCAGGTTGAACAGTTGCTG GTGGGTTAAAGAGAATCCCCCAGCCTG TTGCTGTGTAGAGA | 12 | 48120416 | P11 |
| 73 | cg09885951 | GTAGAGGGCTTGTTTTTAAAATCCATC CGAAAGGGCCAATCAGACGCGGCAGT CTGAGTG[CG]CAGGCGCGGATTGGTCC GCAGCTACTTAGAGTGACCAATAGGCG TGGAGGTAAGTTTGGT | 1 | 214776469 | CENPF |
| 74 | cg10281002 | TTGGGATGCGATAACTCAGTGCCCTCT TGCAGACTTGCATAGAAATAATTACTG GGTTGT[CG]TGGAGGGGACACGAGAC AGAGGGAGTTCTCCGTAATGTGCCTTG CGGAGAGAAAGGTCCA | 12 | 114846399 | TBX5 |
| 75 | cg10376763 | TCAGGTCTCCTTGGCAGTTCCCCTTCTG CTGTTCTTGTTGCTGCTTGGTGCTGTGT | 2 | 217724363 | TNP1 |

(continued)

| SEQ ID NO. | Probe | Sequence with the CpG site marked with [] | Chro moso me | Position | Gene |
|---|---|---|---|---|---|
| | | GAAG[CG]CACCAGGGCAGAGCCCGCTGGGGGCTCACAAGTGGGAGCGGTAATTGCGATTGGCTGTGG | | | |
| 76 | cg10377274 | AAAAGGAAAAGGAGGAAGTGGAATGCTGGCTTTTCAGGTGTCGCTTGGCCAAATCTAAAG[CG]TGGCAACTTCAGGAATTTCAGGTTGTCCCCATTGTCAGATTCCAGGCACCCACAGGTAAG | 11 | 125616888 | PATE1 |
| 77 | cg10486998 | CGACCCATCCCGCTAGAATCCGTCCAGTCTCTGCTCGCGCACCGTGACTTCTAAGGGGCG[CG]GATTTCAGCCGAGCTGTTTTCGCCTCTCAGTTGCAGCAGAGAAGCCCCTGGCACCCGACT | 18 | 74961787 | GALR1 |
| 78 | cg10523019 | CTCGCTGCTTCTCCCCTAGTCTTCGGGTCCCTTGAACGCAGGTCGCTTGTTTGCCTTACG[CG]TAGTCAGCGGCCAGTGGCTATTTATGGCAGTAAGGAATATTATCCACATTTCACATGGAG | 2 | 227700458 | RHBDD1 |
| 79 | cg10920957 | TACCTGTTGGCCAGGGCGCAGGGCGCACGGAATTCGGGTGACTTTGCTCCAAGATACACG[CG]TGTGTCCCGACTCTCACTCAATTTATAGGGGAGAGGGACTCGCCAAATCCCTGTTTTCTG | 16 | 87635473 | JPH3 |
| 80 | cg11932564 | CCCTACACACGGAACTCACCGTCCTTGTCTCCGTCGGGGGCCTCTGCGGAGGACGCGCCG[CG]AAGCCGCCGCTGTCGCCGCCTCCAGCTCACCAGACCCACCAGGACCAGCGCCAGGACCAG | 22 | 42322146 | TNFRSF13 C |
| 81 | cg12351433 | CCCTTCCACACACCCTTCCCTGCCGGCCCGCCCCTGCCCTCCCCCTCTTACCGCGCACCC[CG]CTGAGTCTGCTCTGCCTTGACCTGCGACAGTGCCCAGTGACCCAATAACCTCCTTCCTGC | 2 | 48982957 | LHCGR |

(continued)

| SEQ ID NO. | Probe | Sequence with the CpG site marked with [] | Chro moso me | Position | Gene |
|---|---|---|---|---|---|
| 82 | cg12373771 | TGGCGATCCAGGAGCACCAGTACAGGT CGGTGACGGCGATGAGGTACAGGTCCA GCAGGC[CG]CCCTGCGCCAGCAGCAGC ACCACGGACAGCGCCTGGTAGCCCCAG CGGCACCTGGGACTG | 22 | 17601381 | CECR6 |
| 83 | cg12768605 | TTTGGGACGGCGCGTCCCAAGGGTTTC TGGAAGTTGTAACCTGTGCTCCGAGTG CGTAGG[CG]CAGGAACCCTTCGGGGGA ATCCCTTTAGCAGGGAGCGTATATTGA AGAGTGCGTGCGGAG | 19 | 44324951 | LYPD5 |
| 84 | cg12830694 | CCACTGGCCCGGTTCAACGAATATCTA TTAAGTATCCACTCTATACCAGACACT GCTTTA[CG]CTCCAGGGATAGAGCAGG GAACAAAACAGACAAAACCAGTCCCA CGCAGTTGACAGTTGT | 19 | 38747796 | PPP1R14A |
| 85 | cg12946225 | CCGGCGGGCGGCAAGGCTCCGGGCCA GCATGGGGGCTTCGTGGTGACTGTCAA GCAAGAG[CG]CGGCGAGGGTCCACGC GCGGGCGAGAAGGGGTCCCACGAGGA GGAGGTGAGAGTCCCTGC | 19 | 3573751 | HMG20B |
| 86 | cg13038560 | GACCTCAAGTGATCCACCGACCTGGGC CTCCCAAAATGTTAGGATTACTGGCAT GAACCA[CG]GCGCCCAGCCCATCCGAC TTTTGTAACACTCAGAATTGTAGTTTTG TTTGTTTGTTTGAG | 2 | 200819113 | C2orf60; C2orf47 |
| 87 | cg13216057 | TACCTGGGGTGGACCAAGCACAGGTCA GCCCCCTCCCCTTGGCGTCGGGTCCTA CTCGAG[CG]CCCCGCCCCACATCCACC AAGAGAGGCTGAGCTCAGCAGAGTCG TCCCCTCCCCCGCCGC | 11 | 12030643 | DKK3 |

(continued)

| SEQ ID NO. | Probe | Sequence with the CpG site marked with [] | Chro moso me | Position | Gene |
|---|---|---|---|---|---|
| 88 | cg13319175 | AGAAAGCTCCCTCACCGGCTCCCCTGC TCCTGCTCAACAGGCCCTGGTGGCTGC AGATGT[CG]TGCCCCCCAGTTGGTTCC ATGGTGAACACACTCCAGTAGCGGATT ACTTTTGCCCTTTGT | 1 | 19746564 | CAPZB |
| 89 | cg13460409 | ATCTCTCACCTTGCTACTTTCTCGGTAG CCGTTTCTGTTGTCCCTGGATTGGGGG CTCGG[CG]TTCGCTGTCCCTGGGCACC AACCCTTTTAAAGACAGTAACGTTGTA GGAAATCAAATTAG | 21 | 38379570 | DSCR6 |
| 90 | cg13682722 | AGTGGTTGGGACCCTGTGAGAACCGGA ACTGCGAAAACCGGAGAAGGGAATTG TTGACCG[CG]AAAGGGACTAAGGAAA TTGGGATTCCAGTTCGACCCCTAAATT CACACCATCCTTGCTAA | 14 | 90798568 | C14orf102 |
| 91 | cg13836627 | CCTCACAGGCTGAGTGGAGTGTTTTGC AGTCTCAAAGCCTTATCGCTGGCGTGC GCATAC[CG]CAGGGAGTGACATCAGAT CGAAACTACAGGGTTTCGCCGGGGACC AACCACTCCTCCAAA | 15 | 30113723 | TJP1 |
| 92 | cg13854874 | AATAATAAATAATAATGAATCCATTCT TCCTTCGGTCGTGGGTCTGGCAGGCAT AAATTC[CG]GCCGGGATTCCGACCCCA GGGCCAGAGCAGGACTCGCCTTGGCGT CTATGAGTGGGCGGG | 21 | 37757525 | CHAF1B |
| 93 | cg13899108 | GGGCTGAAGAGACCCCCCCCCAACAC ACCAGCCCCGAAAACCGTCTGCCGTCC CCTATAG[CG]CTGCATGGAAAAGAACC AAGACAAGGACTTGGAGTGGAGAAGA CAGAAATTGTCCACTGA | 19 | 18344322 | PDE4C |
| 94 | cg13975369 | CCATTTGAGGGCAAGGGCTGTGTCTTT GGGTACTTCGCTCCTCGCAGTCACAAG TACTGG[CG]TGCGTACGCGGGGAGAGA | 7 | 130080553 | TSGA14 |

(continued)

| SEQ ID NO. | Probe | Sequence with the CpG site marked with [] | Chro moso me | Position | Gene |
|---|---|---|---|---|---|
| | | TCGCTCCTCAAAACGGGGTCCTGAACG CTGCCCCGCGGCCCC | | | |
| 95 | cg14258236 | GTCTTCCCTCTGAGGACTGGATCCTCA AGATGGTGGAGATTATGCAAATGTAGG AAAGTA[CG]ATACAAAGGAAAGGAGT CCAACCAATGAAGACCCCAGTGGATA GCAGTGCCAACTCATTG | 6 | 29323330 | OR5V1 |
| 96 | cg14308452 | CTGGGGGCCTGTTTGGGAGATGCCACA AGAACCTTGCCATTGGGGGGCCCCTTT GGGGGA[CG]ACATAGATATTGCTTTGG GGCCCTGGCTGGGTGATGGATGACACA GAGCTTGTCTTTGGG | 19 | 5784184 | PRR22 |
| 97 | cg14329157 | TTCCTTTTGGGAAACGCAGTGTGCTAA AAAAGTGCATGCAGCCCAGGCTGTGGC CTAGGC[CG]TCGGTTCCCGGCCATGCC TAGCTCCTCTGAGGTCGCCCTTAGTGA GGACACGAGGTGCCC | 2 | 228736135 | WDR69 |
| 98 | cg14424579 | TAAGCGATAAGGAGTTTCACACGATGT CTTTTTATTTCGCAGTTGAGTCCCAGTT TCTGC[CG]CTTTATCTTTCCCGCCTCCC GGCAGGCAGGCCGTTAACCGTCTTCCG GAAGACGCTGCTA | 2 | 27274309 | AGBL5 |
| 99 | cg14501253 | GAAGGGCCACGCCGAGAGAGGCAGGC AACAAGGGCACGGCTGGAGGCCGGAA GGTCACCC[CG]TCCCCGGCGGGGCGGG CGCGGCCCAGCCTCACTTCCCGGGCAC GTTCGGGCGGGGCGATT | 8 | 12809014 | C8orf79 |
| 100 | cg14658362 | GAAGGGTGGGCTTAGGGCCAGGGGTG CAAATCCCTCGGTAAAAGCCGGCAAAC TAAAAGT[CG]CACACATCCCAGGTCCC GGTCCAGGCCCCGGCGGGGCAGGGTC CCCGAAGTCCCGGGGCG | 8 | 30241661 | RBPMS |

(continued)

| SEQ ID NO. | Probe | Sequence with the CpG site marked with [] | Chro moso me | Position | Gene |
|---|---|---|---|---|---|
| 101 | cg14723032 | CTGGGGTTCTAGGCTGGAGCAGGCTTTGTGGACCCCAGCGGCCTGGTGGTGAGCAGTACC[CG]CCTTCCACTTCCTAAATCGGGATGCAGAGATTCTAGTGGACAGGCCTTGTGGTCCGGGGA | 17 | 6460572 | PITPNM3 |
| 102 | cg14894144 | GCGGACAGAGATAGAAAGGCTCTCAGAGATCCGAGCCTCACCGCGAACACCCGGGGCAAA[CG]ACATTGCGGTGCATGTTAAGCAGCATCTTGCAGTGCCTGGCCCTTACTCACAGGTCTCAG | 18 | 21270554 | LAMA3 |
| 103 | cg14992253 | CTGCTGGGCCCAGGTCGGCTCATGAACCCGCTGCAGGCCGGCGGAGGCCCGCTTCAGCAG[CG]GCTGCGTGCCACCCCACAGAGCGGCCACCAGCACCAGAGCCAACACCTGCCCTGAATGCA | 1 | 32687567 | EIF3I; Clorf91 |
| 104 | cg15341340 | GCAGCGGGATCATAGCTGCTATGGGGCTGAGATCCAGGAATCTGTGTCGGGACTGCGGGG[CG]CTGGGTTACATCAGAGGCCAGGACTGGCACCTGGCGCCTTTCACTTCCCTAAACTTGCCT | 19 | 12992237 | DNASE2 |
| 105 | cg15381769 | GCAGCCTGGGCCCCGCCGCCAGCCGCTGCTCGGAGGGAGCGAGCGAGAAAGGGGAGCCGG[CG]CAGCTCGCTGCCCTGTTCCAGAACTCAGAATTTGAGAGGCGAGAGTTCGGTAAGCCGTGC | 6 | 128841972 | PTPRK |
| 106 | cg15547534 | CTCCTCCTCTTGAAAACTCTGCTATGGCTGAGTTACCCAGAGGAATCTTAGTCCTGCTAG[CG]CTGCGATGCCCATTGCCCAGTGTGTCAGTCCTCATTCTGGGGCGCCAAATGGGGCAGCAT | 7 | 100034410 | C7orf47 |

(continued)

| SEQ ID NO. | Probe | Sequence with the CpG site marked with [] | Chro moso me | Position | Gene |
|---|---|---|---|---|---|
| 107 | cg15661409 | TTGTTAATCTTTAATTTAATTAAAGAATTTATCCCCCAAATAGGAAAGAAAGCAGCGGAG[CG]GCTAAAGCGTCATTTGATTTTTCTGTCGATGACTTGAGTTGCCTTTGAAGGGGGTGAATA | 14 | 57960976 | C14orf105 |
| 108 | cg15974053 | TGAGGCCGTCGCATCAAATCCTCAATAGAGGCTGGATCCTGGAAGTCCGGCCTCGGGGGG[CG]TTGCCAGGAAGGCTAGAGACCTGGAAGTTTGTCCCCAGCCCCTCCTCCCTCAGACACTCC | 19 | 49339789 | HSD17B14 |
| 109 | cg15988232 | CCTTCTAGTCTCCGGGCAGCCTGGGGAGCGGCCTTTAATCCTGGTCCCTTCTCCGGGATA[CG]TCGTCCCCCAGGTGTCTCAGACCACCAAAACTCAGGTTCCTGGGTAGACCAGGGGGGTCT | 3 | 47621127 | CSPG5 |
| 110 | cg16150435 | TGTGGTCTGTGGCAACAGGTGTCACTTGAATGAATGTCCCAGAGGAAGCTGGGTGTCTCC[CG]CCCTGGCTCCTTTCCTTGACCTCCCTGCCCCTTCTTGGCCCAGGTGTCCTGGCTCACAGC | 6 | 31080529 | C6orf15 |
| 111 | cg16241714 | GGCACAGCTCCAGGGTGGGCACGGCGGCCATGGAGTCGATGTAGGCGCTGAAGTCGATGG[CG]CTCTCGTCGTCGTACATGGCGGGGGCGGCGGCGCCTGGCTCGCCTAGGGCCCCTGGCTCG | 8 | 48650511 | CEBPD |
| 112 | cg16494477 | CTCCCGCCCAGCGATGTATTCAGCGCCCTCCGCCTGCACTTGCCTGTAAGCGCCCGCGCG[CG]GGGCTGCCCACCTTGCCTGGCTGTCTGTCCGTATGCCTGTGCCCTGTACCTCTGTCTGCC | 5 | 170847251 | FGF18 |
| 113 | cg16547529 | CACTGGCTTGTTAACTCTTCAAGGGCAGAATTATGGGCACCGAGCCTCTAAAATGTTGAA[CG]AATGACTGAATATCATCAAGAGGCAGTACTAAAAGATGATGAAA | 11 | 75140681 | KLHL35 |

(continued)

| SEQ ID NO. | Probe | Sequence with the CpG site marked with [] | Chro moso me | Position | Gene |
|---|---|---|---|---|---|
| | | GAATGAATGAGCGGTG | | | |
| 114 | cg16579101 | GCAGAAATGGGAGAAGGTGGCGTCGCGCGTGTCGGAGGGAACGGCAGAACGCACGCTTGG[CG]TATTATAGTGGGAAAGGGCACAGCCTCAACTCAGCACCCGCAACTCACTCAGCACTCCCG | 12 | 6677158 | NOP2 |
| 115 | cg17063929 | GCCTGTTGTTGTGGCTGCTGCTGTTCAGGATGTCCCGGGTGGGAACTTGGAGGCGTCCCC[CG]CAGCCTCTACCCAGGCCTGCCAGGCTCCAAAATACTGGCAAACATGTGAACAATGCTACT | 11 | 89224799 | NOX4 |
| 116 | cg17099569 | TTTAACTCAGAGTTCTTAACCTTTTCTGCGCCGTGGGCCCCTTGGCAAGCAAGTGAAGTT[CG]TGGACTCCTACAATAATGCTATAAATGCATAGAAGAAAAGACACAGGACTGTGAAAGAAA | 2 | 121549866 | |
| 117 | cgl7285325 | CCGTGTCTGCCTCCCGCTTCCCCGCCTCGCGACTTGAGCCCCGCCCGTACCTGCTTAGGG[CG]CTGCCCTCGCCCGCTTGCTCCGGATCCCAGCCCAGGTACCCGGCCTCGCCCGCGGGTCGG | 22 | 50968343 | TYMP |
| 118 | cg17408647 | GGGGGGAAGACGGAGACTCTTATACCGCGGGAGACTAACCTGTGAGCAACAGAAGCACCA[CG]CTACAAAGAGCATGACGAGTTCTTCCAGGCTTGGGAAAGCACGGGTAAATGCCCGCGGTC | 7 | 43769049 | C7orf44 |
| 119 | cg17655614 | AAACAAAAGAACTCAGCCAAGTGTAAAAGCCCTTTCTGATCCCAGGTCTTAGTGAGCCAC[CG]GCGGGGCTGGGATTCGAACCCAGTGGAATCAGAACCGTGCAGGTCCCATAACCCACCTAG | 16 | 68770944 | CDH1 |

(continued)

| SEQ ID NO. | Probe | Sequence with the CpG site marked with [] | Chro moso me | Position | Gene |
|---|---|---|---|---|---|
| 120 | cg17729667 | CGCAAATCTCAGGGCGGCTCTGGCCAG TTTGGAGCCTGGGGTGACCCTTGGAGC TGACCT[CG]CTGGTCCCTGTCGGAGCC CTGCGCGCTGCGGAGCTTGGCGGTTCG CAGCTCTCGGGGTAG | 20 | 25566382 | NINL |
| 121 | cg17853587 | AGTTGCTGGCCTTCCACTTGTCTTCAGG AGCTGAAACACATGGCATTTGAAAAAA ACTGG[CG]AACAGAGGAAACTCTTGCA GCCTCGCAGCCGCCCTGGTCCAGTGCC AACGGCAGGAGCAC | 4 | 118954386 | NDST3 |
| 122 | cg17960516 | GAAGGAGCCCCGCCCGCGCCGGCCCTG GAGTCGCCGGTGTCGCCGCCCTGCCCG CGGGCC[CG]CCCTCCTGGCCCAGCCCA GGGCCCTGCGAGCTATTTTGAAAGTGA CCCTGGGCTGGGGCG | 4 | 3465004 | DOK7 |
| 123 | cg18055007 | TCTGGCCGGCCCTGGCGACGGGGCTGC AAACGCTTCGTAGACCTCAGAACAGCG CAACGG[CG]GACCGGCGGACCGGCAC GAAACATAGCAGCCCCACCACAAACA TTTCCCTTCTTAATTCC | 6 | 31698226 | DDAH2 |
| 124 | cg18180783 | AGCCAGGATCTGCCTTTTAACCTCCAT TTGCTGTTGAGATGCTCAGTTCAACCT GCTGTG[CG]GGATAGACATCGATGTCT CCCTGAGAAGCACATATAGGCTCTCTG AGGTTTCTTTTCTTC | 10 | 75402320 | MYOZ1 |
| 125 | cg18440048 | GTAGCCCTGTTCCTGTCTGCCCTCCCCG CCCCCACAGAAATAGAGATGAGAAGG GGCAGG[CG]AAGAACTAGGAGTGTCT GCGAGACCATCCCAGGACCCTGAGCCC CCCAACTCTCTGCATC | 22 | 24093826 | ZNF70 |

(continued)

| SEQ ID NO. | Probe | Sequence with the CpG site marked with [] | Chro moso me | Position | Gene |
|---|---|---|---|---|---|
| 126 | cg18573383 | GCCGTGAATGGAGTGGAGACTGGCCGC AGGTCAGGAGAGCTCACCACTTGAAGG TGAAGT[CG]CCCTGCTCGGATTCCATC TGCAGATTTTGTTTCTCCCCCAAATCAG CCACTGCTGGAGCT | 12 | 75603401 | KCNC2 |
| 127 | cg18983672 | GGCAGCCAGAAAGGCAGCTCCAAGTT GTGGATTTCCTGGGGGCTCTTCATTTAA AGCGGC[CG]CACCACTTTCCACAATTC TGTTTTTTCAGAGAATGCTCTCAAGGC CTGGAGGGAGGGCAT | 1 | 47881256 | FOXE3 |
| 128 | cg18984151 | TCCCTTGGCCTCGCTCTCTGCCCAGCCC CGGGCTCCTTTTCTCCACACGTGGCTGT CAAG[CG]CCTTCTGTATGCCCCACACT CCTGGGAGCTTGGGCTACATCGATGAA CAAAAACAAAGGA | 3 | 47555476 | C3orf75 |
| 129 | cg19008809 | GCGCGCGTGCCGCCGCCGCGGGCACTG CGCCCGTTTGCCTGCCCCTCGTCGGGG ATCGGG[CG]CTCCCTCTGAGACCTGAA AGGGCACCCAAGTGCCCCCTGTCTGCG AAGTCCGGCGCGGGC | 3 | 53080682 | SFMBT1 |
| 130 | cg19167673 | TTTTCTCTTTGCAGCGAGGCTGGAGGG TGGGCTTTTTTTTTTTTTTCCTTTTTG CGCG[CG]TATGTATGTGTGTGCGCGCA AAGTATCTCTATCTAGGGAATGAAAAA TGGGCGCTGGCGG | 22 | 39640835 | PDGFB |
| 131 | cgl9273182 | GGGCGGGGCTGAGACCTGCGAGAGGC AGGCTGGGAAGCGGCGCCATATTGGCG TCGGCCG[CG]CTGTATTGTCATAAATA GAGCCGGTTTTGTGGTGTTTTCACTACT CGGTTGGATGCCTCA | 2 | 60983417 | PAPOLG |

(continued)

| SEQ ID NO. | Probe | Sequence with the CpG site marked with [] | Chro moso me | Position | Gene |
|---|---|---|---|---|---|
| 132 | cg19305227 | AAAACATATAATATTTAACTTGAGAGG TGCAGTCCTCCTCTACATTGAGGGCAG GCTCAG[CG]AAGGAGGGCCCAAGACA TAAAACTAACCAATGGCAGGAAAGCC CCCATGCCCCACCCAAG | 15 | 45544335 | SLC28A2 |
| 133 | cg19346193 | ATCCAGCCCATCAGTAAATCCTGTTAT CCAGACATTTCTCAGCACTAATTCTGA GACCAT[CG]TAGTCCACACCTCTATCA TCTCTTGCCTGGACTACTATTTAATGTA ACAGCTTTTAACCG | 10 | 127513190 | BCCIP; UROS |
| 134 | cg19478743 | AAGCAGGAGCAGGAGCACGCGGGACC CGGGCCGCAAGTCCCGTCCCATCTCGG GGCTCCG[CG]GACTCTGCGGGGATGGA GCCACCTCGCTCTGACTCCCAGACATG CTCCGGCGCGTGACGT | 17 | 4642647 | ZMYND 15 ; CXCL16 |
| 135 | cg19514928 | GGGTGCAAACCTTTGGGCATCCAGGGA GAGCTTTCTTGTTAGAGCCCACACACA ATCGGG[CG]CATCAAGTGGGTAAGTCC CCCTCCCCCGCCGCCACCTTCTGAAAC AAGTAGCTCTTATTT | 1 | 95583636 | TMEM56 |
| 136 | cg19692710 | CAAAATAAAACAGAGCCCTGTGAGTCT TCAATTTCCGAGTTGAGTGACCTTTCA CAGGGT[CG]CAGAATCAGCCCCAGCTC TCCCCCAGTCCTTTCACTGACTCCTCTC TGTGGCAGAGCTGA | 11 | 73661920 | DNAJB 13 |
| 137 | cg19945840 | GCGCGCCCTGGAGCGGGAGCAGGCGC GGCACGGGGACCTGCTGCTGCTGCCCG CGCTGCG[CG]ACGCCTACGAAAACCTC ACGGCCAAGGTGCTGGCCATGCTGGCC TGGCTGGACGAGCACG | 1 | 1168036 | SDF4; B3GALT6 |

(continued)

| SEQ ID NO. | Probe | Sequence with the CpG site marked with [] | Chro moso me | Position | Gene |
|---|---|---|---|---|---|
| 138 | cg20295671 | TCGGACGCAGGCTGGCTGGGCAGGGA CACTCGGCCGGCGGGGCTGGCGGTGGT GGTCACT[CG]TTCCTCCGGCTCGCGGG GATGGGCCGAGGGCGTGCAGGGCCCG CAGCTCCAGAGGCTGAG | 22 | 22090486 | YPEL1 |
| 139 | cg20305610 | GGTTGGGGACGAGGAGGGGGCGCTCC TCGGGCAGGGATGGCTCCTCAGGTGCT TTCTGGG[CG]CGGAGCGGCGGAGGTGG GAGAGCAGCTTGGGAAAAGGAGCGCC CGGAAAAGGGCAGCGCT | 4 | 95373302 | PDLIM5 |
| 140 | cg20524216 | TCGGGGGTGGTGTTAAGCAGGTTATTA AGTTCCACGAACATTCCGAGCTCCTGG GACTAG[CG]CTCTGGAGGAGAACCCGG AGTGCTGCAGAGACGACGGAGGCTGG AGAGCAAAACACACCC | 3 | 47555100 | C3orf75 |
| 141 | cg20692569 | CGACCCGGAGCGCGGGCGCGGGGCTG CGCCGTGCCAGGCGGTGGAGATCCCCA TGTGCCG[CG]GCATCGGCTACAACCTG ACCCGCATGCCCAACCTGCTGGGCCAC ACGTCGCAGGGCGAGG | 7 | 72848481 | FZD9 |
| 142 | cg20761322 | CACCTGGTAGTTGTCTAGCTGCTCTTCG GTGAAGATGGTCTGCTTGTTCCCCATG GTGGC[CG]CCGCGCCGCCGCTCGCCCG CCCGGGCTCCGACTCCCATCAGCGGCC GCCAGACCCGGAGC | 15 | 78423564 | CIB2 |
| 143 | cg20795863 | TTTTCCTTGTGCAGCTTTTGCCCTTCTC AGTTTTATTTTCTCACATCGTCCTAATA TTAA[CG]TTCACTGTGGTTGAATGAAA GACTGATAGATTACATTTATTTCTCAA AGAAGCTAAGTTT | 2 | 233896119 | NEU2 |

(continued)

| SEQ ID NO. | Probe | Sequence with the CpG site marked with [] | Chro moso me | Position | Gene |
|---|---|---|---|---|---|
| 144 | cg20828084 | GACTCCATATGCCCTAGGGATGTGTTG TGATGAACTTTTCCTACTGGTACTGTTT CCTCC[CG]CGAGGGAATGTCTAGACCA GCCGCACCTTCTTGCTTTGACCCTTCAG AACTTTGGCCTGT | 15 | 81070851 | KIAA1199 |
| 145 | cg20914508 | AGAGCACCAGAGAGAGAGGGAGAGAG AGAGAGAGCGCTAGAGAGAGGGAGCG AGCATGTG[CG]ATGAGCAATAGCTGTG GACCTTACAGTTGCTGCTAACTGCCCT GGTGTGTGTGAGGGAGA | 3 | 115342333 | GAP43 |
| 146 | cg20947775 | CCGCCCGGGGGCGGGTGGAAGGTGGC TCCCGGGGCAGGGAGCCTGCAGGGCG GCTCACAG[CG]CTTCTGCTCTTGTGTGT GTGTGACCCCCAAAATGCCTTTTATGG TATTTTTCCAGTCCCC | 4 | 83720240 | SCD5 |
| 147 | cg20999813 | GGGCCCCGCTTGGGGAGGGCGTGGAG GGCGCCGAAGGGGTTAACCTCCCTGGG GCTGGAC[CG]CGGGGCGAGCCCGGGG TGTGGAGTGGGGCCCTCCCCGCCGCGC CGGCCGGGGGAGGCGGC | 16 | 84734014 | USP10 |
| 148 | cg21096399 | CTGACTGGCCGAGGTGGCAGCGAGGA GAAGCTGTCCCGGATGCCCGGAGTCGC CCCGGGT[CG]AAGCCAGCCAGGCTCAC CGCTGCTCAGCCCCTGCCAGCCAATGT AGCCCCTAGGGGACCT | 11 | 119188145 | MCAM |
| 149 | cg21378206 | AAATAGGGGAGTCTACACCCTGTGGAG CTCAAGATGGTCCTGAGTGGGGCGCTG TGCTTC[CG]GTGAGTGTATGAGGCCCT GGTTTGGTGGTGTCCTCCGGAGGAAGT GAGTTCTGGATAGAC | 2 | 113817043 | IL1F5 |

(continued)

| SEQ ID NO. | Probe | Sequence with the CpG site marked with [] | Chro moso me | Position | Gene |
|---|---|---|---|---|---|
| 150 | cg21460081 | CGGGGCGACCCCCTCCTTGCCTCGCTC TCTCCGGGATCAGAGAGAGAGCGAGA GAGAGAG[CG]CGCGCAGGTTGCGACT GGAGGGCCTGTTGGGGCGCTAGGCAG AGCGCAAACCCTAGATCC | 17 | 46656012 | HOXB4 |
| 151 | cg21801378 | CCACGAAGAGCTTGATGGCGTCGTGGT CCTTCATGGGTACGGCGGGACCGGGGT TTAGCC[CG]CTCATGCCGACGCCGCTG TCCGCGGTGCTGAAACCCAGGCGCGGG CCGGGGCCAGCGGGC | 15 | 72612125 | BRUNOL6 |
| 152 | cg21870884 | GGGCCCGCGGCGGCTGGTGGATACCTT CGTGCTGCACCTGGCGGCAGCTGACCT GGGCTT[CG]TGCTCACGCTGCCGCTGT GGGCCGCGGCGGCGGCGCTAGGCGGC CGCTGGCCGTTCGGCG | 1 | 200842429 | GPR25 |
| 153 | cg22006386 | ACACGGGTGCGATCGCAGGCAGAAGC AGTACGGGGGAACTTAAGAGGGGGAC TGTCAAAG[CG]AGAAATAGAAACCAA GACCAGGTGAAGAGCAAGAGTGGAAT ACAGGGAGGGGGCGGAATA | 19 | 38827378 | CATSPER G |
| 154 | cg22289837 | TTTTCATGAACAGAGGTACAGCTCAGG GAGTGTGGCTAAATCAGTCCCAGTCTC CAGCTC[CG]CGTGAACCTGGGATCCAG ACATCTCCTGGATATCTGGCGCTCTCT GAGATCCAGCCCTCG | 8 | 86350278 | CA3 |
| 155 | cg22432269 | AGGCCGAGCCGGGAGAGCCCCCGCCC CGGGAGGAAGGGGAGGAGGCCGAGTG TTTCCTGG[CG]CATTCCCGGCCAGCCC GAGTGACTCACTCGGCCAAGGAAACTC CCAGGGCCCGCCCAGGA | 15 | 22892697 | CYFIP 1 |

(continued)

| SEQ ID NO. | Probe | Sequence with the CpG site marked with [] | Chro moso me | Position | Gene |
|---|---|---|---|---|---|
| 156 | cg22449114 | GGGCCTGGGCATTAAGTCAGTGGTTCT GGGCTTGGGGTGCCGCACCCAGCACGA ATTCCA[CG]TCGCTTCCCCCTGGCCTCG TTGGGGACCCCTGCACCTCTCCGGTTC CCGCAGAGGCGCTG | 20 | 590243 | TCF15 |
| 157 | cg22679120 | AAAAAAATTACCGGGCGTAACTGCACG CGCCCGTAGTCCCAGCACTTTGGGAGG CTAAGG[CG]GAGGATCACTTGAAAGA GAGAGAAAAGCAGCTACACATCTATA GATTCGGTTCACAGATG | 7 | 2353402 | SNX8 |
| 158 | cg22736354 | TGCGCCAGGGCGGCCACGCAGGCCAG GCAGACCACGTGGCCGCAGGACAGGT TGCGCGGG[CG]CCGCTGCTGCCGGTGG CCAAACTTCTCAAAGCACACCTTGCAC TCGAGCAGGCTGATCTC | 6 | 18122719 | NHLRC1 |
| 159 | cg22809047 | TCACATCTGTCATCTCTCAGGTCATATC CAACACACTGGGCCACCCACGCACAG GGACGA[CG]CGACAGCCCTGTGGCTCC ACCGCACAGGACAGCCACGACTGGCA ATCCTGTGCCGGCCCT | 2 | 101618261 | RPL31 |
| 160 | cg22901840 | GTGCAGGGAAAGCACACCGTGGCTGC AGCCCAGCAACTGGCAGTAGGTATTTT CAATGGT[CG]GCAGGTACTCATGACGG AAGTTGCCGCTCGCCCACTTGTGCAGC AGCGTACTTTTCCCCA | 1 | 68512777 | DIRAS3 |
| 161 | cg22920873 | CGAAGATCCGGCCAATTTGCCCAGCGC GCTGTGCTCCGCGACGGCGCATGCCCG CTTTTG[CG]CAGGCGCGGGGACTACGG CGCAGGCGCGGAGACTATTGCGCAGG CAAGCGCGTACGCAGA | 7 | 139025153 | C7orf55 |

(continued)

| SEQ ID NO. | Probe | Sequence with the CpG site marked with [] | Chro moso me | Position | Gene |
|---|---|---|---|---|---|
| 162 | cg23517605 | CTCCAGTGCCGGCAGGTGGGAGGGCTGAGGTGGCACAGGCTGCTCCGCCACCTCGGACTG[CG]GCTCCTACTCGGCCACTGGCCAGAGTCCCTCCAGCCAACTGCCCCTGGTGAGACCACCGT | 6 | 3228365 | TUBB2B |
| 163 | cg23662675 | TGGCTGCCCCGGCAAATCGGAGTGTAAAGCCGCCCCGGATTGGCTGAAACACTTCCTGAG[CG]ATTATCTTTGTGAGGCTCGGGTGAGCAAGAGCCATCCTGTGCATAGAAAAAGACAGGCTA | 20 | 45985596 | ZMYND8 |
| 164 | cg23941599 | CTGAGATCTCGCTGGCTCTTCTCCTCTCGGATTTTCGGGGTGCTCCCTTAGGGAATCTTT[CG]GTCCCATCTCAGAGACCCCAGAAGGGAAGTGTATTAGTGCGTTTTCACGCTGCTGATAAA | 5 | 114880796 | FEM1C |
| 165 | cg24116886 | CTGGTTTATACTGCCACATTCATTCTTGGAGGTGAGTACATTTCGATCTTGGTCCGGCTG[CG]CAGAGAGTCAAAGCAGGAAAATCACAGATTCTTCCCAGCAGTCTACAGCCTACACAGCGG | 20 | 137877 | DEFB127 |
| 166 | cg24126851 | GCAAGCAATCTTAAAGGAACTGGGAAGAGTTCTGACTCCTGTCCTTCTTCCTTAGGACTG[CG]AGTAGACTGTGAGAAAAACAGGTTTTCTGGACTTGAGATGTGTACAAATGGCACAAAGAA | 11 | 6678143 | DCHS1 |
| 167 | cg24254120 | GTTGGAGTGCAGACCCAGTCAGTCTCAGAATAAGACGAGAAGCCGTTGGAGCATTTTGAG[CG]GAGATGACACCATGTGATTTACTTTCTAGCTGGCTTAAGATTTCTCGATGTCATTGTCAT | 13 | 34392869 | RFC3 |

(continued)

| SEQ ID NO. | Probe | Sequence with the CpG site marked with [] | Chro moso me | Position | Gene |
|---|---|---|---|---|---|
| 168 | cg24262469 | CTCTGCAAGCTCCATGAGGACAGGCGTGAAGTTCAGGCTACATGCCTGGTACGTAATAGA[CG]CTCTGACAGACATTTGCTGAATGAATAAGTTAGTCACTACGGCGTTTGTGGGCTTTAAAA | 3 | 156391694 | TIPARP; LOC10028 7227 |
| 169 | cg24450312 | GGGGCGCGCGAGGGGCGCAGCGCCCGGAGGGCTGCCCGGGGGAACCTGGAGCCCCCGCCC[CG]GGCCTCCCGACCCGCTCGCCCGCTCCGGCCTGGTCTGCAGCAGAGACTGCGGCGGCGGCC | 1 | 206681158 | RASSF5 |
| 170 | cg24580001 | TCTTCTGAAGGATTTGATGCTGGTGCTTTTCAGGTGTGGGTCCTGACAGTGATGTTGGGA[CG]GCAGCTAGCCAGACAGCAACTGTACCATGTAAACTCACTTCAGAGGTGTAGAATGGGGGC | 11 | 64106532 | CCDC88B |
| 171 | cg24834740 | GGGATGAGGATGGGGCGGGGAGGTGGTCCCAGCCTGCTATCACCTAGCTGGGGGCCGGGG[CG]CTTTGGCCAAGGGACGATAGCTTGAGATAAATGGGAGTGTGGGGACTCTGGAAAGACGGG | 20 | 37434552 | PPP1R16B |
| 172 | cg25070637 | TGCCAATCGGCGTGTAATCCTGTAGGAATTTCTCCCGGGTTTATCTGGGAGTCACACTGC[CG]CCTCCTCTCCCCAGTCGCCCAGGGGAGCCCGGAGAAGCAGGCTCAGGAGGGAGGGAGCCA | 8 | 97505868 | SDC2 |
| 173 | cg25148589 | GGGTGAGTGTGTGTGAGTGCATGGGAGGGTGCTGAATATTCCGAGACACTGGGACCACAG[CG]GCAGCTCCGCTGAAAACTGCATTCAGCCAGTCCTCCGGACTTCTGGAGCGGGGACAGGGC | 4 | 158141936 | GRIA2 |

(continued)

| SEQ ID NO. | Probe | Sequence with the CpG site marked with [] | Chro moso me | Position | Gene |
|---|---|---|---|---|---|
| 174 | cg25505610 | GAGGCGCCAGCGGGAGGCAACATCAA<br>TGCAGTTAGCTACACGGGCCTGAAAAC<br>TGGAGGC[CG]CGACAAGCGTCGCTGAG<br>TGGAGGCCCAGTAAGTCCCACCCACTA<br>GGCCAGCCCGAGCGCG | 11 | 32605184 | EIF3M |
| 175 | cg25552492 | GCAGGGGGGCGTCTTGGGGGGCCTCTT<br>AGCGCTGACTTGCAGCATGAGGCAGAA<br>GCCGAG[CG]CGGAGAGCGCCAGCAGC<br>CCCGGCCCCGGGCCCCCCCTGGCCCGC<br>AGCCCCGCCATGCTGC | 8 | 22013999 | LGI3 |
| 176 | cg25683012 | ATCCTCCCAAACTGTGAGCTGGGAACT<br>AGCAAGAATCAAAAAGCCAGTGTATG<br>CTTCCTG[CG]AACCACACAGCCTGAAC<br>TGCTGTAGGGTGATGTCCCTGTGTGAC<br>AGACTGGGGTGGGGAG | 12 | 57030113 | BAZ2A |
| 177 | cg25771195 | GATAAGCGCCTAATATACATCCCTGCC<br>TGTCATTATTCACATTGTGGCATGCAGT<br>CAAAG[CG]ACACTCTGAGGAAAATGTA<br>TCGCCTTAAATACATTGATTAGAAAAT<br>AAGAAAGCCCGAAC | 16 | 58163814 | C16orf80 |
| 178 | cg25781123 | GGGGAAGCACTCTCTAAACGTTAGCAA<br>ATACCATGGTAGGACACAAGGCCCCTG<br>ACTCTC[CG]CTTTCAGCTTACTGAAGA<br>TCCTCAAAACCAACAGCACACAGCTTC<br>CAGCGCATGCTCCTT | 3 | 9404598 | THUMPD3 |
| 179 | cg26003813 | TTGTTGAGAGGCGGACACTGACTCGGG<br>AGGTCTGGGGTAGGGCCTGAACGTTTG<br>CCTTTG[CG]GTTCTAACAAGCTCTCAG<br>GTGATGGCGATGCTACTGTTCCCTGGC<br>CCCGAGGTAGAGGAA | 16 | 23689802 | PLK1 |

(continued)

| SEQ ID NO. | Probe | Sequence with the CpG site marked with [] | Chro moso me | Position | Gene |
|---|---|---|---|---|---|
| 180 | cg26005082 | AGCTCTCCACCGACCGAAGGAGGAGAATGCTATTTATTTCAGCACCAAATATCCGGACAG[CG]CCTCTCGGGAGGTCCGAGAAGAGAACCGCGATCTGTTTCAGCACCGGGGCTCAGGACAGT | 19 | 4769660 | MIR7-3; C19orf30 |
| 181 | cg26045434 | GGGCTTCCTAACTTTCAGGTGTCAGAATGTGTGGCCCAGCCCACAGGGGCACGGGGAACA[CG]CTCCGTACGGGCACCGCAGGCTCGGCTCAGAAATCCCCCGCCACGAGTGTCCCCAGACGG | 8 | 21987861 | HR;HR |
| 182 | cg26297688 | ATAAGCCACGTCTCTCCTCACCCCTAGCACTTAATCACAAAGGCCTGTAGAGAGTCCCGA[CG]AGAACTTCTGAGCAGGCCCCGCTGTCAGTCCCTGAGGACAGCATGCAAGGGAGGTTGACG | 12 | 107349093 | C12orf23 |
| 183 | cg26372517 | CCGGCGCCTCTGCCCGCAGCGCTCGCCGTCGGGCTAGGGCTCCGCCGCCGCCACGCCTCG[CG]CCCGGCACTCACCGCCCCATGCTGGTGCACACCTACTCCGCCATGGTGAGTAGTCTCGGG | 1 | 36039159 | TFAP2E |
| 184 | cg26453588 | GGCTGCCCACCCGCCCACCCCGCCTGGAAGCTTTCTGATTTCTCTGTTCGCCCCGCCAGG[CG]CTGTGGGGTCCGTCTCACCAGGTCTGCACGTGAGCCCCCTGCCCCCAATCCCTCCCAGTC | 22 | 43506021 | BIK |
| 185 | cg26620959 | GGTGGGAAGGAAATGTCCCTGAGAGCCGGGACGCGCTGCCTCCGCTGCCTGGAGGAGCTG[CG]CTGTCCTGCCAGCTAACTTTTGCCCACGGTTTCCACTGCCCGGGTGACCTTTCTGAGCGG | 6 | 152958489 | SYNE1 |

(continued)

| SEQ ID NO. | Probe | Sequence with the CpG site marked with [] | Chro moso me | Position | Gene |
|---|---|---|---|---|---|
| 186 | cg26842024 | CGACGACGACCTCAACAGCGTGCTGGACTTCATCCTGTCCATGGGGCTGGATGGCCTGGG[CG]CCGAGGCCGCCCCGGAGCCGCCGCCGCCGCCCCCGCCGCCTGCGTTCTATTACCCCGAAC | 19 | 16436122 | KLF2 |
| 187 | cg26845300 | CGCAACACCCCAGGCGTGGGGCAAAGACAGCGGGGTTGCGGGGCTCCTGTCTGCCCGGGG[CG]TCGAGAGTTCCTGCCGCCCCCTCCCGCCTCATGCACGGAAAGCGCCGAGCCACGGCGTGC | 6 | 158243833 | SNX9 |
| 188 | cg27092035 | GTGTGACCACGGAACGGCCCTGCTGGTGCCGGGAGCTTGGGGGGTCGAGGGCTTGGCAGC[CG]CAGCGCACAGGCCCCGCGCGGGTGGGCGGTCAGAGCCCGGGAACCGAGGAACGGGTGGGT | 5 | 175792880 | ARL10 |
| 189 | cg27169020 | GACGGAATGAAATGAAGTGCCCTGGAGAAGCCAACTGGAGGTGGTGGCCCCGAGAGTAGA[CG]CGGAGGGGCTGAGGCCGCAGGATCCTGGAGCCCAGGAGCTGACGGAGATCGCCCACAGCT | 15 | 83954229 | BNC1 |
| 190 | cg27319898 | GGAATTCCTGATTCCCTGGTGGACCCTGGAAGTTGTCCTTAAATAAATATATCGCTGGCC[CG]CGGTTGAGCAGCCACCTCGTCAGAGCAGCATGTGGACTGGCTCGCCGGGTCCCCTCCGTG | 7 | 88389003 | ZNF804B |
| 191 | cg27377450 | CTACACAAAGGCGCTCACACTTTATCCGAAACAGCAGTGGGGCTTGGGTGCGGTGGCTCA[CG]CCTATAATCCCAGCACTTTGGGAGGCCGAGGAGGGTGGATCATCTGAGGTCAGGAGTTCA | 19 | 7446301 | |

(continued)

| SEQ ID NO. | Probe | Sequence with the CpG site marked with [] | Chro moso me | Position | Gene |
|---|---|---|---|---|---|
| 192 | cg27413543 | GAAACCAAGACTAGGGGCGCGCCGTC ACCAGAGACCGGGCCTCAGGCTGGTGC GGGGCAG[CG]GAGACCCAGGCTGCGG TCCCAGTTTTGGCCTGGGCTCTACCTCA AAGCTTAAGGACCGGC | 4 | 83812148 | SEC31A |
| 193 | cg27494383 | CAAGCCTAGGAAAGTGCCTCAGGCTGG ACGGTCCCCTGACCGCCAGATAGCACT TACCCG[CG]GCTCCGAACCACACCAGC AGCTGTCCCCAGCAGCCCATCCCTGTT GGGTCCACCCGGCAA | 15 | 41805868 | LTK |
| 194 | cg00091693 | CTCCTCCTCTGCTGACATGTCACTAGG ATTGGCACCACAGTCCACCTTGCCTTA CTTCCA[CG]CCCCCCGCTTTGTATAGC AATATGTTAATATGCTTAATTCAATTCC AGAAAATACCACTA | 17 | 39041602 | KRT20 |
| 195 | cg00168942 | CTTTGCTTTCTTATCTCCAGCTCACACC TTTAAGTCTTATGTAGTTAAAGGACAT TTATC[CG]CCTCCTTGGAGAACACAGC CCTCCAGTGTCTCCTGCAGCCTGGAGC CTGGGACATTCTGG | 10 | 35894430 | GJD4 |
| 196 | cg00431549 | TAACTGCTGGACCTGACTGTGTTACAC AGGATGCTGCTCTGGTGCAGAAGTTTT GGCCAT[CG]TATGCTTGGGGACAGACC TGGGCAAAAGCCCACAGAGGAAGTTG CCACAAACACATGATC | 12 | 15039025 | MGP |
| 197 | cg00436603 | CTCACCAGGTCACTGGCTGGAACCCCT GGGGGCCACCATTGCGGGAATCAGCCT TTGAAA[CG]ATGGCCAACAGCAGCTAA TAATAAACCAGTAATTTGGGATAGACG AGTAGCAAGAGGGCA | 10 | 135340740 | CYP2E1 |

(continued)

| SEQ ID NO. | Probe | Sequence with the CpG site marked with [] | Chro moso me | Position | Gene |
|---|---|---|---|---|---|
| 198 | cg01027805 | CGGTTTGGAGACGGGGGGCGCTGTCGGAGGGAGGGAGGAAGGGAGGGAGCGGGGGTGGGG[CG]CACAGAGGATTCCAACAGGAGACTGGAAGAGATTTTGAAAGGTCATCTCGTCCTTCCCCC | 14 | 21566863 | ZNF219; C14orf176 |
| 199 | cg01234063 | AAGCCGGATCCTCTCCGTTCCCTTGGAGTGAGCAAGCGGGACAGTTCTGCGGAAAGTTTC[CG]CCCCCAATCCCCCAGCCCTGCGCCCGGACTGAAGCGGCGGCCCCCACCTCCAGCATCCTC | 11 | 126226007 | ST3GAL4 |
| 200 | cg01262913 | GTTCCAAGAAATCTGCCACCAGCTCCAAGCCTCATGTCCTGAAGTGCCACCTCATTCCCG[CG]GGGTGAGCCAGCAGCCTCTGAAAAGAGGAAGCCATTGAACAGATCACACTGTGCCTCCCG | 21 | 38580486 | DSCR9 |
| 201 | cg01407797 | TGATTATATGTACTATTATTATCTCATTTTACTACTGTGGAAACTGAGATACGAAACTTG[CG]GAGTGAGGATTTGAACCTAGGTCATACTCTTGGCCAGCCAGAGACACCCTAAGCCCCAGC | 22 | 29168514 | CCDC117 |
| 202 | cg01459453 | GCAAGTTTAAAAGTACTCACAAAATCTAATAGGCAATTCAACATAAAACTCCATGGCTAT[CG]CTGTTCCTCACTTTCTGAACCTTTACCTGCCTGACTTTACTCCATACCACTCCAACTCAC | 1 | 169599212 | SELP |
| 203 | cg01485645 | CCCCCGCCCGGTCCTGGAAGACCGGGTCAGGCATTGTTTTCTTGCCTATTGTTCCAGTTC[CG]CGCCCCCCACCCTAAGTTGAGGGAGTTTGGGGAGAGTCTAGGGAGCAATGAGTGAACTCC | 17 | 36862199 | MLLT6 |

(continued)

| SEQ ID NO. | Probe | Sequence with the CpG site marked with [] | Chro moso me | Position | Gene |
|---|---|---|---|---|---|
| 204 | cg01511567 | GTAGTTTTATTGTATCAGACTTAGTACA GGGGTGGGGTGGGGGTGTGTATTGGAA TGATG[CG]TGCCCGTTTCTCTGCAAAA TAGTTTCTATGTCATGGAAAGGAGTCG ATGGGACAAGAAGA | 11 | 57103631 | SSRP1 |
| 205 | cg01560871 | GGTTTTAGCCAGAGAGAAGCGGATGG AGGCGGAACGCTGGCAGAGGACGTTG GTGGGCTG[CG]TCCCAGCTTCGTCAGC CCCACCTGGCCTGACCCCACCACACAG GGGTCGGCTTCCATGCA | 10 | 72545424 | C10orf27 |
| 206 | cg01570885 | GGAGGAGGGTTGGAGAGCAGGGCCGT GTTGCAAGGCTCTCTGGGTGGCCACAG CAGCTTG[CG]CTGCGCCCACATTGCTT CTGCGTGTTTACAGTTGGGCACGAGAA GGCTCAGCACGCACGC | 6 | 3849272 | FAM50B |
| 207 | eg01820374 | GGGAGGCTCAGTTCCTGGGCTTGCTGT TTCTGCAGCCGCTTTGGGTGGCTCCAG GTAAAA[CG]GGGATGGCGGGAGGGTT GACCTCCAGCCCCACAGGAGGGGACC AGCAGGGATCTCTGTGG | 12 | 6882083 | LAG3 |
| 208 | cg02047577 | AGCCTGCCGGCCTGGTGTGTCTCGGGC CGTAGGTGGCGACGTGGGCGAAGGAT CAGCGTC[CG]CGCGGGCCGGGGGCGC AGCCATGGCGCTCGGAGGCCTCTTTGC GGGCCTGGCCGGGCGGC | 20 | 62587702 | UCKL1AS ; UCKL1 |
| 209 | cg02071305 | TGCCTGATGGATAATCCATCACTTGCT TTTCTAGTATGAATGGTCTATTTACGGG TCCAG[CG]CCCCTGCTGGCTTACGACC TTTTCCAGGGCGGGGAGGGGCTGTCCT CATCTCTGTGACCC | 15 | 41185973 | VPS18 |

(continued)

| SEQ ID NO. | Probe | Sequence with the CpG site marked with [] | Chro moso me | Position | Gene |
|---|---|---|---|---|---|
| 210 | cg02275294 | GTTTGAATGTTGCTGAAGGACGCTGGTTTTCAAACGGTAAGGAATCTCCTGATAAAGGCA[CG]AATCTTGGTGTGCAGATAAGCCAGCGATTCTTGCTTCTGGCTAGTTCTACGTTGTTCCTG | 1 | 179262462 | SOAT1 |
| 211 | cg02335441 | CCCTGCGAGGGGGAAGGTAATGGTTTCAAGCTGCCCGGGCTGGGTTCCGAATCTCTAGGA[CG]CCATGGCTGCGATCTCCTCGCTTTCCTGGACATCTTACCTCCGGATGTACTCCAGTCTCA | 3 | 130745948 | NEK11 ; ASTE1 |
| 212 | cg03019000 | TGAGCATAGTTGTCACCTTCCCCACCTCCCACCAAAAGTCCGGGATTTTCACGAGGGGAG[CG]TTTTATCTTTGGGCCCCTAGAAGAGTGCTTTGTAGTTTGTAGGTCCTCAGAAATTTGAGG | 3 | 51704351 | TEX264 |
| 213 | cg03286783 | TTTCCCCGCCTCCCAACCGTGAGGTGTTGGGTTTGGGGGACGCTGGCAGCTGGGTTCTCC[CG]GTTCCCTTGGGCAGGTGCAGGGTCGGGTTCAAAGCCTCCGGAACGCGTTTTGGCCTGATT | 15 | 44580973 | CASC4 |
| 214 | cg03330058 | ATAATCGGCCTCCGGTCCCTGAGGATTCGGAAACTCCTGACGCAGCTAAAGTGAATCTGG[CG]CTGAGATGCCCCCTCCATGGGCCGGACGCGGAGGGAAGGGGTGCCCAGTTGGGTTCTGGG | 3 | 127392403 | ABTB1 |
| 215 | cg03578041 | TGAATGAATAAAGGGAGCTATTGAAATGTCAGGATGTTCTAAAACACTGCCACCTTTTCA[CG]TGTAACTTCAAATTGAGTTCCATCTCACCTCTCCAAATGTGACCCAGAAACTAGGGACAG | 15 | 71147307 | LARP6 |

(continued)

| SEQ ID NO. | Probe | Sequence with the CpG site marked with [] | Chro moso me | Position | Gene |
|---|---|---|---|---|---|
| 216 | cg03682823 | TGGCAGAGCAGGCTGCCTGCCTACTTG<br>TGCTTGATTGAAGTGGCGGTGTAGTTG<br>TGGTGG[CG]CGAATCAGCGTCCAGCAA<br>CAGTTTGTGGAAACTGTGGGTTTGCTG<br>AGTATGGCGGGGGAA | 7 | 94286953 | SGCE; PEG10 |
| 217 | cg03891319 | ACCATCTCACACTGTCACATACACAAT<br>CATATCCACTGATAGACTGCACACGCA<br>GTGGCA[CG]CTTAAACCGTCACACGTG<br>CTCTTGTCCATGCATTCATTCCCATTCT<br>AGGCACTGTCCGGG | 3 | 52016838 | ACY1 |
| 218 | cg03947362 | CTGCCCCGCGCGAGGGCCTCACCTGTG<br>GGTAGAGGTGCTGCATGAACTGCTCCC<br>GAGAAA[CG]CCCTCCAGCCGGGGTACC<br>GGGAGGTGCTGCCCGGCCATGGTTGCT<br>CACGCCTGCCCTCTT | 2 | 200820154 | C2orf60; C2orf47 |
| 219 | cg04005032 | GGTGGCGGCCCCGGCACGGCGGCTGCT<br>GCTGCTGCTACAGCTCCGGACGCCCGG<br>GCCGCG[CG]TGCCTGCTCCAAATCCCC<br>GGGAAATGCCTGACTCATACAGGAGG<br>AAGAGGAGGAGGAGGC | 3 | 32022767 | OSBPL10; ZNF860 |
| 220 | cg04094160 | CTCTGACCAATCACCCTTTGCCTTACA<br>ACATGTAAAACGGTTATCAAATGCCTT<br>TTAGGG[CG]GGATTTATCACTAAACTG<br>CTCCAGGTTTGGACTATAGAAATGCGG<br>CTGTTCGCTGCAACC | 9 | 37465712 | ZBTB5 |
| 221 | cg04121983 | AGCTTACGTCAGTTTCTCGGTGGCAGC<br>GAATTTACTGCCAGAGTCTTGTGGCAT<br>GAGATC[CG]CGCAGGCCTGGGGCCCTG<br>GCCGGGAACCCCTCACTCCCCAAACGT<br>CCCAAGCCCAACCCA | 17 | 73511085 | CASKIN2 |

(continued)

| SEQ ID NO. | Probe | Sequence with the CpG site marked with [] | Chro moso me | Position | Gene |
|---|---|---|---|---|---|
| 222 | cg04268405 | TGACGTTACGTACTGGAAGTCCCAGGA GGAATGCCCAGCAAGTGGAATCCAAG ACGTTCT[CG]CCTTCTCGGGGACAGGG CCATCACCAGGATTCGGAAAGGAACA GGGAGGTTCGGTTTGTG | 10 | 73723221 | CHST3 |
| 223 | cg04431054 | GATGACCTTGGCTAACTGATCTTATCC CTTGGGCCGCTGTGGCACAGGATGAGT GAGCTA[CG]CCTGGTAACAAGAGTGCC ACTCTCGTGTAAGGGGGCTGCGAAGTA GAAAGGAGGCCAGCC | 5 | 126853024 | PRRC1 |
| 224 | cg04452713 | CCTCTCTACCGCTCATCTAAGGGCGTC TCCGGACTGTCGCCCACCCCACCATCC TCCCTG[CG]CTGGGGGTACTAAATCCC GTGCAAAAAGACCTGGTCCATTCCCAA GACTGGTCCAGACAC | 6 | 56707687 | DST |
| 225 | cg04474832 | CCAGCCAAGTGGCCTTGATCGTTTTCC CAATGCCCCCGAGCCTGTTTCCTGCCA GTAGAG[CG]GGTCAGATGTTGCCAACC TCTGCAGAGTAGCAATAAGCAGTAAAC GCCACGCTCTGCACA | 3 | 52008487 | ABHD14B |
| 226 | cg04999691 | GAGGGAGCCGCGGAGGACTGGCAGCT GCAGATGCTGGAGCAGGCCAGCCTGTG GCTGGGC[CG]TAGCTTCCTGCTGGCAG GCTTCCTGGTATCGAGCAGCTGCCCCA GCCTGGAGCAGGCGGC | 7 | 150027050 | C7orf29; LRRC61 |
| 227 | cg05442902 | GCCAGGTCACCCTCTCACTCTGTGCCT CTTAGTTATCTTGCATGCTCTGGTCTTT GCATA[CG]CTGCTCCCTGCACCAGGAA CCTCCATCCCCATCTTTGTCTGCTTGTC GAACTTCAGAAAT | 22 | 21369010 | MGC1670 3; P2RX6 |

(continued)

| SEQ ID NO. | Probe | Sequence with the CpG site marked with [] | Chro moso me | Position | Gene |
|---|---|---|---|---|---|
| 228 | cg05590257 | GCAGCCAGCGCAGCACCCAAGGCAGC GCCTCCAGAGTCAGAGCCAGGCCCACA GCCGCCG[CG]GCCGCCACCTGCCAACT CAACCGTCCCATGCCGCCGCTAATCCG GGACCCACAGCCACGC | 17 | 17109570 | PLD6 |
| 229 | cg05847778 | TCGACCTGTCCGCGCAGTGAGTTTCCA AGATTCCCGAGGGATCTTCAACCCTGT AGAGGG[CG]CCGCCGTGCGCGTTAGGG ACCCGCGGGCGGAGACTGCACCTCCGC AGCTCGCGGCCCTGG | 2 | 170336167 | BBS5 |
| 230 | cg05903609 | GGGTTACCCGGCCCTCGATAAGGAAAC ACTCCGGCCATATCCGGAGAATCTGGG GAGCGG[CG]GGATAGAAAAATTCACT AACCACAGGCCCGGGCCCACAAGAAG CGCAGCAGAAAGGCGTC | 17 | 1587888 | PRPF8 |
| 231 | cg06044899 | ATATCGGGTTTGTCAGACATGGTTGCG GAGGAAAAGCGGAGCGAGGCGCGCGA GTACGAG[CG]AAGTCTGGTCTGCGCAG TGGCCACCACCGAGTTGTCGCCATAAT ATTTTTAATAATGTTT | 4 | 91760229 | TMSL3 ; FAM190A |
| 232 | cg06117855 | TGGGGAGGGTTTCCTGGACAGAGGTCC TTTGGCTGCTGCCTTAAGACGTGCAGC CTGGGC[CG]TGGCTGTCACTGCGTTCG GACCCAGACCCGCTGCAGGCAGCAGC AGCCCCCGCCCGCGCA | 3 | 45067788 | CLEC3B |
| 233 | cg06513075 | AGGGGGAGTAATTTCATTTGACGACCA TATACAGGCCTAATGGGAGCCTGCAAA GTACAG[CG]GCCGCAGTCATGGGTAGA TTACAGGATTCCCATCTGTAAGATCAG TACTGTGGGGGTGGA | 11 | 34126714 | NAT10 |

(continued)

| SEQ ID NO. | Probe | Sequence with the CpG site marked with [] | Chro moso me | Position | Gene |
|---|---|---|---|---|---|
| 234 | cg06688848 | AACGAGCCGGAGAGACTTGATTGGGCCATTCACGCCTCAGGATGAGGACTGGCCAGTCTG[CG]CCTGGAGGGCGGGCCGGTCCCGCTGATCACGTGACACGATTTTTGAAAGGTGATTGGCTG | 16 | 57220097 | RSPRY1; FAM192A |
| 235 | cg06836772 | CAGAATAAGTAGAGGAGGACAATTCAAGAGAGCACAGAGCTGCGTGCATTCTCCCTGTGC[CG]CGACCTGTATCCAAAAGCCTCAGACGAGACTTGAGGAGCTTCCTAGAGGCTCTCCTGCCA | 1 | 57110403 | PRKAA2 |
| 236 | cg06926735 | CGTCACAGCCGGTCCCCAGAGCAGGATTCCTTCCGGCGCCTGCGCCTGATCACCGCTCTG[CG]CTTGAGCTGATAAACTCAGCTGATGGGATAAGAGTCTTGTTTTATCGGATTTTGGGGAAG | 20 | 48732667 | UBE2V1; TMEM189 -UBE2V1 |
| 237 | cg07158339 | TACAGGGCTTAACTCATTTTATCCTTACCACAATCCTATGAAGTAGGAACTTTTATAAAA[CG]CATTTTATAAACAAGGCACAGAGAGGTTAATTAACTTGCCCTCTGGTCACACAGCTAGGA | 9 | 71650237 | FXN |
| 238 | cg07388493 | GGGAGCCAGTGTTCTTTCTCTCCTGTGACTTTGGTGAAGTCTCTCACCACTCAGTGTTGT[CG]TGAGCATGCTAGGCAGAGTGCAAGAAAGGAGCAAGAACTCACTAATGGCTAGGCCTTCCC | 1 | 39491459 | NDUFS5 |
| 239 | cg07408456 | GGCCTGGAGACCAGGTGGTTCAGACTCCATAAACTCTGCCCATTCTCCAGTGAGGTGGAC[CG]AGGCAACCCCTCAAGTCCTGTCCCTCCCCATAGTGACGGCTCTGTAGCCGCTGCTGGCCA | 19 | 15590532 | PGLYRP2 |

(continued)

| SEQ ID NO. | Probe | Sequence with the CpG site marked with [] | Chro moso me | Position | Gene |
|---|---|---|---|---|---|
| 240 | cg07498421 | GATGGTGCTTATGGGGCAGGTTCCCTA ACAGTCAGGATTCCGGTTGCAGTTTTT CTCCCC[CG]CCCCAAAGATACGTGGTT GCAGACGTAAGTAACAGGAATCCATCT TTCTTTGAAAGTCCT | 12 | 94071223 | CRADD |
| 241 | cg07663789 | TGGTAACACGCTCAGCCGCTGCCACGC TATTTAAACGCGGGCTATGGATCCAGG AACCGG[CG]CGAATCAATGAGATCAA ATGCGAGGGAGATGCACCGTCAATTAC AAACACTTGGACAAGT | 5 | 32711429 | NPR3 |
| 242 | cg07730301 | AGTGGGCCAGCAGTCGGGCCAGAGTC CAGCTCAGCAACTCCGGGTTACAGGCA GCCCAGG[CG]GGCCTAGCCACCGGCA GCTGCACTCAGAGGCCACTGTGTCCTG GCTGAGCTCATCTGCCT | 11 | 67777952 | ALDH3B1 |
| 243 | cg07770222 | CTCTCTTCCTATTTTGTGATTAGGATGC TCCATCAGTTTCTGCCACCAGCTTGCTG GAGA[CG]CTGCGTGTCCCTGACTCCTC TCAAAGGGTGAAAAGCTCAGTCGCACC CGAGACCTGCTCC | 8 | 144120106 | C8orf31 |
| 244 | cg07849904 | AGCAGCAACAAGTTTTGCATTTCAGCA ATCAATTTCAGCCATTACATTTGCACC AATCAG[CG]CCGCCCAAGTTCCGGGCT CGGGGCGGGGCTCGCTCTTAAGGTGGT CCGGGGTCCTGGCTG | 22 | 28197796 | MN1 |
| 245 | cg08186124 | GCTAACGGAAACCGAGGCACGTGGAC TGCAATTATGCATTTTCATTGGTCCTCA GGATCA[CG]CGACAGGAAGTATTGCGT AACCGGTTGACTGCCACATGCGCATTG GCTTCCAGGGCCGGA | 3 | 45883676 | LZTFL1 |

(continued)

| SEQ ID NO. | Probe | Sequence with the CpG site marked with [] | Chro moso me | Position | Gene |
|---|---|---|---|---|---|
| 246 | cg08331960 | TCGGGGTCCCTTGGCCTGGAGACCCTT TGTCCAACCCGTCGCCCACCTCAAGAC CTGCCT[CG]ATGCTGCGCATACAGTAG GTATCCAATAAATGTTCCTGGGATAGA AGGCAAAGGCGCTGG | 16 | 2076597 | SLC9A3R2 |
| 247 | cg09133026 | TCACTAACATCGCGCTCCAGGGCCAGC CGGATCTGCGTGGCCGCATCCACCAGA TAGTCA[CG]TTTTGTCATGTCAGGCACT CCCAGAGCCACCCTGTTGCGAATCTGC TCCAGGTACACGTG | 14 | 75388105 | RPS6KL1 |
| 248 | cg09441152 | GCAGAAACGCGGGGCGGCCTCTCCCCA TCCCCGTGTAGTTCTCCGGGCTGAACC GTTGGG[CG]CCTATTTGCAGAAAAGGC AGCTCCTGAGCCTCAAGACAGACTCGG GGGCCAGGCGTGCGT | 18 | 77712293 | PQLC1 |
| 249 | cg09646392 | TCACTATTCTTAGTCCACAGGGGAGTA GTGACTACCCAGGGCTTGGTAAGTGCT CAGTAA[CG]TTTGTTGAAAGATGAATC AATATTTCAATGCTGGGGCAAAGCAGT GAAAAACTGGGGAAT | 13 | 108921052 | TNFSF13B |
| 250 | cg09722397 | TCGGGGTATTTTTAGGCCGGCGATAAA TAATTCATAGGGAACGTGGCATCAGGC TCCCCC[CG]CGGGAGGAGGGGGCGCG AGCAGCGAGAGCCACCGTCACCCGCG GCTCAAGGACACTCGCG | 17 | 72855943 | GRIN2C |
| 251 | cg09722555 | ATCAGCATTAGGGGTTGGGACTGAGGT CAGAGTCAGGGGTATCAGGGGTGGGA GCTCACA[CG]AAAGCCTGGAGGTGACA GTCCCCGTCAGCCTCCTGCAGTTCCAC CTGGATGACCTTCCTC | 9 | 34662282 | CCL27 |

(continued)

| SEQ ID NO. | Probe | Sequence with the CpG site marked with [] | Chro moso me | Position | Gene |
|---|---|---|---|---|---|
| 252 | cg09809672 | CCCCAGAGAGCTTTCATCTAGAAGGTTTGACTCTGGCCAGACAACCAGCGAGCATCTTCT[CG]CAATCTGTTGCTTCTTCCATGGCAAACTCCAGAGAATTAAGAAGCCAAACTCAACATCGC | 1 | 236557682 | EDARAD D |
| 253 | cg10045881 | TCACAAGTCTGCCAGGGGAAGTCCCTGGACTTCTTGCTTCTTTCGTGTAGGACAGGCTGT[CG]AAACCTCAGTGGATAAAAGACCTAGAGAATGTGTATCCCAGAAGAAGCTGGCCAAGGATA | 1 | 111770291 | CHI3L2 |
| 254 | cg10266490 | TGGGGGTGCCTGGAGTTTGGCTGGGGCTGGGTGCCCAGTGGGCGGGCACAGGCCCCTTGA[CG]TGGCTGTGGCCTAGCTGGCAGCCTCGTCCTTCCTCTCCGCTAGGCGGGCACTGGAGCTTT | 1 | 55013709 | ACOT11 |
| 255 | cg10345936 | AACGGGGAAGAGGCTGAGATTGTATGACTCCCAGCCACAGTTTGCTGGGCAAGATACTGG[CG]CCAGGAGGTGGTGAGATTTGTCTAAGGTCACACATGAAATCCAGGATAGAACTCTGCAGC | 5 | 150727812 | SLC36A2 |
| 256 | cg10865119 | ACTCTGGGGCTCGAGCTTAGGATAACTTCAGGTTCAGCTGAGGCCTCTGAACTGTGACTC[CG]CCCCGTGGCCGCATGCGTCGGAACTCCTACCTGCCCTTTGCCCTTCTCGAGGCCGGTGCT | 6 | 170190112 | C6orf122; C6orf208 |
| 257 | cg10940099 | TCTTGCCCTCAGATTACCAGACACGACGCAGCTGGACTTGTCTCATGCCTGCGATAGGGA[CG]GCCCCCACCCTGACTTGCATGGAACAGTCGACATAATGTGGCCTACTGCTTCCACCTGAG | 6 | 109703938 | CD164 |

(continued)

| SEQ ID NO. | Probe | Sequence with the CpG site marked with [] | Chro moso me | Position | Gene |
|---|---|---|---|---|---|
| 258 | cg11025793 | TGGTCTCCCCTGGAGGGTGGGCGGGTT ATCTGAGGGAGTCCTCGGAGGGTCGCC CCCTTG[CG]CGTCAGAGTTGCTGCGTG GGGTCTCAGAGATAGCGCCTGGGCTGG GGAAATCATTGTGGG | 19 | 13262015 | IER2; STX10 |
| 259 | cg11299964 | TGTTAGGCTTCTCCATCGAATCTTCTTT CTCCCCATTTCCACGGAGAAAAGCCCT TAGTT[CG]TCCAGAAATGAGTGATGAG GCAGCTCAGCCTCTCTGAGAAAGACCT GGGTTCAAATGCCA | 9 | 128469783 | MAPKAP1 |
| 260 | cg11314684 | AAATGCTCAAAATCAAGAATTACAAAA AAATCCCTTAATAACAAGCAAATTCCT AACACA[CG]TTAAATATATCATTTCTCT CTTACTAGACATAGCATGACACAGTTT AACAGTATCAGAAA | 1 | 244006288 | AKT3 |
| 261 | cg11388238 | GGTCTTGTGTGTTCAGAGGCTGGTTTTA CAGGTGAAGAGAAGAAACAGCCGCAG AAGTTG[CG]ATTGTCCAAGGTCACTTA ATAAGTGGCAAGAATTAGGATGTTAAG TGTTCTCACCCCCAG | 2 | 201375098 | KCTD18 |
| 262 | cg11653266 | ACCCCTGGACGCTGCGTCCTGATTTCC CCAGGGACGCAGGCCTGGTTGGGAGA AGGGGTG[CG]AGCTCCGATTCCGGACT CTGCTTGGGTTTAAAACCCAGATTGAG GGCTGGGCGCGGTGGC | 17 | 73901339 | MRPL38 |
| 263 | cg12413566 | ACCAGGGGGTGATGCCAGACATTGCTC ACTTTTTCCATGTAGTCAATGTCAGTCC TGCAG[CG]TCAGCTGGGATGGGGGTAA GGACATCTGGGAACCCCCTCTTCCTGG TCTCCCTCCCTCTT | 3 | 39235366 | XIRP1 |

(continued)

| SEQ ID NO. | Probe | Sequence with the CpG site marked with [] | Chro moso me | Position | Gene |
|---|---|---|---|---|---|
| 264 | cg12616277 | GGGCCCCGAGCTGCGCCTGTCCAGCCAGCTGCTGCCCGAGCTCTGTACCTTCGTGGTGCG[CG]TGCTGTTCTACCTGGGGCCTGTCTACCTAGCTGGCTACCTGGGGCTCAGCATAACCTGGT | 3 | 138153763 | ESYT3 |
| 265 | cg12941369 | TCACATGTTTCGTTTCTAGTCCTGAAACATGGTTAAGTGCTTGCCTCCTAGGGCCTCTGC[CG]CAGGCTTTTGGTTTGGAGGCTCTCCTTTGCCACTCCACCCCTCTCCACTCTTCTCCTCTT | 3 | 33839389 | PDCD6IP |
| 266 | cg12985418 | ATTCACATTTAGTTCGCCTAGGAAAACTAGCAGTTAGTGAAAAACTGGCCACATCACAGC[CG]CACAGCTCCAGCAGCCCGGGTAGCTTCCCCACCCTCACTTTCTCCAGCCCCGCCTCCAGG | 18 | 19320538 | MIB1 |
| 267 | cg13129046 | CTACTCAAGGGGCATCCACGGAGCTGGGTCAGCAAACATAACACTGGTCATCTGAGCCTG[CG]CCCGCCCTTCCTCCCAGGCCAGGGCGCCCCCACCCCCTGGGTTTTTCCTCCGTGGACGCC | 10 | 71389696 | C10orf35 |
| 268 | cg13269407 | CAGACACCGAGCCGCGGCCACAGGGCCAGCCGCACAGTCGGAGGAAGGGCCGGAGCGAGG[CG]GGGCCCGGGGCTGTCAAGGAGAAAAACATCCCAAGGCCTGCAAATTGCTGCTCTCAGCTT | 22 | 46450107 | C22orf26; LOC15038 1 |
| 269 | cg13302154 | AAGGGTTCATCAGGATGGAGATATCCGGTGCACCATGAGTTCTGTTTCCTTAATCAACAC[CG]TTGTAACTTGCCCATCCAGTTTTGTGACATTAATTCAAACCTGTGCCCTAGTCCTCTTTT | 12 | 15039432 | MGP |

(continued)

| SEQ ID NO. | Probe | Sequence with the CpG site marked with [] | Chro moso me | Position | Gene |
|---|---|---|---|---|---|
| 270 | cg13547237 | GCAGTGCATCGAGCTGGAGCAGCAGTT TGACTTCTTGAAGGACCTGGTGGCATC TGTTCC[CG]ACATGCAGGGGGACGGGG AAGACAACCACATGGATGGGGACAAG GGCGCCCGCAGGTGGG | 11 | 65687877 | C11orf68; DRAP1 |
| 271 | cg13828047 | TCAACATACTACATGATTTGCTTACAA TACTTGTCTGTCTTGCCTTCACCAGAAT GTAAG[CG]CTCTACAAAGGCAGAGGG AAGGCTATCTTGCTCTCTGATGTATCCT CCAGCCCTTAGAAC | 15 | 75182130 | MPI |
| 272 | cg13931228 | GGTGTGAATCACACTGCCCGGTCGGGC CTTTGGGAAAAAATTAATGAAGGACAC AGTCAG[CG]CCGTAGAACCTGCCAAAT ACACATCAGATCCAGTGGAGTCTGTGA AGGGGGAGGGGGAGA | 7 | 24612418 | MPP6 |
| 273 | cg14060828 | GCCTTTCTCGGGATCTATCTTTCTGTGT CTCTTTCCCTTGCTGATTTTCTGTCCAT TTCC[CG]CACCACCACTACCACCAAAC CCTCCTCCCGCCTTCCCCCACCCCTAGT CTCTGTCTTCTC | 19 | 49926276 | PTH2 |
| 274 | cg14163776 | ACTTTGCTCCTGGTGGTTTTCACTGTTC TGCCATGGTGGGGTTCTGAAGACCAGG CTCAT[CG]TACTCACCTTGCAACACCT GCCCCTCTAATCCACACTTTTTCTAGAA GCACTTTAAGATA | 3 | 195164580 | ACAP2 |
| 275 | cg14175438 | CGCACAAAATCCCAGCCTCAAGGGCA GAACATTTTAAATGACCCACCCATCCT AGAGATG[CG]CCAGTTAGGTCATCTTA TATATCTTGAGATAGCTGAGATGGTCA GATCAACCAAGGACCT | 7 | 121036729 | FAM3C |

(continued)

| SEQ ID NO. | Probe | Sequence with the CpG site marked with [] | Chro moso me | Position | Gene |
|---|---|---|---|---|---|
| 276 | cg14408969 | ACTGACAATGCTATAGCATCCTGGCCATATCCAGTTTTGAAAACACTACGGTGTCAGCCA[CG]CACCATTTAGGACGGGGAGAATGGAAAGCCAGTTTGGAGAACAGACGCTTTCTTAAGAGT | 8 | 42396118 | C8orf40; SLC20A2 |
| 277 | cg14409958 | TCCCTAGTATCACATTCTCAGCTACTTCTGCCTCCTTGAAAGTTTCTCATGATGAAATTT[CG]CAAAATTGTAACTAACATAAAAGATAACATTATTTTCCCCATGCTGTGGTTCAAGTTTAG | 8 | 120651652 | ENPP2 |
| 278 | cg14423778 | GTCAGTGTTCTTTTAGTTTGCTTAAACTGTGTGGGTACTTGAGTCCTTTTAAACGATTAA[CG]CTGGGAAGAGGCACCATTTAATTAATTAATTTGTTCTGGAAGGGATCAGTGTACAATTTT | 3 | 151985433 | MBNL1 ; LOC40109 3 |
| 279 | cg14597908 | GGAGACAGAACTTTCCCCTTTTTTCCCATCCCTTCTTCTTGCTCAGAGAGGCAAGCAAGG[CG]CGGAGCTTTAGAAAGTTCTTAAGTGGTCAGGAAGGTAGGTGCTTCCCTTTTTCTCCTCAC | 20 | 57414960 | GNASAS; GNAS |
| 280 | cg14654875 | TGTCCTTTGTGTCTTGAGCGGATGGTGGGGCCGTGGAACATGAAGGAGTATCTTTGTGTA[CG]TTCACAACGTTCACATCGGTGTAGGCCAGGTTGCTGGACTCTGACTCAAAGTGTTATAGA | 16 | 3493997 | NAT15; ZNF597 |
| 281 | cg14727952 | CCAACTTCGAGACTTGCAGTCAAAGCGATTTTTAAAATGACTTGTTTTCAAGCCTCTGGC[CG]CCGCCCACTCTTCTGGCCCTTGGACTTTGACCAAGATGTTTTCTCGCAGTTTTTGCAAGG | 11 | 102218358 | BIRC2 |

(continued)

| SEQ ID NO. | Probe | Sequence with the CpG site marked with [] | Chro moso me | Position | Gene |
|---|---|---|---|---|---|
| 282 | cg15185286 | CCCCCTCGCCCGGCCCGGCGCCCACTA GCCACAGGGCCCGCTTCCCCCTGGAGA TCAGCG[CG]CACTTCCCGAGCCCTCGT AGCACTCAGAGGTCGCATCCACACCTG GGATGCCTAGGGGGC | 6 | 143381675 | AIG1 |
| 283 | cg15262928 | GGAGTCCTGGCTCCCATTGGCTGCAGC GGGAAATGGTGAACCAATGCTCATAGA CCTTAA[CG]CCCTCCTCTCGGGATCAC TTCCGCCTCTGGGGTCAGGCTCCGCCC AGCTTGCCCGGCATC | 1 | 201924572 | TIMM17A |
| 284 | cg15703512 | CCAGAAATTGGGCGGCAGTGAGGTCGC CGCAAGGCTTCCCGTGGACCCTGCAAA ACGTGG[CG]TGGGCATTGCACACCATT GTACTGTATGGAAACTTCTGCAGAGGT TAGCACCGTGCCTGA | 16 | 22012565 | C16orf65 |
| 285 | cg15804973 | GGCTAAATTGATCAGGTTCTCCCATGT ACTTTTCCTTTTAAAATTTCCAGTGGCT CATTC[CG]TTATCAGTAATGAGTAATT GATTAGTGCCAACTGCCGAAGGACTTA GTATTCTCATTTAG | 6 | 137114513 | MAP3K5 |
| 286 | cg16034652 | GTTGAAAAAGCTAAGTAATTCTGTAAA AATGTCTACTTTCTCATTACAGTAAGAT GTTTT[CG]CAGAGTTAACAGTGCTCTG GTGTAGATAACCAAGACTGCTTCTGTA AATTAGGCCTACTC | 14 | 93798309 | BTBD7; KIAA1409 |
| 287 | cg16168311 | CCTCAGCCAGGAGGAGGCCCAGGCCG TGGACCAGGAGCTATTTAACGAATACC AGTTCAG[CG]TGGACCAACTTATGGAA CTGGCCGGGCTGAGCTGTGCTACAGCC ATCGCCAAGGTCAGTG | 1 | 156561947 | APOA1BP |

(continued)

| SEQ ID NO. | Probe | Sequence with the CpG site marked with [] | Chro moso me | Position | Gene |
|---|---|---|---|---|---|
| 288 | cg16358826 | CCGCACTCTAGTCCCAGTATTTGCTAAGCTATTGCTTTAAAGACACCCCATTTCTTTACC[CG]CCTCCACCAGACACGCGCACACCCTCCGCTTTGCTGCTCCATCCTTTTCTGGAGAGGAGG | 4 | 46996264 | GABRA4 |
| 289 | cg16408394 | TTATCCCCAAAGCAGCCCACGCCCGGGTGGGCAGGGTCCCCCGGGGCTGTATGAACAGAA[CG]TCAGACCTGGGAAGGCCCCATTCCAGAAATGGGGCCCCTCACTCTGGCACCCCCGGGTGT | 9 | 137219075 | RXRA |
| 290 | cg16419345 | CCCGCAACCTGGCAGTTACTAGAGGTCTTGGAATCCAGACTTCTTTGCTTTCGCCATCAC[CG]TCATCAAAGTGGGAAATGCACACTTACTGTTAAAACCTAGTGTAGGGCCGGGCGCGGTGG | 17 | 73976089 | ACOX1; C17orf106 |
| 291 | cg16744741 | CAGCTGGATGCACTTGTTCTGGAGCTCCTCTGTGAGTTCAGCAATGGCCACAGTCTGCTT[CG]ACAGCTGCTCCCGCAGCTCCTTCAAATGGTACTCCCGCTCCTGGATCTCAGCATCCTTCC | 4 | 82126025 | PRKG2 |
| 292 | cg16899442 | CGGTGCTGCCTCCACGCCCGGCTTCCCCATGGCTGCTGCTGCCACTGGCACTGCTAAGTG[CG]TTGCCAAGGCCTCTGTTGGTCCCAGGTGACTCCCAGGGCACCGCCCACAGGGGCCGGCCA | 16 | 776458 | CCDC78; HAGHL |
| 293 | cg16984944 | TTTCTTCAAATTAAATTGCTACAGCAGGAAATTACTGAACTGTGGCTCTTCTCCTACGTC[CG]CCTTCCCTATGTCAATTCCCATTTCCCTTGCTTTCTCCAATAGTTAGGACTGTAAATTCT | 3 | 99979425 | TBC1D23 |

(continued)

| SEQ ID NO. | Probe | Sequence with the CpG site marked with [] | Chro moso me | Position | Gene |
|---|---|---|---|---|---|
| 294 | cg17274064 | AAAATAATAATTAAAACTCCCTCAACTTTTAAGGCCGAGCAACATAATCTATTAATTGGT[CG]CTATTAACATGCAGTTTTATTGACCATAGCACACAGAAGTCTGATTGTGAGGGAGGAGTG | 21 | 40033892 | ERG; ERG |
| 295 | cg17324128 | CCCTCCCCCGCCAGCCTGGCGCATTGCGGGCCTCGGGCTCATTGCTGAGAGGGGGCACTG[CG]CCTGGCACCTCTGTTAAGCAATTTAGGGGCTACAACCTGAGCAAGACAGATGAGCCCGGC | 10 | 45455500 | RASSF4 |
| 296 | cg17338403 | TGGAAGGTGCTGTTTCCTGGTACCTGTCCAGCCCTCTGAGCTTTTCTCTCAGCTTCCAAA[CG]CTGCAGTTGAGAACTAGCAGATCCTATTGGTAGTGCCCTGTGGCCCACACTCCTTGGTAA | 15 | 92395836 | SLCO3A1 |
| 297 | cg17589341 | CCAGGGGACCAGTTCCTTGGTGTTGCTTTGGCATTGATGCCTGAAGTGGGAGGAGAAAGC[CG]AGCCCACAAACACACAGAGCAGAGTGGGGCTCTGAGTATATAACTGTTAGGTGCCTCCCT | 18 | 43304079 | SLC14A1 |
| 298 | cg17686885 | TCTGAGGTTTGTGTTATTAACCCCCTATTATCTTTGGTCTACCCAGGGCAGCCAAAGAGG[CG]CAGAGAAGAATGACAAGGTGCCCAGCAAGCGGCAGGATCAAAGCCTGGGTCTCTAATTCC | 17 | 52977769 | TOM1L1 |
| 299 | cg18031008 | GGCGATTCCGTAATTTCCGCTTCCGGTAGTGAGAACCCTTCCGGTGGGCTAGGTACTGAG[CG]CGCGAGGTGAGGAGTTGTGCAGGGTTTGGGGAAAGGAAGGCTGGCTTGGCGAGAGGGCAG | 1 | 150266311 | MRPS21 |

(continued)

| SEQ ID NO. | Probe | Sequence with the CpG site marked with [] | Chro moso me | Position | Gene |
|---|---|---|---|---|---|
| 300 | cg18139769 | GCAGAGCAGGCTGCCTGCCTACTTGTG CTTGATTGAAGTGGCGGTGTAGTTGTG GTGGCG[CG]AATCAGCGTCCAGCAACA GTTTGTGGAAACTGTGGGTTTGCTGAG TATGGCGGGGGAATT | 7 | 94286955 | SGCE; PEG10 |
| 301 | cg18328933 | CCAGTAGAGCGGGTCAGATGTTGCCAA CCTCTGCAGAGTAGCAATAAGCAGTAA ACGCCA[CG]CTCTGCACAGCCTCCCAG TGCTGGGCCTGGTCGCCACGCGGAGCC TTGGGCTGGGACAGG | 3 | 52008538 | ABHD14B |
| 302 | cg18956095 | ACTGCTGGATCGTGAGAGGTAAGCATG CTGGCTTCTACTGAAACGCCCCTTGTC ATCACA[CG]CCCATCCCCTGGGGCGAC ACGACCCAGGCCCCGCCCCTCGGGGGG CTGCTGCGAGTCCGG | 8 | 124287111 | ZHX1 |
| 303 | cg19044674 | CTCGACCTCGGCTTGGGAGGCAGCGGC CACGACAGCCAGCAGTGTGGTCAGCA GCTTCAA[CG]CGCGTACCGCCATCGCT CCCTCAGACCTAACGGAACCGCCAGCC ACCCGCCACCAAGGCC | 1 | 43232628 | LEPRE1; Clorf50 |
| 304 | cg19046959 | CAGTAGCAGCAGCAGCAGCGAAGACA GGGGTGTCAGAGTCCCCAGCATGGCGT CCGTGGA[CG]TGCTGCAAAGAAGAAC AGAGAAAGTCATCAAGCCAGCCCTGG GTGGTTTGGCACTAGGCC | 1 | 36565856 | COL8A2 |
| 305 | cg19420968 | CGATTATCTGTACCCAAAACAGTATGA GTGGGTCCTCATCGCAGCCTATGTGGC TGTGTT[CG]TCGTGGCCCTGGTGGGCA ACACGCTGGGTAGGTCCAGGGCTTGCC CGGCAGTGCTGCCGG | 1 | 32084964 | HCRTR1 |

(continued)

| SEQ ID NO. | Probe | Sequence with the CpG site marked with [] | Chro moso me | Position | Gene |
|---|---|---|---|---|---|
| 306 | cg19569684 | GGGCCCTCCATGCCATCGGAGCTGGCATCTCCAGCTAGAAAATGGCCAGTTGTTCTGATT[CG]TAGCTCTCCTAGTCAGCTTCCAGTCCAGGGCAGAGGGCAGGGACTGCTAGGGACCTGGGC | 5 | 138726419 | MGC2950 6 |
| 307 | cg19706682 | ATAACAATAATAATAATGGTAGCAAGCAACGCTCTGCAGTAGGGGCTTCTCTCGCCATTT[CG]TACTGAGGAGGAAACATACTTAAGAGGTTACAAAACTTGCACCAAACAGATAACCCTCGG | 16 | 84179331 | LRRC50; HSDL1 |
| 308 | cg19722847 | TCTGCTTACAGCTGCTTCCAAATTAAGCATATCTGGATGGTGTGACACTTTTTGTTAGTC[CG]AGAACTGTATGGGCATCGCAACTGGGCCTGTTCCAAGATAGACTTGTTGGGACCTTCAAA | 12 | 30849114 | IPO8 |
| 309 | cg19724470 | CATTCTTATGCGACTGTGTGTTCAGAATATAGCTCTGATGCTAGGCTGGAGGTCTGGACA[CG]GGTCCAAGTCCACCGCCAGCTGCTTGCTAGTAACATGACTTGTGTAAGTTATCCCAGCTG | 9 | 5450936 | CD274 |
| 310 | cg19761273 | GGACAAAGCCACCACCTTTCACAAAATGAGGCCAGACCACCTGCCTCCCTCCAGTCCCTG[CG]GCCTGGAGACGGAGTCAACATTCTTATCTGTGTTGGATCTGAATGTTCCTCCTTGCAAAG | 17 | 80232096 | CSNK1D |
| 311 | cg19853760 | AAAAGGGTGGGAGCGTCCGGGGGCCCATCTCTCTCGGGTGGAGTCTTCTGACAGCTGGTG[CG]CCTGCCCGGGAACATCCTCCTGGACTCAATCATGGCTTGTGTGAGTGTGGGGACCCCCCC | 22 | 38071677 | LGALS1 |

(continued)

| SEQ ID NO. | Probe | Sequence with the CpG site marked with [] | Chro moso me | Position | Gene |
|---|---|---|---|---|---|
| 312 | cg20100381 | GACTAGCATTTTATTTCCATTGGACAG CGCTGGCTGAGAACAAAACCTAACCCT CTGTGC[CG]CCCTCGCGGCCGGGATGC GGTGCGCCCCGGGCCTCCCCATTCGGA AAACGAGGAGCCTGG | 16 | 66864408 | NAE1 |
| 313 | cg20240860 | ACTGCGATGAAAGGCCATAAGGATGCT CACACCCGAATCTAAAAAGCCCTTTGT GTGGGC[CG]CAGCCAAGCATACTTTGG CAAGAAATTTCTGTGGCTCTAACCTCC TTTGAAAACTGGAGA | 11 | 44087423 | ACCS |
| 314 | cg21211748 | GACGGAGACAGAGGGTGGTTCCGGGA TTCACAGTGCAGAGGCGGCCAGAGCA GTGCACAG[CG]CCCCGAGAAATGGGC CCGGATTCCCTGGGATTGAAGGGAAAC ATTTTGGCGCGGGGTCCC | 1 | 23858035 | E2F2 |
| 315 | cg21305265 | GTAGTCCCCGAGGTCACAAGGCAGTGG CAGGTGTCTGTAGTCCTCGGGTTGACT GCAGCT[CG]CGGTGGTCCCTCTCCGAG CCCAGGAAGCCACTCCAGTGCCGAGG GAGAGGCCTGGGAGCG | 8 | 25316571 | KCTD9; CDCA2 |
| 316 | cg21370143 | AGACCCAACCCCAGTCCTAAAGCTACC TGGCTTCTTCCCCGGCTCAGGCATCCT GAGAGA[CG]TCACACCAGGCACGAAG CAGGCACAGGTCACCCAAAGAGGGAC TGAGTGGGGTCCTGTCC | 11 | 47374208 | MYBPC3 |
| 317 | cg21395782 | GGCCTGCGCAACACCCCAGAGGCAAG GTGAACGCGAGGGCCTATAATGCAAG AACCAAGG[CG]AGTCACGCCCTGTCTG GGCAAAAGAGGAGTAAAGACCCCTCA GCTGCAGCCCGGCAGCGC | 19 | 19626814 | NDUFA13; TSSK6 |

(continued)

| SEQ ID NO. | Probe | Sequence with the CpG site marked with [] | Chro moso me | Position | Gene |
|---|---|---|---|---|---|
| 318 | cg21950518 | GTCGGCCTGGCAGGCGCGGCCCCCGGTTCAGCTGCGCCGGGGCGGCCCAGCGCGACTCCG[CG]GGCCTTTTGGCTGCTCGCCCCGGCTCCGGAACACTGTCAGATCCTTCTCCGCAGAGGTAG | 5 | 55290746 | IL6ST |
| 319 | cg22171829 | CTGTGTCCCCTCTCACCAAAGTCCAGTAGCTGCTTCATGGACAGCGGGGACGGGCTGTAG[CG]CGAGAAATGCTCCACCTCTCGGGGCACCAGGCCGGCGCCGTTGAGCGAGCCAGCGCTGCG | 7 | 95225520 | PDK4 |
| 320 | cg22190114 | TTTTATTGTTTTATGTCTCTGCAGGTCTCGTGTTTCTCTCTTCCAATCGGTTGTCTTTAT[CG]TGGACACTGAGGTGTTCTCTGCCTTGACTAAAGATGAGTGACGTGAATCCACCCTCTGAC | 19 | 56459234 | NLRP8 |
| 321 | cg22197830 | GAAGGCTCCTGGGCCTTTCTGGCTCTGGGAATGAAGCGTGGAAAACCCTCCTTAGGCGGG[CG]CAGTGCTTCAAGTAGCCAAGCTCTGACTTCCGAGGGAAGAAAGGAGGCCATGGGCCTCTG | 5 | 134209784 | TXNDC15 |
| 322 | cg22568540 | GACCACGAGCATGGACATGATGGTCGCGCTCACTCCGGTGCAGTGAGTGTCTGGGGTGAG[CG]TCTGCAGCAATGAGGCCCCAAGGGAGGGCGGTGGGGTGGCTCGGGCACTGACCTCTTCCC | 19 | 58864846 | NCRNA00 181; A1BG |
| 323 | cg22613010 | ATTAGGGTAGGCCCCTGGTCCTCGCGCTTCCCAGGGTAACCTGGAGCAGGGGTCCCGGAG[CG]CACTCCTGGGGCTCAGCTCAGCTTCACTTACCAGGGTCTGCTCGTACTGCAGCGCCCGTG | 3 | 184079172 | CLCN2 |

(continued)

| SEQ ID NO. | Probe | Sequence with the CpG site marked with [] | Chro moso me | Position | Gene |
|---|---|---|---|---|---|
| 324 | cg22637507 | GCCTGTGATTGGGAGTTGCTGGAGTCG GTGCTTCACTCTTAAGGTTCCGATCAC AGACTG[CG]GAGTGGGTCAGGGGCTGC GAGGGCTGCCCCAAGTCCTACCGGGTT TGCACGGGCGCGCCC | 11 | 43902407 | ALKBH3 |
| 325 | cg22947000 | TAGCTATGACACATGGCTTGGAAATTA ACCTTTAACCAAACATCTTATAAGTAA CGCCAG[CG]CAGCTTCCCTTGTGAATG TAAAGAGATCCAGGGCTCTTGGAGAGG GACAAGTGAGAGCCA | 16 | 81272281 | BCMO1 |
| 326 | cg23092072 | CAAAAAAGGCGGGCTGTTTTGTAAATA TTTGTCTCTATGTAAGGAAATCAAAAC TGAAAG[CG]GAGTAACACCAAGTATGC CCGTTTCTTGAGCTCAAGCACTGGAAG GATCAAAAGTAGCGA | 4 | 87927706 | AFF1 |
| 327 | cg23124451 | TCAGTCTCCCCATATTTACAATAAAAG GGGAGCGAGGTGGGATGGCGCTGAGG ATCCCTA[CG]TCCGATCCTAATCTCCA GCTCAGGCAGGCTCGGCCGCCACTAGC ATCCTGGAGCGACAAC | 22 | 39548131 | CBX7 |
| 328 | cg23180365 | AACCCCGGCATGACCACCAGCCTCCCG GCTCTGCAGTCGGCGCCCAGGCCGGCC GCTTCG[CG]TCACTTGACTAAGGACCC ACGGCCTGGCACCGCCCCTCGTCGGCC CAGCAGCCAGCCCTC | 3 | 33138627 | GLB1; TMPPE |
| 329 | cg23786576 | AGAGACTCCCAGCTCTGACACCAATTA GCTGTGTGATCTTGGGCAAGTGACCTA GCCTCG[CG]GAGCCTGGCTACATCATC TGAAGAGCTGGGACAGTACTAGTGCCC ACCTCACAGGGCTGT | 1 | 47133596 | ATPAF1 |

(continued)

| SEQ ID NO. | Probe | Sequence with the CpG site marked with [] | Chromosome | Position | Gene |
|---|---|---|---|---|---|
| 330 | cg24058132 | GGGCCATGAGTGGCCCTACCATGGCTCTTCCCCAGCATCTCAGGGAGTATCTACCTCGTG[CG]AGGACCAGGCTTGGACACCAGGTCCCGATTCCATTGTCATCTTGGTGGAATCACTTTGCT | 14 | 88459866 | GALC |
| 331 | cg24081819 | CGCGCTGGGCTTGCAGCCCAGCTTTCAGATTGCTCCTGTGCCGGAGCCCTGCGAATCATG[CG]AATCATGAAACTGAAGACCTGGCCCTGAAGTCCCAGTGCATATGAGGAGATCCGTTGTCT | 8 | 27348940 | EPHX2 |
| 332 | cg24471894 | TTTTTCTTGTGCTGTCTTTGTACTCTTTCCTGTGAATTGCTTTTTCCCTTTAACTTCCAT[CG]TAGCAACTCTGGAAAACCAAAACCAAAACCAAAAACAATCACTGCAGTTCTCTTCATCAA | 9 | 2838508 | KIAA0020 |
| 333 | cg24888049 | AGCATTGCTGGTTCTATTTAATGGACATGAGATAATGTTAGAGGTTTTAAAGTGATTAAA[CG]TGCAGACTATGCAAACCAGGCCCAGTCTCCAGTGTGGTACCGTTGCTCCTGCATCGCAGC | 15 | 91426667 | FES; FURIN |
| 334 | cg24899750 | GGAGGAACTGGCTATCCTAAAGGTGATTTTAAACCGGGGTAGCTAGAGCCCAAAGAAGGG[CG]AAACCAGGACTAACTGCCCCATAGCATGAGGGGCAGCGCCTGTAAAATTACATAGGATTT | 20 | 16710314 | SNRPB2 |
| 335 | cg25101936 | CTGGCCCACCCGTGAGTCACGGACAGAACATGCAGACTCAGGCCTTGGTGACATAAGCTC[CG]CATTGCTAAAACCGCGTGACCTCGAGGGCTGACTGGCCTGAGAACCCTGGATGGCGCTCT | 11 | 113929164 | ZBTB16 |

(continued)

| SEQ ID NO. | Probe | Sequence with the CpG site marked with [] | Chro moso me | Position | Gene |
|---|---|---|---|---|---|
| 336 | cg25159610 | GCCATCTTGTGGAATGTTCCGGAATGC CGTTAGGTGTCGAAGTGGGCAGCGGTT GACAAC[CG]TGGGCCTTTGACAGTTAC TAGTACTAAACATCGATGCCGATTGTG AGTTTCCAATCAGAG | 5 | 57756802 | PLK2 |
| 337 | cg25166896 | CGTGGTCCCTGCAGGGTGTGTGGGCTG CTCGGCCTTGGCCAGCATCAGGGACAG CTCTGG[CG]CCCGGTCACTCTGCCCCC TACCCGCGGCCTGCTGCGGGCCAGCAG GGTGACAGCTAATGT | 22 | 20009063 | C22orf25 |
| 338 | cg25411725 | TCTACCTGTCTCATTTGAGTTGAGTGTG AATTGTTTAGGATATTGCAATTAGAGG TGGTG[CG]GGCTGGCTGGTTGCTATAA GCCATCTTAACATTTGGCTAAGCTCAC TCCTGTGTGCTGGG | 3 | 38306672 | SLC22A13 |
| 339 | cg25564800 | GATGGAATGAATGATGGAATGATTGAA GGCTGAGGGAGTATTACAAAATTAGTA GGTCAG[CG]CCTCGTGTCTAAAGGGCT CACATGCAGCATGAATGCAGGAAGCTT CTGGACATTCCTTTT | 3 | 122234134 | KPNA1 |
| 340 | cg25657834 | CGAGCTGCCTGGTTAGTGAGCACCTCC TCTTCTCTGGGAACCTCTAGAACTGGG AGGACA[CG]CCCCCGAAAGGGTGTCCC TGAGCCAACGTGGGACCGCGAGTGCC AGCCCGTTAGCGTCGG | 2 | 11810365 | NTSR2 |
| 341 | cg25809905 | ACTTGATTCTGGTTGGGGGCTTTGCCTA GGGGAGCCTTCCCTGACTCCTCAGGCT GGCCG[CG]TGGGCTAACACACGTAGGC ACAGCATTGAGCACACTGTTTACTCTT GGTCCGTTCACAGG | 17 | 42467728 | ITGA2B |

(continued)

| SEQ ID NO. | Probe | Sequence with the CpG site marked with [] | Chro moso me | Position | Gene |
|---|---|---|---|---|---|
| 342 | cg25928579 | AATGAGTTGTTTCATATTTTGCACTGTCTTTTCATGATCATTTGCATCCATTAGAGACCC[CG]CATCCTATTGGCTTCTTCGTACTCCTCCCGGACAGAACGCAGAGCGAGGGTGAGAGCGAG | 17 | 46692534 | HOXB8 |
| 343 | cg26043391 | AACTCCTGCCTCCCTCTCCCCCCGGCCGAGGTCTGGGAGATGAGAAGGGAGCGCGTTCCC[CG]GGAAGGGAGCCCCCCGCGAGCCCCAGCCGGCTACAGATCTGGGAGGGAGCCGCTCCCGTC | 1 | 224302174 | FBXO28 |
| 344 | cg26162695 | AAGCGCCCACATGCGCCCGTCTCCACCAAAACTGAGAAAGCCGCCGGTCACCTACGCCCG[CG]TTTCCCGTGCACCACCTAGCCGCTCCGCATGGCGGATCCAGCCAATCAGCGCGCCGTGCA | 17 | 12921313 | ELAC2 |
| 345 | cg26394940 | TAAATAAATAAGGGCTTTTGTTTGTTTGCCGGCTCCTGCACATGGCTGCTGGGACTCAAG[CG]CTCGTGTTGTCTGCGCCTCTGTGGGACTCTGGGGACGGGAGGCAGGGGAGGCCCCCGCAG | 22 | 46449461 | C22orf26; LOC15038 1 |
| 346 | cg26456957 | CCGGGTAAAGGGGATGAATAGCAGACTGCCCCGGGGCAGTTAGGAATTCGACTGGACAGC[CG]CGTGGGAGGGAGTGCGGGGAGAGGCAGAGTTGTTTTGTTATTGTTGTTTTATTTTGTTTT | 19 | 55629363 | PPP1R12C |
| 347 | cg26614073 | CTTGGGCAACGTAGGAGACCTCCGTCTCCACAAGTAAAATTAATTAGCCGGCTGTGGTGG[CG]CGCACCTGTGGTCCCAGCTACTCAGGAGGCTGAGGTAGGAGGATCACCTGAGCCCGGGAG | 3 | 47517819 | SCAP |

(continued)

| SEQ ID NO. | Probe | Sequence with the CpG site marked with [] | Chro moso me | Position | Gene |
|---|---|---|---|---|---|
| 348 | cg26723847 | AGCCTGCAGGTGGGTTTGTTAGGGGGA GACCGCTCTGCCAATACTGGCTTTCCC ATCGCC[CG]GCCATCTGCAACTGCCAG ACGCAAAGTGAGGCTCGTCCACCGAGC CCCACTTCCCAGAGC | 11 | 134095652 | VPS26B; NCAPD3 |
| 349 | cg26824091 | GGACTGGTACAGGACAGGCATCTTTGA ACCTATTTCTGGGAGTTCTGAAACTAC TGTTCT[CG]TGGGCCTTGGCGACTGATT TGGGAAAGCTGACCCTGGGTTGGCCTG GCTTCCAGCCACCG | 6 | 38670437 | GLO1 |
| 350 | cg27015931 | TGTTTTTGTGGGAGGCCTTCTGCATGGT CCCGGGAGGTCAGGCAGCCCGGGAGG GCCTCC[CG]GAGCAGAGGCTGGAGTCA GTCCCAATGCCAACAGTTTCGAACCTT GCCCGCGGGCACTGC | 16 | 22012404 | C16orf65 |
| 351 | cg27016307 | TCTCTCCCTGGCCAGGAGACGGTGGCC AAGGGACTTGACTTTGAACTACCAACA AGCTCA[CG]TTTGGCAGCTGCAAAGAC AAAGGCTAGACTTTTAGCAGGTTTTTG GGGGAGCCTGGGGCA | 19 | 49658913 | HRC |
| 352 | cg27202708 | CGGGCAAGGTCTGAAGACTGCGAGGA CCCAGCTGCCAGGCGCATTGTGAAGTG GCCCGAG[CG]TCACAGGCGACCCGGA CCTCGGGACCGGGGGGCAGGGCGGGT GTCTGCAGCGTCCTCGGG | 1 | 223566709 | Clorf65 |
| 353 | cg27544190 | GAACCCTCGACTGGGGGCAGCCGCACC AGTGGACACGGCGGGGTAGGATTAAA GTTGAGG[CG]TGCTCACAGACACTTGT CTGGTGTGAGCCCTTGGCATATAGATG GCTGCGAGTGAAGTGG | 21 | 33785434 | C21orf63 |

(continued)

| SEQ ID NO. | Probe | Sequence with the CpG site marked with [] | Chro moso me | Position | Gene |
|---|---|---|---|---|---|
| 354 | cg21296230 | GGTGCGTTGTTCGCGGGGGTGAATTGT GAAGAACCATCGCGGGGTCCTTCCTGC TGAGGC[CG]CGGACACCGTGACCTCGC TGCTCTGGGTCTGCAGGGAAACGTAGG AAAAAAAGTTGTCAG | 15 | 33010536 | GREM1 |

TABLE 4: Listing of 110 CpGs Subset

| Probe | Sequence with the CpG site marked with [ ] | Chromosome | Position | Gene |
|---|---|---|---|---|
| cg00075967 | GGTGTGGCCAGGAGCCACCCCCACCCC CGCACCTGACTTCACACACATACCTGC CTTCAG[CG]CCTGCCCCAGAGCTCCCA AGCCCCTGCCCGCCACATCTGCAGTGC CGCACACAGACAGGA | 15 | 74495354 | STRA6 |
| cg01511567 | GTAGTTTTATTGTATCAGACTTAGTACA GGGGTGGGGTGGGGGTGTGTATTGGAA TGATG[CG]TGCCCGTTTCTCTGCAAAA TAGTTTCTATGTCATGGAAAGGAGTCG ATGGGACAAGAAGA | 11 | 57103631 | SSRP1 |
| cg27544190 | GAACCCTCGACTGGGGGCAGCCGCACC AGTGGACACGGCGGGGTAGGATTAAA GTTGAGG[CG]TGCTCACAGACACTTGT CTGGTGTGAGCCCTTGGCATATAGATG GCTGCGAGTGAAGTGG | 21 | 33785434 | C21orf63 |
| cg19761273 | GGACAAAGCCACCACCTTTCACAAAAT GAGGCCAGACCACCTGCCTCCCTCCAG TCCCTG[CG]GCCTGGAGACGGAGTCAA CATTCTTATCTGTGTTGGATCTGAATGT TCCTCCTTGCAAAG | 17 | 80232096 | CSNK1D |
| cg17324128 | CCCTCCCCCGCCAGCCTGGCGCATTGC GGGCCTCGGGCTCATTGCTGAGAGGGG GCACTG[CG]CCTGGCACCTCTGTTAAG CAATTTAGGGGCTACAACCTGAGCAAG ACAGATGAGCCCGGC | 10 | 45455500 | RASSF4 |
| cg27015931 | TGTTTTTGTGGGAGGCCTTCTGCATGGT CCCGGGAGGTCAGGCAGCCCGGGAGG GCCTCC[CG]GAGCAGAGGCTGGAGTCA GTCCCAATGCCAACAGTTTCGAACCTT GCCCGCGGGCACTGC | 16 | 22012404 | C16orf65 |
| cg26614073 | CTTGGGCAACGTAGGAGACCTCCGTCT CCACAAGTAAAATTAATTAGCCGGCTG TGGTGG[CG]CGCACCTGTGGTCCCAGC TACTCAGGAGGCTGAGGTAGGAGGATC ACCTGAGCCCGGGAG | 3 | 47517819 | SCAP |
| cg02275294 | GTTTGAATGTTGCTGAAGGACGCTGGT TTTCAAACGGTAAGGAATCTCCTGATA AAGGCA[CG]AATCTTGGTGTGCAGATA AGCCAGCGATTCTTGCTTCTGGCTAGT TCTACGTTGTTCCTG | 1 | 179262462 | SOAT1 |
| cg19722847 | TCTGCTTACAGCTGCTTCCAAATTAAG CATATCTGGATGGTGTGACACTTTTTGT TAGTC[CG]AGAACTGTATGGGCATCGC AACTGGGCCTGTTCCAAGATAGACTTG TTGGGACCTTCAAA | 12 | 30849114 | IPO8 |
| cg19167673 | TTTTCTCTTTGCAGCGAGGCTGGAGGG TGGGCTTTTTTTTTTTTTTTTCCTTTTTG CGCG[CG]TATGTATGTGTGTGCGCGCA AAGTATCTCTATCTAGGGAATGAAAAA TGGGCGCTGGCGG | 22 | 39640835 | PDGFB |

(continued)

| Probe | Sequence with the CpG site marked with [ ] | Chromosome | Position | Gene |
|---|---|---|---|---|
| cg07388493 | GGGAGCCAGTGTTCTTTCTCTCCTGTG ACTTTGGTGAAGTCTCTCACCACTCAG TGTTGT[CG]TGAGCATGCTAGGCAGAG TGCAAGAAAGGAGCAAGAACTCACTA ATGGCTAGGCCTTCCC | 1 | 39491459 | NDUFS5 |
| cg08331960 | TCGGGGTCCCTTGGCCTGGAGACCCTT TGTCCAACCCGTCGCCCACCTCAAGAC CTGCCT[CG]ATGCTGCGCATACAGTAG GTATCCAATAAATGTTCCTGGGATAGA AGGCAAAGGCGCTGG | 16 | 2076597 | SLC9A3R2 |
| cg05442902 | GCCAGGTCACCCTCTCACTCTGTGCCT CTTAGTTATCTTGCATGCTCTGGTCTTT GCATA[CG]CTGCTCCCTGCACCAGGAA CCTCCATCCCCATCTTTGTCTGCTTGTC GAACTTCAGAAAT | 22 | 21369010 | MGC1670 3; P2RX6 |
| cg01459453 | GCAAGTTTAAAAGTACTCACAAAATCT AATAGGCAATTCAACATAAAACTCCAT GGCTAT[CG]CTGTTCCTCACTTTCTGAA CCTTTACCTGCCTGACTTTACTCCATAC CACTCCAACTCAC | 1 | 169599212 | SELP |
| cg03286783 | TTTCCCCGCCTCCCAACCGTGAGGTGT TGGGTTTGGGGGACGCTGGCAGCTGGG TTCTCC[CG]GTTCCCTTGGGCAGGTGC AGGGTCGGGTTCAAAGCCTCCGGAACG CGTTTTGGCCTGATT | 15 | 44580973 | CASC4 |
| cg03019000 | TGAGCATAGTTGTCACCTTCCCCACCT CCCACCAAAAGTCCGGGATTTTCACGA GGGGAG[CG]TTTTATCTTTGGGCCCCT AGAAGAGTGCTTTGTAGTTTGTAGGTC CTCAGAAATTTGAGG | 3 | 51704351 | TEX264 |
| cg16744741 | CAGCTGGATGCACTTGTTCTGGAGCTC CTCTGTGAGTTCAGCAATGGCCACAGT CTGCTT[CG]ACAGCTGCTCCCGCAGCT CCTTCAAATGGTACTCCCGCTCCTGGA TCTCAGCATCCTTCC | 4 | 82126025 | PRKG2 |
| cg07158339 | TACAGGGCTTAACTCATTTTATCCTTAC CACAATCCTATGAAGTAGGAACTTTTA TAAAA[CG]CATTTTATAAACAAGGCAC AGAGAGGTTAATTAACTTGCCCTCTGG TCACACAGCTAGGA | 9 | 71650237 | FXN |
| cg11388238 | GGTCTTGTGTGTTCAGAGGCTGGTTTTA CAGGTGAAGAGAAGAAACAGCCGCAG AAGTTG[CG]ATTGTCCAAGGTCACTTA ATAAGTGGCAAGAATTAGGATGTTAAG TGTTCTCACCCCCAG | 2 | 201375098 | KCTD18 |
| cg25070637 | TGCCAATCGGCGTGTAATCCTGTAGGA ATTTCTCCCGGGTTTATCTGGGAGTCA CACTGC[CG]CCTCCTCTCCCCAGTCGC CCAGGGGAGCCCGGAGAAGCAGGCTC AGGAGGGAGGGAGCCA | 8 | 97505868 | SDC2 |

(continued)

| Probe | Sequence with the CpG site marked with [ ] | Chromosome | Position | Gene |
|---|---|---|---|---|
| cg13547237 | GCAGTGCATCGAGCTGGAGCAGCAGTTTGACTTCTTGAAGGACCTGGTGGCATCTGTTCC[CG]ACATGCAGGGGGACGGGGAAGACAACCACATGGATGGGGACAAGGGCGCCCGCAGGTGGG | 11 | 65687877 | C11orf68; DRAP1 |
| cg13931228 | GGTGTGAATCACACTGCCCGGTCGGGCCTTTGGGAAAAAATTAATGAAGGACACAGTCAG[CG]CCGTAGAACCTGCCAAATACACATCAGATCCAGTGGAGTCTGTGAAGGGGGAGGGGGAGA | 7 | 24612418 | MPP6 |
| cg22947000 | TAGCTATGACACATGGCTTGGAAATTAACCTTTAACCAAACATCTTATAAGTAACGCCAG[CG]CAGCTTCCCTTGTGAATGTAAAGAGATCCAGGGCTCTTGGAGAGGGACAAGTGAGAGCCA | 16 | 81272281 | BCMO1 |
| cg00431549 | TAACTGCTGGACCTGACTGTGTTACACAGGATGCTGCTCTGGTGCAGAAGTTTTGGCCAT[CG]TATGCTTGGGGACAGACCTGGGCAAAAGCCCACAGAGGAAGTTGCCACAAACACATGATC | 12 | 15039025 | MGP |
| cg25809905 | ACTTGATTCTGGTTGGGGGCTTTGCCTAGGGGAGCCTTCCCTGACTCCTCAGGCTGGCCG[CG]TGGGCTAACACACGTAGGCACAGCATTGAGCACACTGTTTACTCTTGGTCCGTTCACAGG | 17 | 42467728 | ITGA2B |
| cg26394940 | TAAATAAATAAGGGCTTTTGTTTGTTTGCCGGCTCCTGCACATGGCTGCTGGGACTCAAG[CG]CTCGTGTTGTCTGCGCCTCTGTGGGACTCTGGGGACGGGAGGCAGGGGAGGCCCCCGCAG | 22 | 46449461 | C22orf26;LOC150381 |
| cg08090772 | TCTTACTCCGTGGGAAAATGGCCCTGAGCCCGACTGGCTTGAGGCTTAGACAGGTGACCC[CG]CGAAGCGGGTGGGCAGGCGCGGCCGAGGGGCGGGAGGCGGGCAGCCTCCGTGATTGGCCG | 8 | 67344640 | ADHFE1 |
| cg01027805 | CGGTTTGGAGACGGGGGGCGCTGTCGGAGGGAGGGAGGAAGGGAGGGAGCGGGGGTGGGG[CG]CACAGAGGATTCCAACAGGAGACTGGAAGAGATTTTGAAAGGTCATCTCGTCCTTCCCCC | 14 | 21566863 | ZNF219;C14orf176 |
| cg04474832 | CCAGCCAAGTGGCCTTGATCGTTTTCCCAATGCCCCCGAGCCTGTTTCCTGCCAGTAGAG[CG]GGTCAGATGTTGCCAACCTCTGCAGAGTAGCAATAAGCAGTAAACGCCACGCTCTGCACA | 3 | 52008487 | ABHD14B |
| cg24899750 | GGAGGAACTGGCTATCCTAAAGGTGATTTTAAACCGGGGTAGCTAGAGCCCAAAGAAGGG[CG]AAACCAGGACTAACTGCCCCATAGCATGAGGGGCAGCGCCTGTAAAATTACATAGGATTT | 20 | 16710314 | SNRPB2 |

(continued)

| Probe | Sequence with the CpG site marked with [ ] | Chromosome | Position | Gene |
|---|---|---|---|---|
| cg04268405 | TGACGTTACGTACTGGAAGTCCCAGGA GGAATGCCCAGCAAGTGGAATCCAAG ACGTTCT[CG]CCTTCTCGGGGACAGGG CCATCACCAGGATTCGGAAAGGAACA GGGAGGTTCGGTTTGTG | 10 | 73723221 | CHST3 |
| cg12413566 | ACCAGGGGGTGATGCCAGACATTGCTC ACTTTTTCCATGTAGTCAATGTCAGTCC TGCAG[CG]TCAGCTGGGATGGGGGTAA GGACATCTGGGAACCCCCTCTTCCTGG TCTCCCTCCCTCTT | 3 | 39235366 | XIRP1 |
| cg01820374 | GGGAGGCTCAGTTCCTGGGCTTGCTGT TTCTGCAGCCGCTTTGGGTGGCTCCAG GTAAAA[CG]GGGATGGCGGGAGGGTT GACCTCCAGCCCCACAGGAGGGGACC AGCAGGGATCTCTGTGG | 12 | 6882083 | LAG3 |
| cg06557358 | AGCATCGAGACAGCGGGCGAACGGGC GTCCGGGGACAGGGTGGGGGCGGCGG GGAGGAGG[CG]TCGGAGACTCTGAAC CCCAGAAAAGTTCAAGGTTTGTGCAGG TTCCCCCAGGGAAGGCGA | 17 | 32907002 | TMEM132 E; C17orf10 2 |
| cg09809672 | CCCCAGAGAGCTTTCATCTAGAAGGTT TGACTCTGGCCAGACAACCAGCGAGCA TCTTCT[CG]CAATCTGTTGCTTCTTCCA TGGCAAACTCCAGAGAATTAAGAAGCC AAACTCAACATCGC | 1 | 236557682 | EDARAD D |
| cg18328933 | CCAGTAGAGCGGGTCAGATGTTGCCAA CCTCTGCAGAGTAGCAATAAGCAGTAA ACGCCA[CG]CTCTGCACAGCCTCCCAG TGCTGGGCCTGGTCGCCACGCGGAGCC TTGGGCTGGGACAGG | 3 | 52008538 | ABHD14B |
| cg22197830 | GAAGGCTCCTGGGCCTTTCTGGCTCTG GGAATGAAGCGTGGAAAACCCTCCTTA GGCGGG[CG]CAGTGCTTCAAGTAGCCA AGCTCTGACTTCCGAGGGAAGAAAGG AGGCCATGGGCCTCTG | 5 | 134209784 | TXNDC15 |
| cg13828047 | TCAACATACTACATGATTTGCTTACAA TACTTGTCTGTCTTGCCTTCACCAGAAT GTAAG[CG]CTCTACAAAGGCAGAGGG AAGGCTATCTTGCTCTCTGATGTATCCT CCAGCCCTTAGAAC | 15 | 75182130 | MPI |
| cg19724470 | CATTCTTATGCGACTGTGTGTTCAGAAT ATAGCTCTGATGCTAGGCTGGAGGTCT GGACA[CG]GGTCCAAGTCCACCGCCAG CTGCTTGCTAGTAACATGACTTGTGTA AGTTATCCCAGCTG | 9 | 5450936 | CD274 |
| cg01407797 | TGATTATATGTACTATTATTATCTCATT TTACTACTGTGGAAACTGAGATACGAA ACTTG[CG]GAGTGAGGATTTGAACCTA GGTCATACTCTTGGCCAGCCAGAGACA CCCTAAGCCCCAGC | 22 | 29168514 | CCDC117 |

(continued)

| Probe | Sequence with the CpG site marked with [ ] | Chromosome | Position | Gene |
|---|---|---|---|---|
| cg07408456 | GGCCTGGAGACCAGGTGGTTCAGACTC CATAAACTCTGCCCATTCTCCAGTGAG GTGGAC[CG]AGGCAACCCCTCAAGTCC TGTCCCTCCCCATAGTGACGGCTCTGT AGCCGCTGCTGGCCA | 19 | 15590532 | PGLYRP2 |
| cg27202708 | CGGGCAAGGTCTGAAGACTGCGAGGA CCCAGCTGCCAGGCGCATTGTGAAGTG GCCCGAG[CG]TCACAGGCGACCCGGA CCTCGGGACCGGGGGGCAGGGCGGGT GTCTGCAGCGTCCTCGGG | 1 | 223566709 | Clorf65 |
| cg01570885 | GGAGGAGGGTTGGAGAGCAGGGCCGT GTTGCAAGGCTCTCTGGGTGGCCACAG CAGCTTG[CG]CTGCGCCCACATTGCTT CTGCGTGTTTACAGTTGGGCACGAGAA GGCTCAGCACGCACGC | 6 | 3849272 | FAM50B |
| cg24058132 | GGGCCATGAGTGGCCCTACCATGGCTC TTCCCCAGCATCTCAGGGAGTATCTAC CTCGTG[CG]AGGACCAGGCTTGGACAC CAGGTCCCGATTCCATTGTCATCTTGGT GGAATCACTTTGCT | 14 | 88459866 | GALC |
| cg11025793 | TGGTCTCCCCTGGAGGGTGGGCGGGTT ATCTGAGGGAGTCCTCGGAGGGTCGCC CCCTTG[CG]CGTCAGAGTTGCTGCGTG GGGTCTCAGAGATAGCGCCTGGGCTGG GGAAATCATTGTGGG | 19 | 13262015 | IER2;STX 10 |
| cg19853760 | AAAAGGGTGGGAGCGTCCGGGGGCCC ATCTCTCTCGGGTGGAGTCTTCTGACA GCTGGTG[CG]CCTGCCCGGGAACATCC TCCTGGACTCAATCATGGCTTGTGTGA GTGTGGGGACCCCCCC | 22 | 38071677 | LGALS1 |
| cg02217159 | TATTTCCGATGACCTACATCTCAGGGA CGCAGTAGGATGTTCATTGATAAACAA ATAAAG[CG]GCTCGAAGAAATATTGTG CAGAGACATGATTGAGGTGTACAATCA TTAGGATATTGAATT | 6 | 62996697 | KHDRBS2 |
| cg27319898 | GGAATTCCTGATTCCCTGGTGGACCCT GGAAGTTGTCCTTAAATAAATATATCG CTGGCC[CG]CGGTTGAGCAGCCACCTC GTCAGAGCAGCATGTGGACTGGCTCGC CGGGTCCCCTCCGTG | 7 | 88389003 | ZNF804B |
| cg13269407 | CAGACACCGAGCCGCGGCCACAGGGC CAGCCGCACAGTCGGAGGAAGGGCCG GAGCGAGG[CG]GGGCCCGGGGCTGTC AAGGAGAAAAACATCCCAAGGCCTGC AAATTGCTGCTCTCAGCTT | 22 | 46450107 | C22orf26;L OC150381 |
| cg14654875 | TGTCCTTTGTGTCTTGAGCGGATGGTG GGGCCGTGGAACATGAAGGAGTATCTT TGTGTA[CG]TTCACAACGTTCACATCG GTGTAGGCCAGGTTGCTGGACTCTGAC TCAAAGTGTTATAGA | 16 | 3493997 | NAT15;ZN F597 |

(continued)

| Probe | Sequence with the CpG site marked with [ ] | Chromosome | Position | Gene |
|---|---|---|---|---|
| cg13129046 | CTACTCAAGGGGCATCCACGGAGCTGG GTCAGCAAACATAACACTGGTCATCTG AGCCTG[CG]CCCGCCCTTCCTCCCAGG CCAGGGCGCCCCCACCCCCTGGGTTTT TCCTCCGTGGACGCC | 10 | 71389696 | C10orf35 |
| cg12941369 | TCACATGTTTCGTTTCTAGTCCTGAAAC ATGGTTAAGTGCTTGCCTCCTAGGGCC TCTGC[CG]CAGGCTTTTGGTTTGGAGG CTCTCCTTTGCCACTCCACCCCTCTCCA CTCTTCTCCTCTT | 3 | 33839389 | PDCD6IP |
| cg09191327 | GCTCCGTGCTCCCGGCTGAGGCCCTGG TGCTCAAGACCGGGCTGAAGGCGCCG GGACTGG[CG]CTGGCCGAGGTTATCAC CTCCGACATCCTGCACAGCTTCCTGTA CGGCCGCTGGCGCAAC | 9 | 133540108 | PRDM12 |
| cg22171829 | CTGTGTCCCCTCTCACCAAAGTCCAGT AGCTGCTTCATGGACAGCGGGGACGGG CTGTAG[CG]CGAGAAATGCTCCACCTC TCGGGGCACCAGGCCGGCGCCGTTGAG CGAGCCAGCGCTGCG | 7 | 95225520 | PDK4 |
| cg17338403 | TGGAAGGTGCTGTTTCCTGGTACCTGT CCAGCCCTCTGAGCTTTTCTCTCAGCTT CCAAA[CG]CTGCAGTTGAGAACTAGCA GATCCTATTGGTAGTGCCCTGTGGCCC ACACTCCTTGGTAA | 15 | 92395836 | SLCO3A1 |
| cg09722397 | TCGGGGTATTTTTAGGCCGGCGATAAA TAATTCATAGGGAACGTGGCATCAGGC TCCCCC[CG]CGGGAGGAGGGGGCGCG AGCAGCGAGAGCCACCGTCACCCGCG GCTCAAGGACACTCGCG | 17 | 72855943 | GRIN2C |
| cg02489552 | CTCCTCCCCCCACCTCTGGAATTCCACC TCCCTTGTTGCGCCCATCGCTATGGTG ACGGG[CG]CTCTCAGTACACTGTCTCT ACAGGCCAGGAAAGAGTTGTGTGTCTT TGGGGTCCCTTCCG | 19 | 15121531 | CCDC105 |
| cg15661409 | TTGTTAATCTTTAATTTAATTAAAGAAT TTATCCCCCAAATAGGAAAGAAAGCAG CGGAG[CG]GCTAAAGCGTCATTTGATT TTTCTGTCGATGACTTGAGTTGCCTTTG AAGGGGGTGAATA | 14 | 57960976 | C14orf105 |
| cg06810647 | TGCCGCGGGGGAGAGGAACCCCTCGC CCCAGCCGGGCTCCACCCTAGCTCACC CATCCCG[CG]GCCTACACTGAGGCTCT CAATTTGGGTGGCACTTATGGGGCATG TGTCCCCTCTCTCCTT | 16 | 1665094 | CRAMP 1L |
| cg02388150 | AACCTATGAAAATAAACAAAAGCTGCT CCAAGCATTCTCTCGGCCTTTCTGAACT TTCTA[CG]CTTTGGGTTTTTGTTTTTCC TCCCGTCTCAGAGGTTAAAAACTTCGA TAGGGACTCGGA | 8 | 41165699 | SFRP1 |

(continued)

| Probe | Sequence with the CpG site marked with [ ] | Chromosome | Position | Gene |
|---|---|---|---|---|
| cg18983672 | GGCAGCCAGAAAGGCAGCTCCAAGTTGTGGATTTCCTGGGGGCTCTTCATTTAAAGCGGC[CG]CACCACTTTCCACAATTCTGTTTTTTCAGAGAATGCTCTCAAGGCCTGGAGGGAGGGCAT | 1 | 47881256 | FOXE3 |
| cg06993413 | GAGGCGCGGGGTGGAGACTGGGCCGAGCAGGGGATAGAGATGAACTCCAGAAAGGAACAG[CG]ACTTGCTGAAAGTCACAGGGCAAAATGTGGCGCGTCTGTAGTCAATAAATAATATATATT | 15 | 65810204 | DPP8 |
| cg26842024 | CGACGACGACCTCAACAGCGTGCTGGACTTCATCCTGTCCATGGGGCTGGATGGCCTGGG[CG]CCGAGGCCGCCCCGGAGCCGCCGCCGCCGCCCCCGCCGCCTGCGTTCTATTACCCCGAAC | 19 | 16436122 | KLF2 |
| cg21870884 | GGGCCCGCGGCGGCTGGTGGATACCTTCGTGCTGCACCTGGCGGCAGCTGACCTGGGCTT[CG]TGCTCACGCTGCCGCTGTGGGCCGCGGCGGCGGCGCTAGGCGGCCGCTGGCCGTTCGGCG | 1 | 200842429 | GPR25 |
| cg18984151 | TCCCTTGGCCTCGCTCTCTGCCCAGCCCCGGGCTCCTTTTCTCCACACGTGGCTGTCAAG[CG]CCTTCTGTATGCCCCACACTCCTGGGAGCTTGGGCTACATCGATGAACAAAAACAAAGGA | 3 | 47555476 | C3orf75 |
| cg18180783 | AGCCAGGATCTGCCTTTTAACCTCCATTTGCTGTTGAGATGCTCAGTTCAACCTGCTGTG[CG]GGATAGACATCGATGTCTCCCTGAGAAGCACATATAGGCTCTCTGAGGTTTCTTTTCTTC | 10 | 75402320 | MYOZ1 |
| cg16547529 | CACTGGCTTGTTAACTCTTCAAGGGCAGAATTATGGGCACCGAGCCTCTAAAATGTTGAA[CG]AATGACTGAATATCATCAAGAGGCAGTACTAAAAGATGATGAAAGAATGAATGAGCGGTG | 11 | 75140681 | KLHL35 |
| cg22901840 | GTGCAGGGAAAGCACACCGTGGCTGCAGCCCAGCAACTGGCAGTAGGTATTTTCAATGGT[CG]GCAGGTACTCATGACGGAAGTTGCCGCTCGCCCCACTTGTGCAGCAGCGTACTTTTCCCCA | 1 | 68512777 | DIRAS3 |
| cg02332492 | CGGGGCAGCTGTCAGTGAAGCTCTACGGTATGTGGGGGCCAGCCTCTGTGACCAGGCAGG[CG]CTCAAGCTCTGCACACTCACTGGGCCACCCCGAGGGGCTGGGTGAGCCCATGGGGACACA | 9 | 139840678 | C8G |
| cg24262469 | CTCTGCAAGCTCCATGAGGACAGGCGTGAAGTTCAGGCTACATGCCTGGTACGTAATAGA[CG]CTCTGACAGACATTTGCTGAATGAATAAGTTAGTCACTACGGCGTTTGTGGGCTTTAAAA | 3 | 156391694 | TIPARP;LOC100287227 |

(continued)

| Probe | Sequence with the CpG site marked with [ ] | Chromosome | Position | Gene |
|---|---|---|---|---|
| cg15547534 | CTCCTCCTCTTGAAAACTCTGCTATGGC TGAGTTACCCAGAGGAATCTTAGTCCT GCTAG[CG]CTGCGATGCCCATTGCCCA GTGTGTCAGTCCTCATTCTGGGGCGCC AAATGGGGCAGCAT | 7 | 100034410 | C7orf47 |
| cg20828084 | GACTCCATATGCCCTAGGGATGTGTTG TGATGAACTTTTCCTACTGGTACTGTTT CCTCC[CG]CGAGGGAATGTCTAGACCA GCCGCACCTTCTTGCTTTGACCCTTCAG AACTTTGGCCTGT | 15 | 81070851 | KIAA1199 |
| cg02580606 | AACCTAAATTTTGGGAGCACCTACTCT GCATGAAGCACTGTGCTCCATGCCTGT GCACAG[CG]TGACTCTGTCATTGGTGA TGGGTCCTGCTTGCTGAGCCTCCACTG TGCACCAGGCACAGT | 17 | 39526726 | KRT33B |
| cg05675373 | AAGGAGGAGATGGCCAAGGGCGAGGC GTCGGAGAAGATCATCATCAACGTGGG CGGCACG[CG]ACATGAGACCTACCGCA GCACCCTGCGCACCCTACCGGGAACCC GCCTCGCCTGGCTGGC | 1 | 110754257 | KCNC4 |
| cg26453588 | GGCTGCCCACCCGCCCACCCCGCCTGG AAGCTTTCTGATTTCTCTGTTCGCCCCG CCAGG[CG]CTGTGGGGTCCGTCTCACC AGGTCTGCACGTGAGCCCCCTGCCCCC AATCCCTCCCAGTC | 22 | 43506021 | BIK |
| cg13682722 | AGTGGTTGGGACCCTGTGAGAACCGGA ACTGCGAAAACCGGAGAAGGGAATTG TTGACCG[CG]AAAGGGACTAAGGAAA TTGGGATTCCAGTTCGACCCCTAAATT CACACCATCCTTGCTAA | 14 | 90798568 | C14orf102 |
| cg01353448 | GCCCAGCCTCGGTGAGCACACACGCCC TCCCTGTCTCTCGCCTTCGCTTCCCTGC ATCTG[CG]CTGATTGGTAAGTGCTTCA GATTTTTACTCCAAGAACTTTTGTGGTG AGAAAAGCAAGTT | 7 | 31726912 | C7orf16 |
| cg24580001 | TCTTCTGAAGGATTTGATGCTGGTGCTT TTCAGGTGTGGGTCCTGACAGTGATGT TGGGA[CG]GCAGCTAGCCAGACAGCA ACTGTACCATGTAAACTCACTTCAGAG GTGTAGAATGGGGGC | 11 | 64106532 | CCDC88B |
| cg18440048 | GTAGCCCTGTTCCTGTCTGCCCTCCCCG CCCCCACAGAAATAGAGATGAGAAGG GGCAGG[CG]AAGAACTAGGAGTGTCT GCGAGACCATCCCAGGACCCTGAGCCC CCCAACTCTCTGCATC | 22 | 24093826 | ZNF70 |
| cg13460409 | ATCTCTCACCTTGCTACTTTCTCGGTAG CCGTTTCTGTTGTCCCTGGATTGGGGG CTCGG[CG]TTCGCTGTCCCTGGGCACC AACCCTTTTAAAGACAGTAACGTTGTA GGAAATCAAATTAG | 21 | 38379570 | DSCR6 |

(continued)

| Probe | Sequence with the CpG site marked with [ ] | Chromosome | Position | Gene |
|---|---|---|---|---|
| cg01968178 | CTGCAGCGGCCCCGTTTGCAGGGCAGGGACCCGGGTGCTGCCCCACCCTCAGCGTTCCAG[CG]GAGAAACTGAAGTCCGAACCTGAACCTCGGGAATCTGTCTGCACCTGTCTAGGTGGGATG | 2 | 86565038 | REEP1 |
| cg13038560 | GACCTCAAGTGATCCACCGACCTGGGCCTCCCAAAATGTTAGGATTACTGGCATGAACCA[CG]GCGCCCAGCCCATCCGACTTTTGTAACACTCAGAATTGTAGTTTTGTTTGTTTGTTTGAG | 2 | 200819113 | C2orf60;C2orf47 |
| cg23517605 | CTCCAGTGCCGGCAGGTGGGAGGGCTGAGGTGGCACAGGCTGCTCCGCCACCTCGGACTG[CG]GCTCCTACTCGGCCACTGGCCAGAGTCCCTCCAGCCAACTGCCCCTGGTGAGACCACCGT | 6 | 3228365 | TUBB2B |
| cg13975369 | CCATTTGAGGGCAAGGGCTGTGTCTTTGGGTACTTCGCTCCTCGCAGTCACAAGTACTGG[CG]TGCGTACGCGGGGAGAGATCGCTCCTCAAAACGGGGTCCTGAACGCTGCCCCGCGGCCCC | 7 | 130080553 | TSGA14 |
| cg19008809 | GCGCGCGTGCCGCCGCCGCGGGCACTGCGCCCGTTTGCCTGCCCCTCGTCGGGGATCGGG[CG]CTCCCTCTGAGACCTGAAAGGGCACCCAAGTGCCCCCTGTCTGCGAAGTCCGGCGCGGGC | 3 | 53080682 | SFMBT1 |
| cg12830694 | CCACTGGCCCGGTTCAACGAATATCTATTAAGTATCCACTCTATACCAGACACTGCTTTA[CG]CTCCAGGGATAGAGCAGGGAACAAAACAGACAAAACCAGTCCCACGCAGTTGACAGTTGT | 19 | 38747796 | PPP1R14A |
| cg23662675 | TGGCTGCCCCGGCAAATCGGAGTGTAAAGCCGCCCCGGATTGGCTGAAACACTTCCTGAG[CG]ATTATCTTTGTGAGGCTCGGGTGAGCAAGAGCCATCCTGTGCATAGAAAAAGACAGGCTA | 20 | 45985596 | ZMYND8 |
| cg02331561 | CAGCGGCGGTAGCCGAGCGAGGGCGCGGTGGCCTCTGACAGGAATGACTCTGCGCACGTG[CG]TTTCGCAGCAGTGGAAGTCTTCACACCCGGAAACTCGACTTTGGCCGTTTCTCCATTTCT | 16 | 2391081 | ABCA17P; ABCA3 |
| cg10523019 | CTCGCTGCTTCTCCCCTAGTCTTCGGGTCCCTTGAACGCAGGTCGCTTGTTTGCCTTACG[CG]TAGTCAGCGGCCAGTGGCTATTTATGGCAGTAAGGAATATTATCCACATTTCACATGGAG | 2 | 227700458 | RHBDD1 |
| cg27377450 | CTACACAAAGGCGCTCACACTTTATCCGAAACAGCAGTGGGGCTTGGGTGCGGTGGCTCA[CG]CCTATAATCCCAGCACTTTGGGAGGCCGAGGAGGGTGGATCATCTGAGGTCAGGAGTTCA | 19 | 7446301 | |

(continued)

| Probe | Sequence with the CpG site marked with [ ] | Chromosome | Position | Gene |
|---|---|---|---|---|
| cg06144905 | CTGACCTCACCACCCACCAGGGAGGTGGGTCTTATTCTGGGCATCGTGCCAAGTTCTTAG[CG]GGGCCCTCTAGAATCTCTAAAGCAAATCAGGCTGAAGAGGGGAAAACCAGCAGGGGGAGG | 17 | 27369780 | PIPOX |
| cg26845300 | CGCAACACCCCAGGCGTGGGGCAAAGACAGCGGGGTTGCGGGGCTCCTGTCTGCCCGGGG[CG]TCGAGAGTTCCTGCCGCCCCCTCCCGCCTCATGCACGGAAAGCGCCGAGCCACGGCGTGC | 6 | 158243833 | SNX9 |
| cg25771195 | GATAAGCGCCTAATATACATCCCTGCCTGTCATTATTCACATTGTGGCATGCAGTCAAAG[CG]ACACTCTGAGGAAAATGTATCGCCTTAAATACATTGATTAGAAAATAAGAAAGCCCGAAC | 16 | 58163814 | C16orf80 |
| cg12946225 | CCGGCGGGCGGCAAGGCTCCGGGCCAGCATGGGGGCTTCGTGGTGACTGTCAAGCAAGAG[CG]CGGCGAGGGTCCACGCGCGGGCGAGAAGGGGTCCCACGAGGAGGAGGTGAGAGTCCCTGC | 19 | 3573751 | HMG20B |
| cg26005082 | AGCTCTCCACCGACCGAAGGAGGAGAATGCTATTTATTTCAGCACCAAATATCCGGACAG[CG]CCTCTCGGGAGGTCCGAGAAGAGAACCGCGATCTGTTTCAGCACCGGGGCTCAGGACAGT | 19 | 4769660 | MIR7-3; C19orf30 |
| cg21378206 | AAATAGGGGAGTCTACACCCTGTGGAGCTCAAGATGGTCCTGAGTGGGGCGCTGTGCTTC[CG]GTGAGTGTATGAGGCCCTGGTTTGGTGGTGTCCTCCGGAGGAAGTGAGTTCTGGATAGAC | 2 | 113817043 | IL1F5 |
| cg10281002 | TTGGGATGCGATAACTCAGTGCCCTCTTGCAGACTTGCATAGAAATAATTACTGGGTTGT[CG]TGGAGGGGACACGAGACAGAGGGAGTTCTCCGTAATGTGCCTTGCGGAGAGAAAGGTCCA | 12 | 114846399 | TBX5 |
| cg22920873 | CGAAGATCCGGCCAATTTGCCCAGCGCGCTGTGCTCCGCGACGGCGCATGCCCGCTTTTG[CG]CAGGCGCGGGGACTACGGCGCAGGCGCGGAGACTATTGCGCAGGCAAGCGCGTACGCAGA | 7 | 139025153 | C7orf55 |
| cg19945840 | GCGCGCCCTGGAGCGGGAGCAGGCGCGGCACGGGGACCTGCTGCTGCTGCCCGCGCTGCG[CG]ACGCCTACGAAAACCTCACGGCCAAGGTGCTGGCCATGCTGGCCTGGCTGGACGAGCACG | 1 | 1168036 | SDF4;B3GALT6 |
| cg04084157 | AGGGTGCCTGCCTCTCCCGGCCTGCGCCTGCGCGCTGGGGCCTTCGGCTGAAGGGGTGTG[CG]CTAGCGGAGCTCCGGGAAATGAATGAATGAATGAATGAATGAAATGCTGAAGCGGGCAGG | 7 | 100809049 | VGF |

(continued)

| Probe | Sequence with the CpG site marked with [ ] | Chromosome | Position | Gene |
|---|---|---|---|---|
| cg20692569 | CGACCCGGAGCGCGGGCGCGGGGCTGCGCCGTGCCAGGCGGTGGAGATCCCCATGTGCCG[CG]GCATCGGCTACAACCTGACCCGCATGCCCAACCTGCTGGGCCACACGTCGCAGGGCGAGG | 7 | 72848481 | FZD9 |
| cg26297688 | ATAAGCCACGTCTCTCCTCACCCCTAGCACTTAATCACAAAGGCCTGTAGAGAGTCCCGA[CG]AGAACTTCTGAGCAGGCCCCGCTGTCAGTCCCTGAGGACAGCATGCAAGGGAGGTTGACG | 12 | 107349093 | C12orf23 |
| cg04528819 | GCAGCCCGGGAAGGGGCATTGGTGGCGCTTGGCAGCAGGTGTGACAGACCTCCTCCGGGG[CG]CCTGATCCGCGGCGGGGGCGGGGCCTGCCCCTAGGGCCCCTCCAGAGAACCCACCAGAGG | 7 | 130418315 | KLF14 |
| cg06493994 | GGAGAGCAAGTCAAGAAATACGGTGAAGGAGTCCTTCCCAAAGTTGTCTAGGTCCTTCCG[CG]CCGGTGCCTGGTCTTCGTCGTCAACACCATGGACAGCTCCCGGGAACCGACTCTGGGGCG | 6 | 25652602 | SCGN |
| cg25505610 | GAGGCGCCAGCGGGAGGCAACATCAATGCAGTTAGCTACACGGGCCTGAAAACTGGAGGC[CG]CGACAAGCGTCGCTGAGTGGAGGCCCAGTAAGTCCCACCCACTAGGCCAGCCCGAGCGCG | 11 | 32605184 | EIF3M |
| cg00864867 | AGTACAAGACCGTATTATTTGAGAGAAAGTCTCGAACGCTGCTGGCTAAGGGGAAAAGTG[CG]ATAACTTGTGATGATTCAGGGAATGACTAGACAGGATGGGAAAATACCCACGTGTCTCTT | 12 | 80085268 | PAWR |
| cg02479575 | GAGGGACAGCTCTCCACCGACCGAAGGAGGAGAATGCTATTTATTTCAGCACCAAATATC[CG]GACAGCGCCTCTCGGGAGGTCCGAGAAGAGAACCGCGATCTGTTTCAGCACCGGGGCTCA | 19 | 4769653 | MIR7-3; C19orf30 |
| cg22736354 | TGCGCCAGGGCGGCCACGCAGGCCAGGCAGACCACGTGGCCGCAGGACAGGTTGCGCGGG[CG]CCGCTGCTGCCGGTGGCCAAACTTCTCAAAGCACACCTTGCACTCGAGCAGGCTGATCTC | 6 | 18122719 | NHLRC1 |
| cg14424579 | TAAGCGATAAGGAGTTTCACACGATGTCTTTTTATTTCGCAGTTGAGTCCCAGTTTCTGC[CG]CTTTATCTTTCCCGCCTCCCGGCAGGCAGGCCGTTAACCGTCTTCCGGAAGACGCTGCTA | 2 | 27274309 | AGBL5 |
| cg16241714 | GGCACAGCTCCAGGGTGGGCACGGCGGCCATGGAGTCGATGTAGGCGCTGAAGTCGATGG[CG]CTCTCGTCGTCGTACATGGCGGGGGCGGCGGCGCCTGGCTCGCCTAGGGCCCCTGGCTCG | 8 | 48650511 | CEBPD |

TABLE 5: Listing of 38 CpGs Subset

| Probe | Sequence with the CpG site marked with [ ] | Chromosome | Position | Gene |
|---|---|---|---|---|
| cg00431549 | TAACTGCTGGACCTGACTGTGTTACAC AGGATGCTGCTCTGGTGCAGAAGTTTT GGCCAT[CG]TATGCTTGGGGACAGACC TGGGCAAAAGCCCACAGAGGAAGTTG CCACAAACACATGATC | 12 | 15039025 | MGP |
| cg00864867 | AGTACAAGACCGTATTATTTGAGAGAA AGTCTCGAACGCTGCTGGCTAAGGGGA AAAGTG[CG]ATAACTTGTGATGATTCA GGGAATGACTAGACAGGATGGGAAAA TACCCACGTGTCTCTT | 12 | 80085268 | PAWR |
| cg01353448 | GCCCAGCCTCGGTGAGCACACACGCCC TCCCTGTCTCTCGCCTTCGCTTCCCTGC ATCTG[CG]CTGATTGGTAAGTGCTTCA GATTTTTACTCCAAGAACTTTTGTGGTG AGAAAAGCAAGTT | 7 | 31726912 | C7orf16 |
| cg01459453 | GCAAGTTTAAAAGTACTCACAAAATCT AATAGGCAATTCAACATAAAACTCCAT GGCTAT[CG]CTGTTCCTCACTTTCTGAA CCTTTACCTGCCTGACTTTACTCCATAC CACTCCAACTCAC | 1 | 169599212 | SELP |
| cg01511567 | GTAGTTTTATTGTATCAGACTTAGTACA GGGGTGGGGTGGGGGTGTGTATTGGAA TGATG[CG]TGCCCGTTTCTCTGCAAAA TAGTTTCTATGTCATGGAAAGGAGTCG ATGGGACAAGAAGA | 11 | 57103631 | SSRP1 |
| cg02275294 | GTTTGAATGTTGCTGAAGGACGCTGGT TTTCAAACGGTAAGGAATCTCCTGATA AAGGCA[CG]AATCTTGGTGTGCAGATA AGCCAGCGATTCTTGCTTCTGGCTAGT TCTACGTTGTTCCTG | 1 | 179262462 | SOAT1 |
| cg02479575 | GAGGGACAGCTCTCCACCGACCGAAG GAGGAGAATGCTATTTATTTCAGCACC AAATATC[CG]GACAGCGCCTCTCGGGA GGTCCGAGAAGAGAACCGCGATCTGTT TCAGCACCGGGGCTCA | 19 | 4769653 | MIR7-3; C19orf30 |
| cg04084157 | AGGGTGCCTGCCTCTCCCGGCCTGCGC CTGCGCGCTGGGGCCTTCGGCTGAAGG GGTGTG[CG]CTAGCGGAGCTCCGGGAA ATGAATGAATGAATGAATGAATGAAAT GCTGAAGCGGGCAGG | 7 | 100809049 | VGF |
| cg04528819 | GCAGCCCGGGAAGGGGCATTGGTGGC GCTTGGCAGCAGGTGTGACAGACCTCC TCCGGGG[CG]CCTGATCCGCGGCGGGG GCGGGGCCTGCCCCTAGGGCCCCTCCA GAGAACCCACCAGAGG | 7 | 130418315 | KLF14 |
| cg05442902 | GCCAGGTCACCCTCTCACTCTGTGCCT CTTAGTTATCTTGCATGCTCTGGTCTTT GCATA[CG]CTGCTCCCTGCACCAGGAA CCTCCATCCCCATCTTTGTCTGCTTGTC GAACTTCAGAAAT | 22 | 21369010 | MGC1670 3; P2RX6 |

(continued)

| Probe | Sequence with the CpG site marked with [ ] | Chromosome | Position | Gene |
|---|---|---|---|---|
| cg06117855 | TGGGGAGGGTTTCCTGGACAGAGGTCCTTTGGCTGCTGCCTTAAGACGTGCAGCCTGGGC[CG]TGGCTGTCACTGCGTTCGGACCCAGACCCGCTGCAGGCAGCAGCAGCCCCCGCCCGCGCA | 3 | 45067788 | CLEC3B |
| cg06493994 | GGAGAGCAAGTCAAGAAATACGGTGAAGGAGTCCTTCCCAAAGTTGTCTAGGTCCTTCCG[CG]CCGGTGCCTGGTCTTCGTCGTCAACACCATGGACAGCTCCCGGGAACCGACTCTGGGGCG | 6 | 25652602 | SCGN |
| cg07158339 | TACAGGGCTTAACTCATTTTATCCTTACCACAATCCTATGAAGTAGGAACTTTTATAAAA[CG]CATTTTATAAACAAGGCACAGAGAGGTTAATTAACTTGCCCTCTGGTCACACAGCTAGGA | 9 | 71650237 | FXN |
| cg07388493 | GGGAGCCAGTGTTCTTTCTCTCCTGTGACTTTGGTGAAGTCTCTCACCACTCAGTGTTGT[CG]TGAGCATGCTAGGCAGAGTGCAAGAAAGGAGCAAGAACTCACTAATGGCTAGGCCTTCCC | 1 | 39491459 | NDUFS5 |
| cg08331960 | TCGGGGTCCCTTGGCCTGGAGACCCTTTGTCCAACCCGTCGCCCACCTCAAGACCTGCCT[CG]ATGCTGCGCATACAGTAGGTATCCAATAAATGTTCCTGGGATAGAAGGCAAAGGCGCTGG | 16 | 2076597 | SLC9A3R2 |
| cg10281002 | TTGGGATGCGATAACTCAGTGCCCTCTTGCAGACTTGCATAGAAATAATTACTGGGTTGT[CG]TGGAGGGGACACGAGACAGAGGGAGTTCTCCGTAATGTGCCTTGCGGAGAGAAAGGTCCA | 12 | 114846399 | TBX5 |
| cg10523019 | CTCGCTGCTTCTCCCCTAGTCTTCGGGTCCCTTGAACGCAGGTCGCTTGTTTGCCTTACG[CG]TAGTCAGCGGCCAGTGGCTATTTATGGCAGTAAGGAATATTATCCACATTTCACATGGAG | 2 | 227700458 | RHBDD1 |
| cg13547237 | GCAGTGCATCGAGCTGGAGCAGCAGTTTGACTTCTTGAAGGACCTGGTGGCATCTGTTCC[CG]ACATGCAGGGGGACGGGGAAGACAACCACATGGATGGGGACAAGGGCGCCCGCAGGTGGG | 11 | 65687877 | C11orf68; DRAP1 |
| cg14424579 | TAAGCGATAAGGAGTTTCACACGATGTCTTTTTATTTCGCAGTTGAGTCCCAGTTTCTGC[CG]CTTTATCTTTCCCGCCTCCCGGCAGGCAGGCCGTTAACCGTCTTCCGGAAGACGCTGCTA | 2 | 27274309 | AGBL5 |
| cg16744741 | CAGCTGGATGCACTTGTTCTGGAGCTCCTCTGTGAGTTCAGCAATGGCCACAGTCTGCTT[CG]ACAGCTGCTCCCGCAGCTCCTTCAAATGGTACTCCCGCTCCTGGATCTCAGCATCCTTCC | 4 | 82126025 | PRKG2 |

(continued)

| Probe | Sequence with the CpG site marked with [ ] | Chromosome | Position | Gene |
|---|---|---|---|---|
| cg17324128 | CCCTCCCCCGCCAGCCTGGCGCATTGC GGGCCTCGGGCTCATTGCTGAGAGGGG GCACTG[CG]CCTGGCACCTCTGTTAAG CAATTTAGGGGCTACAACCTGAGCAAG ACAGATGAGCCCGGC | 10 | 45455500 | RASSF4 |
| cg19722847 | TCTGCTTACAGCTGCTTCCAAATTAAG CATATCTGGATGGTGTGACACTTTTTGT TAGTC[CG]AGAACTGTATGGGCATCGC AACTGGGCCTGTTCCAAGATAGACTTG TTGGGACCTTCAAA | 12 | 30849114 | IPO8 |
| cg19724470 | CATTCTTATGCGACTGTGTGTTCAGAAT ATAGCTCTGATGCTAGGCTGGAGGTCT GGACA[CG]GGTCCAAGTCCACCGCCAG CTGCTTGCTAGTAACATGACTTGTGTA AGTTATCCCAGCTG | 9 | 5450936 | CD274 |
| cg19761273 | GGACAAAGCCACCACCTTTCACAAAAT GAGGCCAGACCACCTGCCTCCCTCCAG TCCCTG[CG]GCCTGGAGACGGAGTCAA CATTCTTATCTGTGTTGGATCTGAATGT TCCTCCTTGCAAAG | 17 | 80232096 | CSNK1D |
| cg19945840 | GCGCGCCCTGGAGCGGGAGCAGGCGC GGCACGGGGACCTGCTGCTGCTGCCCG CGCTGCG[CG]ACGCCTACGAAAACCTC ACGGCCAAGGTGCTGGCCATGCTGGCC TGGCTGGACGAGCACG | 1 | 1168036 | SDF4;B3G ALT6 |
| cg20692569 | CGACCCGGAGCGCGGGCGCGGGGCTG CGCCGTGCCAGGCGGTGGAGATCCCCA TGTGCCG[CG]GCATCGGCTACAACCTG ACCCGCATGCCCAACCTGCTGGGCCAC ACGTCGCAGGGCGAGG | 7 | 72848481 | FZD9 |
| cg21801378 | CCACGAAGAGCTTGATGGCGTCGTGGT CCTTCATGGGTACGGCGGGACCGGGGT TTAGCC[CG]CTCATGCCGACGCCGCTG TCCGCGGTGCTGAAACCCAGGCGCGGG CCGGGGCCAGCGGGC | 15 | 72612125 | BRUNOL6 |
| cg22736354 | TGCGCCAGGGCGGCCACGCAGGCCAG GCAGACCACGTGGCCGCAGGACAGGT TGCGCGGG[CG]CCGCTGCTGCCGGTGG CCAAACTTCTCAAAGCACACCTTGCAC TCGAGCAGGCTGATCTC | 6 | 18122719 | NHLRC1 |
| cg22947000 | TAGCTATGACACATGGCTTGGAAATTA ACCTTTAACCAAACATCTTATAAGTAA CGCCAG[CG]CAGCTTCCCTTGTGAATG TAAAGAGATCCAGGGCTCTTGGAGAGG GACAAGTGAGAGCCA | 16 | 81272281 | BCMO1 |
| cg23517605 | CTCCAGTGCCGGCAGGTGGGAGGGCTG AGGTGGCACAGGCTGCTCCGCCACCTC GGACTG[CG]GCTCCTACTCGGCCACTG GCCAGAGTCCCTCCAGCCAACTGCCCC TGGTGAGACCACCGT | 6 | 3228365 | TUBB2B |

(continued)

| Probe | Sequence with the CpG site marked with [ ] | Chromosome | Position | Gene |
|---|---|---|---|---|
| cg24899750 | GGAGGAACTGGCTATCCTAAAGGTGAT TTTAAACCGGGGTAGCTAGAGCCCAAA GAAGGG[CG]AAACCAGGACTAACTGC CCCATAGCATGAGGGGCAGCGCCTGTA AAATTACATAGGATTT | 20 | 16710314 | SNRPB2 |
| cg25771195 | GATAAGCGCCTAATATACATCCCTGCC TGTCATTATTCACATTGTGGCATGCAGT CAAAG[CG]ACACTCTGAGGAAAATGTA TCGCCTTAAATACATTGATTAGAAAAT AAGAAAGCCCGAAC | 16 | 58163814 | C16orf80 |
| cg25809905 | ACTTGATTCTGGTTGGGGGCTTTGCCTA GGGGAGCCTTCCCTGACTCCTCAGGCT GGCCG[CG]TGGGCTAACACACGTAGGC ACAGCATTGAGCACACTGTTTACTCTT GGTCCGTTCACAGG | 17 | 42467728 | ITGA2B |
| cg26005082 | AGCTCTCCACCGACCGAAGGAGGAGA ATGCTATTTATTTCAGCACCAAATATC CGGACAG[CG]CCTCTCGGGAGGTCCGA GAAGAGAACCGCGATCTGTTTCAGCAC CGGGGCTCAGGACAGT | 19 | 4769660 | MIR7-3; C19orf30 |
| cg26394940 | TAAATAAATAAGGGCTTTTGTTTGTTTG CCGGCTCCTGCACATGGCTGCTGGGAC TCAAG[CG]CTCGTGTTGTCTGCGCCTCT GTGGGACTCTGGGGACGGGAGGCAGG GGAGGCCCCCGCAG | 22 | 46449461 | C22orf26;L OC150381 |
| cg26453588 | GGCTGCCCACCCGCCCACCCCGCCTGG AAGCTTTCTGATTTCTCTGTTCGCCCCG CCAGG[CG]CTGTGGGGTCCGTCTCACC AGGTCTGCACGTGAGCCCCCTGCCCCC AATCCCTCCCAGTC | 22 | 43506021 | BIK |
| cg26614073 | CTTGGGCAACGTAGGAGACCTCCGTCT CCACAAGTAAAATTAATTAGCCGGCTG TGGTGG[CG]CGCACCTGTGGTCCCAGC TACTCAGGAGGCTGAGGTAGGAGGATC ACCTGAGCCCGGGAG | 3 | 47517819 | SCAP |
| cg27015931 | TGTTTTTGTGGGAGGCCTTCTGCATGGT CCCGGGAGGTCAGGCAGCCCGGGAGG GCCTCC[CG]GAGCAGAGGCTGGAGTCA GTCCCAATGCCAACAGTTTCGAACCTT GCCCGCGGGCACTGC | 16 | 22012404 | C16orf65 |

TABLE 6: Listing of 17 CpGs Subset

| Probe | Sequence with the CpG site marked with [ ] | Chromosome | Position | Gene |
|---|---|---|---|---|
| cg00431549 | TAACTGCTGGACCTGACTGTGTTACAC AGGATGCTGCTCTGGTGCAGAAGTTTT GGCCAT[CG]TATGCTTGGGGACAGACC TGGGCAAAAGCCCACAGAGGAAGTTG CCACAAACACATGATC | 12 | 15039025 | MGP |

(continued)

| Probe | Sequence with the CpG site marked with [ ] | Chromosome | Position | Gene |
|---|---|---|---|---|
| cg01459453 | GCAAGTTTAAAAGTACTCACAAAATCTAATAGGCAATTCAACATAAAACTCCATGGCTAT[CG]CTGTTCCTCACTTTCTGAACCTTTACCTGCCTGACTTTACTCCATACCACTCCAACTCAC | 1 | 169599212 | SELP |
| cg01511567 | GTAGTTTTATTGTATCAGACTTAGTACAGGGGTGGGGTGGGGGTGTGTATTGGAATGATG[CG]TGCCCGTTTCTCTGCAAAATAGTTTCTATGTCATGGAAAGGAGTCGATGGGACAAGAAGA | 11 | 57103631 | SSRP1 |
| cg02275294 | GTTTGAATGTTGCTGAAGGACGCTGGTTTTCAAACGGTAAGGAATCTCCTGATAAAGGCA[CG]AATCTTGGTGTGCAGATAAGCCAGCGATTCTTGCTTCTGGCTAGTTCTACGTTGTTCCTG | 1 | 179262462 | SOAT1 |
| cg04528819 | GCAGCCCGGGAAGGGGCATTGGTGGCGCTTGGCAGCAGGTGTGACAGACCTCCTCCGGGG[CG]CCTGATCCGCGGCGGGGGCGGGGCCTGCCCCTAGGGCCCCTCCAGAGAACCCACCAGAGG | 7 | 130418315 | KLF14 |
| cg06117855 | TGGGGAGGGTTTCCTGGACAGAGGTCCTTTGGCTGCTGCCTTAAGACGTGCAGCCTGGGC[CG]TGGCTGTCACTGCGTTCGGACCCAGACCCGCTGCAGGCAGCAGCAGCCCCCGCCCGCGCA | 3 | 45067788 | CLEC3B |
| cg06493994 | GGAGAGCAAGTCAAGAAATACGGTGAAGGAGTCCTTCCCAAAGTTGTCTAGGTCCTTCCG[CG]CCGGTGCCTGGTCTTCGTCGTCAACACCATGGACAGCTCCCGGGAACCGACTCTGGGGCG | 6 | 25652602 | SCGN |
| cg07158339 | TACAGGGCTTAACTCATTTTATCCTTACCACAATCCTATGAAGTAGGAACTTTTATAAAA[CG]CATTTTATAAACAAGGCACAGAGAGGTTAATTAACTTGCCCTCTGGTCACACAGCTAGGA | 9 | 71650237 | FXN |
| cg07388493 | GGGAGCCAGTGTTCTTTCTCTCCTGTGACTTTGGTGAAGTCTCTCACCACTCAGTGTTGT[CG]TGAGCATGCTAGGCAGAGTGCAAGAAAGGAGCAAGAACTCACTAATGGCTAGGCCTTCCC | 1 | 39491459 | NDUFS5 |
| cg10523019 | CTCGCTGCTTCTCCCCTAGTCTTCGGGTCCCTTGAACGCAGGTCGCTTGTTTGCCTTACG[CG]TAGTCAGCGGCCAGTGGCTATTTATGGCAGTAAGGAATATTATCCACATTTCACATGGAG | 2 | 227700458 | RHBDD1 |
| cg17324128 | CCCTCCCCCGCCAGCCTGGCGCATTGCGGGCCTCGGGCTCATTGCTGAGAGGGGGCACTG[CG]CCTGGCACCTCTGTTAAGCAATTTAGGGGCTACAACCTGAGCAAGACAGATGAGCCCGGC | 10 | 45455500 | RASSF4 |

(continued)

| Probe | Sequence with the CpG site marked with [ ] | Chromosome | Position | Gene |
|---|---|---|---|---|
| cg19722847 | TCTGCTTACAGCTGCTTCCAAATTAAG CATATCTGGATGGTGTGACACTTTTTGT TAGTC[CG]AGAACTGTATGGGCATCGC AACTGGGCCTGTTCCAAGATAGACTTG TTGGGACCTTCAAA | 12 | 30849114 | IPO8 |
| cg22736354 | TGCGCCAGGGCGGCCACGCAGGCCAG GCAGACCACGTGGCCGCAGGACAGGT TGCGCGGG[CG]CCGCTGCTGCCGGTGG CCAAACTTCTCAAAGCACACCTTGCAC TCGAGCAGGCTGATCTC | 6 | 18122719 | NHLRC1 |
| cg25809905 | ACTTGATTCTGGTTGGGGGCTTTGCCTA GGGGAGCCTTCCCTGACTCCTCAGGCT GGCCG[CG]TGGGCTAACACACGTAGGC ACAGCATTGAGCACACTGTTTACTCTT GGTCCGTTCACAGG | 17 | 42467728 | ITGA2B |
| cg26394940 | TAAATAAATAAGGGCTTTTGTTTGTTTG CCGGCTCCTGCACATGGCTGCTGGGAC TCAAG[CG]CTCGTGTTGTCTGCGCCTCT GTGGGACTCTGGGGACGGGAGGCAGG GGAGGCCCCCGCAG | 22 | 46449461 | C22orf26;LOC150381 |
| cg26614073 | CTTGGGCAACGTAGGAGACCTCCGTCT CCACAAGTAAAATTAATTAGCCGGCTG TGGTGG[CG]CGCACCTGTGGTCCCAGC TACTCAGGAGGCTGAGGTAGGAGGATC ACCTGAGCCCGGGAG | 3 | 47517819 | SCAP |
| cg27015931 | TGTTTTTGTGGGAGGCCTTCTGCATGGT CCCGGGAGGTCAGGCAGCCCGGGAGG GCCTCC[CG]GAGCAGAGGCTGGAGTCA GTCCCAATGCCAACAGTTTCGAACCTT GCCCGCGGGCACTGC | 16 | 22012404 | C16orf65 |

TABLE 7: Listing of 6 CpGs Subset

| Probe | Sequence with the CpG site marked with [ ] | Chromosome | Position | Gene |
|---|---|---|---|---|
| cg01511567 | GTAGTTTTATTGTATCAGACTTAGTACA GGGGTGGGGTGGGGGTGTGTATTGGAA TGATG[CG]TGCCCGTTTCTCTGCAAAA TAGTTTCTATGTCATGGAAAGGAGTCG ATGGGACAAGAAGA | 11 | 57103631 | SSRP1 |
| cg07388493 | GGGAGCCAGTGTTCTTTCTCTCCTGTG ACTTTGGTGAAGTCTCTCACCACTCAG TGTTGT[CG]TGAGCATGCTAGGCAGAG TGCAAGAAAGGAGCAAGAACTCACTA ATGGCTAGGCCTTCCC | 1 | 39491459 | NDUFS5 |
| cg19722847 | TCTGCTTACAGCTGCTTCCAAATTAAG CATATCTGGATGGTGTGACACTTTTTGT TAGTC[CG]AGAACTGTATGGGCATCGC AACTGGGCCTGTTCCAAGATAGACTTG TTGGGACCTTCAAA | 12 | 30849114 | IPO8 |

(continued)

| Probe | Sequence with the CpG site marked with [ ] | Chromosome | Position | Gene |
|---|---|---|---|---|
| cg22736354 | TGCGCCAGGGCGGCCACGCAGGCCAG GCAGACCACGTGGCCGCAGGACAGGT TGCGCGGG[CG]CCGCTGCTGCCGGTGG CCAAACTTCTCAAAGCACACCTTGCAC TCGAGCAGGCTGATCTC | 6 | 18122719 | NHLRC1 |
| cg26394940 | TAAATAAATAAGGGCTTTTGTTTGTTTG CCGGCTCCTGCACATGGCTGCTGGGAC TCAAG[CG]CTCGTGTTGTCTGCGCCTCT GTGGGACTCTGGGGACGGGAGGCAGG GGAGGCCCCCGCAG | 22 | 46449461 | C22orf26;LOC150381 |
| cg26614073 | CTTGGGCAACGTAGGAGACCTCCGTCT CCACAAGTAAAATTAATTAGCCGGCTG TGGTGG[CG]CGCACCTGTGGTCCCAGC TACTCAGGAGGCTGAGGTAGGAGGATC ACCTGAGCCCGGGAG | 3 | 47517819 | SCAP |

Edaradd (NCBI Reference Sequence: NM_080738.3):

TTGTATGGGAACTCTGGTGAATGCGAATCATTTTTAAATTACTTTTTTTGTAAAGTGCAAAACAACAATAG
CACCCATTTGCGTCATACTTTATAGTTCGCAAAGCACATGGGAAAAATAAAGGTAATGATGGGGATCGTTG
CAATTCATAGGAAAGGAGGCACGAGGAAATGAAAATGAAAGGGAGTAATAACTACGTAACTAGTCAATCTT
CCTTAAAAAAAAAAACCCTTAAAATATACCACCATCTTCTATTTGATATAATGCAGAATGGGAATGATAAA
AACATGAATTACATTTCAGAGTTTCAAAAAGCAAACCAGCTTTATAGCAATGCTTGAGGTTGGGCTGCTAA
CAAGCTCACTCAACTAGTGTTTCCTGACGGCCAACGTCAGAATAATTCCATCTCCATGAGAAGTACAGAAA
GAACCACAAACCAAACCTCCAAATTGATTCTAAGATAAAATACCCTTAAAAAAAATTTCCCTTCCTATCCG
GGCGGCAGACCAAGAGGAAGTTTATCCTCCCACCTACAAATTCCCCAGAGAGCTTTCATCTAGAAGGTTTG
ACTCTGGCCAGACAACCAG*CG*AGCATCTTCT<u>CG</u>CAATCTGTTGCTTCTTCCATGGCAAACTCCAGAGAATT
AAGAAGCCAAACTCAACAT*CG*CCATGGGCCTCAGGACGACTAAACAGATGGGGAGAGGCACTGGCAGACCA
AGAGGAAGTTTATCCTCCCACCTACAAATTCCCCAGAGAGCTTTCATCTAGAAGGTTTGACTCTGGCCAGA
CAACCAGCGAGCATCTTCTCGCAATCTGTTGCTTCTTCCATGGCAAACTCCAGAGAATTAAGAAGCCAAAC
TCAACATCGCCATGGGCCTCAGGACGACTAAACAGATGGGGAGAGGCACTAAAGCTCCTGGTCACCAAGAG

GGTATGTAGGCATTTGCTGTCTTCCTGGATTTCTCAGAGCTGAGTTTTTAGCCAGAGGTTGCTTATTTACG
ATAATTCTTGGATATATTATACACTAAATACTATTATTATCTTTTTCGACCCGACTTTTATCTTTCTGTTC
TTATGTGTGAAGGCAGAGAAAGATTATTTAGAGCTCTTCAAAGATTCCTATTTAATTTAAAATGCCTGTCG
CCTTCCTATAATAGGCTTATGATGGATGATAGCTTTAGTTAAAATGTAGCAATCTTAAATATATT (SEQ ID NO: 355)

GREM1 NCBI REFERENCE SEQUENCE: XM_006725542.1

ATTTAAACGGGAGACGGCGCGATGCCTGGCACTCGGTGCGCCTTCCGCGGACCGGGCGAC
CCAGTGCACGGCCGCCGCGTCACTCTCGGTCCCGCTGACCCCGCGCCGAGCCCCGGCGGC
TCTGGCCGCGGCCGCACTCAGCGCCACGCGTCGAAAGCGCAGGCCCCGAGGACCCGCCGC
ACTGACAGTATGAGCCGCACAGCCTACACGGTGGGAGCCCTGCTTCTCCTCTTGGGGACC
CTGCTGCCGGCTGCTGAAGGGAAAAAGAAAGGGTCCCAAGGTGCCATCCCCCCGCCAGAC
AAGGCCCAGCACAATGACTCAGAGCAGACTCAGTCGCCCCAGCAGCCTGGCTCCAGGAAC
CGGGGGCGGGGCCAAGGGCGGGGCACTGCCATGCCCGGGGAGGAGGTGCTGGAGTCCAGC
CAAGAGGCCCTGCATGTGACGGAGCGCAAATACCTGAAGCGAGACTGGTGCAAAACCCAG
CCGCTTAAGCAGACCATCCACGAGGAAGGCTGCAACAGTCGCACCATCATCAACCGCTTC
TGTTACGGCCAGTGCAACTCTTTCTACATCCCCAGGCACATCCGGAAGGAGGAAGGTTCC
TTTCAGTCCTGCTCCTTCTGCAAGCCCAAGAAATTCACTACCATGATGGTCACACTCAAC
TGCCCTGAACTACAGCCACCTACCAAGAAGAAGAGAGTCACACGTGTGAAGCAGTGTCGT
TGCATATCCATCGATTTGGATTAAGCCAAATCCAGGTGCACCCAGCATGTCCTAGGAATG
CAGCCCCAGGAAGTCCCAGACCTAAAACAACCAGATTCTTACTTGGCTTAAACCTAGAGG
CCAGAAGAACCCCCAGCTGCCTCCTGGCAGGAGCCTGCTTGTGCGTAGTTCGTGTGCATG
AGTGTGGATGGGTGCCTGTGGGTGTTTTTAGACACCAGAGAAAACACAGTCTCTGCTAGA
GAGCACTCCCTATTTTGTAAACATATCTGCTTTAATGGGGATGTACCAGAAACCCACCTC
ACCCCGGCTCACATCTAAAGGGGCGGGGCCGTGGTCTGGTTCTGACTTTGTGTTTTTGTG
CCCTCCTGGGGACCAGAATCTCCTTTCGGAATGAATGTTCATGGAAGAGGCTCCTCTGAG
GGCAAGAGACCTGTTTTAGTGCTGCATTCGACATGGAAAAGTCCTTTTAACCTGTGCTTG
CATCCTCCTTTCCTCCTCCTCCTCACAATCCATCTCTTCTTAAGTTGATAGTGACTATGT
CAGTCTAATCTCTTGTTTGCCAAGGTTCCTAAATTAATTCACTTAACCATGATGCAAATG
TTTTTCATTTTGTGAAGACCCTCCAGACTCTGGGAGAGGCTGGTGTGGGCAAGGACAAGC
AGGATAGTGGAGTGAGAAAGGGAGGGTGGAGGGTGAGGCCAAATCAGGTCCAGCAAAAGT
CAGTAGGGACATTGCAGAAGCTTGAAAGGCCAATACCAGAACACAGGCTGATGCTTCTGA
GAAAGTCTTTTCCTAGTATTTAACAGAACCCAAGTGAACAGAGGAGAAATGAGATTGCCA

GAAAGTGATTAACTTTGGCCGTTGCAATCTGCTCAAACCTAACACCAAACTGAAAACATA
AATACTGACCACTCCTATGTTCGGACCCAAGCAAGTTAGCTAAACCAAACCAACTCCTCT
GCTTTGTCCCTCAGGTGGAAAAGAGAGGTAGTTTAGAACTCTCTGCATAGGGGTGGGAAT
TAATCAAAAACCGCAGAGGCTGAAATTCCTAATACCTTTCCTTTATCGTGGTTATAGTCA
GCTCATTTCCATTCCACTATTTCCCATAATGCTTCTGAGAGCCACTAACTTGATTGATAA
AGATCCTGCCTCTGCTGAGTGTACCTGACAGTAGTCTAAGATGAGAGAGTTTAGGGACTA
CTCTGTTTTAGCAAGAGATATTTTGGGGGTCTTTTTGTTTTAACTATTGTCAGGAGATTG
GGCTAAAGAGAAGACGACGAGAGTAAGGAAATAAAGGGAATTGCCTCTGGCTAGAGAGTA
GTTAGGTGTTAATACCTGGTAGAGATGTAAGGGATATGACCTCCCTTTCTTTATGTGCTC
ACTGAGGATCTGAGGGGACCCTGTTAGGAGAGCATAGCATCATGATGTATTAGCTGTTCA
TCTGCTACTGGTTGGATGGACATAACTATTGTAACTATTCAGTATTTACTGGTAGGCACT
GTCCTCTGATTAAACTTGGCCTACTGGCAATGGCTACTTAGGATTGATCTAAGGGCCAAA
GTGCAGGGTGGGTGAACTTTATTGTACTTTGGATTTGGTTAACCTGTTTTCTTCAAGCCT
GAGGTTTTATATACAAACTCCCTGAATACTCTTTTTGCCTTGTATCTTCTCAGCCTCCTA
GCCAAGTCCTATGTAATATGGAAAACAAACACTGCAGACTTGAGATTCAGTTGCCGATCA
AGGCTCTGGCATTCAGAGAACCCTTGCAACTCGAGAAGCTGTTTTTATTTCGTTTTTGTT
TTGATCCAGTGCTCTCCCATCTAACAACTAAACAGGAGCCATTTCAAGGCGGGAGATATT
TTAAACACCCAAAATGTTGGGTCTGATTTTCAAACTTTTAAACTCACTACTGATGATTCT
CACGCTAGGCGAATTTGTCCAAACACATAGTGTGTGTGTTTTGTATACACTGTATGACCC
CACCCCAAATCTTTGTATTGTCCACATTCTCCAACAATAAAGCACAGAGTGGATTTAATT
AAGCACACAAATGCTAAGGCAGAATTTTGAGGGTGGGAGAGAAGAAAAGGGAAAGAAGCT
GAAAATGTAAAACCACACCAGGGAGGAAAAATGACATTCAGAACCAGCAAACACTGAATT
TCTCTTGTTGTTTTAACTCTGCCACAAGAATGCAATTTCGTTAACGGAGATGACTTAAGT
TGGCAGCAGTAATCTTCTTTTAGGAGCTTGTACCACAGTCTTGCACATAAGTGCAGATTT
GGCTCAAGTAAAGAGAATTTCCTCAACACTAACTTCACTGGGATAATCAGCAGCGTAACT
ACCCTAAAAGCATATCACTAGCCAAAGAGGGAAATATCTGTTCTTCTTACTGTGCCTATA
TTAAGACTAGTACAAATGTGGTGTGTCTTCCAACTTTCATTGAAAATGCCATATCTATAC
CATATTTTATTCGAGTCACTGATGATGTAATGATATATTTTTTCATTATTATAGTAGAAT
ATTTTTATGGCAAGATATTTGTGGTCTTGATCATACCTATTAAAATAATGCCAAACACCA
AATATGAATTTTATGATGTACACTTTGTGCTTGGCATTAAAAGAAAAAAACACACATCCT
GGAAGTCTGTAAGTTGTTTTTTGTTACTGTAGGTCTTCAAAGTTAAGAGTGTAAGTGAAA
AATCTGGAGGAGAGGATAATTTCCACTGTGTGGAATGTGAATAGTTAAATGAAAAGTTAT
GGTTATTTAATGTAATTATTACTTCAAATCCTTTGGTCACTGTGATTTCAAGCATGTTTT

CTTTTTCTCCTTTATATGACTTTCTCTGAGTTGGGCAAAGAAGAAGCTGACACACCGTAT
GTTGTTAGAGTCTTTTATCTGGTCAGGGGAAACAAAATCTTGACCCAGCTGAACATGTCT
TCCTGAGTCAGTGCCTGAATCTTTATTTTTTAAATTGAATGTTCCTTAAAGGTTAACATT
TCTAAAGCAATATTAAGAAAGACTTTAAATGTTATTTTGGAAGACTTACGATGCATGTAT
ACAAACGAATAGCAGATAATGATGACTAGTTCACACATAAAGTCCTTTTAAGGAGAAAAT
CTAAAATGAAAAGTGGATAAACAGAACATTTATAAGTGATCAGTTAATGCCTAAGAGTGA
AAGTAGTTCTATTGACATTCCTCAAGATATTTAATATCAACTGCATTATGTATTATGTCT
GCTTAAATCATTTAAAAACGGCAAAGAATTATATAGACTATGAGGTACCTTGCTGTGTAG
GAGGATGAAAGGGGAGTTGATAGTCTCATAAAACTAATTTGGCTTCAAGTTTCATGAATC
TGTAACTAGAATTTAATTTTCACCCCAATAATGTTCTATATAGCCTTTGCTAAAGAGCAA
CTAATAAATTAAACCTATTCTTTC (SEQ ID NO: 356)

NHLRC NCBI Reference Sequence: NM_198586.2

GCACAGGACGCGCCATGGCGGCCGAAGCCTCGGAGAGCGGGCCAGCGCTGCATGAGCTCA
TGCGCGAGGCGGAGATCAGCCTGCTCGAGTGCAAGGTGTGCTTTGAGAAGTTTGGCCACC
GGCAGCAGCGGCGCCCGCGCAACCTGTCCTGCGGCCACGTGGTCTGCCTGGCCTGCGTGG
CCGCCCTGGCGCACCCGCGCACTCTGGCCCTCGAGTGCCCATTCTGCAGGCGAGCTTGCC
GGGGCTGCGACACCAGCGACTGCCTGCCGGTGCTGCACCTCATAGAGCTCCTGGGCTCAG
CGCTTCGCCAGTCCCCGGCCGCCCATCGCGCCGCCCCCAGCGCCCCCGGAGCCCTCACCT
GCCACCACACCTTCGGCGGCTGGGGGACCCTGGTCAACCCCACCGGACTGGCGCTTTGTC
CCAAGACGGGGCGTGTCGTGGTGGTGCACGACGGCAGGAGGCGTGTCAAGATTTTTGACT
CAGGGGGAGGATGCGCGCATCAGTTTGGAGAGAAGGGGGACGCTGCCCAAGACATTAGGT
ACCCTGTGGATGTCACCATCACCAACGACTGCCATGTGGTTGTCACTGACGCCGGCGATC
GCTCCATCAAAGTGTTTGATTTTTTTGGCCAGATCAAGCTTGTCATTGGAGGCCAATTCT
CCTTACCTTGGGGTGTGGAGACCACCCCTCAGAATGGGATTGTGGTAACTGATGCGGAGG
CAGGGTCCCTGCACCTCCTGGACGTCGACTTCGCGGAAGGGGTCCTTCGGAGAACTGAAA
GGTTGCAAGCTCATCTGTGCAATCCCCGAGGGGTGGCAGTGTCTTGGCTCACCGGGGCCA
TTGCGGTCCTGGAGCACCCCCTGGCCCTGGGGACTGGGGTTTGCAGCACCAGGGTGAAAG
TGTTTAGCTCAAGTATGCAGCTTGTCGGCCAAGTGGATACCTTTGGGCTGAGCCTCTACT
TTCCCTCCAAAATAACTGCCTCCGCTGTGACCTTTGATCACCAGGGAAATGTGATTGTTG
CAGATACATCTGGTCCAGCTATCCTTTGCTTAGGAAAACCTGAGGAGTTTCCAGTACCGA
AGCCCATGGTCACTCATGGTCTTTCGCATCCTGTGGCTCTTACCTTCACCAAGGAGAATT
CTCTTCTTGTGCTGGACACAGCATCTCATTCTATAAAAGTCTATAAAGTTGACTGGGGGT

GATGGGCTGGGGTGGGTCCCTGGAATCAGAAGCACTAGTGCTGCCATTAATGAATTGTTT
AACCCTGGATAAGTCACTTAAACTCATCTATCCAGGCAGGGATAATTAAAACCATCTGGC
AGACTTACAAAGCTTGGGACAGTTATTGGAGATTAATCTACCATTTATTGAATGCATACT
CTGTGCAAGGAAATTTGCAAATATTAGCTTATTTAATCTGTACTATCCAGTGAGGTAATT
TCTTCCCCCCCAAGATAGAGTCAAGCTCTGTCACCCAGGCTGGAGTGCAGAAGCATGATC
ACAGCTCACTACAGTTTCAACGTCCCCCGCTCAGGTGGTCCTTCCACCTCAGCCTCCCAA
GTAGCTGGGACCACAAGTGTGCATTACCACACTCAGCTAATTTTTGTATTTTGGCAGAGA
TGGGGTTTCACCATGTTGCCCAGGCTGGTCTCAAACTCCTGAGTTCAAGCAATCCACCTT
CCTCGGCCTCCCAAAGTACTAGGAGTACAGGCATAGCCACTTGCTCAGCCATAATTTTTA
TTATTAATCTCATTGTACAAGTGAGAAAACTGAGACCCAGAGAGCTTAAGTGACTTCCTC
GAGGTCATAGTTACTTACTGCCTTAGTCCCAATTTGAATTCAATTCTGATTCCAAATAAG
TTGCGCTTAAATAAGACAACAGATGTGGGAAAAATATGTGAATGTGTAGTGTTGCTATGT
GTACTGTCTTTACAAGTAGCTAATTATTTTAGCACAAAGATGTGCAAAGAAAGGAGACTT
TATGGAGAGTTCAGGAGAAAAAGGATTTTGTGGTGGCCATCACTTTCATTCAATTTGCGA
CTGCTCTGATGGCACATTAGATGAAGTTACTGTTGATCCTGAGTTACGTGAATAAGAAAA
ACAATTGAACTGCTTATTAAAAAAGTAAACATGT (SEQ ID NO: 357)

SCGN NCBI Reference Sequence: NM_006998.3

CAGCCGCTGGTTTTGCTGAGGGCTGAGGGACGGCTCAGCGACGCCACGGCCAGCAGCGCT
CGCGTCCTCCCCAGCAACAGTTACTCAAAGCTAATCAGATAGCGAAAGAAGCAGGAGAGC
AAGTCAAGAAATACGGTGAAGGAGTCCTTCCCAAAGTTGTCTAGGTCCTTCCGCGCCGGT
GCCTGGTCTTCGTCGTCAACACCATGGACAGCTCCCGGGAACCGACTCTGGGGCGCTTGG
ACGCCGCTGGCTTCTGGCAGGTCTGGCAGCGCTTTGATGCGGATGAAAAAGGTTACATAG
AAGAGAAGGAACTCGATGCTTTCTTTCTCCACATGTTGATGAAACTGGGTACTGATGACA
CGGTCATGAAAGCAAATTTGCACAAGGTGAAACAGCAGTTTATGACTACCCAAGATGCCT
CTAAAGATGGTCGCATTCGGATGAAAGAGCTTGCTGGTATGTTCTTATCTGAGGATGAAA
ACTTTCTTCTGCTCTTTCGCCGGGAAAACCCACTGGACAGCAGCGTGGAGTTTATGCAGA
TTTGGCGCAAATATGACGCTGACAGCAGTGGCTTTATATCAGCTGCTGAGCTCCGCAACT
TCCTCCGAGACCTCTTTCTTCACCACAAAAAGGCCATTTCTGAGGCTAAACTGGAAGAAT
ACACTGGCACCATGATGAAGATTTTTGACAGAAATAAAGATGGTCGGTTGGATCTAAATG
ACTTAGCAAGGATTCTGGCTCTTCAGGAAAACTTCCTTCTCCAATTTAAAATGGATGCTT
GTTCTACTGAAGAAAGGAAAAGGGACTTTGAGAAAATCTTTGCCTACTATGATGTTAGTA
AAACAGGAGCCCTGGAAGGCCCAGAAGTGGATGGGTTTGTCAAAGACATGATGGAGCTTG

TCCAGCCCAGCATCAGCGGGGTGGACCTTGATAAGTTCCGCGAGATTCTCCTGCGTCACT
GCGACGTGAACAAGGATGGAAAAATTCAGAAGTCTGAGCTGGCTTTGTGTCTTGGGCTGA
AAATCAACCCATAATCCCAGACTGCTTTGCCTTTTGCTCTTACTATGTTTCTGTGATCTT
GCTGGTAGAATTGTATCTGTGCATTGATGTTGGGAACACAGTGGGCAAACTCACAAATGG
TGTGCTATTCTTGGGCAAGAACAGGGACGCTAGGGCCTTCCTTCCACCGGCGTGATCTAT
CCCTGTCTCACTGAAAGCCCCTGTGTAGTGTCTGTGTTGTTTTCCCTTGACCCTGGGCTT
TCCTATCCTCCCAAAGACTCAGCTCCCCTGTTAGATGGCTCTGCCTGTCCTTCCCCAGTC
ACCAGGGTGGGGGGGACAGGGGCAGCTGAGTGCATTCATTTTGTGCTTTTCTTGTGGGCT
TTCTGCTTAGTCTGAAAGGTGTGTGGCATTCATGGCAATCCTGTAACTTCAACATAGATT
TTTTTGTGTGTGTGGAAATAAATCTGCAATTGGAAACAAAAAAAAAAAAAAA (SEQ ID NO: 358)

## Claims

**1.** A method for determining an age of a biological sample, the method comprising:

selectively measuring the methylation levels of a set of methylation markers in genomic DNA of the biological sample, said set of methylation markers comprising the 6 CpG methylation markers in Table 7; and
comparing the measured methylation marker levels to reference levels for the methylation markers to determine the age of the sample based on said methylation levels.

**2.** The method of claim 1, wherein:

(a) the biological sample is a solid tissue, blood, urine, fecal, saliva, epidermis, brain, kidney or liver sample that comprises genomic DNA;
(b) the biological sample is a sample comprising tissue culture cells or pluripotent stem cells;
(c) determining the age of the biological sample comprises applying a statistical prediction algorithm to the measured methylation marker levels; optionally wherein determining the age of the biological sample comprises

(a) obtaining a linear combination of the methylation marker levels, and
(b) applying a transformation to the linear combination to determine the age of the biological sample;

(d) the set of methylation markers comprises markers in each of the genes of Table 3; optionally at the CpG positions of Table 3;
(e) measuring a methylation level of a set of methylation markers comprises treatment of genomic DNA from the sample with bisulfite to convert unmethylated cytosines of CpG dinucleotides to uracil; or
(f) the age of an individual is determined based on the age of the biological sample.

**3.** A kit comprising probes for detecting methylation markers consisting of each of the CpG positions of Table 3.

**4.** The method of claim 1 further comprising reporting the age of the sample.

**5.** The method of claim 4, wherein said reporting comprises preparing a written or electronic report.

**6.** A tangible computer-readable medium comprising computer-readable code that, when executed by a computer, causes the computer to perform operations comprising:

a) receiving information corresponding to methylation levels of a set of methylation markers in a biological sample, said markers comprising the 6 CpG methylation markers in Table 7; and
b) determining the age of the biological sample by comparing the measured methylation marker levels to reference marker levels and by applying a statistical prediction algorithm to the measured methylation marker levels.

**7.** The tangible computer-readable medium of claim 6, wherein:

(a) the reference levels are stored in said tangible computer-readable medium;
(b) the receiving information comprises receiving from a tangible data storage device information corresponding to the methylation levels of the set of methylation markers in the biological sample;
(c) the tangible computer-readable medium further comprises computer-readable code that, when executed by a computer, causes the computer to perform one or more additional operations comprising: sending information corresponding to the methylation levels of the set of methylation markers in the biological sample to a tangible data storage device; or
(d) determining the age of the biological sample comprises applying a linear regression model to predict sample age based on a weighted average of the methylation marker levels plus an offset.

**8.** The method of claim 1 or the tangible computer-readable medium of claim 6, wherein the set of methylation markers consists of the 354 CpG methylation markers of Table 3.

**Patentansprüche**

**1.** Verfahren zum Bestimmen eines Alters einer biologischen Probe, wobei das Verfahren Folgendes umfasst:

selektives Messen der Methylierungsniveaus einer Reihe von Methylierungsmarkern in genomischer DNA der biologischen Probe, wobei die Reihe von Methylierungsmarkern die 6 CpG-Methylierungsmarker in Tabelle 7 umfasst; und
Vergleichen der gemessenen Methylierungsmarker-Niveaus mit Referenz-Niveaus für die Methylierungsmarker, um das Alter der Probe basierend auf den Methylierungsniveaus zu bestimmen.

**2.** Verfahren nach Anspruch 1, wobei:

(a) die biologische Probe eine Festgewebe-, Blut-, Urin-, Stuhl-, Speichel-, Epidermis-, Hirn-, Nieren- oder Leberprobe ist, die genomische DNA umfasst;
(b) die biologische Probe eine Probe ist, die Gewebekulturzellen oder pluripotente Stammzellen umfasst;
(c) Bestimmen des Alters der biologischen Probe das Anwenden eines Algorithmus zur statistischen Vorhersage auf die gemessenen Methylierungsmarker-Niveaus umfasst; wobei optional das Bestimmen des Alters der biologischen Probe Folgendes umfasst

(a) Erhalten einer Linearkombination der Methylierungsmarker-Niveaus und
(b) Anwenden einer Transformation auf die Linearkombination, um das Alter der biologischen Probe zu bestimmen;

(d) die Reihe von Methylierungsmarkern Marker in jedem der Gene aus Tabelle 3 umfasst; optional an den CpG-Positionen aus Tabelle 3;
(e) Messen eines Methylierungsniveaus einer Reihe von Methylierungsmarkern die Behandlung von genomischer DNA aus der Probe mit Bisulfit umfasst, um unmethylierte Cytosine von CpG-Dinukleotiden zu Uracil umzuwandeln; oder
(f) das Alter eines Individuums basierend auf dem Alter der biologischen Probe bestimmt wird.

**3.** Kit, umfassend Sonden zum Erfassen von Methylierungsmarkern, die jeweils aus den CpG-Positionen aus Tabelle 3 bestehen.

**4.** Verfahren nach Anspruch 1, ferner umfassen das Berichten des Alters der Probe.

**5.** Verfahren nach Anspruch 4, wobei das Berichten das Herstellen eines geschriebenen oder elektronischen Berichts umfasst.

**6.** Materieller, computerlesbarer Datenträger, umfassend einen computerlesbaren Code, der, wenn er durch einen Computer ausgeführt wird, verursacht, dass der Computer Vorgänge durchführt, die Folgendes umfassen:

a) Empfangen von Informationen, die den Methylierungsniveaus einer Reihe von Methylierungsmarkern in einer biologischen Probe entsprechen, wobei die Marker die 6 CpG-Methylierungsmarker in Tabelle 7 umfassen; und
b) Bestimmen des Alters der biologischen Probe durch Vergleichen der gemessenen Methylierungsmarker-Niveaus mit Referenz-Markerniveaus und durch Anwenden eines Algorithmus zur statistischen Vorhersage auf die gemessenen Methylierungsmarker-Niveaus.

**7.** Materieller, computerlesbarer Datenträger nach Anspruch 6, wobei:

(a) die Referenz-Niveaus auf dem materiellen, computerlesbaren Datenträger gespeichert werden;
(b) das Empfangen der Informationen das Empfangen von Informationen von einer materiellen Datenspeichervorrichtung umfasst, die den Methylierungsniveaus der Reihe von Methylierungsmarkern in der biologischen Probe entsprechen;
(c) der materielle, computerlesbare Datenträger ferner computerlesbaren Code umfasst, der, wenn er durch einen Computer ausgeführt wird, verursacht, dass der Computer einen oder mehrere zusätzliche Vorgänge durchführt, umfassend: Senden von Informationen, die den Methylierungsniveaus der Reihe von Metyhlierungsmarkern in der biologischen Probe entsprechen, an eine materielle Datenspeichervorrichtung; oder
(d) Bestimmen des Alters der biologischen Probe das Anwenden eines linearen Regressionsmodells umfasst, um das Probenalter basierend auf einem gewichteten Durchschnitt der Methylierungsmarker-Niveaus zuzüglich eines Versatzes vorherzusagen.

**8.** Verfahren nach Anspruch 1 oder materieller, computerlesbarer Datenträger nach Anspruch 6, wobei die Reihe von Methylierungsmarkern aus den 354 CpG-Methylierungsmarkern aus Tabelle 3 besteht.

**Revendications**

**1.** Procédé de détermination d’un âge d’un échantillon biologique, le procédé comprenant :

la mesure sélective des niveaux de méthylation d’un ensemble de marqueurs de méthylation dans l’ADN génomique de l'échantillon biologique, ledit ensemble de marqueurs de méthylation comprenant les 6 marqueurs de méthylation CpG du tableau 7 ; et
la comparaison des niveaux de marqueurs de méthylation mesurés aux niveaux de référence pour les marqueurs de méthylation afin de déterminer l'âge de l'échantillon sur la base desdits niveaux de méthylation.

**2.** Procédé selon la revendication 1, dans lequel :

(a) l'échantillon biologique est un échantillon de tissu solide, de sang, d'urine, de selles, de salive, d'épiderme, de cerveau, de rein ou de foie qui comprend de l'ADN génomique ;
(b) l'échantillon biologique est un échantillon comprenant des cellules de culture tissulaire ou des cellules souches pluripotentes ;
(c) la détermination de l'âge de l'échantillon biologique comprend l'application d'un algorithme de prédiction statistique aux niveaux de marqueurs de méthylation mesurés ; éventuellement dans lequel la détermination de l'âge de l'échantillon biologique comprend (a) l'obtention d'une combinaison linéaire des niveaux de marqueurs de méthylation, et (b) l'application d'une transformation à la combinaison linéaire pour déterminer l'âge de l'échantillon biologique ;
(d) l'ensemble de marqueurs de méthylation comprend des marqueurs dans chacun des gènes du tableau 3 ; éventuellement aux positions CpG du tableau 3 ;
(e) la mesure d'un niveau de méthylation d'un ensemble de marqueurs de méthylation comprend le traitement de l'ADN génomique de l'échantillon avec du bisulfite pour convertir les cytosines non méthylées des dinucléotides CpG en uracile ; ou
(f) l'âge d'un individu est déterminé sur la base de l'âge de l'échantillon biologique.

**3.** Kit comprenant des sondes de détection de marqueurs de méthylation constitués de chacune des positions CpG du tableau 3.

**4.** Procédé selon la revendication 1, comprenant en outre le rapport de l'âge de l'échantillon.

**5.** Procédé selon la revendication 4, dans lequel ledit rapport comprend la préparation d'un rapport écrit ou électronique.

**6.** Support lisible par ordinateur tangible comprenant un code lisible par ordinateur qui, lorsqu'il est exécuté par un ordinateur, amène l'ordinateur à effectuer des opérations comprenant :

a) la réception d'informations correspondant aux niveaux de méthylation d'un ensemble de marqueurs de méthylation dans un échantillon biologique, lesdits marqueurs comprenant les 6 marqueurs de méthylation CpG du tableau 7 ; et
b) la détermination de l'âge de l'échantillon biologique en comparant les niveaux de marqueurs de méthylation mesurés aux niveaux de marqueurs de référence et en appliquant un algorithme de prédiction statistique aux niveaux de marqueurs de méthylation mesurés.

**7.** Support tangible lisible par ordinateur selon la revendication 6, dans lequel :

(a) les niveaux de référence sont stockés dans ledit support lisible par ordinateur tangible ;
(b) la réception d'informations comprend la réception à partir d'un dispositif de stockage de données tangibles d'informations correspondant aux niveaux de méthylation de l'ensemble de marqueurs de méthylation dans l'échantillon biologique ;
(c) le support lisible par ordinateur tangible comprend en outre un code lisible par ordinateur qui, lorsqu'il est exécuté par un ordinateur, amène l'ordinateur à effectuer une ou plusieurs opérations supplémentaires comprenant : l'envoi d'informations correspondant aux niveaux de méthylation de l'ensemble de marqueurs de méthylation dans l'échantillon biologique à un dispositif de stockage de données tangibles ; ou
(d) la détermination de l'âge de l'échantillon biologique comprend l'application d'un modèle de régression linéaire pour prédire l'âge de l'échantillon sur la base d'une moyenne pondérée des niveaux de marqueurs de méthylation plus un décalage.

**8.** Procédé selon la revendication 1 ou support tangible lisible par ordinateur selon la revendication 6, dans lequel l'ensemble de marqueurs de méthylation est constitué des 354 marqueurs de méthylation CpG du tableau 3.

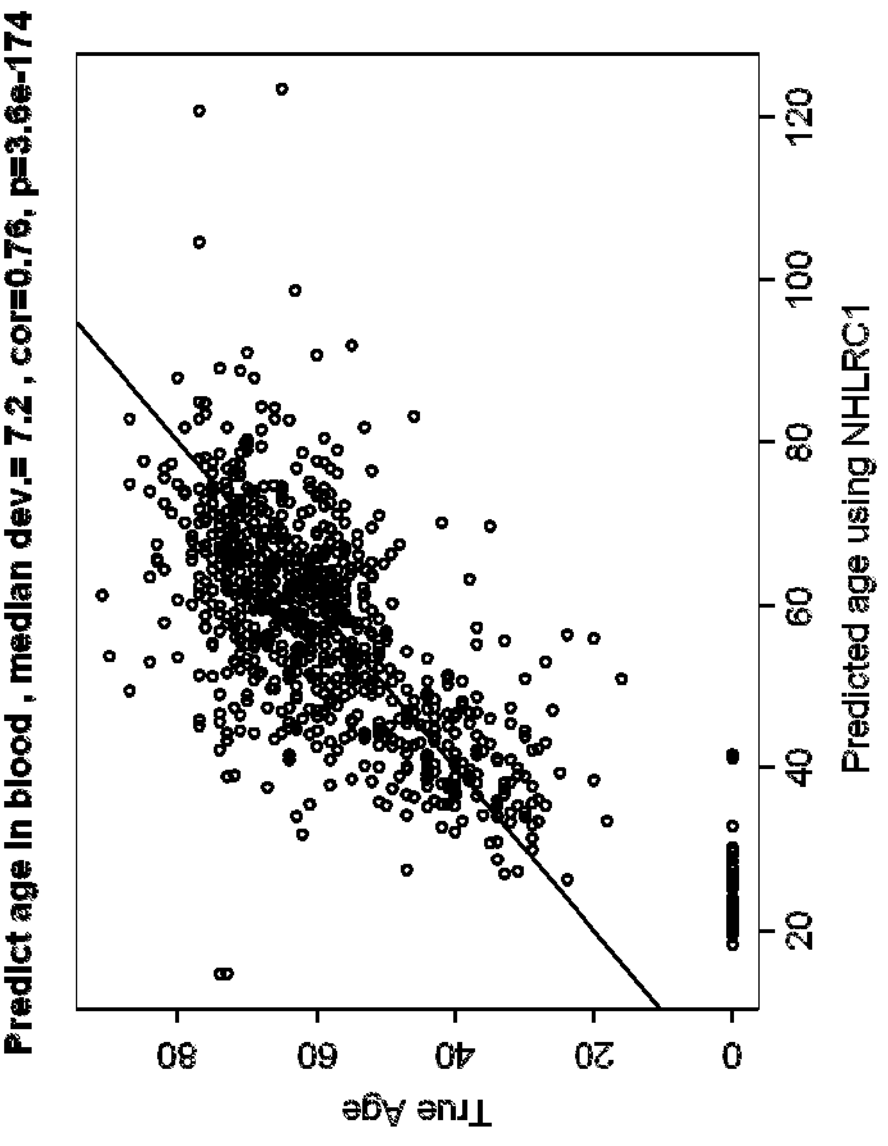

Predict age in blood , median dev.= 7.2 , cor=0.76, p=3.6e-174
True Age
0
20
40
60
80
Predicted age using NHLRC1
20
40
60
80
100
120


FIG. 1

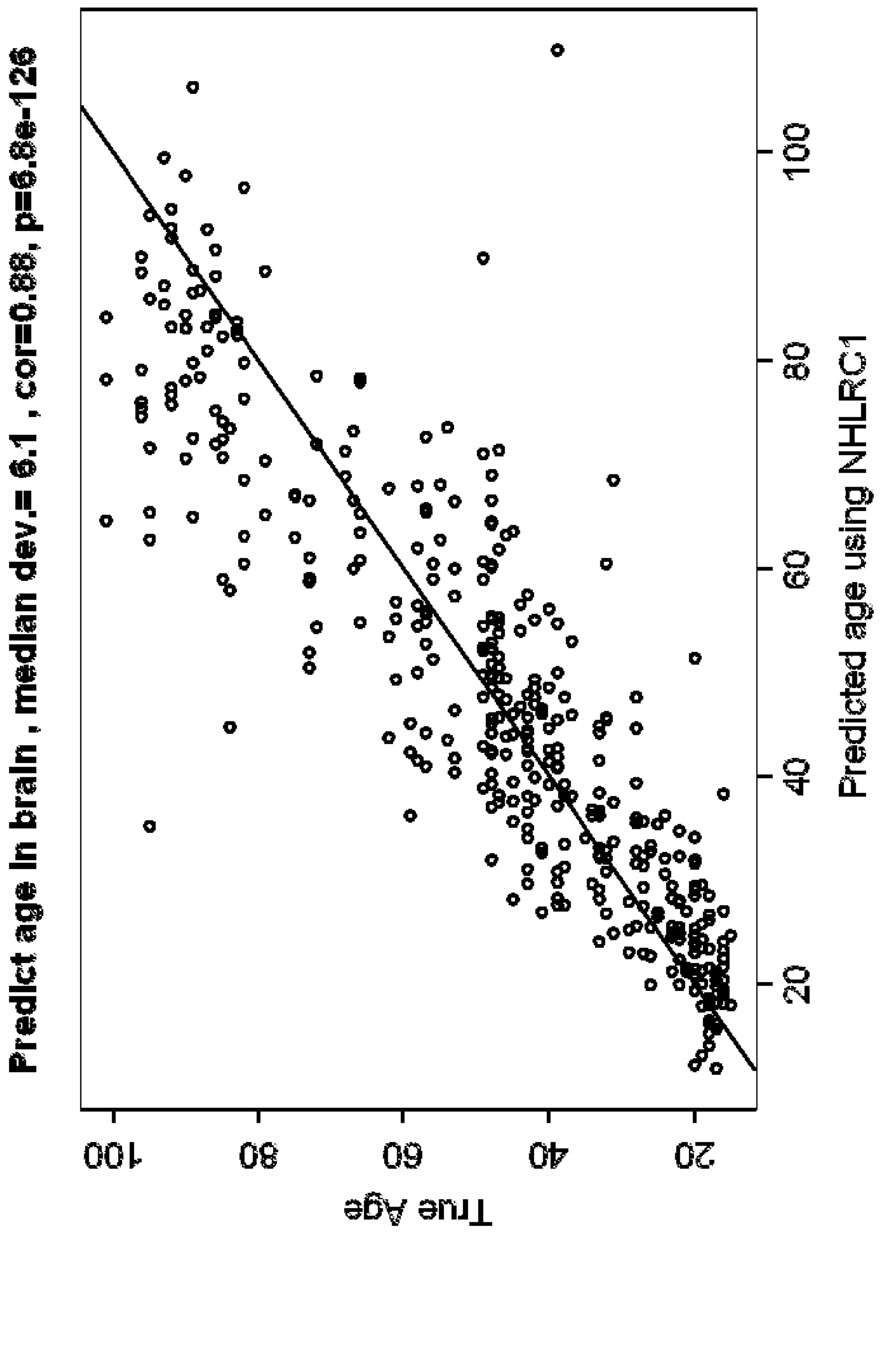
Predict age in brain , median dev.= 6.1 , cor=0.88, p=6.8e-126
Predicted age using NHLRC1
True Age
20
40
60
80
100

FIG. 2

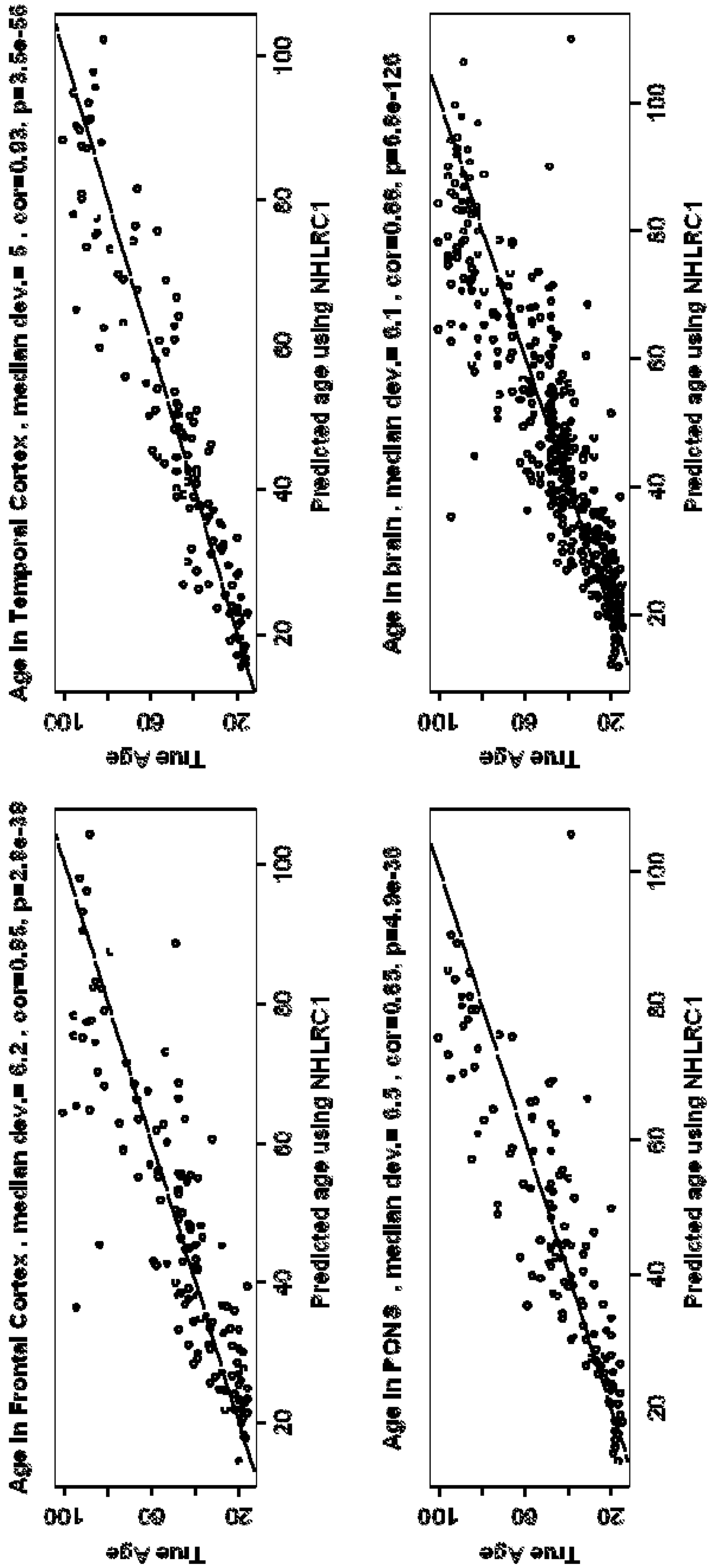
Age in Frontal Cortex , median dev.= 6.2 , cor=0.85, p=2.8e-39
Age in Temporal Cortex , median dev.= 5 , cor=0.93, p=3.5e-56
Age in PONS , median dev.= 6.5 , cor=0.85, p=4.9e-30
Age in brain , median dev.= 6.1 , cor=0.88, p=6.8e-120
True Age
Predicted age using NHLRC1

FIG. 3

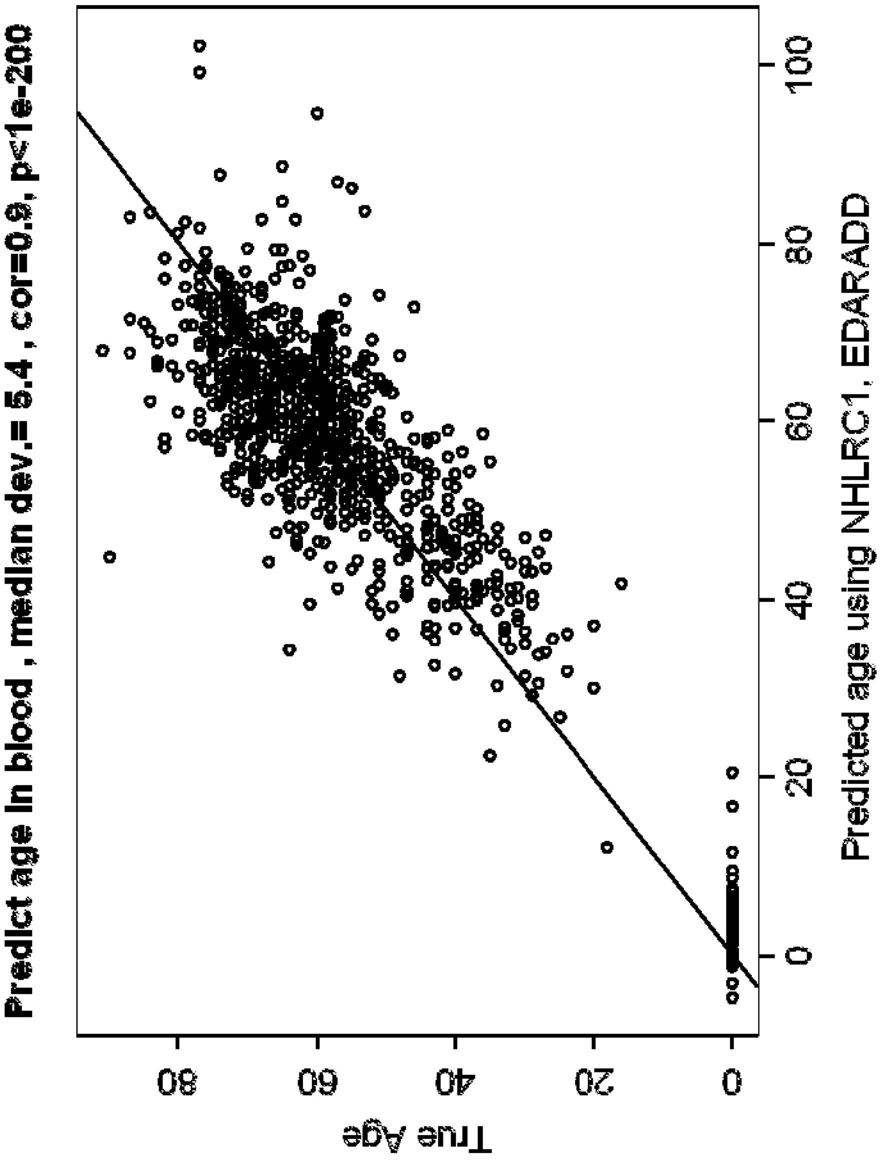
Predict age in blood , median dev.= 5.4 , cor=0.9, p<1e-200
True Age
0
20
40
60
80
100
Predicted age using NHLRC1, EDARADD

FIG. 4

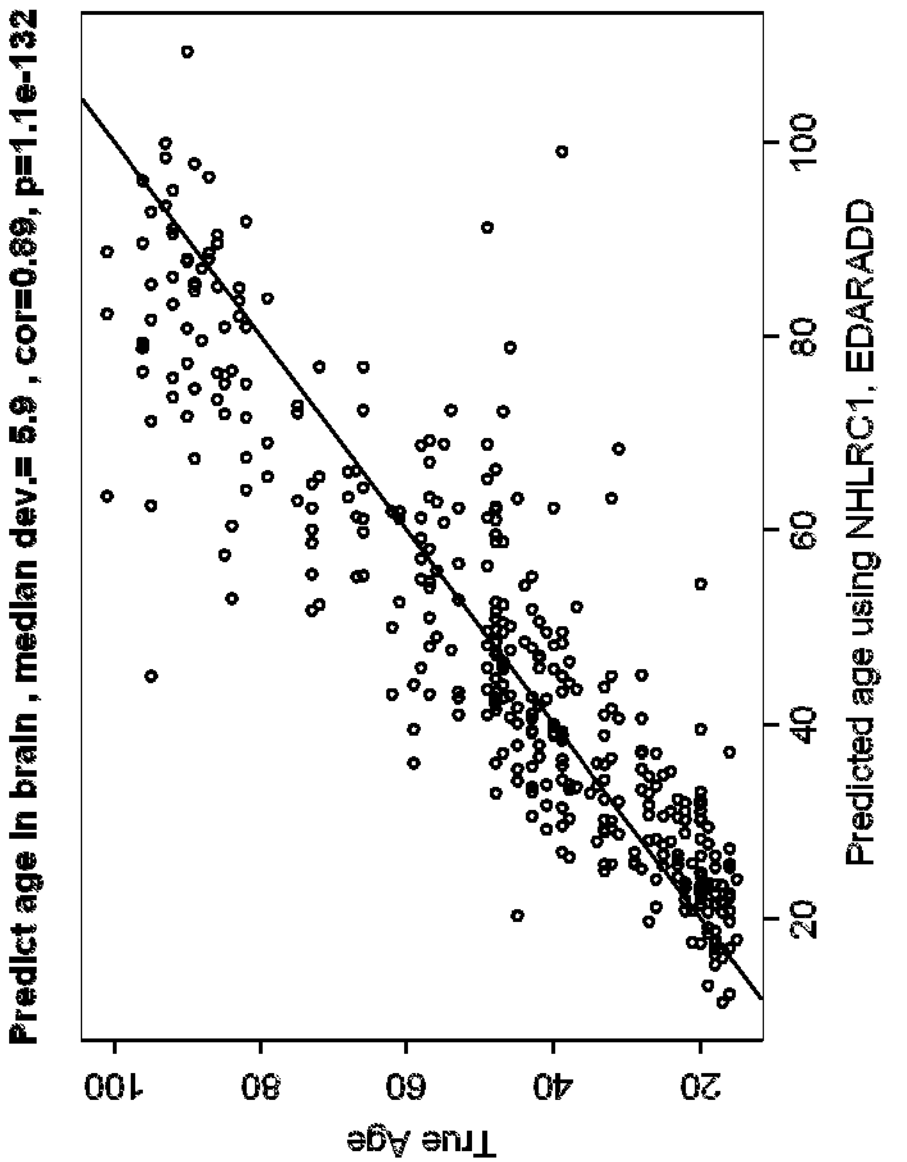
Predict age in brain , median dev.= 5.9 , cor=0.89, p=1.1e-132
True Age
100
80
60
40
20
Predicted age using NHLRC1, EDARADD
20
40
60
80
100

FIG. 5

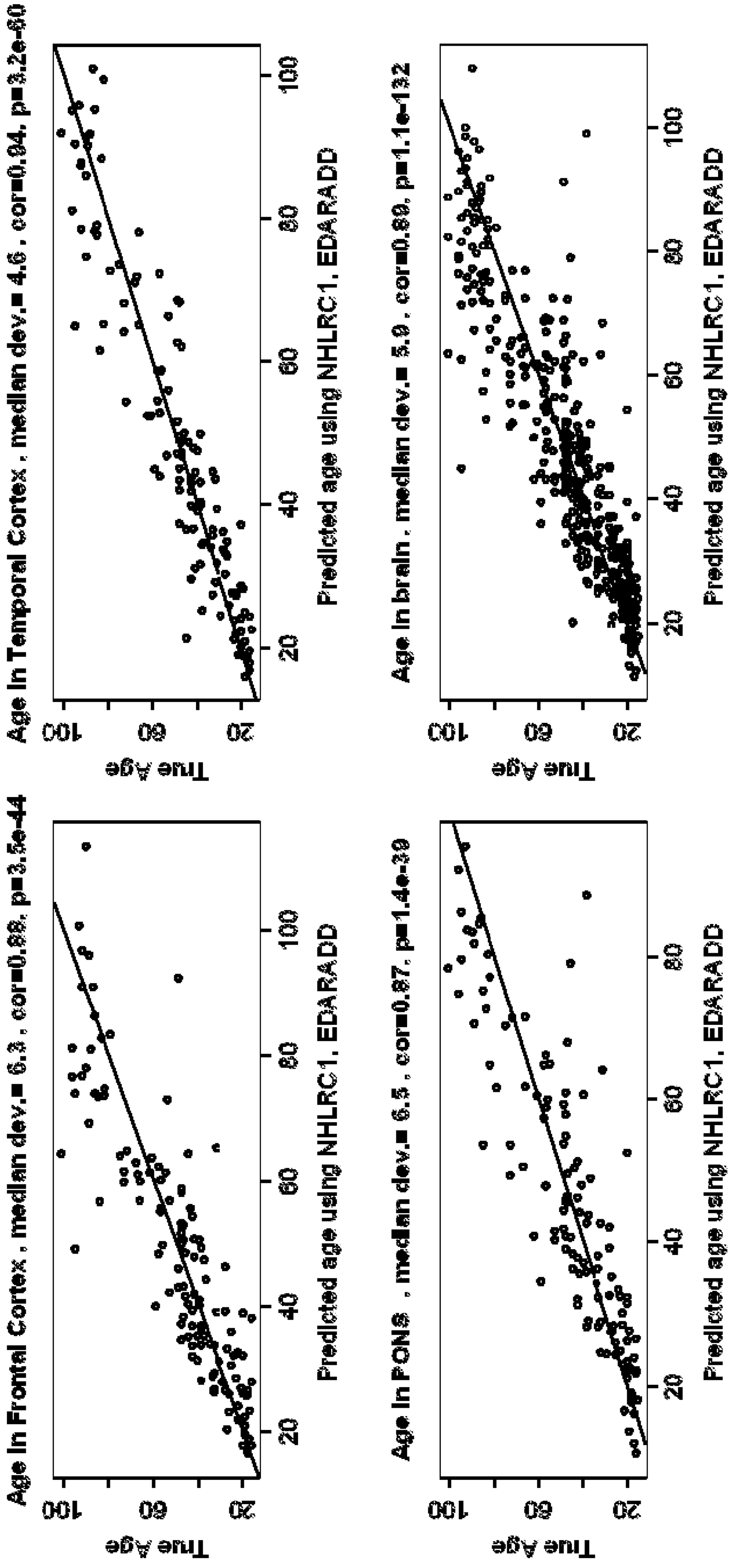
Age in Frontal Cortex , median dev.= 6.3 , cor=0.88, p=3.5e-44
True Age
Predicted age using NHLRC1, EDARADD
Age in Temporal Cortex , median dev.= 4.6 , cor=0.94, p=3.2e-60
True Age
Predicted age using NHLRC1, EDARADD
Age in PONS , median dev.= 6.5 , cor=0.87, p=1.4e-39
True Age
Predicted age using NHLRC1, EDARADD
Age in brain , median dev.= 5.9 , cor=0.89, p=1.1e-132
True Age
Predicted age using NHLRC1, EDARADD

FIG. 6

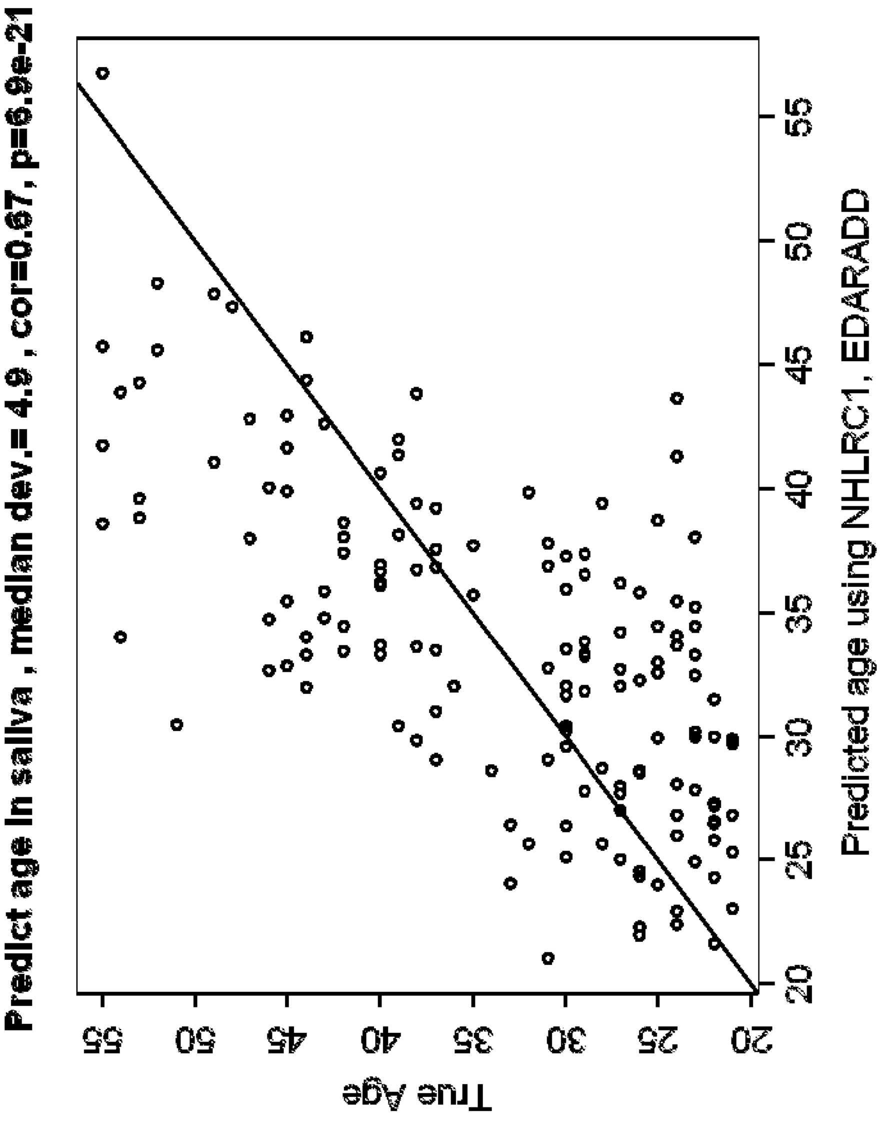
Predict age in saliva , median dev.= 4.9 , cor=0.67, p=6.9e-21
True Age
Predicted age using NHLRC1, EDARADD
20
25
30
35
40
45
50
55

FIG. 7

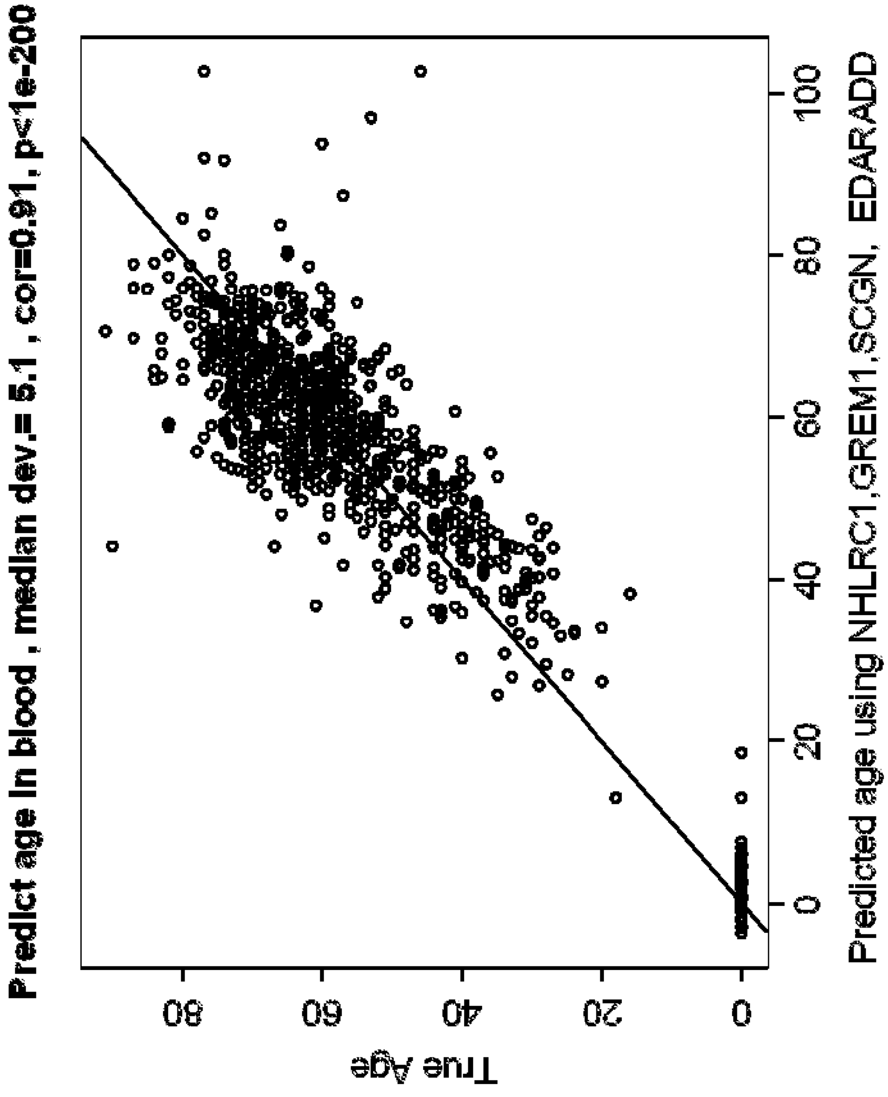
Predict age in blood , median dev.= 5.1 , cor=0.91, p<1e-200
True Age
0
20
40
60
80
100
Predicted age using NHLRC1,GREM1,SCGN, EDARADD

FIG. 8

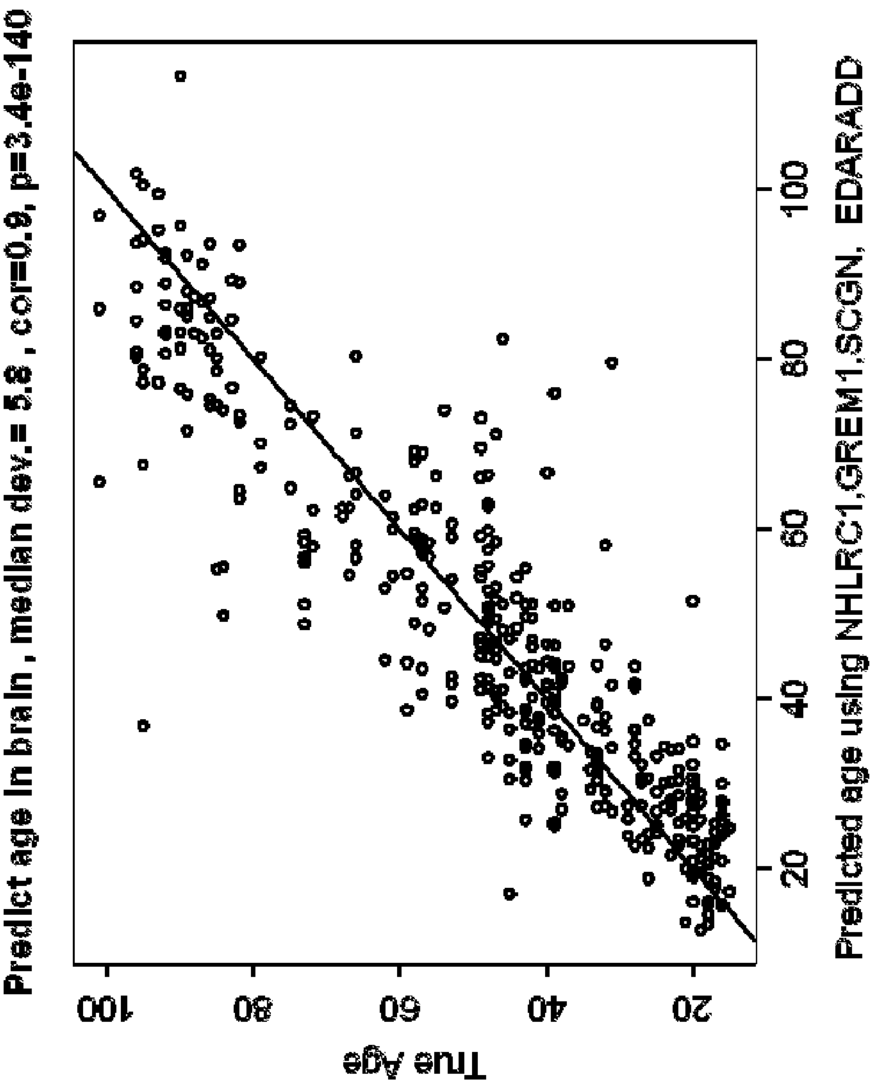
Predict age in brain , median dev.= 5.8 , cor=0.9, p=3.4e-140
True Age
20
40
60
80
100
Predicted age using NHLRC1,GREM1,SCGN, EDARADD
20
40
60
80
100

FIG. 9

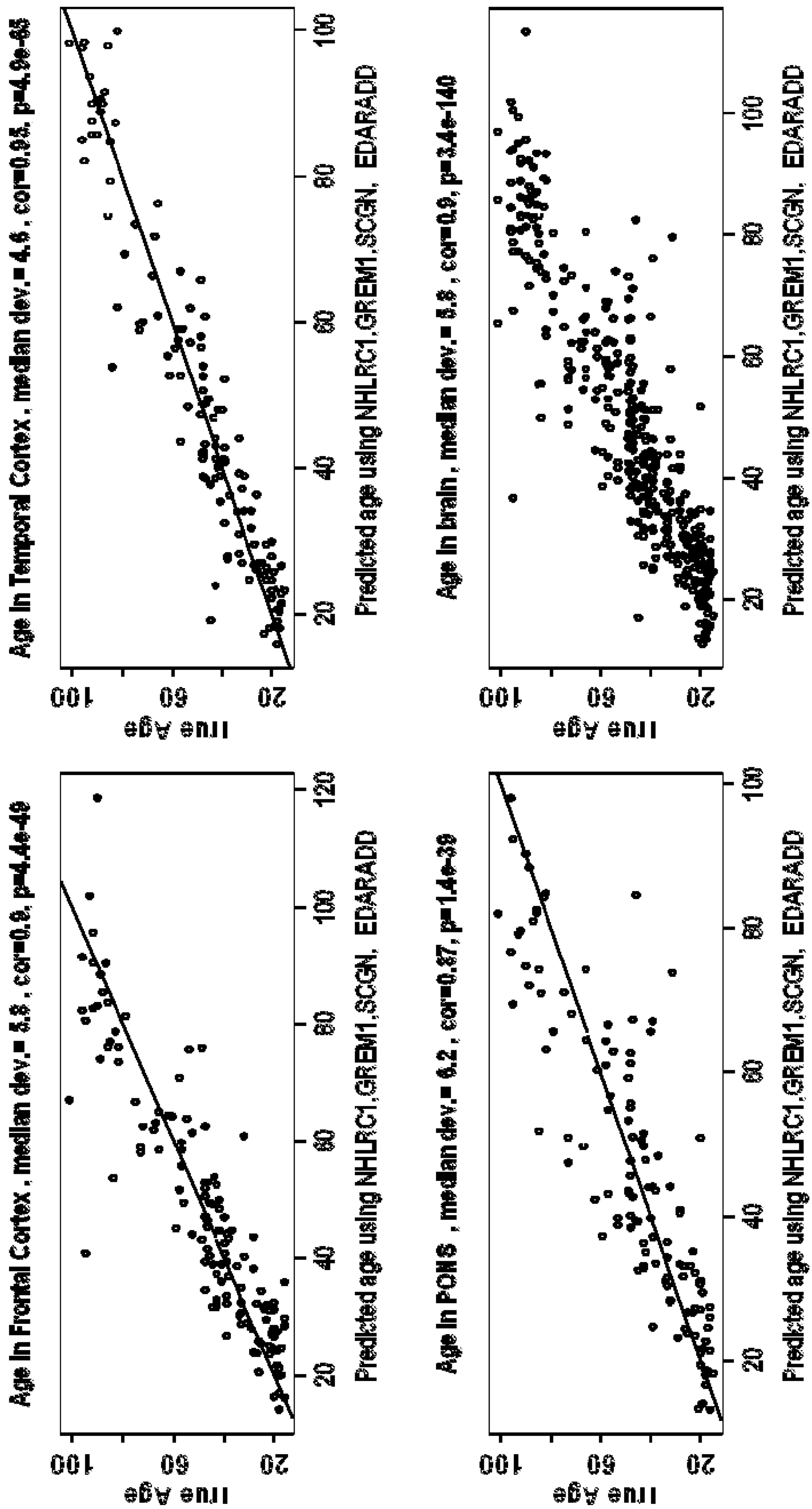
Age in Frontal Cortex , median dev.= 5.5 , cor=0.9, p=4.4e-49
True Age
Predicted age using NHLRC1,GREM1,SCGN, EDARADD
Age in Temporal Cortex , median dev.= 4.6 , cor=0.95, p=4.9e-65
True Age
Predicted age using NHLRC1,GREM1,SCGN, EDARADD
Age in PONS , median dev.= 6.2 , cor=0.87, p=1.4e-39
True Age
Predicted age using NHLRC1,GREM1,SCGN, EDARADD
Age in brain , median dev.= 5.8 , cor=0.9, p=3.4e-140
True Age
Predicted age using NHLRC1,GREM1,SCGN, EDARADD

FIG. 10

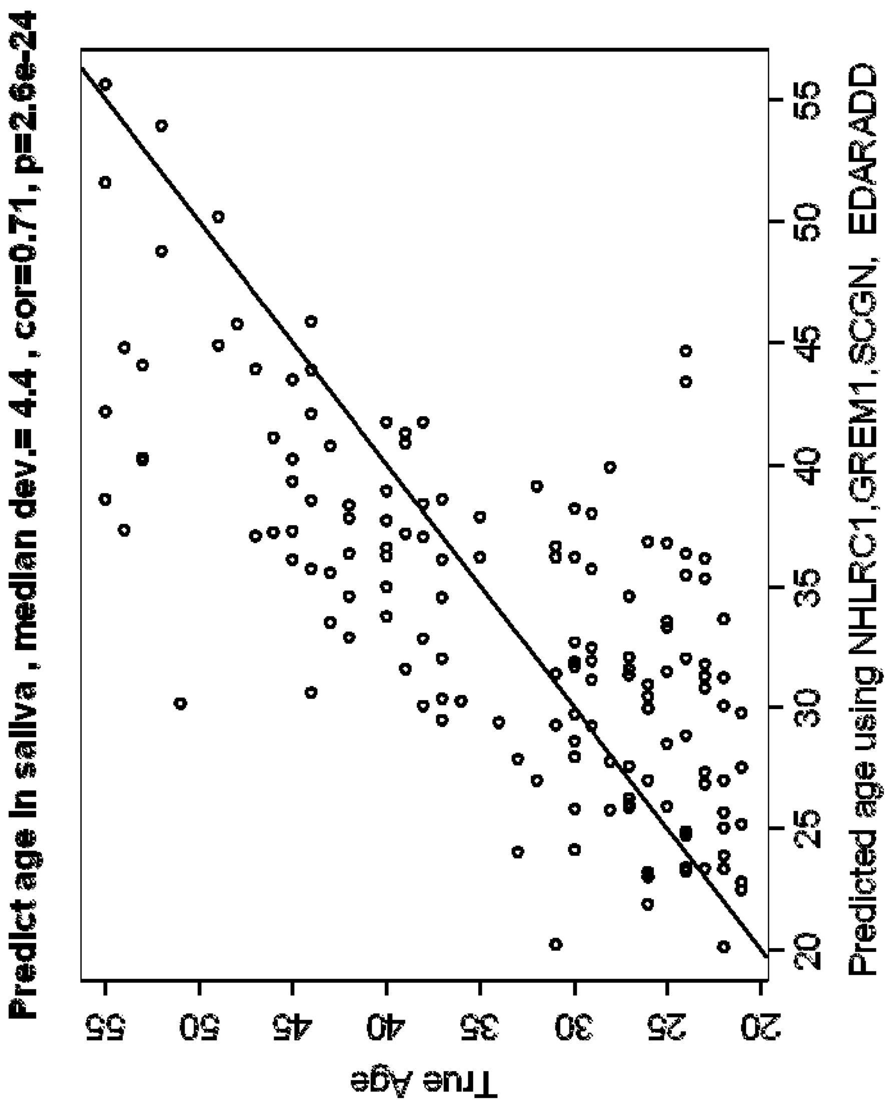
Predict age in saliva , median dev.= 4.4 , cor=0.71, p=2.6e-24
True Age
55
50
45
40
35
30
25
20
Predicted age using NHLRC1,GREM1,SCGN, EDARADD
20
25
30
35
40
45
50
55

FIG. 11

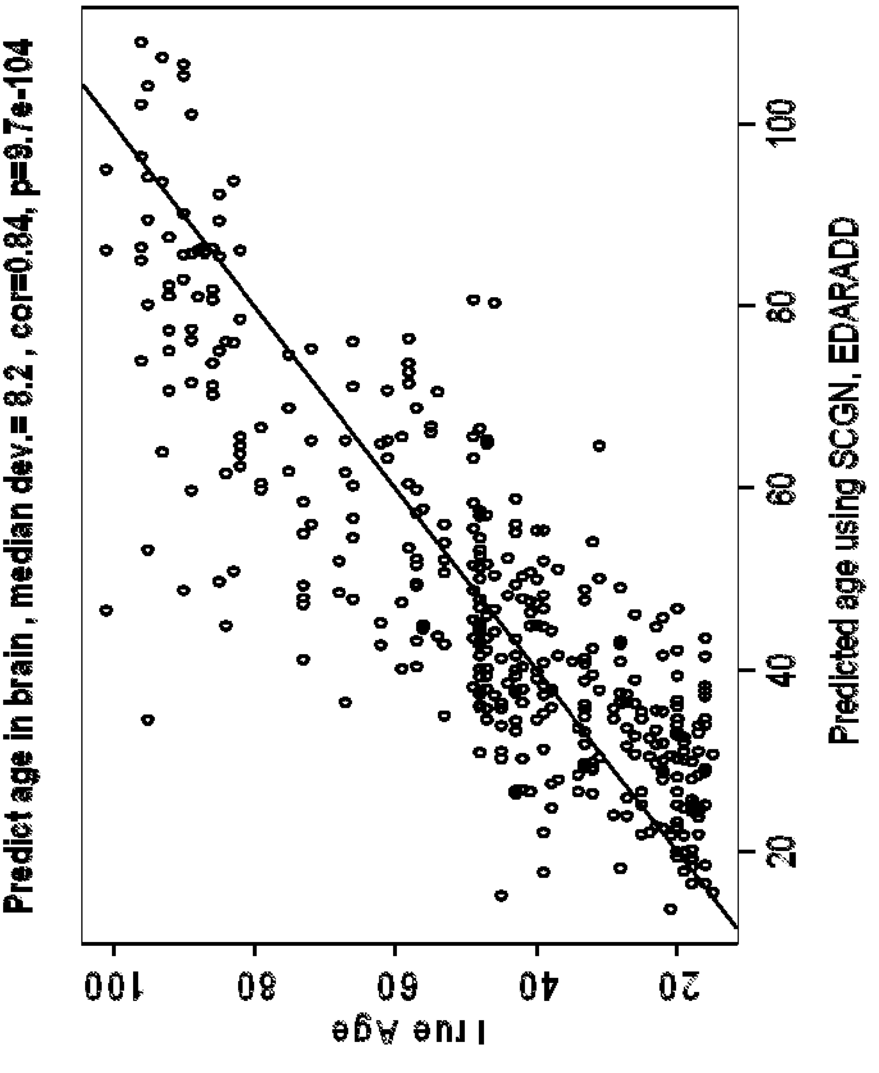
Predict age in brain , median dev.= 8.2 , cor=0.84, p=9.7e-104
True Age
Predicted age using SCGN, EDARADD
20
40
60
80
100

FIG. 12

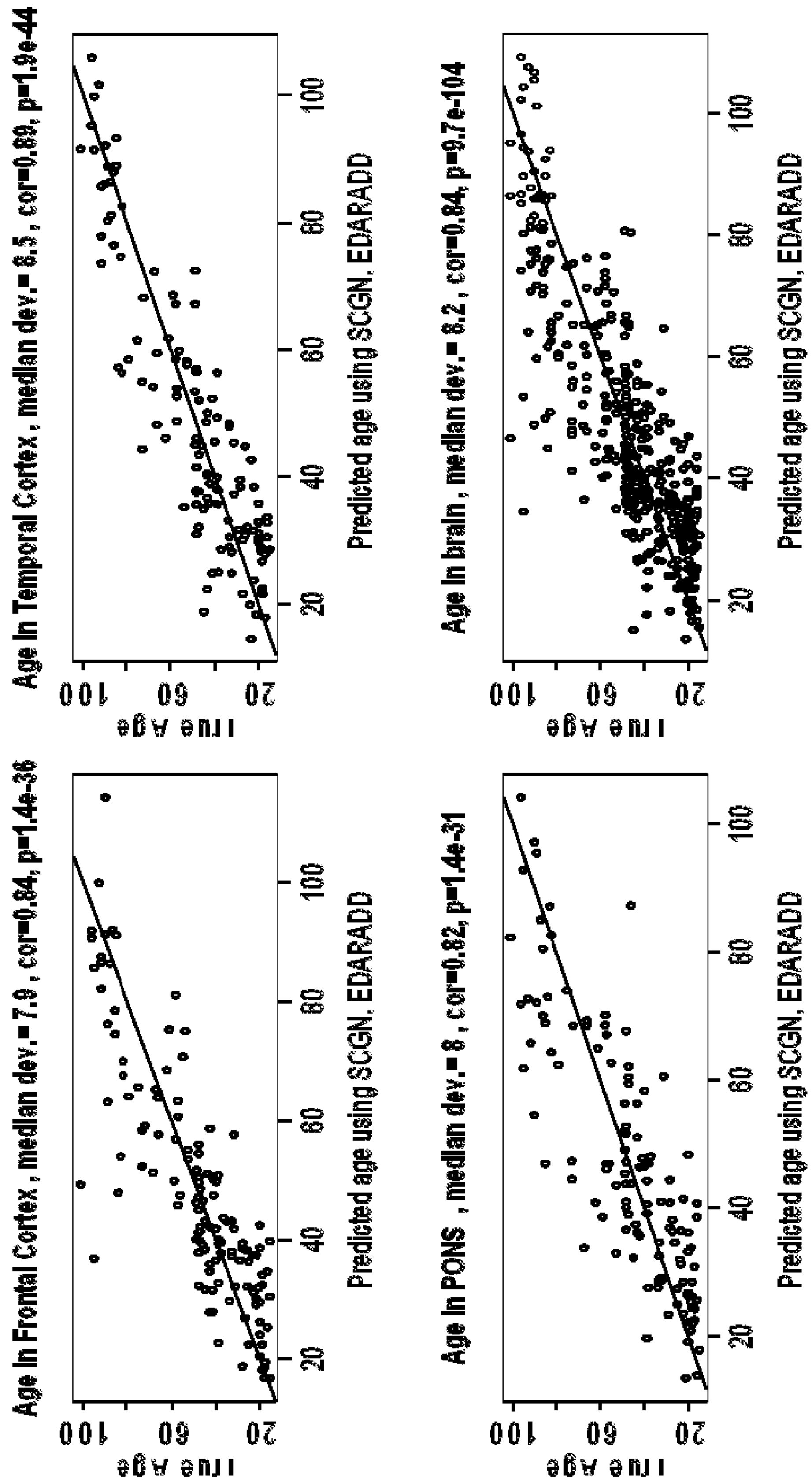
Age in Frontal Cortex , median dev.= 7.9 , cor=0.84, p=1.4e-36
Age in Temporal Cortex , median dev.= 8.5 , cor=0.89, p=1.9e-44
Age in PONS , median dev.= 6 , cor=0.82, p=1.4e-31
Age in brain , median dev.= 8.2 , cor=0.84, p=9.7e-104
True Age
Predicted age using SCGN, EDARADD

FIG. 13

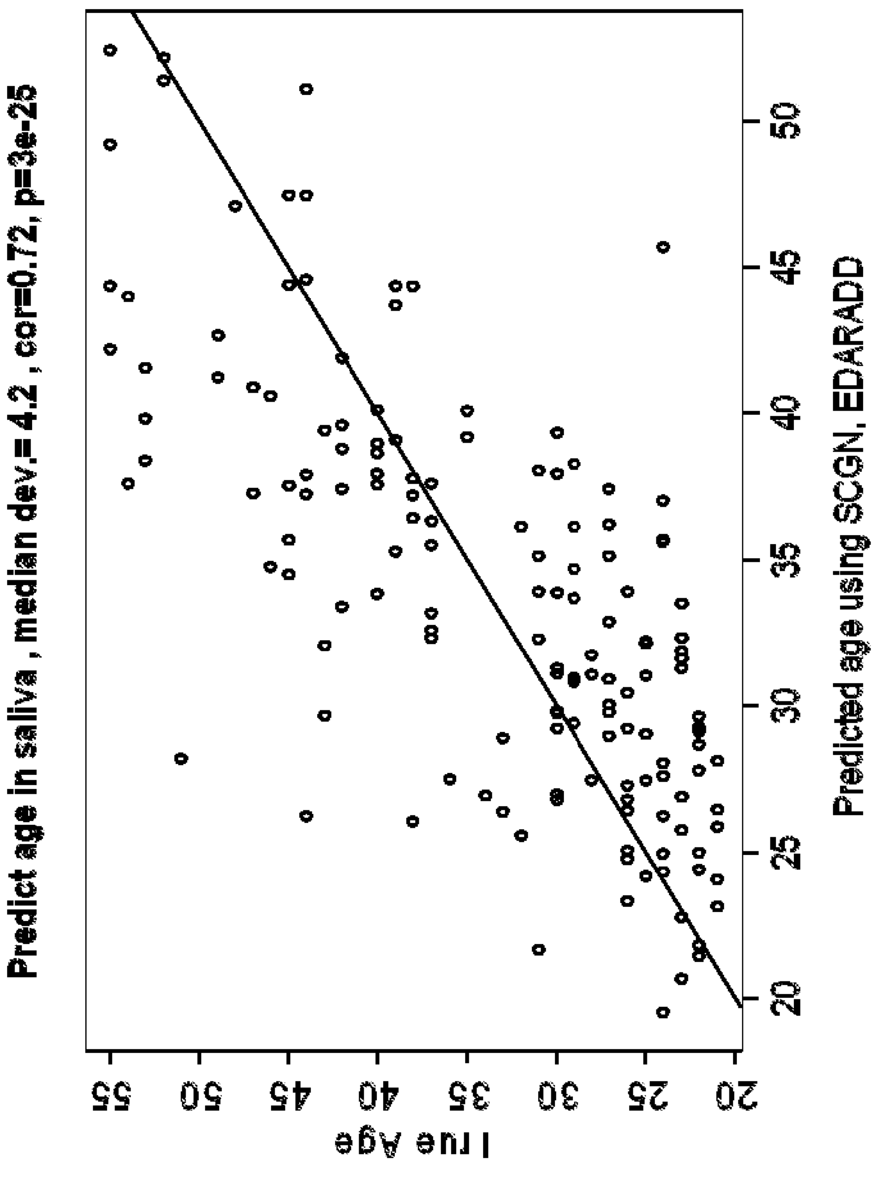
Predict age in saliva , median dev.= 4.2 , cor=0.72, p=3e-25
True Age
55
50
45
40
35
30
25
20
Predicted age using SCGN, EDARADD
20
25
30
35
40
45
50

FIG. 14

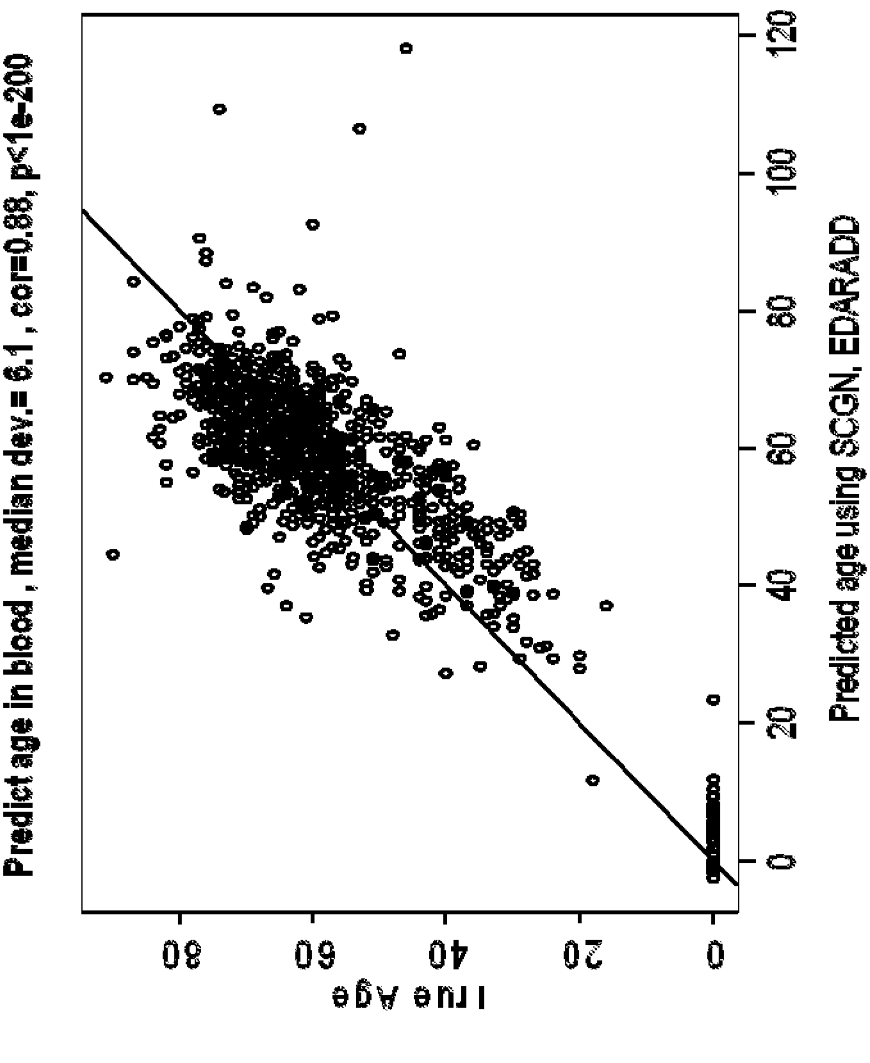
Predict age in blood , median dev.= 6.1 , cor=0.88, p<1e-200
True Age
0
20
40
60
80
Predicted age using SCGN, EDARADD
0
20
40
60
80
100
120

FIG. 15

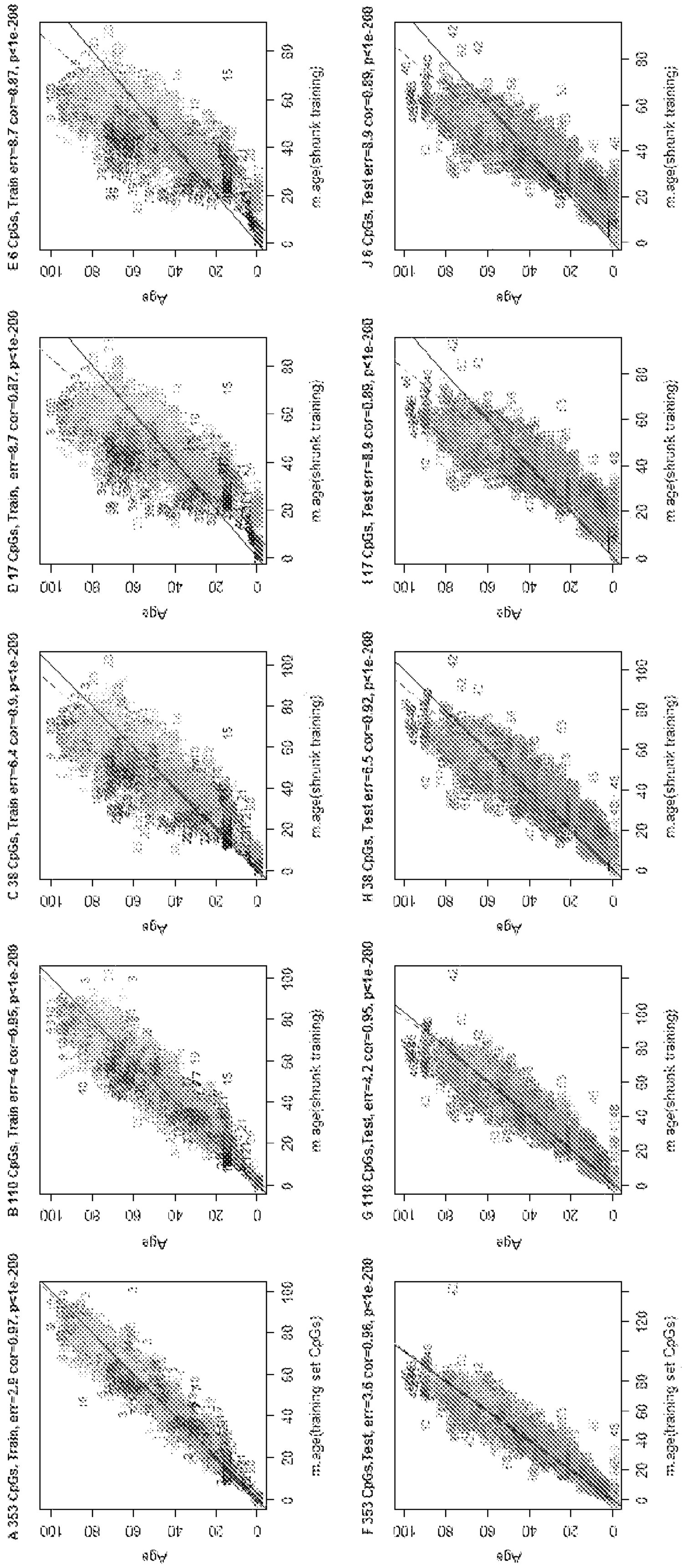

A 353 CpGs, Train, err=2.9 cor=0.97, p<1e-200
B 110 CpGs, Train err=4 cor=0.95, p<1e-200
C 38 CpGs, Train err=6.4 cor=0.9, p<1e-200
D 17 CpGs, Train, err=8.7 cor=0.87, p<1e-200
E 6 CpGs, Train err=8.7 cor=0.87, p<1e-200
F 353 CpGs,Test, err=3.6 cor=0.96, p<1e-200
G 110 CpGs,Test, err=4.2 cor=0.95, p<1e-200
H 38 CpGs, Test err=6.5 cor=0.92, p<1e-200
I 17 CpGs, Test err=8.9 cor=0.89, p<1e-200
J 6 CpGs, Test err=8.9 cor=0.89, p<1e-200
Age
m.age(training set CpGs)
m.age(shrunk training)


FIG. 16

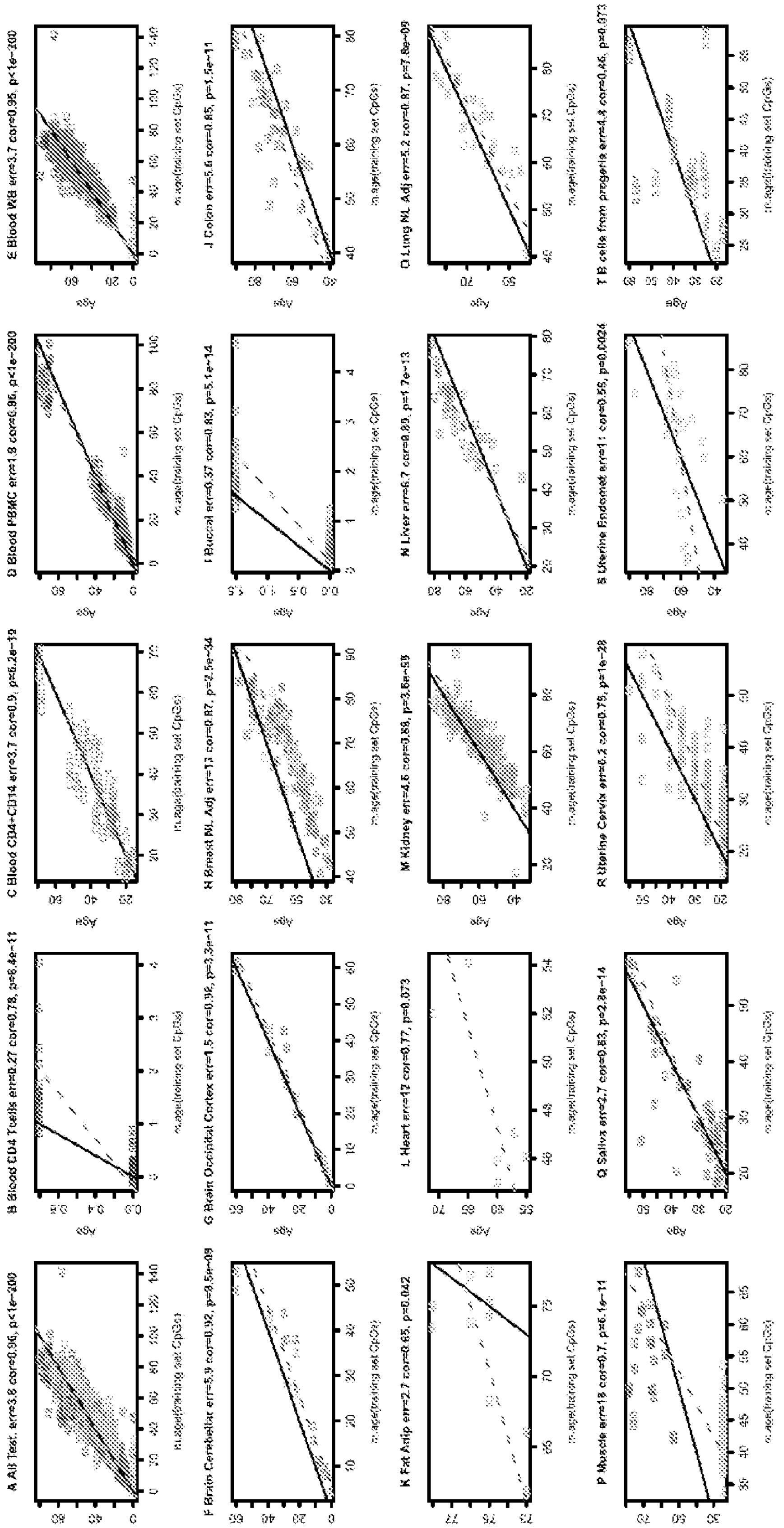
A All Test. err=3.8 cor=0.96, p<1e-200
B Blood CD4 Tcells err=0.27 cor=0.78, p=6.4e-11
C Blood CD34+CD14 err=3.7 cor=0.9, p=6.2e-19
D Blood PBMC err=1.8 cor=0.96, p<1e-200
E Blood WB err=3.7 cor=0.95, p<1e-200
F Brain Cerebellar err=5.9 cor=0.92, p=3.5e-09
G Brain Occipital Cortex err=1.5 cor=0.98, p=3.3e-11
H Breast NL Adj err=13 cor=0.87, p=2.5e-34
I Buccal err=0.37 cor=0.83, p=5.1e-14
J Colon err=5.6 cor=0.85, p=1.5e-11
K Fat Adip err=2.7 cor=0.65, p=0.042
L Heart err=12 cor=0.77, p=0.073
M Kidney err=4.6 cor=0.88, p=3.6e-55
N Liver err=6.7 cor=0.89, p=1.7e-13
O Lung NL Adj err=5.2 cor=0.87, p=7.8e-09
P Muscle err=18 cor=0.7, p=6.1e-11
Q Saliva err=2.7 cor=0.83, p=2.8e-14
R Uterine Cervix err=6.2 cor=0.75, p=1e-28
S Uterine Endomet err=11 cor=0.56, p=0.0024
T B cells from progeria err=4.4 cor=0.46, p=0.073
Age
m.age(training set CpGs)

FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6287778 B
- US 6582908 B
- US 5744305 A
- US 20140228231, Eric Vilain
- US 20060292585
- US 6214556 B
- US 5786146 A
- US 6017704 A
- US 6265171 B
- US 6200756 B
- US 6251594 B
- US 5912147 A
- US 6331393 B
- US 6605432 B
- US 6300071 B
- US 20030148327
- US 20030148326
- US 20030143606
- US 20030082609
- US 20050009059
- US 20050196792 A
- US 4683202 A
- US 4683195 A
- US 4800159 A
- US 4965188 A
- US 5333675 A
- US 6300070 B
- WO 04096825 A1
- US 7501253 B
- US 7238486 B
- US 6929914 B
- US 6251592 B
- US 5576180 A
- WO 61883875 A


### Non-patent literature cited in the description


- **BESTOR.** *Hum. Mol. Genet.,* 2000, vol. 9, 2395-2402
- *Gene Expression Technical Manual,* 2004
- *GeneChip Mapping Assay Manual,* 2004
- **BOCKLAND et al.** *PLoS ONE,* 2011, vol. 6 (6), e14821
- **KOCH et al.** *AGING,* 2011, vol. 3 (10), 1, , 018-1, 027
- **STEVE HORVATH.** DNA methylation age of human tissues and cell types. *Genome Biology,* 2013, vol. 14, R115
- **OAKELEY, E. J.** *Pharmacology & Therapeutics,* 1999, vol. 84, 389-400
- PCR Technology: Principles and Applications for DNA Amplification. Freeman Press, 1992
- PCR Protocols: A Guide to Methods and Applications. Academic Press, 1990
- **MATTILA et al.** *Nucleic Acids Res.,* 1991, vol. 19, 4967
- **ECKERT et al.** *PCR Methods and Applications,* 1991, vol. 1, 17
- PCR. IRL Press
- **OLEK et al.** *Nuc. Acids Res.,* 1994, vol. 24, 5064-6
- **SYVANEN.** *Nature Rev. Gen.,* 2001, vol. 2, 930-942
- **GIBBS JR ; VAN DER BRUG MP ; HERNANDEZ DG ; TRAYNOR BJ ; NALLS MA et al.** Abundant Quantitative Trait Loci Exist for DNA Methylation and Gene Expression in Human Brain. *PLoS Genet,* 2010, vol. 6 (5), e1000952
- **KOCH CM ; SUSCHEK CV ; LIN Q ; BORK S ; GOERGENS M ; JOUSSEN S ; PALLUA N ; HO AD ; ZENKE M ; WAGNER W.** Specific Age-Associated DNA Methylation Changes in Human Dermal Fibroblasts. *PLoS ONE,* 2011, vol. 6, e16679
- **KOCH C ; WAGNER W.** Epigenetic-aging-signature to determine age in different tissues. *Aging,* 2011, vol. 3, 1018-1027
- **BOCKLANDT S ; LIN W ; SEHL ME ; SANCHEZ FJ ; SINSHEIMER JS ; HORVATH S ; VILAIN E.** Epigenetic predictor of age. *PLoS ONE,* 2011, vol. 6, e14821
- **ESTELLER M.** Epigenetic lesions causing genetic lesions in human cancer: promoter hypermethylation of DNA repair genes. *European Journal of Cancer,* 2000, vol. 36, 2294-2300
- **USHIJIMA T.** Detection and interpretation of altered methylation patterns in cancer cells. *Nat Rev Cancer,* 2005, vol. 5, 223-231

- **SO K ; TAMURA G ; HONDA T ; HOMMA N ; WAKI T ; TOGAWA N ; NISHIZUKA S ; MOTOYAMA T.** Multiple tumor suppressor genes are increasingly methylated with age in non-neoplastic gastric epithelia. *Cancer Science,* 2006, vol. 97, 1155-1158
- **FRAGA MF ; ESTELLER M.** Epigenetics and aging: the targets and the marks. *Trends in Genetics,* 2007, vol. 23, 413-418
- **FRAGA MF ; AGRELO R ; ESTELLER M.** Cross-Talk between Aging and Cancer. *Annals of the New York Academy of Sciences,* 2007, vol. 1100, 60-74
- **BJORNSSON HT ; SIGURDSSON MI ; FALLIN MD ; IRIZARRY RA ; ASPELUND T ; CUI H ; YU W ; RONGIONE MA ; EKSTRÖM TJ ; HARRIS TB et al.** Intra-individual Change Over Time in DNA Methylation With Familial Clustering. *JAMA: The Journal of the American Medical Association,* 2008, vol. 299, 2877-2883
- **CHRISTENSEN B ; HOUSEMAN E ; MARSIT C ; ZHENG S ; WRENSCH M ; WIEMELS J ; NELSON H ; KARAGAS M ; PADBURY J ; BUENO R et al.** Aging and Environmental Exposures Alter Tissue-Specific DNA Methylation Dependent upon CpG Island Context. *PLoS Genet,* 2009, vol. 5, e1000602
- **RODRIGUEZ-RODERO S ; FERNÁNDEZ-MORERA J ; FERNANDEZ A ; MENÉNDEZ-TORRE E ; FRAGA M.** Epigenetic regulation of aging. *Discov Med,* 2010, vol. 10, 225-233
- **TESCHENDORFF AE ; MENON U ; GENTRY-MAHARAJ A ; RAMUS SJ ; WEISENBERGER DJ ; SHEN H ; CAMPAN M ; NOUSHMEHR H ; BELL CG ; MAXWELL AP et al.** Age-dependent DNA methylation of genes that are suppressed in stem cells is a hallmark of cancer. *Genome Res,* 2010, vol. 20, 440-446
- **HORVATH S ; ZHANG Y ; LANGFELDER P ; KAHN R ; BOKS M ; VAN EIJK K ; VAN DEN BERG L ; OPHOFF RA.** Aging effects on DNA methylation modules in human brain and blood tissue. *Genome Biology,* 2012, 13
- **ISSA J-PJ ; OTTAVIANO YL ; CELANO P ; HAMILTON SR ; DAVIDSON NE ; BAYLIN SB.** Methylation of the oestrogen receptor CpG island links ageing and neoplasia in human colon. *Nat Genet,* 1994, vol. 7, 536-540
- **MAEGAWA S ; HINKAL G ; KIM HS ; SHEN L ; ZHANG L ; ZHANG J ; ZHANG N ; LIANG S ; DONEHOWER LA ; ISSA J-PJ.** Widespread and tissue specific age-related DNA methylation changes in mice. *Genome Res,* 2010, vol. 20, 332-340
- **HANNUM G ; GUINNEY J ; ZHAO L ; ZHANG L ; HUGHES G ; SADDA S ; KLOTZLE B ; BIBIKOVA M ; FAN J-B ; GAO Y et al.** Genome-wide Methylation Profiles Reveal Quantitative Views of Human Aging Rates. *Molecular cell,* 2012
- **ALISCH RS ; BARWICK BG ; CHOPRA P ; MYRICK LK ; SATTEN GA ; CONNEELY KN ; WARREN ST.** Age-associated DNA methylation in pediatric populations. *Genome Res,* 2012, vol. 22, 623-632
- **HARRIS R ; NAGY-SZAKAL D ; PEDERSEN N ; OPEKUN A ; BRONSKY J ; MUNKHOLM P ; JESPERSGAARD C ; ANDERSEN P ; MELEGH B ; FERRY G et al.** Genome-wide peripheral blood leukocyte DNA methylation microarrays identified a single association with inflammatory bowel diseases. *Inflamm Bowel Dis,* 2012, vol. 18, 2334-2341
- **ADKINS RM ; KRUSHKAL J ; TYLAVSKY FA ; THOMAS F.** Racial differences in gene-specific DNA methylation levels are present at birth. *Birth Defects Research Part A: Clinical and Molecular Teratology,* 2011, vol. 91, 728-736
- **GIBBS JR ; VAN DER BRUG MP ; HERNANDEZ DG ; TRAYNOR BJ ; NALLS MA ; LAI S-L ; AREPALLI S ; DILLMAN A ; RAFFERTY IP ; TRONCOSO J et al.** Abundant Quantitative Trait Loci Exist for DNA Methylation and Gene Expression in Human Brain. *PLoS Genet,* 2010, vol. 6, e1000952
- **NUMATA S ; YE T ; HYDE THOMAS M ; GUITART-NAVARRO X ; TAO R ; WININGER M ; COLANTUONI C ; WEINBERGER DANIEL R ; KLEINMAN JOEL E ; LIPSKA BARBARA K.** DNA Methylation Signatures in Development and Aging of the Human Prefrontal Cortex. *The American Journal of Human Genetics,* 2012, vol. 90, 260-272
- **GUINTIVANO J ; ARYEE MJ ; KAMINSKY ZA.** A cell epigenotype specific model for the correction of brain cellular heterogeneity bias and its application to age, brain region and major depression. *Epigenetics,* 2013, vol. 8, 290-302
- **ZHUANG J ; JONES A ; LEE S-H ; NG E ; FIEGL H ; ZIKAN M ; CIBULA D ; SARGENT A ; SALVESEN HB ; JACOBS IJ et al.** The Dynamics and Prognostic Potential of DNA Methylation Changes at Stem Cell Gene Loci in Women's Cancer. *PLoS Genet,* 2012, vol. 8, e1002517
- **ESSEX MJ ; THOMAS BOYCE W ; HERTZMAN C ; LAM LL ; ARMSTRONG JM ; NEUMANN SMA ; KOBOR MS.** Epigenetic Vestiges of Early Developmental Adversity: Childhood Stress Exposure and DNA Methylation in Adolescence. *Child Development,* 2011, vol. 84, 58-75
- **RAKYAN VK ; DOWN TA ; MASLAU S ; ANDREW T ; YANG TP ; BEYAN H ; WHITTAKER P ; MCCANN OT ; FINER S ; VALDES AM et al.** Human aging-associated DNA hypermethylation occurs preferentially at bivalent chromatin domains. *Genome Res,* 2010, vol. 20, 434-439

- **MARTINO DJ ; TULIC MK ; GORDON L ; HODDER M ; RICHMAN T ; METCALFE J ; PRESCOTT SL ; SAFFERY R.** Evidence for age-related and individual-specific changes in DNA methylation profile of mononuclear cells during early immune development in humans. *Epigenetics: official journal of the DNA Methylation Society,* 2011, 6
- **FERNÁNDEZ-TAJES J ; SOTO-HERMIDA A ; VÁZQUEZ-MOSQUERA ME ; CORTÉS-PEREIRA E ; MOSQUERA A ; FERNÁNDEZ-MORENO M ; OREIRO N ; FERNÁNDEZ-LÓPEZ C ; FERNÁNDEZ JL ; REGO-PÉREZ I.** Genome-wide DNA methylation analysis of articular chondrocytes reveals a cluster of osteoarthritic patients. *Annals of the Rheumatic Diseases,* 2013
- **HARRIS RA ; NAGY-SZAKAL D ; KELLERMAYER R.** Human metastable epiallele candidates link to common disorders. *Epigenetics,* 2013, vol. 8, 157-163
- **GRÖNNIGER E ; WEBER B ; HEIL O ; PETERS N ; STAB F ; WENCK H ; KORN B ; WINNEFELD M ; LYKO F.** Aging and Chronic Sun Exposure Cause Distinct Epigenetic Changes in Human Skin. *PLoS Genet,* 2010, vol. 6, e1000971
- **ZOURIDIS H ; DENG N ; IVANOVA T ; ZHU Y ; WONG B ; HUANG D ; WU YH ; WU Y ; TAN IB ; LIEM N et al.** Methylation Subtypes and Large-Scale Epigenetic Alterations in Gastric Cancer. *Science Translational Medicine,* 2012, vol. 4, 156ra140
- **HAAS J ; FRESE KS ; PARK YJ ; KELLER A ; VOGEL B ; LINDROTH AM ; WEICHENHAN D ; FRANKE J ; FISCHER S ; BAUER A et al.** Alterations in cardiac DNA methylation in human dilated cardiomyopathy. *EMBO Molecular Medicine,* 2013, vol. 5, 413-429
- **SHEN J ; WANG S ; ZHANG Y-J ; KAPPIL M ; WU H-C ; KIBRIYA MG ; WANG Q ; JASMINE F ; AHSAN H ; LEE P-H et al.** Genome-wide DNA methylation profiles in hepatocellular carcinoma. *Hepatology,* 2012, vol. 55, 1799-1808
- **BORK S ; PFISTER S ; WITT H ; HORN P ; KORN, B ; HO A ; WAGNER W.** DNA methylation pattern changes upon long-term culture and aging of human mesenchymal stromal cells. *Aging Cell,* 2010, vol. 9, 54-63
- **GORDON L ; JOO JE ; POWELL JE ; OLLIKAINEN M ; NOVAKOVIC B ; LI X ; ANDRONIKOS R ; CRUICKSHANK MN ; CONNEELY KN ; SMITH AK et al.** Neonatal DNA methylation profile in human twins is specified by a complex interplay between intrauterine environmental and genetic factors, subject to tissue-specific influence. *Genome Res,* 2012, vol. 22, 1395-1406
- **KOBAYASHI Y ; ABSHER DM ; GULZAR ZG ; YOUNG SR ; MCKENNEY JK ; PEEHL DM ; BROOKS JD ; MYERS RM ; SHERLOCK G.** DNA methylation profiling reveals novel biomarkers and important roles for DNA methyltransferases in prostate cancer. *Genome Res,* 2011, vol. 21, 1017-1027
- **LIU J ; MORGAN M ; HUTCHISON K ; CALHOUN VD.** A Study of the Influence of Sex on Genome Wide Methylation. *PLoS ONE,* 2010, vol. 5, e10028
- **SONG H ; RAMUS SJ ; TYRER J ; BOLTON KL ; GENTRY-MAHARAJ A ; WOZNIAK E ; ANTON-CULVER H ; CHANG-CLAUDE J ; CRAMER DW ; DICIOCCIO R et al.** A genome-wide association study identifies a new ovarian cancer susceptibility locus on 9p22.2. *Nat Genet,* 2009, vol. 41, 996-1000
- **LIU Y ; ARYEE MJ ; PADYUKOV L ; FALLIN MD ; HESSELBERG E ; RUNARSSON A ; REINIUS L ; ACEVEDO N ; TAUB M ; RONNINGER M et al.** Epigenome-wide association data implicate DNA methylation as an intermediary of genetic risk in rheumatoid arthritis. *Nat Biotech,* 2013, vol. 31, 142-147
- **HEYN H ; LI N ; FERREIRA HJ ; MORAN S ; PISANO DG ; GOMEZ A ; DIEZ J ; SANCHEZ-MUT JV ; SETIEN F ; CARMONA FJ et al.** Distinct DNA methylomes of newborns and centenarians. *Proceedings of the National Academy of Sciences,* 2012, vol. 109, 10522-10527
- **LAM LL ; EMBERLY E ; FRASER HB ; NEUMANN SM ; CHEN E ; MILLER GE ; KOBOR MS.** Factors underlying variable DNA methylation in a human community cohort. *Proceedings of the National Academy of Sciences,* 2012, vol. 109, 17253-17260
- **KHULAN B ; COOPER WN ; SKINNER BM ; BAUER J ; OWENS S ; PRENTICE AM ; BELTEKI G ; CONSTANCIA M ; DUNGER D ; AFFARA NA.** Periconceptional maternal micronutrient supplementation is associated with widespread gender related changes in the epigenome: a study of a unique resource in the Gambia. *Human Molecular Genetics,* 2012, vol. 21, 2086-2101
- **MARTINO D ; MAKSIMOVIC J ; JOO JH ; PRESCOTT SL ; SAFFERY R.** Genome-scale profiling reveals a subset of genes regulated by DNA methylation that program somatic T-cell phenotypes in humans. *Genes Immun,* 2012, vol. 13, 388-398
- **HEYN H ; MORAN S ; ESTELLER M.** Aberrant DNA methylation profiles in the premature aging disorders Hutchinson-Gilford Progeria and Werner syndrome. *Epigenetics,* 2013, vol. 8, 28-33

- **GINSBERG MR ; RUBIN RA ; FALCONE T ; TING AH ; NATOWICZ MR.** Brain Transcriptional and Epigenetic Associations with Autism. *PLoS ONE,* 2012, vol. 7, e44736
- **MARTINO D ; LOKE Y ; GORDON L ; OLLIKAINEN M ; CRUICKSHANK M ; SAFFERY R ; CRAIG J.** Longitudinal, genome-scale analysis of DNA methylation in twins from birth to 18 months of age reveals rapid epigenetic change in early life and pair-specific effects of discordance. *Genome Biology,* 2013, vol. 14, R42
- **RIBEL-MADSEN R ; FRAGA MF ; JACOBSEN S ; BORK-JENSEN J ; LARA E ; CALVANESE V ; FERNANDEZ AF ; FRIEDRICHSEN M ; VIND BF ; HØJLUND K et al.** Genome-Wide Analysis of DNA Methylation Differences in Muscle and Fat from Monozygotic Twins Discordant for Type 2 Diabetes. *PLoS ONE,* 2012, vol. 7, e51302
- **PAI AA ; BELL JT ; MARIONI JC ; PRITCHARD JK ; GILAD Y.** A Genome-Wide Study of DNA Methylation Patterns and Gene Expression Levels in Multiple Human and Chimpanzee Tissues. *PLoS Genet,* 2011, vol. 7, e1001316
- **JACOBSEN SC ; BRØNS C ; BORK-JENSEN J ; RIBEL-MADSEN R ; YANG B ; LARA E ; HALL E ; CALVANESE V ; NILSSON E ; JORGENSEN SW et al.** Effects of short-term high-fat overfeeding on genome-wide DNA methylation in the skeletal muscle of healthy young men. *Diabetologia,* 2012, vol. 55, 3341-3349
- **BLAIR JD ; YUEN RKC ; LIM BK ; MCFADDEN DE ; VON DADELSZEN P ; ROBINSON WP.** Widespread DNA hypomethylation at gene enhancer regions in placentas associated with early-onset pre-eclampsia. *Molecular Human Reproduction,* 2013
- **TESCHENDORFF A ; JONES A ; FIEGL H ; SARGENT A ; ZHUANG J ; KITCHENER H ; WIDSCHWENDTER M.** Epigenetic variability in cells of normal cytology is associated with the risk of future morphological transformation. *Genome Medicine,* 2012, vol. 4, 24
- **HERNANDO-HERRAEZ I ; PRADO-MARTINEZ J ; GARG P ; FERNANDEZ-CALLEJO M ; HEYN H ; HVILSOM C ; NAVARRO A ; ESTELLER M ; SHARP A ; MARQUES-BONET T.** Dynamics of DNA Methylation in Recent Human and Great Apes Evolution. *PLoS Genet,* 2013
- **PACHECO SE ; HOUSEMAN EA ; CHRISTENSEN BC ; MARSIT CJ ; KELSEY KT ; SIGMAN M ; BOEKELHEIDE K.** Integrative DNA Methylation and Gene Expression Analyses Identify DNA Packaging and Epigenetic Regulatory Genes Associated with Low Motility Sperm. *PLoS ONE,* 2011, vol. 6, e20280
- **KRAUSZ C ; SANDOVAL J ; SAYOLS S ; CHIANESE C ; GIACHINI C ; HEYN H ; ESTELLER M.** Novel Insights into DNA Methylation Features in Spermatozoa: Stability and Peculiarities. *PLoS ONE,* 2012, vol. 7, e44479
- **NAZOR KRISTOPHER L ; ALTUN G ; LYNCH C ; TRAN H ; HARNESS JULIE V ; SLAVIN I ; GARITAONANDIA I ; MÜLLER F-J ; WANG Y-C ; BOSCOLO FRANCESCA S et al.** Recurrent Variations in DNA Methylation in Human Pluripotent Stem Cells and Their Differentiated Derivatives. *Cell stem cell,* 2012, vol. 10, 620-634
- **SHAO K ; KOCH C ; GUPTA MK ; LIN Q ; LENZ M ; LAUFS S ; DENECKE B ; SCHMIDT M ; LINKE M ; HENNIES HC et al.** Induced Pluripotent Mesenchymal Stromal Cell Clones Retain Donor-derived Differences in DNA Methylation Profiles. *Mol Ther,* 2012
- **CALVANESE V ; FERNÁNDEZ AF ; URDINGUIO RG ; SUÁREZ-ALVAREZ B ; MANGAS C ; PEREZ-GARCIA V ; BUENO C ; MONTES R ; RAMOS-MEJIA V ; MARTINEZ-CAMBLOR P et al.** A promoter DNA demethylation landscape of human hematopoietic differentiation. *Nucleic Acids Research,* 2012, vol. 40, 116-131
- **RAMOS-MEJIA V ; FERNANDEZ A ; AYLLON V ; REAL P ; BUENO C ; ANDERSON P ; MARTIN F ; FRAGA M ; MENENDEZ P.** Maintenance of human embryonic stem cells in mesenchymal stem cell-conditioned media augments hematopoietic specification. *Stem Cells Dev,* 2012, vol. 21, 1549-1558
- **REINIUS LE ; ACEVEDO N ; JOERINK M ; PERSHAGEN G ; DAHLÉN S-E ; SÖDERHÄLL C ; SCHEYNIUS A ; KERE J.** Differential DNA Methylation in Purified Human Blood Cells: Implications for Cell Lineage and Studies on Disease Susceptibility. *PLoS ONE,* 2012, vol. 7, e41361
- **STURM D ; WITT H ; HOVESTADT V ; KHUONG-QUANG D-A ; JONES DAVID TW ; KONERMANN C ; PFAFF E ; TÖNJES M ; SILL M ; BENDER S et al.** Hotspot Mutations in H3F3A and IDH1 Define Distinct Epigenetic and Biological Subgroups of Glioblastoma. *Cancer Cell,* 2012, vol. 22, 425-437
- **FACKLER MJ ; UMBRICHT CB ; WILLIAMS D ; ARGANI P ; CRUZ L-A ; MERINO VF ; TEO WW ; ZHANG Z ; HUANG P ; VISVANANTHAN K et al.** Genome-wide Methylation Analysis Identifies Genes Specific to Breast Cancer Hormone Receptor Status and Risk of Recurrence. *Cancer Research,* 2011, vol. 71, 6195-6207

- **DEDEURWAERDER S ; DESMEDT C ; CALONNE E ; SINGHAL SK ; HAIBE-KAINS B ; DEFRANCE M ; MICHIELS S ; VOLKMAR M ; DEPLUS R ; LUCIANI J et al.** DNA methylation profiling reveals a predominant immune component in breast cancers. *EMBO Molecular Medicine,* 2011, vol. 3, 726-741
- **HINOUE T ; WEISENBERGER DJ ; LANGE CPE ; SHEN H ; BYUN H-M ; VAN DEN BERG D ; MALIK S ; PAN F ; NOUSHMEHR H ; VAN DIJK CM et al.** Genome-scale analysis of aberrant DNA methylation in colorectal cancer. *Genome Res,* 2012, vol. 22, 271-282
- **LAUSS M ; AINE M ; SJÖDAHL G ; VEERLA S ; PATSCHAN O ; GUDJONSSON S ; CHEBIL G ; LÖVGREN K ; FERNÖ M ; MÅNSSON W et al.** DNA methylation analyses of urothelial carcinoma reveal distinct epigenetic subtypes and an association between gene copy number and methylation status. *Epigenetics,* 2012, vol. 7, 858-867
- **WEISENBERGER D ; DEN BERG D ; PAN F ; BERMAN B ; LAIRD P.** Comprehensive DNA methylation analysis on the Illumina Infinium assay platform. *Technical report Illumina, Inc, San Diego,* 2008
- **DUNNING M ; BARBOSA-MORAIS N ; LYNCH A ; TAVARE S ; RITCHIE M.** Statistical issues in the analysis of Illumina data. *BMC Bioinformatics,* 2008, vol. 9, 85
- **DEDEURWAERDER S ; DEFRANCE M ; CALONNE E ; DENIS H ; SOTIRIOU C ; FUKS F.** Evaluation of the Infinium Methylation 450K technology. *Epigenomics,* 2011, vol. 3, 771-784
- **MAKSIMOVIC J ; GORDON L ; OSHLACK A.** SWAN: Subset-quantile Within Array Normalization for Illumina Infinium HumanMethylation450 BeadChips. *Genome Biology,* 2012, vol. 13, R44
- **TESCHENDORFF AE ; MARABITA F ; LECHNER M ; BARTLETT T ; TEGNER J ; GOMEZ-CABRERO D ; BECK S.** A beta-mixture quantile normalization method for correcting probe design bias in Illumina Infinium 450 k DNA methylation data. *Bioinformatics,* 2013, vol. 29, 189-196
- **ERNST J ; KHERADPOUR P ; MIKKELSEN TS ; SHORESH N ; WARD LD ; EPSTEIN CB ; ZHANG X ; WANG L ; ISSNER R ; COYNE M et al.** Mapping and analysis of chromatin state dynamics in nine human cell types. *Nature,* 2011, vol. 473, 43-49
- **LANGFELDER P ; MISCHEL PS ; HORVATH S.** When is hub gene selection better than standard meta-analysis?. *PLoS ONE,* 2013, vol. 8, e61505
- **GÖRING H ; CURRAN J ; JOHNSON M ; DYER T ; CHARLESWORTH J ; COLE S ; JOWETT J ; ABRAHAM L ; RAINWATER D ; COMUZZIE A et al.** Discovery of expression QTLs using large-scale transcriptional profiling in human lymphocytes. *Nat Genet,* 2007, vol. 39, 1208-1216
- **PANKLA R ; BUDDHISA S ; BERRY M ; BLANKENSHIP DM ; BANCROFT GJ ; BANCHEREAU J ; LERTMEMONGKOLCHAI G ; CHAUSSABEL D.** Genomic transcriptional profiling identifies a candidate blood biomarker signature for the diagnosis of septicemic melioidosis. *Genome Biol,* 2009, vol. 10, R127
- **DUMEAUX V ; OLSEN KS ; NUEL G ; PAULSSEN RH ; B√ΠRRESEN-DALE A-L ; LUND E.** Deciphering normal blood gene expression variation--the NOWAC postgenome study. *PLoS Genet,* vol. 6, e1000873
- **CAO J-N ; GOLLAPUDI S ; SHARMAN EH ; JIA Z ; GUPTA S.** Age-related alterations of gene expression patterns in human CD8+ T cells. *Aging Cell,* 2010, vol. 9, 19-31
- **WILLINGER T ; FREEMAN T ; HASEGAWA H ; MCMICHAEL AJ ; CALLAN MFC.** Molecular Signatures Distinguish Human Central Memory from Effector Memory CD8 T Cell Subsets. *The Journal of Immunology,* 2005, vol. 175, 5895-5903
- **WILLINGER T ; FREEMAN T ; HERBERT M ; HASEGAWA H ; MCMICHAEL AJ ; CALLAN MFC.** Human Naive CD8 T Cells Down-Regulate Expression of the WNT Pathway Transcription Factors Lymphoid Enhancer Binding Factor 1 and Transcription Factor 7 (T Cell Factor-1) following Antigen Encounter In Vitro and In Vivo. *The Journal of Immunology,* 2006, vol. 176, 1439-1446
- **OLDHAM M ; LANGFELDER P ; HORVATH S.** Network methods for describing sample relationships in genomic datasets: application to Huntington's disease. *BMC Syst Biol,* 2012, vol. 6, 63
- **LU T ; PAN Y ; KAO S-Y ; LI C ; KOHANE I ; CHAN J ; YANKNER BA.** Gene regulation and DNA damage in the ageing human brain. *Nature,* 2004, vol. 429, 883-891
- **MYERS AJ ; GIBBS JR ; WEBSTER JA ; ROHRER K ; ZHAO A ; MARLOWE L ; KALEEM M ; LEUNG D ; BRYDEN L ; NATH P et al.** A survey of genetic human cortical gene expression. *Nat Genet,* 2007, vol. 39, 1494-1499
- **OLDHAM M ; KONOPKA G ; IWAMOTO K ; LANGFELDER P ; KATO T ; HORVATH S ; GESCHWIND D.** Functional organization of the transcriptome in human brain. *Nature Neuroscience,* 2008, vol. 11, 1271-1282
- **RODWELL GE ; SONU R ; ZAHN JM ; LUND J ; WILHELMY J ; WANG L ; XIAO W ; MINDRINOS M ; CRANE E ; SEGAL E et al.** A transcriptional profile of aging in the human kidney. *PLoS Biol,* 2004, vol. 2, e427

- **ZAHN J ; SONU R ; VOGEL H ; CRANE E ; MAZAN-MAMCZARZ K ; RABKIN R ; DAVIS R ; BECKER K ; OWEN A ; KIM S.** Transcriptional profiling of aging in human muscle reveals a common aging signature. *PLoS Genet,* 2006, vol. 2, e115
- **WARNER HR.** The Future of Aging Interventions. *The Journals of Gerontology Series A: Biological Sciences and Medical Sciences,* 2004, vol. 59, B692-B696
- **JOHNSON T.** Recent results: Biomarkers of aging. *Experimental Gerontology,* 2006, vol. 41, 1243-1246
- **MATHER KA ; JORM AF ; PARSLOW RA ; CHRISTENSEN H.** Is Telomere Length a Biomarker of Aging? A Review. *The Journals of Gerontology Series A: Biological Sciences and Medical Sciences,* 2011, vol. 66A, 202-213
- **BAKER G ; SPROTT R.** Biomarkers of aging. *Exp Gerontol,* 1988, vol. 23, 223-239
- **OBERDOERFFER P ; SINCLAIR DA.** The role of nuclear architecture in genomic instability and ageing. *Nat Rev Mol Cell Biol,* 2007, vol. 8, 692-702
- **CAMPISI J ; VIJG J.** Does Damage to DNA and Other Macromolecules Play a Role in Aging? If So, How?. *The Journals of Gerontology Series A: Biological Sciences and Medical Sciences,* 2009, vol. 64A, 175-178
- **BERDYSHEV G ; KOROTAEV G ; BOIARSKIKH G ; VANIUSHIN B.** Nucleotide composition of DNA and RNA from somatic tissues of humpback and its changes during spawning. *Biokhimiia,* 1967, vol. 31, 88-993
- **VANYUSHIN B ; NEMIROVSKY L ; KLIMENKO V ; VASILIEV V ; BELOZERSKY A.** The 5 mehylcytosine in DNA of rats. Tissue and age specificity and the changes induced by hydrocortisone and other agents. *Gerontologia,* 1973, vol. 19, 138-152
- **WILSON V ; SMITH R ; MA S ; CUTLER R.** Genomic 5-methyldeoxycytidine decreases with age. *J Biol Chem,* 1987, vol. 262, 9948-9951
- **BOLLATI V ; SCHWARTZ J ; WRIGHT R ; LITONJUA A ; TARANTINI L ; SUH H ; SPARROW D ; VOKONAS P.** Baccarelli A: Decline in genomic DNA methylation through aging in a cohort of elderly subjects. *Mechanisms of Ageing and Development,* 2009, vol. 130, 234-239
- **MUGATROYD C ; WU Y ; BOCKMÜHL Y ; SPENGLER D.** The Janus face of DNA methylation in aging. *AGING,* 2010, 2
- **BELL JT ; TSAI P-C ; YANG T-P ; PIDSLEY R ; NISBET J ; GLASS D ; MANGINO M ; ZHAI G ; ZHANG F ; VALDES A et al.** Epigenome-Wide Scans Identify Differentially Methylated Regions for Age and Age-Related Phenotypes in a Healthy Ageing Population. *PLoS Genet,* 2012, vol. 8, e1002629
- **BERNSTEIN BE ; STAMATOYANNOPOULOS JA ; COSTELLO JF ; REN B ; MILOSAVLJEVIC A ; MEISSNER A ; KELLIS M ; MARRA MA ; BEAUDET AL ; ECKER JR et al.** The NIH Roadmap Epigenomics Mapping Consortium. *Nat Biotech,* 2010, vol. 28, 1045-1048
- **ILLINGWORTH R ; KERR A ; DESOUSA D ; JORGENSEN H ; ELLIS P ; STALKER J ; JACKSON D ; CLEE C ; PLUMB R ; ROGERS J et al.** A Novel CpG Island Set Identifies Tissue-Specific Methylation at Developmental Gene Loci. *PLoS Biol,* 2008, vol. 6, e22
- **LI Y ; ZHU J ; TIAN G ; LI N ; LI Q ; YE M ; ZHENG H ; YU J ; WU H ; SUN J et al.** The DNA Methylome of Human Peripheral Blood Mononuclear Cells. *PLoS Biol,* 2010, vol. 8, e1000533
- **THOMPSON RF ; ATZMON G ; GHEORGHE C ; LIANG HQ ; LOWES C ; GREALLY JM ; BARZILAI N.** Tissue-specific dysregulation of DNA methylation in aging. *Aging Cell,* 2010, vol. 9, 506-518
- **HERNANDEZ DG ; NALLS MA ; GIBBS JR ; AREPALLI S ; VAN DER BRUG M ; CHONG S ; MOORE M ; LONGO DL ; COOKSON MR ; TRAYNOR BJ.** Distinct DNA methylation changes highly correlated with chronological age in the human brain. *Human Molecular Genetics,* 2011, vol. 20, 1164-1172
- **BOCKLANDT S ; LIN W ; SEHL ME ; SANCHEZ FJ ; SINSHEIMER JS ; HORVATH S ; VILAIN E.** Epigenetic predictor of age. *PLoS ONE,* 2011, vol. 6, e148215
- **LAIRD PW.** The power and the promise of DNA methylation markers. *Nat Rev Cancer,* 2003, vol. 3, 253-266
- **BELL J ; PAI A ; PICKRELL J ; GAFFNEY D ; PIQUE-REGI R ; DEGNER J ; GILAD Y ; PRITCHARD J.** DNA methylation patterns associate with genetic and gene expression variation in HapMap cell lines. *Genome Biology,* 2011, vol. 12, R10
- **FRASER H ; LAM L ; NEUMANN S ; KOBOR M.** Population-specificity of human DNA methylation. *Genome Biology,* 2012, vol. 13, R8
- **VAN EIJK K ; DE JONG S ; BOKS M ; LANGEVELD T ; COLAS F ; VELDINK J ; DE KOVEL C ; JANSON E ; STRENGMAN E ; LANGFELDER P et al.** Genetic Analysis of DNA Methylation and Gene Expression Levels in Whole Blood of Healthy Human Subjects. *BMC Genomics,* 2012, vol. 13, 636
- **JONES M ; FEJES A ; KOBOR M.** DNA methylation, genotype and gene expression: who is driving and who is along for the ride?. *Genome Biology,* 2013, vol. 14, 126
- **SHIBATA D ; TAVARÉ S.** Counting Divisions in a Human Somatic Cell Tree: How, What and Why. *Cell Cycle,* 2006, vol. 5, 610-614
- **RICHARDSON B.** Impact of aging on DNA methylation. *Ageing Research Reviews,* 2003, vol. 2, 245-261

- **KIM JY ; TAVARÉ S ; SHIBATA D.** Counting human somatic cell replications: Methylation mirrors endometrial stem cell divisions. *Proceedings of the National Academy of Sciences of the United States of America,* 2005, vol. 102, 17739-17744
- **THOMSON JA ; ITSKOVITZ-ELDOR J ; SHAPIRO SS ; WAKNITZ MA ; SWIERGIEL JJ ; MARSHALL VS ; JONES JM.** Embryonic Stem Cell Lines Derived from Human Blastocysts. *Science,* 1998, vol. 282, 1145-1147
- **SCHWARTZENTRUBER J ; KORSHUNOV A ; LIU X-Y ; JONES DTW ; PFAFF E ; JACOB K ; STURM D ; FONTEBASSO AM ; QUANG D-AK ; TONJES M et al.** Driver mutations in histone H3.3 and chromatin remodelling genes in paediatric glioblastoma. *Nature,* 2012, vol. 482, 226-231
- **BERNSTEIN BE ; MIKKELSEN TS ; XIE X ; KAMAL M ; HUEBERT DJ ; CUFF J ; FRY B ; MEISSNER A ; WERNIG M ; PLATH K et al.** A Bivalent Chromatin Structure Marks Key Developmental Genes in Embryonic Stem Cells. *Cell,* 2006, vol. 125, 315-326
- **KOLASINSKA-ZWIERZ P ; DOWN T ; LATORRE I ; LIU T ; LIU XS ; AHRINGER J.** Differential chromatin marking of introns and expressed exons by H3K36me3. *Nat Genet,* 2009, vol. 41, 376-381
- **BJERKE L ; MACKAY A ; NANDHABALAN M ; BURFORD A ; JURY A ; POPOV S ; BAX DA ; CARVALHO D ; TAYLOR KR ; VINCI M et al.** Histone H3.3 Mutations Drive Pediatric Glioblastoma through Upregulation of MYCN. *Cancer Discovery,* 2013
- **STURM D ; WITT H ; HOVESTADT V ; KHUONG-QUANG D-A ; JONES DAVID TW ; KONERMANN C ; PFAFF E ; TÖNJES M ; SILL M ; BENDER S et al.** Hotspot Mutations in H3F3A and IDH1 Define Distinct Epigenetic and Biological Subgroups of Glioblastoma. *Cancer Cell,* 2012, vol. 22, 425-437, http://labs.genetics.ucla.edu/horvath/dnamage
- **FRIEDMAN J ; HASTIE T ; TIBSHIRANI R.** Regularization Paths for Generalized Linear Models via Coordinate Descent. *Journal of Statistical Software,* 2010, vol. 33, 1-22
- **HEYN H ; LI N ; FERREIRA HJ ; MORAN S ; PISANO DG ; GOMEZ A ; DIEZ J ; SANCHEZ-MUT JV ; SETIEN F ; CARMONA FJ et al.** Distinct DNA methylomes of newborns and centenarians. *Proceedings of the National Academy of Sciences,* 2012, vol. 109, 10211-10527
- **GINSBERG MR ; RUBIN RA ; FALCONE T ; TING AH ; NATOWICZ MR.** Brain Transcriptional and Epigenetic Associations with Autism. *PLoS ONE,* 2012
- **TESCHENDORFF A ; JONES A ; FIEGL H ; SARGENT A ; ZHUANG J ; KITCHENER H ; WIDSCHWENDTER M.** Epigenetic variability in cells of normal cytology is associated with the risk of future morphological transformation. *Genome Medicine,* 2012
- **REINIUS LE ; ACEVEDO N ; JOERINK M ; PERSHAGEN G ; DAHLÉN S-E ; GRECO D ; SÖDERHÄLL C ; SCHEYNIUS A ; KERE J.** Differential DNA Methylation in Purified Human Blood Cells: Implications for Cell Lineage and Studies on Disease Susceptibility. *PLoS ONE,* 2012, vol. 7, e41361
- **LEE TI ; JENNER RG ; BOYER LA ; GUENTHER MG ; LEVINE SS ; KUMAR RM ; CHEVALIER B ; JOHNSTONE SE ; COLE MF ; ISONO K-I et al.** Control of Developmental Regulators by Polycomb in Human Embryonic Stem Cells. *Cell,* 2006, vol. 125, 301-313
- **MILLER JA ; CAI C ; LANGFELDER P ; GESCHWIND DH ; KURIAN SM ; SALOMON DR ; HORVATH S.** Strategies for aggregating gene expression data: The collapseRows R function. *BMC Bioinformatics,* 2011, vol. 12, 322
- **TESCHENDORFF AE ; MENON U ; GENTRY-MAHARAJ A ; RAMUS SJ et al.** Age-dependent DNA methylation of genes that are suppressed in stem cells is a hallmark of cancer. *Genome Res,* April 2010, vol. 20 (4), 440-6
- **RAKYAN VK ; DOWN TA ; MASLAU S ; ANDREW T et al.** Human aging-associated DNA hypermethylation occurs preferentially at bivalent chromatin domains. *Genome Res,* April 2010, vol. 20 (4), 434-9
- **GIBBS JR ; VAN DER BRUG MP ; HERNANDEZ DG ; TRAYNOR BJ ; NALLS MA ; LAI SL ; AREPALLI S ; DILLMAN A ; RAFFERTY IP ; TRONCOSO J.** Abundant quantitative trait loci exist for DNA methylation and gene expression in human brain. *PLoS Genet,* 13 May 2010, vol. 6 (5), e1000952
- **BOCKLANDT S ; LIN W ; SEHL ME ; SANCHEZ FJ ; SINSHEIMER JS et al.** Epigenetic Predictor of Age. *PLoS ONE,* 2011, vol. 6 (6), e14821
- **PACHECO SE ; HOUSEMAN EA ; CHRISTENSEN BC ; MARSIT CJ et al.** Integrative DNA methylation and gene expression analyses identify DNA packaging and epigenetic regulatory genes associated with low motility sperm. *PLoS One,* 2011, vol. 6 (6), e20280
- **SONG H ; RAMUS SJ ; TYRER J ; BOLTON KL ; GENTRY-MAHARAJ A ; WOZNIAK E ; ANTON-CULVER H ; CHANG-CLAUDE J ; CRAMER DW ; DICIOCCIO R et al.** A genome-wide association study identifies a new ovarian cancersusceptibility locus on 9p22.2. *Nat Genet,* 2009, vol. 41, 996-1000

- **ADKINS RM ; THOMAS F ; TYLAVSKY FA ; JULIA KRUSHKAL.** Parental ages and levels of DNA methylation in the newborn are correlated. *BMC Med Genet,* 2011, vol. 12, 47
- **LIU J ; MORGAN M ; HUTCHISON K ; CALHOUN VD.** A study of the influence of sex on genome wide methylation. *PLoS One,* 06 April 2010, vol. 5 (4), e10028
- **ADKINS, RM ; KRUSHKAL, J ; TYLAVSKY, FA ; THOMAS, F.** Racial differences in gene-specific DNA methylation levels are present at birth. *Birth Defects Research Part A: Clinical and Molecular Teratology,* 2011, vol. 91, 728-736
- **BOCKLANDT S ; LIN W ; SEHL ME ; SANCHEZ FJ ; SINSHEIMER JS ; HORVATH S ; VILAIN E.** Epigenetic Predictor of Age. *PLoS ONE,* 2011, vol. 6 (6), e14821